(19) Europäisches Patentamt
European Patent Office
Office européen des brevets

(11) **EP 4 056 581 A1**

(12) # EUROPEAN PATENT APPLICATION
published in accordance with Art. 153(4) EPC


(43) Date of publication:
**14.09.2022 Bulletin 2022/37**

(21) Application number: **20883820.1**

(22) Date of filing: **06.11.2020**

(51) International Patent Classification (IPC):
***C07K 7/06*** *(2006.01)* ***A61K 38/07*** *(2006.01)*
***A61K 38/08*** *(2019.01)* ***A61P 35/00*** *(2006.01)*
***A61P 43/00*** *(2006.01)* ***C12N 9/99*** *(2006.01)*

(52) Cooperative Patent Classification (CPC):
**A61K 38/07; A61K 38/08; A61P 35/00; A61P 43/00; C07K 7/06; C12N 9/99**

(86) International application number:
**PCT/JP2020/042350**

(87) International publication number:
**WO 2021/090959 (14.05.2021 Gazette 2021/19)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.11.2019 JP 2019203338**



(71) Applicant: **Taisho Pharmaceutical Co., Ltd.**
**Tokyo 170-8633 (JP)**

(72) Inventors:
- **HAYASHI, Masato**
**Tokyo 170-8633 (JP)**
- **TAKEUCHI, Tomoki**
**Tokyo 170-8633 (JP)**
- **NOMURA, Yusaku**
**Tokyo 170-8633 (JP)**
- **TAMITA, Tomoko**
**Tokyo 170-8633 (JP)**
- **SHIMONO, Rie**
**Tokyo 170-8633 (JP)**

(74) Representative: **Hoffmann Eitle**
**Patent- und Rechtsanwälte PartmbB**
**Arabellastraße 30**
**81925 München (DE)**


(54) **POLYPEPTIDE HAVING MMP2-INHIBITORY EFFECT**


(57) The present invention provides a substituted polypeptide or a pharmaceutically acceptable salt thereof with an effect to inhibit MMP2, the substituted polypeptide represented by formula [I']:

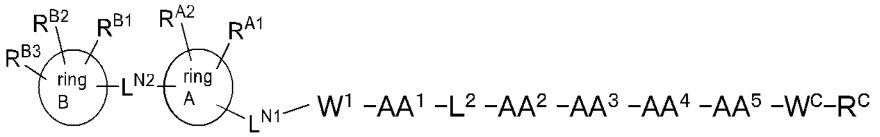

[I']


EP 4 056 581 A1

SEQUENCE LISTING

<110> TAISHO PHARMACEUTICAL CO., LTD.

<120> Polypeptide having MMP2 inhibitory effects

<130> FA0061-20155

<150> JP 2019-203338
<151> 2019-11-08

<160> 47

<170> PatentIn version 3.5

<210> 1
<211> 6
<212> PRT
<213> Artificial Sequence

<220>
<223> peptide chain of [I'-1]: Compound No. 1-39

<220>
<221> MISC_FEATURE
<222> (1)..(1)
<223> gamma-(d)-Glu, beta-Asp, beta-(d)-Asp, or gamma-Glu

<220>
<221> MISC_FEATURE
<222> (2)..(2)
<223> (2S,4S)-(4-amino)Pro

<220>
<221> MISC_FEATURE
<222> (3)..(3)
<223> Leu, Val, Ile, Phe or Trp

<220>
<221> MISC_FEATURE
<222> (4)..(4)
<223> (N-Me)Glu, (N-Me)Ile, (N-Me)Val, (N-Me)Leu, (N-Me)Phe, (N-Me)Tyr, (N-Me)Ser, Pro, (N-Me)Asp or single bond

<220>
<221> MISC_FEATURE
<222> (5)..(5)
<223> Ape, beta-homoPro, Pro, (d)-Pro, beta-Ala, GABA, Acp, Lys, (d)-Lys, Arg, (d)-Arg or single bond

<220>
<221> MISC_FEATURE
<222> (6)..(6)
<223> single bond, Pro, Lys, (d)-Lys, Arg, (d)-Arg (d)-Lys-(d)-Lys

<400> 1

Xaa Xaa Xaa Xaa Xaa Xaa
1               5

<210> 2

<211> 6
<212> PRT
<213> Artificial Sequence

<220>
<223> peptide chain of [I'-2]: Compound No. 40

<220>
<221> MISC_FEATURE
<222> (1)..(1)
<223> gamma-(d)-Glu

<220>
<221> MISC_FEATURE
<222> (2)..(2)
<223> (2S,4S)-(4-amino)Pro

<220>
<221> MISC_FEATURE
<222> (3)..(3)
<223> Leu

<220>
<221> MISC_FEATURE
<222> (4)..(4)
<223> (N-Me)Glu

<220>
<221> MISC_FEATURE
<222> (5)..(5)
<223> Ape

<220>
<221> MISC_FEATURE
<222> (6)..(6)
<223> single bond

<400> 2

Xaa Xaa Xaa Xaa Xaa Xaa
1               5

<210> 3
<211> 6
<212> PRT
<213> Artificial Sequence

<220>
<223> peptide chain of [I'-3]: Compound No. 41

<220>
<221> MISC_FEATURE
<222> (1)..(1)
<223> gamma-(d)-Glu

<220>
<221> MISC_FEATURE
<222> (2)..(2)
<223> (2S,4S)-(4-amino)Pro

<220>
<221> MISC_FEATURE
<222> (3)..(3)
<223> Leu

<220>
<221> MISC_FEATURE
<222> (4)..(4)
<223> (N-Me)Glu

<220>
<221> MISC_FEATURE
<222> (5)..(5)
<223> Ape

<220>
<221> MISC_FEATURE
<222> (6)..(6)
<223> single bond

<400> 3

Xaa Xaa Xaa Xaa Xaa Xaa
1 5

<210> 4
<211> 6
<212> PRT
<213> Artificial Sequence

<220>
<223> peptide chain of [I'-4]: Compound No. 42

<220>
<221> MISC_FEATURE
<222> (1)..(1)
<223> gamma-(d)-Glu

<220>
<221> MISC_FEATURE
<222> (2)..(2)
<223> (2S,4S)-(4-amino)Pro

<220>
<221> MISC_FEATURE
<222> (3)..(3)
<223> Leu

<220>
<221> MISC_FEATURE
<222> (4)..(4)
<223> (N-Me)Glu

<220>
<221> MISC_FEATURE
<222> (5)..(5)
<223> Ape

<220>
<221> MISC_FEATURE
<222> (6)..(6)

<223> single bond

<400> 4

Xaa Xaa Xaa Xaa Xaa Xaa
1 5

<210> 5
<211> 6
<212> PRT
<213> Artificial Sequence

<220>
<223> peptide chain of [I'-5]: Compound No. 43

<220>
<221> MISC_FEATURE
<222> (1)..(1)
<223> gamma-(d)-Glu

<220>
<221> MISC_FEATURE
<222> (2)..(2)
<223> (2S,4S)-(4-amino)Pro

<220>
<221> MISC_FEATURE
<222> (3)..(3)
<223> Leu

<220>
<221> MISC_FEATURE
<222> (4)..(4)
<223> (N-Me)Glu

<220>
<221> MISC_FEATURE
<222> (5)..(5)
<223> Ape

<220>
<221> MISC_FEATURE
<222> (6)..(6)
<223> single bond

<400> 5

Xaa Xaa Xaa Xaa Xaa Xaa
1 5

<210> 6
<211> 6
<212> PRT
<213> Artificial Sequence

<220>
<223> peptide chain of [I'-6]: Compound No. 44

<220>

<221> MISC_FEATURE
<222> (1)..(1)
<223> gamma-(d)-Glu

<220>
<221> MISC_FEATURE
<222> (2)..(2)
<223> (2S,4S)-(4-amino)Pro

<220>
<221> MISC_FEATURE
<222> (3)..(3)
<223> Leu

<220>
<221> MISC_FEATURE
<222> (4)..(4)
<223> (N-Me)Glu

<220>
<221> MISC_FEATURE
<222> (5)..(5)
<223> Ape

<220>
<221> MISC_FEATURE
<222> (6)..(6)
<223> single bond

<400> 6

Xaa Xaa Xaa Xaa Xaa Xaa
1 5

<210> 7
<211> 6
<212> PRT
<213> Artificial Sequence

<220>
<223> peptide chain of [I'-7]: Compound No. 45-55

<220>
<221> MISC_FEATURE
<222> (1)..(1)
<223> beta-(d)-Asp, gamma-(d)-Glu or gamma-Glu

<220>
<221> MISC_FEATURE
<222> (2)..(2)
<223> (2S,4S)-(4-amino)Pro, (S)-piperazine, (2S,4R)-(4-amino)Pro, (2S,4S)-(4-hydroxy)Pro or (2S,4R)-(4-hydroxy)Pro

<220>
<221> MISC_FEATURE
<222> (3)..(3)
<223> Leu

<220>
<221> MISC_FEATURE
<222> (4)..(4)

<223> (N-Me)Ile or (N-Me)Glu

<220>
<221> MISC_FEATURE
<222> (5)..(5)
<223> Ape or beta-homoPro

<220>
<221> MISC_FEATURE
<222> (6)..(6)
<223> single bond

<400> 7

Xaa Xaa Xaa Xaa Xaa Xaa
1               5

<210> 8
<211> 9
<212> PRT
<213> Artificial Sequence

<220>
<223> peptide chain of [I'-8]: Compound No. 56-91

<220>
<221> MISC_FEATURE
<222> (1)..(1)
<223> GABA, (N-Me)GABA or Ape

<220>
<221> MISC_FEATURE
<222> (2)..(2)
<223> Asn, (N-Me)Asn, Glu, Gln, (d)-Ser, homoSer, (d)-Thr or Arg

<220>
<221> MISC_FEATURE
<222> (3)..(3)
<223> Asp

<220>
<221> MISC_FEATURE
<222> (4)..(4)
<223> Ala, Dap, Dab or Orn

<220>
<221> MISC_FEATURE
<222> (5)..(5)
<223> Leu or Dab

<220>
<221> MISC_FEATURE
<222> (6)..(6)
<223> Met, (N-Me)Val, (N-Me)Ile, Glu, (N-Me)Glu or homoGlu

<220>
<221> MISC_FEATURE
<222> (7)..(7)
<223> beta-homoPro or Pro

<220>

<221> MISC_FEATURE
<222> (8)..(8)
<223> single bond, (d)-Lys or (d)-Arg

<220>
<221> MISC_FEATURE
<222> (9)..(9)
<223> -NH2 or -OH

<400> 8

Xaa Xaa Xaa Xaa Xaa Xaa Xaa Xaa Xaa
1 5

<210> 9
<211> 9
<212> PRT
<213> Artificial Sequence

<220>
<223> peptide chain of [I'-9]: Compound No. 92-156

<220>
<221> MISC_FEATURE
<222> (1)..(1)
<223> GABA or (N-Me)GABA

<220>
<221> MISC_FEATURE
<222> (2)..(2)
<223> (N-Me)Ala, (N-Me)Asn, Asn, (d)-Asn, (N-Me)Gly, Aze(2), Pro, homoPro, (N-Me)Val, (N-Me)Leu, (N-Me)Ile, (N-Me)Met, (d)-Lys, (N-Me)Phe, (N-Me)Asp, (N-Me)Glu, (N-Me)Lys, Gln, (N-Me)Tyr, (N-Me)Trp, (N-Me)Thr, Val, Glu or Orn

<220>
<221> MISC_FEATURE
<222> (3)..(3)
<223> Asp

<220>
<221> MISC_FEATURE
<222> (4)..(4)
<223> Ala, Dab, Dap or Dap(Me)

<220>
<221> MISC_FEATURE
<222> (5)..(5)
<223> Leu

<220>
<221> MISC_FEATURE
<222> (6)..(6)
<223> (N-Me)Ile, (N-Me)Glu or (N-Me)Asp

<220>
<221> MISC_FEATURE
<222> (7)..(7)
<223> Aib, Pro or beta-homoPro

<220>

<221>  MISC_FEATURE
<222>  (8)..(8)
<223>  single bond

<220>
<221>  MISC_FEATURE
<222>  (9)..(9)
<223>  -NH2

<400>  9

Xaa Xaa Xaa Xaa Xaa Xaa Xaa Xaa Xaa
1               5

<210>  10
<211>  11
<212>  PRT
<213>  Artificial Sequence

<220>
<223>  peptide chain of [I'-10]: Compound No. 157-160

<220>
<221>  MISC_FEATURE
<222>  (1)..(1)
<223>  gamma-Glu or (d)-Lys

<220>
<221>  MISC_FEATURE
<222>  (2)..(2)
<223>  Adox or (d)-Lys

<220>
<221>  MISC_FEATURE
<222>  (3)..(3)
<223>  Adox or (d)-Lys

<220>
<221>  MISC_FEATURE
<222>  (4)..(4)
<223>  single bond

<220>
<221>  MISC_FEATURE
<222>  (5)..(5)
<223>  single bond

<220>
<221>  MISC_FEATURE
<222>  (6)..(6)
<223>  Lys

<220>
<221>  MISC_FEATURE
<222>  (7)..(7)
<223>  Asp

<220>
<221>  MISC_FEATURE
<222>  (8)..(8)
<223>  Dab

<220>
<221> MISC_FEATURE
<222> (9)..(9)
<223> Leu

<220>
<221> MISC_FEATURE
<222> (10)..(10)
<223> (N-Me)Glu

<220>
<221> MISC_FEATURE
<222> (11)..(11)
<223> beta-homoPro

<400> 10

Xaa Xaa Xaa Xaa Xaa Xaa Xaa Xaa Xaa Xaa Xaa
1 5 10

<210> 11
<211> 9
<212> PRT
<213> Artificial Sequence

<220>
<223> peptide chain of [I'-9]: Compound No. 161-176

<220>
<221> MISC_FEATURE
<222> (1)..(1)
<223> GABA, Ape or Adox

<220>
<221> MISC_FEATURE
<222> (2)..(2)
<223> Asn, Gln, (d)-Ser, Glu or (d)-Thr

<220>
<221> MISC_FEATURE
<222> (3)..(3)
<223> Asp

<220>
<221> MISC_FEATURE
<222> (4)..(4)
<223> Dab

<220>
<221> MISC_FEATURE
<222> (5)..(5)
<223> Leu

<220>
<221> MISC_FEATURE
<222> (6)..(6)
<223> (N-Me)Glu or (N-Me)Ile

<220>
<221> MISC_FEATURE

<222> (7)..(7)
<223> Beta-homoPro

<220>
<221> MISC_FEATURE
<222> (8)..(8)
<223> single bond, (d)-Lys or (d)-Arg

<220>
<221> MISC_FEATURE
<222> (9)..(9)
<223> -NH2 or -OH (only compound No. 161)

<400> 11

Xaa Xaa Xaa Xaa Xaa Xaa Xaa Xaa Xaa
1               5

<210> 12
<211> 9
<212> PRT
<213> Artificial Sequence

<220>
<223> peptide chain of [I'-9]: Compound No. 177-201

<220>
<221> MISC_FEATURE
<222> (1)..(1)
<223> GABA, Ape or Acp

<220>
<221> MISC_FEATURE
<222> (2)..(2)
<223> Asn, Lys, Arg or Glu

<220>
<221> MISC_FEATURE
<222> (3)..(3)
<223> Asp

<220>
<221> MISC_FEATURE
<222> (4)..(4)
<223> Ala or Dab

<220>
<221> MISC_FEATURE
<222> (5)..(5)
<223> Leu

<220>
<221> MISC_FEATURE
<222> (6)..(6)
<223> Met, (N-Me)Glu or (N-Me)Ile

<220>
<221> MISC_FEATURE
<222> (7)..(7)
<223> Pro or beta-homoPro

<220>
<221> MISC_FEATURE
<222> (8)..(8)
<223> single bond

<220>
<221> MISC_FEATURE
<222> (9)..(9)
<223> -NH2

<400> 12

Xaa Xaa Xaa Xaa Xaa Xaa Xaa Xaa Xaa
1 5

<210> 13
<211> 9
<212> PRT
<213> Artificial Sequence

<220>
<223> peptide chain of [I'-11]: Compound No. 202

<220>
<221> MISC_FEATURE
<222> (1)..(1)
<223> GABA

<220>
<221> MISC_FEATURE
<222> (2)..(2)
<223> Asn

<220>
<221> MISC_FEATURE
<222> (3)..(3)
<223> Asp

<220>
<221> MISC_FEATURE
<222> (4)..(4)
<223> Ala

<220>
<221> MISC_FEATURE
<222> (5)..(5)
<223> Leu

<220>
<221> MISC_FEATURE
<222> (6)..(6)
<223> Met

<220>
<221> MISC_FEATURE
<222> (7)..(7)
<223> Pro

<220>
<221> MISC_FEATURE
<222> (8)..(8)

<223> single bond

<220>
<221> MISC_FEATURE
<222> (9)..(9)
<223> -NH2

<400> 13

Xaa Xaa Xaa Xaa Xaa Xaa Xaa Xaa Xaa
1 5

<210> 14
<211> 9
<212> PRT
<213> Artificial Sequence

<220>
<223> peptide chain of [I'-12]: Compound No. 203-205

<220>
<221> MISC_FEATURE
<222> (1)..(1)
<223> Acp

<220>
<221> MISC_FEATURE
<222> (2)..(2)
<223> Asn, Gln or Ala

<220>
<221> MISC_FEATURE
<222> (3)..(3)
<223> Asp

<220>
<221> MISC_FEATURE
<222> (4)..(4)
<223> Dab

<220>
<221> MISC_FEATURE
<222> (5)..(5)
<223> Leu

<220>
<221> MISC_FEATURE
<222> (6)..(6)
<223> (N-Me)Glu

<220>
<221> MISC_FEATURE
<222> (7)..(7)
<223> beta-homoPro

<220>
<221> MISC_FEATURE
<222> (8)..(8)
<223> single bond

<220>

<221> MISC_FEATURE
<222> (9)..(9)
<223> -NH2

<400> 14

Xaa Xaa Xaa Xaa Xaa Xaa Xaa Xaa Xaa
1 5

<210> 15
<211> 9
<212> PRT
<213> Artificial Sequence

<220>
<223> peptide chain of [I'-13]: Compound No. 206-209

<220>
<221> MISC_FEATURE
<222> (1)..(1)
<223> Acp or Ape

<220>
<221> MISC_FEATURE
<222> (2)..(2)
<223> Asn, Gln or Ala

<220>
<221> MISC_FEATURE
<222> (3)..(3)
<223> Asp

<220>
<221> MISC_FEATURE
<222> (4)..(4)
<223> Dab

<220>
<221> MISC_FEATURE
<222> (5)..(5)
<223> Leu

<220>
<221> MISC_FEATURE
<222> (6)..(6)
<223> (N-Me)Glu

<220>
<221> MISC_FEATURE
<222> (7)..(7)
<223> beta-homoPro

<220>
<221> MISC_FEATURE
<222> (8)..(8)
<223> single bond

<220>
<221> MISC_FEATURE
<222> (9)..(9)
<223> -NH2

<400> 15

Xaa Xaa Xaa Xaa Xaa Xaa Xaa Xaa Xaa
1 5

<210> 16
<211> 9
<212> PRT
<213> Artificial Sequence

<220>
<223> peptide chain of [I'-14]: Compound No. 210-315

<220>
<221> MISC_FEATURE
<222> (1)..(1)
<223> GABA, beta-Ala, Ape, epsilon-Lys, epsilon-(d)-Lys, Acp, Adox, gamma-Dab, delta-(d)-Orn, gamma-(d)-Dab, beta-Dap, beta-(d)-Dap or -NH-(CH2)2-O-CH2-CO-

<220>
<221> MISC_FEATURE
<222> (2)..(2)
<223> Asn, Val, Glu, Orn, (N-Me)Asn, Gln, (d)-Asn, (d)-Gln, (d)-Ser, (d)-Ala, Phe, (d)-Phe, Tyr, (d)-Tyr, beta-Dap, beta-(d)-Dap, beta-Ala, Dab, Dap, (d)-Dab, Aze(2), (N-Me)Glu, (N-Me)Ile or (N-Me)Lys

<220>
<221> MISC_FEATURE
<222> (3)..(3)
<223> Asp

<220>
<221> MISC_FEATURE
<222> (4)..(4)
<223> Dab, Dap, Dap(Me), Dab(Me) or Dab(Me)2

<220>
<221> MISC_FEATURE
<222> (5)..(5)
<223> Leu

<220>
<221> MISC_FEATURE
<222> (6)..(6)
<223> (N-Me)Glu, (N-Me)Ile, (N-Me)Asp or (N-Me)Asn

<220>
<221> MISC_FEATURE
<222> (7)..(7)
<223> beta-homoPro

<220>
<221> MISC_FEATURE
<222> (8)..(8)
<223> single bond

<220>
<221> MISC_FEATURE

<222>  (9)..(9)
<223>  NH2, OH, NHEt, piperidin-1-yl, NHMe, azetidin-1-yl, pyrrolidin-1-yl, (4-OH)piperidin-1-yl, NH-(CH2)2-OH or (3-OH)azetidin-1-yl

<400>  16

Xaa Xaa Xaa Xaa Xaa Xaa Xaa Xaa Xaa
1               5

<210>  17
<211>  9
<212>  PRT
<213>  Artificial Sequence

<220>
<223>  peptide chain of [I'-15]: Compound No. 316-317

<220>
<221>  MISC_FEATURE
<222>  (1)..(1)
<223>  Ape

<220>
<221>  MISC_FEATURE
<222>  (2)..(2)
<223>  Asn or (d)-Ser

<220>
<221>  MISC_FEATURE
<222>  (3)..(3)
<223>  Asp

<220>
<221>  MISC_FEATURE
<222>  (4)..(4)
<223>  Dab

<220>
<221>  MISC_FEATURE
<222>  (5)..(5)
<223>  Leu

<220>
<221>  MISC_FEATURE
<222>  (6)..(6)
<223>  (N-Me)Ile

<220>
<221>  MISC_FEATURE
<222>  (7)..(7)
<223>  beta-homoPro

<220>
<221>  MISC_FEATURE
<222>  (8)..(8)
<223>  single bond

<220>
<221>  MISC_FEATURE
<222>  (9)..(9)

<223> -NH2

<400> 17

Xaa Xaa Xaa Xaa Xaa Xaa Xaa Xaa Xaa
1               5

<210> 18
<211> 9
<212> PRT
<213> Artificial Sequence

<220>
<223> peptide chain of [I'-14]: Compound No. 318-586

<220>
<221> MISC_FEATURE
<222> (1)..(1)
<223> Ape, -NH-(CH2)2-O-CH2-CO-, Adox, GABA, Acp, delta-Orn, epsilon-(d)-Lys or epsilon-Lys

<220>
<221> MISC_FEATURE
<222> (2)..(2)
<223> (N-Me)Glu, Thr, (d)-Thr, (d)-Pro, Trp, (N-Me)Leu, (N-Me)Met, (d)-Trp, His, (d)-His, Cys, (d)-Cys, Arg, (d)-Arg, (d)-Glu, (d)-Ser, Lys(Ac), Cit, (d)-Cit, beta-Asp, beta-(d)-Asp, Lys, Met, single bond, (N-Me)Gln, Asn, (N-Me)Arg, (d)-Lys, Gln, Glu, Ala,

<220>
<221> MISC_FEATURE
<222> (2)..(2)
<223> Dap, Dab or Gly

<220>
<221> MISC_FEATURE
<222> (3)..(3)
<223> Asp, aspartimide or beta-Asp

<220>
<221> MISC_FEATURE
<222> (4)..(4)
<223> Dab, Dap or Aze(2)

<220>
<221> MISC_FEATURE
<222> (5)..(5)
<223> Leu, Phe or Ile

<220>
<221> MISC_FEATURE
<222> (6)..(6)
<223> (N-Me)Ile, (N-Me)Glu, Dap, Dab, Orn, (N-Me)Arg, (N-Me)Lys, single bond, (N-Me)Val, (N-Me)Ala or (N-Me)Leu

<220>
<221> MISC_FEATURE
<222> (7)..(7)
<223> beta-homoPro, single bond, Pro, Arg, Lys, beta-Ala, GABA, Ape, His, (d)-Arg, (d)-Lys, Dap, Dab, Orn, homoPro,

(2S,4R)-(4-amino)Pro, beta-Dap, beta-(d)-Dap, gamma-Dab, delta-Orn, delta-(d)-Orn, epsilon-Lys, epsilon-(d)-Lys, Aze(2),

<220>
<221> MISC_FEATURE
<222> (7)..(7)
<223> (d)-Aze(2), (N-Me)beta-Ala, Acp or gamma-(d)-Dab

<220>
<221> MISC_FEATURE
<222> (8)..(8)
<223> single bond, (d)-Lys-(d)-Lys-(d)-Lys, (d)-Lys, (d)-Arg, Lys, Arg, Arg-Arg, (d)-Arg-(d)-Arg, Lys-Lys, (d)-Lys-(d)-Lys, beta-Ala or GABA

<220>
<221> MISC_FEATURE
<222> (9)..(9)
<223> -NH2, -OH, -NH-(CH2)2-NH2, -NH-(CH2)4-NH2 or -NH-(CH2)5-NH2

<400> 18

Xaa Xaa Xaa Xaa Xaa Xaa Xaa Xaa Xaa
1               5

<210> 19
<211> 9
<212> PRT
<213> Artificial Sequence

<220>
<223> peptide chain of [I'-16]: Compound No. 587-589

<220>
<221> MISC_FEATURE
<222> (1)..(1)
<223> beta-Ala or GABA

<220>
<221> MISC_FEATURE
<222> (2)..(2)
<223> Asn

<220>
<221> MISC_FEATURE
<222> (3)..(3)
<223> Asp

<220>
<221> MISC_FEATURE
<222> (4)..(4)
<223> Ala

<220>
<221> MISC_FEATURE
<222> (5)..(5)
<223> Leu

<220>
<221> MISC_FEATURE

<222> (6)..(6)
<223> (N-Me)Met or (N-Me)Val

<220>
<221> MISC_FEATURE
<222> (7)..(7)
<223> Pro

<220>
<221> MISC_FEATURE
<222> (8)..(8)
<223> single bond

<220>
<221> MISC_FEATURE
<222> (9)..(9)
<223> -NH2

<400> 19

```
Xaa Xaa Xaa Xaa Xaa Xaa Xaa Xaa Xaa
1               5
```

<210> 20
<211> 9
<212> PRT
<213> Artificial Sequence

<220>
<223> peptide chain of [I'-17]: Compound No. 590

<220>
<221> MISC_FEATURE
<222> (1)..(1)
<223> Ape

<220>
<221> MISC_FEATURE
<222> (2)..(2)
<223> Asn

<220>
<221> MISC_FEATURE
<222> (3)..(3)
<223> Asp

<220>
<221> MISC_FEATURE
<222> (4)..(4)
<223> Ala

<220>
<221> MISC_FEATURE
<222> (5)..(5)
<223> Leu

<220>
<221> MISC_FEATURE
<222> (6)..(6)
<223> Met

<220>
<221> MISC_FEATURE
<222> (7)..(7)
<223> Pro

<220>
<221> MISC_FEATURE
<222> (8)..(8)
<223> single bond

<220>
<221> MISC_FEATURE
<222> (9)..(9)
<223> -NH2

<400> 20

Xaa Xaa Xaa Xaa Xaa Xaa Xaa Xaa Xaa
1               5

<210> 21
<211> 9
<212> PRT
<213> Artificial Sequence

<220>
<223> peptide chain of [I'-18]: Compound No. 591

<220>
<221> MISC_FEATURE
<222> (1)..(1)
<223> GABA

<220>
<221> MISC_FEATURE
<222> (2)..(2)
<223> Asn

<220>
<221> MISC_FEATURE
<222> (3)..(3)
<223> Asp

<220>
<221> MISC_FEATURE
<222> (4)..(4)
<223> Ala

<220>
<221> MISC_FEATURE
<222> (5)..(5)
<223> Leu

<220>
<221> MISC_FEATURE
<222> (6)..(6)
<223> Met

<220>
<221> MISC_FEATURE
<222> (7)..(7)

<223> Pro

<220>
<221> MISC_FEATURE
<222> (8)..(8)
<223> single bond

<220>
<221> MISC_FEATURE
<222> (9)..(9)
<223> -NH2

<400> 21

Xaa Xaa Xaa Xaa Xaa Xaa Xaa Xaa Xaa
1               5

<210> 22
<211> 9
<212> PRT
<213> Artificial Sequence

<220>
<223> peptide chain of [I'-19]: Compound No. 592

<220>
<221> MISC_FEATURE
<222> (1)..(1)
<223> Ape

<220>
<221> MISC_FEATURE
<222> (2)..(2)
<223> Asn

<220>
<221> MISC_FEATURE
<222> (3)..(3)
<223> Asp

<220>
<221> MISC_FEATURE
<222> (4)..(4)
<223> Ala

<220>
<221> MISC_FEATURE
<222> (5)..(5)
<223> Leu

<220>
<221> MISC_FEATURE
<222> (6)..(6)
<223> Met

<220>
<221> MISC_FEATURE
<222> (7)..(7)
<223> Pro

<220>

<221> MISC_FEATURE
<222> (8)..(8)
<223> single bond

<220>
<221> MISC_FEATURE
<222> (9)..(9)
<223> -NH2

<400> 22

Xaa Xaa Xaa Xaa Xaa Xaa Xaa Xaa Xaa
1 5

<210> 23
<211> 9
<212> PRT
<213> Artificial Sequence

<220>
<223> peptide chain of [I'-20]: Compound No. 593

<220>
<221> MISC_FEATURE
<222> (1)..(1)
<223> single bond

<220>
<221> MISC_FEATURE
<222> (2)..(2)
<223> single bond

<220>
<221> MISC_FEATURE
<222> (3)..(3)
<223> beta-(d)-Asp

<220>
<221> MISC_FEATURE
<222> (4)..(4)
<223> Ala

<220>
<221> MISC_FEATURE
<222> (5)..(5)
<223> Leu

<220>
<221> MISC_FEATURE
<222> (6)..(6)
<223> (N-Me)Glu

<220>
<221> MISC_FEATURE
<222> (7)..(7)
<223> beta-homoPro

<220>
<221> MISC_FEATURE
<222> (8)..(8)
<223> single bond

<220>
<221> MISC_FEATURE
<222> (9)..(9)
<223> -NH2

<400> 23

Xaa Xaa Xaa Xaa Xaa Xaa Xaa Xaa Xaa
1 5

<210> 24
<211> 9
<212> PRT
<213> Artificial Sequence

<220>
<223> peptide chain of [I'-21]: Compound No. 594-595

<220>
<221> MISC_FEATURE
<222> (1)..(1)
<223> single bond

<220>
<221> MISC_FEATURE
<222> (2)..(2)
<223> single bond

<220>
<221> MISC_FEATURE
<222> (3)..(3)
<223> beta-(d)-Asp or gamma-(d)-Glu

<220>
<221> MISC_FEATURE
<222> (4)..(4)
<223> Ala or (2S,4S)-(4-amino)Pro

<220>
<221> MISC_FEATURE
<222> (5)..(5)
<223> Leu

<220>
<221> MISC_FEATURE
<222> (6)..(6)
<223> (N-Me)Ile or (N-Me)Glu

<220>
<221> MISC_FEATURE
<222> (7)..(7)
<223> beta-homoPro or Ape

<220>
<221> MISC_FEATURE
<222> (8)..(8)
<223> single bond

<220>
<221> MISC_FEATURE

<222> (9)..(9)
<223> -NH2

<400> 24

Xaa Xaa Xaa Xaa Xaa Xaa Xaa Xaa Xaa
1 5

<210> 25
<211> 9
<212> PRT
<213> Artificial Sequence

<220>
<223> peptide chain of [I'-22]: Compound No. 596

<220>
<221> MISC_FEATURE
<222> (1)..(1)
<223> GABA

<220>
<221> MISC_FEATURE
<222> (2)..(2)
<223> Asn

<220>
<221> MISC_FEATURE
<222> (3)..(3)
<223> Asp

<220>
<221> MISC_FEATURE
<222> (4)..(4)
<223> Dab

<220>
<221> MISC_FEATURE
<222> (5)..(5)
<223> Leu

<220>
<221> MISC_FEATURE
<222> (6)..(6)
<223> (N-Me)Ile

<220>
<221> MISC_FEATURE
<222> (7)..(7)
<223> Pro

<220>
<221> MISC_FEATURE
<222> (8)..(8)
<223> single bond

<220>
<221> MISC_FEATURE
<222> (9)..(9)
<223> -NH2

<400> 25

Xaa Xaa Xaa Xaa Xaa Xaa Xaa Xaa Xaa
1 5

<210> 26
<211> 9
<212> PRT
<213> Artificial Sequence

<220>
<223> peptide chain of [I'-23]: Compound No. 597

<220>
<221> MISC_FEATURE
<222> (1)..(1)
<223> GABA

<220>
<221> MISC_FEATURE
<222> (2)..(2)
<223> Asn

<220>
<221> MISC_FEATURE
<222> (3)..(3)
<223> Asp

<220>
<221> MISC_FEATURE
<222> (4)..(4)
<223> Ala

<220>
<221> MISC_FEATURE
<222> (5)..(5)
<223> Leu

<220>
<221> MISC_FEATURE
<222> (6)..(6)
<223> Met

<220>
<221> MISC_FEATURE
<222> (7)..(7)
<223> Pro

<220>
<221> MISC_FEATURE
<222> (8)..(8)
<223> single bond

<220>
<221> MISC_FEATURE
<222> (9)..(9)
<223> -NH2

<400> 26

Xaa Xaa Xaa Xaa Xaa Xaa Xaa Xaa Xaa

1 5

<210> 27
<211> 9
<212> PRT
<213> Artificial Sequence

<220>
<223> peptide chain of [I'-24]: Compound No. 598

<220>
<221> MISC_FEATURE
<222> (1)..(1)
<223> GABA

<220>
<221> MISC_FEATURE
<222> (2)..(2)
<223> Asn

<220>
<221> MISC_FEATURE
<222> (3)..(3)
<223> Asp

<220>
<221> MISC_FEATURE
<222> (4)..(4)
<223> Ala

<220>
<221> MISC_FEATURE
<222> (5)..(5)
<223> Leu

<220>
<221> MISC_FEATURE
<222> (6)..(6)
<223> Met

<220>
<221> MISC_FEATURE
<222> (7)..(7)
<223> Pro

<220>
<221> MISC_FEATURE
<222> (8)..(8)
<223> single bond

<220>
<221> MISC_FEATURE
<222> (9)..(9)
<223> -NH2

<400> 27

Xaa Xaa Xaa Xaa Xaa Xaa Xaa Xaa Xaa
1 5

<210> 28
<211> 9
<212> PRT
<213> Artificial Sequence

<220>
<223> peptide chain of [I'-25]: Compound No. 599

<220>
<221> MISC_FEATURE
<222> (1)..(1)
<223> GABA

<220>
<221> MISC_FEATURE
<222> (2)..(2)
<223> Asn

<220>
<221> MISC_FEATURE
<222> (3)..(3)
<223> Asp

<220>
<221> MISC_FEATURE
<222> (4)..(4)
<223> Ala

<220>
<221> MISC_FEATURE
<222> (5)..(5)
<223> Leu

<220>
<221> MISC_FEATURE
<222> (6)..(6)
<223> Met

<220>
<221> MISC_FEATURE
<222> (7)..(7)
<223> Pro

<220>
<221> MISC_FEATURE
<222> (8)..(8)
<223> single bond

<220>
<221> MISC_FEATURE
<222> (9)..(9)
<223> -NH2

<400> 28

Xaa Xaa Xaa Xaa Xaa Xaa Xaa Xaa Xaa
1               5

<210> 29
<211> 9
<212> PRT

<213> Artificial Sequence

<220>
<223> peptide chain of [I'-26]: Compound No. 600-601

<220>
<221> MISC_FEATURE
<222> (1)..(1)
<223> GABA

<220>
<221> MISC_FEATURE
<222> (2)..(2)
<223> Asn

<220>
<221> MISC_FEATURE
<222> (3)..(3)
<223> Asp

<220>
<221> MISC_FEATURE
<222> (4)..(4)
<223> Dab

<220>
<221> MISC_FEATURE
<222> (5)..(5)
<223> Leu

<220>
<221> MISC_FEATURE
<222> (6)..(6)
<223> (N-Me)Ile or (N-Me)Glu

<220>
<221> MISC_FEATURE
<222> (7)..(7)
<223> beta-homoPro

<220>
<221> MISC_FEATURE
<222> (8)..(8)
<223> single bond

<220>
<221> MISC_FEATURE
<222> (9)..(9)
<223> -NH2

<400> 29

Xaa Xaa Xaa Xaa Xaa Xaa Xaa Xaa Xaa
1               5

<210> 30
<211> 9
<212> PRT
<213> Artificial Sequence

<220>

<223> peptide chain of [I'-27]: Compound No. 602-605

<220>
<221> MISC_FEATURE
<222> (1)..(1)
<223> single bond

<220>
<221> MISC_FEATURE
<222> (2)..(2)
<223> single bond

<220>
<221> MISC_FEATURE
<222> (3)..(3)
<223> beta-(d)-Asp

<220>
<221> MISC_FEATURE
<222> (4)..(4)
<223> Dap or Dab

<220>
<221> MISC_FEATURE
<222> (5)..(5)
<223> Leu

<220>
<221> MISC_FEATURE
<222> (6)..(6)
<223> (N-Me)Glu or (N-Me)Ile

<220>
<221> MISC_FEATURE
<222> (7)..(7)
<223> beta-homoPro

<220>
<221> MISC_FEATURE
<222> (8)..(8)
<223> single bond

<220>
<221> MISC_FEATURE
<222> (9)..(9)
<223> -NH2

<400> 30

Xaa Xaa Xaa Xaa Xaa Xaa Xaa Xaa Xaa
1               5

<210> 31
<211> 9
<212> PRT
<213> Artificial Sequence

<220>
<223> peptide chain of [I'-28]: Compound No. 606

<220>
<221> MISC_FEATURE
<222> (1)..(1)
<223> single bond

<220>
<221> MISC_FEATURE
<222> (2)..(2)
<223> single bond

<220>
<221> MISC_FEATURE
<222> (3)..(3)
<223> gamma-(d)-Glu

<220>
<221> MISC_FEATURE
<222> (4)..(4)
<223> (2S,4S)-(4-amino)Pro

<220>
<221> MISC_FEATURE
<222> (5)..(5)
<223> Leu

<220>
<221> MISC_FEATURE
<222> (6)..(6)
<223> (N-Me)Glu

<220>
<221> MISC_FEATURE
<222> (7)..(7)
<223> Ape

<220>
<221> MISC_FEATURE
<222> (8)..(8)
<223> single bond

<220>
<221> MISC_FEATURE
<222> (9)..(9)
<223> -OH

<400> 31

Xaa Xaa Xaa Xaa Xaa Xaa Xaa Xaa Xaa
1 5

<210> 32
<211> 9
<212> PRT
<213> Artificial Sequence

<220>
<223> peptide chain of [I'-29]: Compound No. 607

<220>
<221> MISC_FEATURE
<222> (1)..(1)

<223> single bond

<220>
<221> MISC_FEATURE
<222> (2)..(2)
<223> single bond

<220>
<221> MISC_FEATURE
<222> (3)..(3)
<223> gamma-(d)-Glu

<220>
<221> MISC_FEATURE
<222> (4)..(4)
<223> (2S,4S)-(4-amino)Pro

<220>
<221> MISC_FEATURE
<222> (5)..(5)
<223> Leu

<220>
<221> MISC_FEATURE
<222> (6)..(6)
<223> (N-Me)Glu

<220>
<221> MISC_FEATURE
<222> (7)..(7)
<223> Ape

<220>
<221> MISC_FEATURE
<222> (8)..(8)
<223> single bond

<220>
<221> MISC_FEATURE
<222> (9)..(9)
<223> -OH

<400> 32

Xaa Xaa Xaa Xaa Xaa Xaa Xaa Xaa Xaa
1 5

<210> 33
<211> 9
<212> PRT
<213> Artificial Sequence

<220>
<223> peptide chain of [I'-30]: Compound No. 608

<220>
<221> MISC_FEATURE
<222> (1)..(1)
<223> single bond

<220>

<221> MISC_FEATURE
<222> (2)..(2)
<223> single bond

<220>
<221> MISC_FEATURE
<222> (3)..(3)
<223> gamma-(d)-Glu

<220>
<221> MISC_FEATURE
<222> (4)..(4)
<223> Dab

<220>
<221> MISC_FEATURE
<222> (5)..(5)
<223> Leu

<220>
<221> MISC_FEATURE
<222> (6)..(6)
<223> (N-Me)Ile

<220>
<221> MISC_FEATURE
<222> (7)..(7)
<223> beta-homoPro

<220>
<221> MISC_FEATURE
<222> (8)..(8)
<223> single bond

<220>
<221> MISC_FEATURE
<222> (9)..(9)
<223> -NH2

<400> 33

Xaa Xaa Xaa Xaa Xaa Xaa Xaa Xaa Xaa
1 5

<210> 34
<211> 9
<212> PRT
<213> Artificial Sequence

<220>
<223> peptide chain of [I'-31]: Compound No. 609

<220>
<221> MISC_FEATURE
<222> (1)..(1)
<223> single bond

<220>
<221> MISC_FEATURE
<222> (2)..(2)
<223> single bond

<220>
<221> MISC_FEATURE
<222> (3)..(3)
<223> gamma-(d)-Glu

<220>
<221> MISC_FEATURE
<222> (4)..(4)
<223> Dab

<220>
<221> MISC_FEATURE
<222> (5)..(5)
<223> Leu

<220>
<221> MISC_FEATURE
<222> (6)..(6)
<223> (N-Me)Glu

<220>
<221> MISC_FEATURE
<222> (7)..(7)
<223> Ape

<220>
<221> MISC_FEATURE
<222> (8)..(8)
<223> single bond

<220>
<221> MISC_FEATURE
<222> (9)..(9)
<223> -NH2

<400> 34

Xaa Xaa Xaa Xaa Xaa Xaa Xaa Xaa Xaa
1               5

<210> 35
<211> 9
<212> PRT
<213> Artificial Sequence

<220>
<223> peptide chain of [I'-32]: Compound No. 610-633

<220>
<221> MISC_FEATURE
<222> (1)..(1)
<223> single bond

<220>
<221> MISC_FEATURE
<222> (2)..(2)
<223> single bond

<220>
<221> MISC_FEATURE

<222> (3)..(3)
<223> beta-(d)-Asp or gamma-(d)-Glu

<220>
<221> MISC_FEATURE
<222> (4)..(4)
<223> Ala, Dap, Dab, Orn or (2S,4S)-(4-amino)Pro

<220>
<221> MISC_FEATURE
<222> (5)..(5)
<223> Ala(2-Pyr) or Leu

<220>
<221> MISC_FEATURE
<222> (6)..(6)
<223> Met, (N-Me)Ile or (N-Me)Glu

<220>
<221> MISC_FEATURE
<222> (7)..(7)
<223> Pro, beta-homoPro, Ape or single bond

<220>
<221> MISC_FEATURE
<222> (8)..(8)
<223> single bond

<220>
<221> MISC_FEATURE
<222> (9)..(9)
<223> -NH2

<400> 35

Xaa Xaa Xaa Xaa Xaa Xaa Xaa Xaa Xaa
1               5

<210> 36
<211> 9
<212> PRT
<213> Artificial Sequence

<220>
<223> peptide chain of [I'-32]: Compound No. 634-642

<220>
<221> MISC_FEATURE
<222> (1)..(1)
<223> single bond

<220>
<221> MISC_FEATURE
<222> (2)..(2)
<223> single bond

<220>
<221> MISC_FEATURE
<222> (3)..(3)
<223> beta-(d)-Asp

<220>
<221> MISC_FEATURE
<222> (4)..(4)
<223> Ala, Dap or Dab

<220>
<221> MISC_FEATURE
<222> (5)..(5)
<223> Leu

<220>
<221> MISC_FEATURE
<222> (6)..(6)
<223> (N-Me)Glu or (N-Me)Ile

<220>
<221> MISC_FEATURE
<222> (7)..(7)
<223> beta-homoPro

<220>
<221> MISC_FEATURE
<222> (8)..(8)
<223> single bond

<220>
<221> MISC_FEATURE
<222> (9)..(9)
<223> -NH2

<400> 36

Xaa Xaa Xaa Xaa Xaa Xaa Xaa Xaa Xaa
1               5

<210> 37
<211> 9
<212> PRT
<213> Artificial Sequence

<220>
<223> peptide chain of [I'-33]: Compound No. 643

<220>
<221> MISC_FEATURE
<222> (1)..(1)
<223> single bond

<220>
<221> MISC_FEATURE
<222> (2)..(2)
<223> single bond

<220>
<221> MISC_FEATURE
<222> (3)..(3)
<223> gamma-(d)-Glu

<220>
<221> MISC_FEATURE
<222> (4)..(4)

<223> (2S,4S)-(4-amino)Pro

<220>
<221> MISC_FEATURE
<222> (5)..(5)
<223> Leu

<220>
<221> MISC_FEATURE
<222> (6)..(6)
<223> (N-Me)Glu

<220>
<221> MISC_FEATURE
<222> (7)..(7)
<223> Ape

<220>
<221> MISC_FEATURE
<222> (8)..(8)
<223> single bond

<220>
<221> MISC_FEATURE
<222> (9)..(9)
<223> -OH

<400> 37

Xaa Xaa Xaa Xaa Xaa Xaa Xaa Xaa Xaa
1 5

<210> 38
<211> 9
<212> PRT
<213> Artificial Sequence

<220>
<223> peptide chain of [I'-34]: Compound No. 644-690

<220>
<221> MISC_FEATURE
<222> (1)..(1)
<223> single bond or Ape

<220>
<221> MISC_FEATURE
<222> (2)..(2)
<223> single bond or Glu

<220>
<221> MISC_FEATURE
<222> (3)..(3)
<223> beta-(d)-Asp, beta-Asp, gamma-Glu, gamma-(d)-Glu or Asp

<220>
<221> MISC_FEATURE
<222> (4)..(4)
<223> Dap, Dab or (2S,4S)-(4-amino)Pro

<220>

<221> MISC_FEATURE
<222> (5)..(5)
<223> Leu

<220>
<221> MISC_FEATURE
<222> (6)..(6)
<223> (N-Me)Glu, (N-Me)Ile or (N-Me)Val

<220>
<221> MISC_FEATURE
<222> (7)..(7)
<223> beta-homoPro or Ape

<220>
<221> MISC_FEATURE
<222> (8)..(8)
<223> single bond

<220>
<221> MISC_FEATURE
<222> (9)..(9)
<223> -NH2 or -OH

<400> 38

Xaa Xaa Xaa Xaa Xaa Xaa Xaa Xaa Xaa
1               5

<210> 39
<211> 9
<212> PRT
<213> Artificial Sequence

<220>
<223> peptide chain of [I'-34]: Compound No. 691-704

<220>
<221> MISC_FEATURE
<222> (1)..(1)
<223> single bond

<220>
<221> MISC_FEATURE
<222> (2)..(2)
<223> single bond

<220>
<221> MISC_FEATURE
<222> (3)..(3)
<223> beta-(d)-Asp or gamma-(d)-Glu

<220>
<221> MISC_FEATURE
<222> (4)..(4)
<223> Dap or Dab

<220>
<221> MISC_FEATURE
<222> (5)..(5)
<223> Leu

<220>
<221> MISC_FEATURE
<222> (6)..(6)
<223> (N-Me)Glu or (N-Me)Ile

<220>
<221> MISC_FEATURE
<222> (7)..(7)
<223> beta-homoPro

<220>
<221> MISC_FEATURE
<222> (8)..(8)
<223> single bond

<220>
<221> MISC_FEATURE
<222> (9)..(9)
<223> -NH2

<400> 39

Xaa Xaa Xaa Xaa Xaa Xaa Xaa Xaa Xaa
1 5

<210> 40
<211> 9
<212> PRT
<213> Artificial Sequence

<220>
<223> peptide chain of [I'-35]: Compound No. 705-791

<220>
<221> MISC_FEATURE
<222> (1)..(1)
<223> single bond

<220>
<221> MISC_FEATURE
<222> (2)..(2)
<223> single bond

<220>
<221> MISC_FEATURE
<222> (3)..(3)
<223> beta-(d)-Asp, gamma-(d)-Glu, or gamma-Glu

<220>
<221> MISC_FEATURE
<222> (4)..(4)
<223> Dap, Dab, Ala, (S)-piperazine, or (2S,4S)-(4-amino)Pro

<220>
<221> MISC_FEATURE
<222> (5)..(5)
<223> Leu or Nle

<220>
<221> MISC_FEATURE

<222> (6)..(6)
<223> (N-Me)Glu, (N-Me)Ile, Lys(CO-(CH2)10-CO2H), Lys(CO-(CH2)12-CO2H), Lys(CO-(CH2)14-CO2H), homoSer, (N-Me)Val, (N-Me)Leu, or (N-Me)Asp

<220>
<221> MISC_FEATURE
<222> (7)..(7)
<223> beta-homoPro, single bond, Ala, Thr, Phe, Lys, beta-Ala, GABA, Ape, Acp, beta-Dap, beta-(d)-Dap, gamma-Dab, gamma-(d)-Dab, delta-Orn, epsilon-Lys, epsilon-(d)-Lys, (4)Abz, Pro, or delta-(d)-Orn

<220>
<221> MISC_FEATURE
<222> (8)..(8)
<223> single bond, (d)-Lys, (d)-Arg, (d)-Arg-(d)-Lys, (d)-Arg-(d)-Arg, (d)-Lys-(d)-Arg or (d)-Lys-(d)-Lys

<220>
<221> MISC_FEATURE
<222> (9)..(9)
<223> NH2, NH-(CH2)2-NH2, NH-(CH2)3-NH2, NH-(CH2)4-NH2, NH-(CH2)5-NH2, (4-Me)piperazin-1-yl, morpholin-4-yl, [VI-8], [VI-16], [VI-18] or OH

<400> 40

Xaa Xaa Xaa Xaa Xaa Xaa Xaa Xaa Xaa
1               5

<210> 41
<211> 9
<212> PRT
<213> Artificial Sequence

<220>
<223> peptide chain of [I'-36]: Compound No. 792-890

<220>
<221> MISC_FEATURE
<222> (1)..(1)
<223> single bond

<220>
<221> MISC_FEATURE
<222> (2)..(2)
<223> single bond

<220>
<221> MISC_FEATURE
<222> (3)..(3)
<223> beta-(d)-Asp, gamma-(d)-Glu or gamma-Glu

<220>
<221> MISC_FEATURE
<222> (4)..(4)
<223> Dap, Dab, (S)-piperazine, Pro or (2S,4S)-(4-amino)Pro

<220>
<221> MISC_FEATURE
<222> (5)..(5)

<223> Leu, Ala(cPropyl), Ala(4-Thz), Ile, Val or Tyr

<220>
<221> MISC_FEATURE
<222> (6)..(6)
<223> (N-Me)Glu, (N-Me)Ile, (N-Me)Val, (N-Me)Leu, single bond, (N-Me)Ser, (N-Me)Tyr, (N-Me)Phe, (N-Me)Ala, (N-Me)Asp, (N-Me)Lys, Gly, (d)-Arg, (d)-Pro, (d)-Ser, (d)-Tyr, (d)-Phe, (d)-Ala, (d)-Thr, (N-Me)Arg, (d)-(N-Me)Glu or Glu

<220>
<221> MISC_FEATURE
<222> (7)..(7)
<223> beta-homoPro, Ape, Acp, gamma-Dab, beta-(d)-Dap, delta-Orn, delta-(d)-Orn, epsilon-Lys, epsilon-(d)-Lys, Pro, beta-Ala, GABA, beta-Dap, Aze(2), (d)-Aze(2), Aze(3), cis-NH(3)cPen, single bond or (d)-Lys

<220>
<221> MISC_FEATURE
<222> (8)..(8)
<223> single bond, (d)-Lys, (d)-Arg, (d)-Arg-(d)-Lys, (d)-Arg-(d)-Arg, (d)-Lys-(d)-Lys, (d)-Lys-(d)-Arg, Gly-(d)-Lys, Gly-(d)-Arg, Gly-(d)-Arg-(d)-Arg, Gly-(d)-Lys-(d)-Arg, beta-Ala-(d)-Lys, beta-Ala-(d)-Arg-(d)-Lys, beta-Ala-(d)-Arg-(d)-Arg,

<220>
<221> MISC_FEATURE
<222> (8)..(8)
<223> beta-Ala-(d)-Lys-(d)-Arg, Gly-(d)-Arg-(d)-Lys, Gly-(d)-Lys-(d)-Lys or Ape

<220>
<221> MISC_FEATURE
<222> (9)..(9)
<223> NH2, [VI-14] or OH

<400> 41

Xaa Xaa Xaa Xaa Xaa Xaa Xaa Xaa Xaa
1               5

<210> 42
<211> 8
<212> PRT
<213> Artificial Sequence

<220>
<223> peptide chain of [I'-37]: Compound No. 891-899

<220>
<221> MISC_FEATURE
<222> (1)..(1)
<223> GABA

<220>
<221> MISC_FEATURE
<222> (2)..(2)
<223> crosslinked to Lys at position No. 5 through Gly, GABA or beta-Ala

<220>
<221> MISC_FEATURE
<222> (4)..(4)
<223> Ala or Dab

<220>
<221> MISC_FEATURE
<222> (6)..(6)
<223> (N-Me)Ile or (N-Me)Asp

<220>
<221> MISC_FEATURE
<222> (7)..(7)
<223> beta-homoPro or single bond

<220>
<221> MISC_FEATURE
<222> (8)..(8)
<223> -OH or -NH2

<400> 42

Xaa Glu Asp Xaa Lys Xaa Xaa Xaa
1               5

<210> 43
<211> 9
<212> PRT
<213> Artificial Sequence

<220>
<223> peptide chain of [I'-38]: Compound No. 900-911

<220>
<221> MISC_FEATURE
<222> (1)..(1)
<223> gamma-Glu, Lys or (d)-Lys

<220>
<221> MISC_FEATURE
<222> (2)..(2)
<223> Adox, Lys or (d)-Lys

<220>
<221> MISC_FEATURE
<222> (3)..(3)
<223> Adox, Lys or (d)-Lys

<220>
<221> MISC_FEATURE
<222> (4)..(4)
<223> Lys or (d)-Lys

<220>
<221> MOD_RES
<222> (4)..(4)
<223> GABA

<220>
<221> MISC_FEATURE
<222> (5)..(5)

<223> Asp

<220>
<221> MISC_FEATURE
<222> (6)..(6)
<223> Ala or Dab

<220>
<221> MISC_FEATURE
<222> (7)..(7)
<223> Leu

<220>
<221> MISC_FEATURE
<222> (8)..(8)
<223> (N-Me)Ile or (N-Me)Glu

<220>
<221> MISC_FEATURE
<222> (9)..(9)
<223> beta-homoPro

<400> 43

Xaa Xaa Xaa Xaa Xaa Xaa Xaa Xaa Xaa
1               5

<210> 44
<211> 11
<212> PRT
<213> Artificial Sequence

<220>
<223> peptide chain of [I'-39]: Compound No. 912-955

<220>
<221> MISC_FEATURE
<222> (1)..(1)
<223> (d)-Lys, gamma-Glu, Lys, Arg, (d)-Arg, Adox or single bond

<220>
<221> MISC_FEATURE
<222> (2)..(2)
<223> (d)-Lys, Adox, single bond, Lys, Arg or (d)-Arg

<220>
<221> MISC_FEATURE
<222> (3)..(3)
<223> (d)-Lys, Adox, single bond, Lys, Arg, (d)-Arg or gamma-Glu

<220>
<221> MISC_FEATURE
<222> (4)..(4)
<223> Single bond or (d)-Lys

<220>
<221> MISC_FEATURE
<222> (5)..(5)
<223> Single bond or (d)-Lys

<220>

<221> MOD_RES
<222> (6)..(6)
<223> GABA or Ape

<220>
<221> MISC_FEATURE
<222> (7)..(7)
<223> Asp

<220>
<221> MISC_FEATURE
<222> (8)..(8)
<223> Dab

<220>
<221> MISC_FEATURE
<222> (9)..(9)
<223> Leu

<220>
<221> MISC_FEATURE
<222> (10)..(10)
<223> (N-Me)Ile, (N-Me)Asp or (N-Me)Glu

<220>
<221> MISC_FEATURE
<222> (11)..(11)
<223> beta-homoPro

<400> 44

Xaa Xaa Xaa Xaa Xaa Lys Xaa Xaa Xaa Xaa Xaa
1               5                   10

<210> 45
<211> 12
<212> PRT
<213> Artificial Sequence

<220>
<223> peptide chain of [I'-40]: Compound No. 956-972

<220>
<221> MISC_FEATURE
<222> (1)..(1)
<223> Ape

<220>
<221> MISC_FEATURE
<222> (4)..(4)
<223> Dab

<220>
<221> MISC_FEATURE
<222> (6)..(6)
<223> (N-Me)Ile or (N-Me)Glu

<220>
<221> MISC_FEATURE
<222> (7)..(7)
<223> beta-homoPro

<220>
<221> MISC_FEATURE
<222> (8)..(8)
<223> (d)-Lys or single bond

<220>
<221> MISC_FEATURE
<222> (9)..(9)
<223> (d)-Lys or single bond

<220>
<221> MISC_FEATURE
<222> (10)..(10)
<223> Lys or (d)-Lys

<220>
<221> MOD_RES
<222> (10)..(10)
<223> (d)-Lys or single bond

<220>
<221> MISC_FEATURE
<222> (11)..(11)
<223> (d)-Lys or single bond

<220>
<221> MISC_FEATURE
<222> (12)..(12)
<223> (d)-Lys or single bond

<400> 45

Xaa Glu Asp Xaa Leu Xaa Xaa Xaa Xaa Xaa Xaa Xaa
1               5                   10

<210> 46
<211> 10
<212> PRT
<213> Artificial Sequence

<220>
<223> peptide chain of [I′] recited in claim 1

<220>
<221> MISC_FEATURE
<222> (1)..(1)
<223> L(N1):-C(=O)- or -S(=O)2-

<220>
<221> MISC_FEATURE
<222> (2)..(2)
<223> W(1):L1 or L1′-L1′′; L1 is single bond, and L1′ is Single bond, beta-Ala, GABA, (N-Me)GABA, Ape, Acp, [III-6], [III-7], [III-8], [III-9], [III-10], [III-11], [III-12], [III-13], [IV-23] or [IV-24]

<220>
<221> MISC_FEATURE
<222> (2)..(2)
<223> W(1):L1 or L1′-L1′′; L1′′ is single bond, Gly, (N-Me)Gly, Ala,

(N-Me)Ala, (d)-Ala, Val, (N-Me)Val, (N-Me)Leu, (N-Me)Ile, [IV-27], Pro, (d)-Pro, homoPro, Phe, (N-Me)Phe, (d)-Phe, His, (d)-His, Trp, (N-Me)Trp, (d)-Trp, Tyr, (N-Me)Tyr, (d)-Tyr,

<220>
<221> MISC_FEATURE
<222> (2)..(2)
<223> W(1):L1 or L1'-L1''; L1'' is Thr, (N-Me)Thr, (d)-Thr, Cys, (d)-Cys, Met, (N-Me)Met, (N-Me)Asp, Glu, (N-Me)Glu, (d)-Glu, Asn, (N-Me)Asn, (d)-Asn, Gln, (N-Me)Gln, (d)-Gln, Arg, (N-Me)Arg, (d)-Arg, Cit, (d)-Cit, [IV-7], [IV-9], [IV-10], [IV-13], Lys,

<220>
<221> MISC_FEATURE
<222> (2)..(2)
<223> W(1):L1 or L1'-L1''; L1'' is (N-Me)Lys, (d)-Lys, [IV-14], beta-Alabeta-Aspbeta-(d)-Asp, [III-6], [III-7], (d)-Ser, or homoSer

<220>
<221> MISC_FEATURE
<222> (3)..(3)
<223> AA1: Asp, beta-Asp, beta-(d)-Asp, gamma-Glu or gamma-(d)-Glu

<220>
<221> MISC_FEATURE
<222> (4)..(4)
<223> L2: single bond

<220>
<221> MISC_FEATURE
<222> (5)..(5)
<223> AA2: Ala, [IV-7], [IV-8], [IV-9], [IV-11], [IV-12], [IV-13], [IV-27], Pro, [II-1] or [II-2]

<220>
<221> MISC_FEATURE
<222> (6)..(6)
<223> AA3: Val, Leu, Ile, [IV-2], Phe, Trp, Tyr, Lys, [IV-3], [IV-4], [IV-5] or [IV-9]

<220>
<221> MISC_FEATURE
<222> (7)..(7)
<223> AA4: Single bond, Gly, (d)-Ala, (N-Me)Ala, (N-Me)Val, (N-Me)Leu, (N-Me)Ile, Pro, (d)-Pro, (N-Me)Phe, (d)-Phe, (N-Me)Tyr, (d)-Tyr, (N-Me)Ser, (d)-Ser, homoSer, (d)-Thr, Met, (N-Me)Met, (N-Me)Asp, Glu, (N-Me)Glu, (d)-(N-Me)Glu, homoGlu, (N-Me)Asn,

<220>
<221> MISC_FEATURE
<222> (7)..(7)
<223> AA4: ... (N-Me)Arg, (d)-Arg, [IV-7], [IV-9], [IV-13], Lys or (N-Me)Lys

<220>
<221> MISC_FEATURE
<222> (8)..(8)
<223> AA5: Single bond, Ala, [IV-1], [IV-27], [IV-28], [IV-29], Pro, (d)-Pro, beta-homoPro, homoPro, [II-1'], Phe, His, Thr, Arg, (d)-Arg, [IV-7], [IV-9], [IV-13], Lys, (d)-Lys, beta-Ala, (N-Me)-beta-Ala, GABA, Ape, Acp, [III-6], [III-7], [III-8],

<220>
<221> MISC_FEATURE
<222> (8)..(8)
<223> AA5: ...[III-9], [III-10], [III-11], [III-12], [III-13], [IV-25] or[IV-26]

<220>
<221> MISC_FEATURE
<222> (9)..(9)
<223> Wc: single bond or linker consisting of 1 to 3 amino acids, wherein the 1 to 3 amino acids can be the same or different, and are selected from the group consisting of Gly, Pro, Arg, (d) -Arg, Lys, (d) -Lys, beta-Ala, GABA, and Ape

<220>
<221> MISC_FEATURE
<222> (10)..(10)
<223> Rc: -OH, -NH2, C1-6 alkylamino, or a 4-7 membered saturated heterocyclyl containing one nitrogen and optionally one heteroatom.

<400> 46

Xaa Xaa Xaa Xaa Xaa Xaa Xaa Xaa Xaa Xaa
1               5                   10

<210> 47
<211> 10
<212> PRT
<213> Artificial Sequence

<220>
<223> peptide chain of [I] recited in claim 8

<220>
<221> MISC_FEATURE
<222> (1)..(1)
<223> L(N1):-C(=O)- or -S(=O)2-

<220>
<221> MISC_FEATURE
<222> (2)..(2)
<223> L1: single bond

<220>
<221> MISC_FEATURE
<222> (3)..(3)
<223> AA1: beta-Asp, gamma-Glu or gamma-(d)-Glu

<220>
<221> MISC_FEATURE
<222> (4)..(4)
<223> L2: single bond

<220>
<221> MISC_FEATURE
<222> (5)..(5)
<223> AA2: [II-1] or [II-2]

<220>

<221> MISC_FEATURE
<222> (6)..(6)
<223> AA3: Val, Leu, Ile, Phe or Trp

<220>
<221> MISC_FEATURE
<222> (7)..(7)
<223> AA4: Single bond, Pro, (N-Me)Ala, (N-Me)Val, (N-Me)Leu, (N-Me)Ile, (N-Me)Phe, (N-Me)Tyr, (N-Me)Ser, (N-Me)Asp or (N-Me)Glu

<220>
<221> MISC_FEATURE
<222> (8)..(8)
<223> AA5: Single bond, Pro, (d)-Pro, beta-homoPro, Arg, (d)-Arg, Lys, (d)-Lys, beta-Ala, GABA, Ape or Acp

<220>
<221> MISC_FEATURE
<222> (9)..(9)
<223> Lc: single bond, Pro, Arg, (d) -Arg, Lys, (d) -Lys or (d) -Lys-(d) -Lys

<220>
<221> MISC_FEATURE
<222> (10)..(10)
<223> Rc: -OH or -NH2

<400> 47

```
Xaa Xaa Xaa Xaa Xaa Xaa Xaa Xaa Xaa Xaa
1               5                   10
```

## Description

TECHNICAL FIELD

The present invention relates to a substituted polypeptide compound having an effect to inhibit matrix metalloprotease 2 (hereinafter, appropriately abbreviated as "MMP2").

BACKGROUND ART

Matrix metalloprotease is an endopeptidase having an active center of zinc, and 24 genes are known therefor. MMP degrades the extracellular matrix including collagen and gelatin, thus being involved not only in physiological response such as bone remodeling and wound healing, but also in pathological processes such as inflammation and the progression of cancer (see NPTL 1).

Clinical trials for multiple MMP inhibitors have been previously carried out with focus on the anti-cancer effect by MMP inhibition, but given up because of adverse effects such as skeletal muscle pain and possible promotion of cancer metastasis that are inferred to be caused by the inhibitory effect relatively non-selective to MMP subtypes (see NPTLs 2 and 3).

Activation of MMP2 has been reported to play an important role in the infiltration and metastasis of cancer cells. The infiltration and metastasis of cancer cells are key factors relating to the prognosis of malignant tumor, and inhibition of MMP2 activity can serve as an effective therapeutic means in cancer control. The growth of cancer is suppressed in MMP2-gene-knockout animals, and MMP2 has been reported to play an important role in the growth of cancer (see NPTL 4). Moreover, MMP2 has been reported to be associated with the progression of pathological condition in patients with various types of cancer such as breast cancer, pancreatic cancer, bladder cancer, colorectal cancer, ovarian cancer, prostate cancer, brain tumor, gastric cancer, hepatocellular carcinoma, head and neck cancer, melanoma, uterine cancer, esophageal cancer, renal cell carcinoma, lung cancer, and glioma (see NPTLs 5 and 6). Further, MMP2 has been reported to be involved in the formation of pathological condition even in non-neoplastic diseases.

It has been reported that, in chronic kidney disease, MMP2 causes the epithelial-mesenchymal transition of the renal tubules by converting the structure of the tubular basement membrane, inducing tubular atrophy, fibrogenesis, and renal dysfunction (see NPTL 7). Further, increasing MMP2 concentrations in blood have been found in patients with chronic kidney disease (see NPTLs 8 and 9). Furthermore, it has been reported that renal fibrosis induced by unilateral ureteral obstruction is suppressed in MMP2-gene-knockout animals (see NPTLs 10 and 11). In light of these, inhibition of MMP2 activity to regulate the progression of pathological condition of chronic kidney disease can serve as an effective therapeutic means.

Enhanced expression of MMP2 has been found in alveolar epithelial cells, fibroblasts, and macrophages in idiopathic pulmonary fibrosis, and, in particular, enhanced expression of MMP2 in alveolar lavage fluid has been found in patients with rapidly progressing idiopathic pulmonary fibrosis (see NPTLs 12 and 13). In addition, the effects of a non-selective MMP inhibitor to decrease the lung collagen contents in bleomycin-induced pulmonary fibrosis mice (see NPTL 14) and to prevent the transformation of lung parenchymal fibroblasts induced by TGFβ (see NPTL 15) have been reported. In light of these, inhibition of MMP2 activity to regulate the progression of pathological condition of idiopathic pulmonary fibrosis can serve as an effective therapeutic means.

Moreover, the relationship between MMP2 and non-neoplastic diseases has been reported, the non-neoplastic diseases including multiple sclerosis, cerebral infarction, arteriosclerosis, abdominal aortic aneurysm, peritoneal sclerosis, myocardial infarction, acute kidney injury, diabetic nephropathy, nephrosclerosis, glomerulonephritis, polycystic kidney disease, polycystic liver disease, alcoholic liver disease, nonalcoholic steatohepatitis, cholestatic liver injury, chronic obstructive pulmonary disease, interstitial pneumonia, diabetic retinopathy, age-related macular degeneration, Sjogren's syndrome, meningitis, muscular dystrophy, scleroderma, inflammatory bowel disease, and tuberculosis (see NPTL 16).

In light of the matters described above, finding a means to selectively inhibit MMP2 is an approach of high possibility to establish an effective therapeutic method for diseases in which MMP2 is involved.

A compound with a hydroxamic acid or carboxylic acid introduced as a zinc chelator has been reported as a low-molecular-weight compound having MMP2-inhibitory effect. However, no compound having selective MMP2-inhibitory effect has been known (e.g., see NPTLs 17 and 18).

"β-Amyloid precursor protein (APP-IP, Ile-Ser-Tyr-Gly-Asn-Asp-Ala-Leu-Met-Pro)", a peptide compound consisting of 10 natural amino acids, has been reported to exhibit selective MMP2-inhibitory effect (see NPTL 19). However, peptide compounds are in general quickly metabolized and excreted in vivo, and thus it is known that even when a peptide compound is administered, the expected pharmacological effect is not sustained.

CITATION LIST

NON PATENT LITERATURE

NPL 1: H. J. Ra and W. C. Parks Matrix Biol., 2007, 26(8), 587-596.
NPL 2: A. H. Drummond et al. Ann N Y Acad Sci., 1999, 878, 228-235.
NPL 3: A. D. Baxter et al. Bioorg Med Chem Lett., 2001, 11, 1465-1468.
NPL 4: T. Itoh et al. Cancer Res., 1998, 58, 1048-1051.
NPL 5: R. Roy et al. J Clin Oncol., 2009, 27, 5287-5297.
NPL 6: T. Turpeenniemi-Hujanen Biochimie., 2005, 87, 287-297.
NPL 7: S. Cheng et al. FASEB J., 2006, 20, 1898-1900.
NPL 8: K. Pawlak et al. Clin Biochem., 2011, 44, 838-843.
NPL 9: H. R. Chang et al. Clin Chim Acta., 2006, 366, 243-248.
NPL 10: X. Du et al. Lab Invest., 2012, 92, 1149-1160.
NPL 11: M. K. Tveitaras et al. PLoS One., 2015, 10, e0143390.
NPL 12: M. Selman et al. Am J Physiol Lung Cell Mol Physiol., 2000, 279, L562-L574.
NPL 13: M. Suga et al. Am J Respir Crit Care Med., 2000, 162, 1949-1956.
NPL 14: M. Corbel et al. J Pathol., 2001, 193, 538-545.
NPL 15: J. Michael et al. Am J Respir Cell Mol Biol., 2009, 41, 731-741.
NPL 16: A. Tokito et al. Int J Mol Sci., 2016, 17, E1178.
NPL 17: D. E. Levy et al. J Med Chem., 1998, 41, 199-223.
NPL 18: Y. Tamura et al. J Med Chem., 1998, 41, 640-649.
NPL 19: S. Higashi et al. J Biol Chem., 2003, 278, 14020-14028.

SUMMARY OF INVENTION

TECHNICAL PROBLEM

An object of the present invention is to provide a novel MMP2 inhibitor.

SOLUTION TO PROBLEM

The present inventors diligently examined to achieve the object, and found that a compound represented by formula [I'] (hereinafter, occasionally referred to as compound [I']) has an effect to inhibit MMP2.

Hereinafter, the present invention will be described in detail.

Specifically, embodiments of the present invention are as follows.

(1) Provided as an embodiment of the present invention is a substituted polypeptide represented by formula [I']:

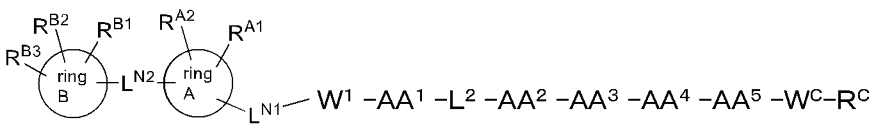

[I'],

or a pharmaceutically acceptable salt thereof,
wherein

$AA^1$ represents:

Asp,
β-Asp, β-(d)-Asp, γ-Glu, or γ-(d)-Glu;

$AA^2$ represents one group selected from the group consisting of:

Ala,

a group represented by any of formulas [IV-7], [IV-8], [IV-9], [IV-11], [IV-12], and [IV-13]:

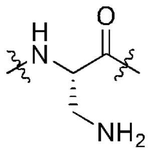 [IV-7], 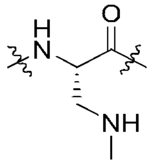 [IV-8],

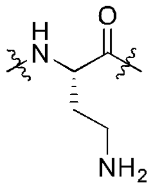 [IV-9], 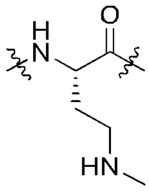 [IV-11], 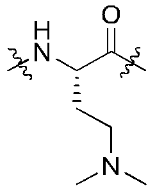 [IV-12],

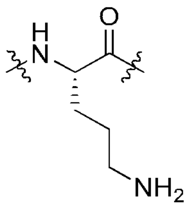 [IV-13]

a group represented by formula [IV-27]:

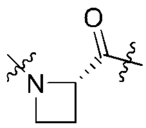 [IV-27]

Pro, and a group represented by any of formulas [II-1] and [II-2]:

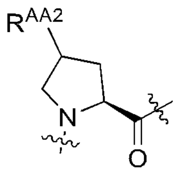 [II-1], 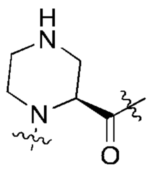 [II-2]

wherein $R^{AA2}$ represents hydroxy or amino; or

$AA^1$ and $AA^2$ may be taken together to form a structure represented by formula [IV-32]:

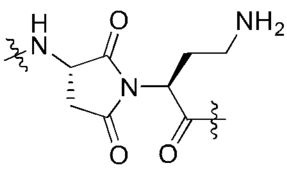 [IV-32];

$AA^3$ represents one group selected from the group consisting of:

Val, Leu, Ile, a group represented by formula [IV-2]:

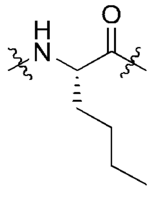 [IV-2]

Phe, Trp,

Tyr, Lys, a group represented by any of formulas [IV-3], [IV-4], and [IV-5]:

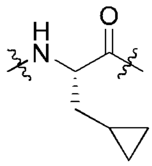 [IV-3], 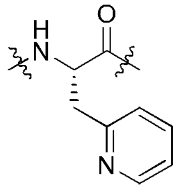 [IV-4], 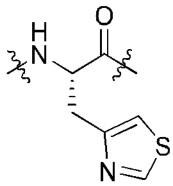 [IV-5]

and

a group represented by formula [IV-9]:

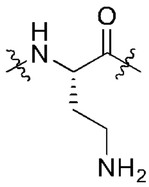 [IV-9];

$AA^4$ represents one group selected from the group consisting of:

a single bond,
Gly, (d)-Ala, (N-Me)Ala, (N-Me)Val, (N-Me)Leu, (N-Me)Ile,
Pro, (d)-Pro,
(N-Me)Phe, (d)-Phe,
(N-Me)Tyr, (d)-Tyr,
(N-Me)Ser, (d)-Ser, homoSer, (d)-Thr,
Met, (N-Me)Met,
(N-Me)Asp, Glu, (N-Me)Glu, (d)-(N-Me)Glu, homoGlu,
(N-Me)Asn,
(N-Me)Arg, (d)-Arg,
a group represented by any of formulas [IV-7], [IV-9], and [IV-13]:

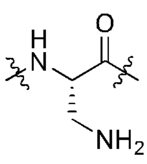 [IV-7], 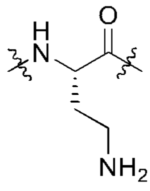 [IV-9], 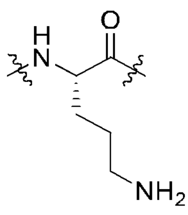 [IV-13]

Lys, and (N-Me)Lys,

wherein if $AA^4$ represents Lys, then
the amino in the side chain of the Lys is optionally substituted with $C_{2-16}$ alkylcarbonyl terminally-substituted with carboxy;

$AA^5$ represents one group selected from the group consisting of:

a single bond,
Ala, a group represented by formula [IV-1]:

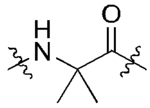
[IV-1]

a group represented by any of formulas [IV-27], [IV-28], and [IV-29]:

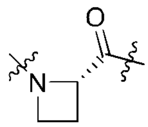
[IV-27],
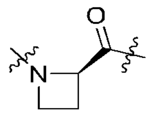
[IV-28],
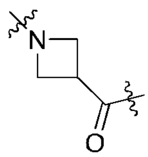
[IV-29]

Pro, (d)-Pro, β-homoPro, homoPro, a group represented by formula [II-1']:

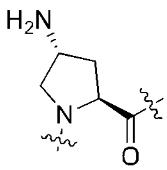
[II-1']

Phe, His,
Thr,
Arg, (d)-Arg,
a group represented by any of formulas [IV-7], [IV-9], and [IV-13]:

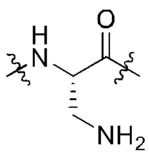
[IV-7],
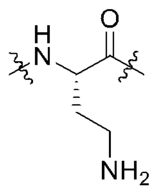
[IV-9],
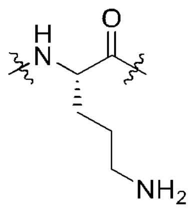
[IV-13]

Lys, (d)-Lys, β-Ala, (N-Me)-β-Ala, GABA, Ape, Acp,
a group represented by any of formulas [III-6] to [III-13]:

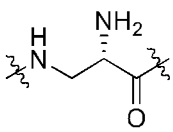
[III-6],
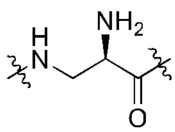
[III-7]

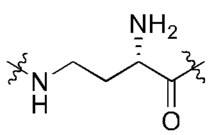
[III-8],
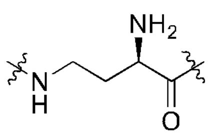
[III-9],

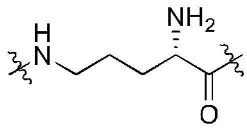 [III-10], 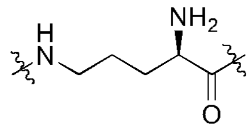 [III-11],

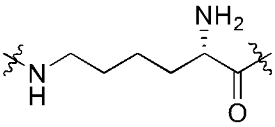 [III-12], 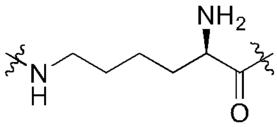 [III-13]

and
a group represented by any of formula [IV-25] and [IV-26]:

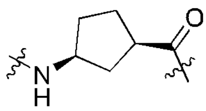 [IV-25], 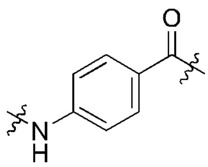 [IV-26];

$W^1$ represents $-L^1-$ or $-L^{1'}-L^{1''}-$; wherein

$L^1$ represents a single bond; and
$L^{1'}$ represents one group selected from the group consisting of:

a single bond,
β-Ala, GABA, (N-Me)GABA, Ape, Acp,
a group represented by any of formulas [III-6] to [III-13]

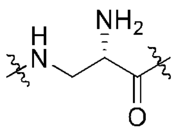 [III-6], 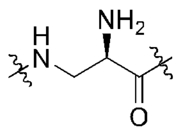 [III-7],

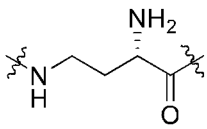 [III-8], 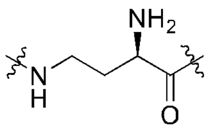 [III-9],

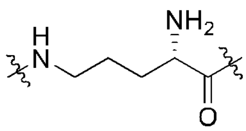 [III-10], 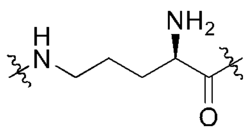 [III-11],

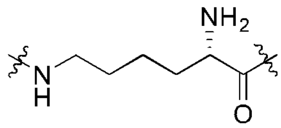 [III-12], 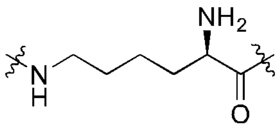 [III-13]

and
a group represented by any of formulas [IV-23] and [IV-24]:

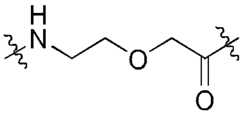 [IV-23], 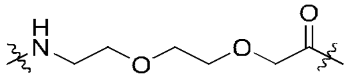 [IV-24];

and

L1" represents one group selected from the group consisting of:

a single bond,
Gly, (N-Me)Gly,
Ala, (N-Me)Ala, (d)-Ala, Val, (N-Me)Val, (N-Me)Leu, (N-Me)Ile,
a group represented by formula [IV-27]:

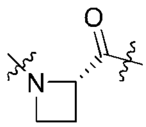 [IV-27]

Pro, (d)-Pro, homoPro, Phe, (N-Me)Phe, (d)-Phe,
His, (d)-His, Trp, (N-Me)Trp, (d)-Trp,
Tyr, (N-Me)Tyr, (d)-Tyr,
(d)-Ser, homoSer, Thr, (N-Me)Thr, (d)-Thr,
Cys, (d)-Cys, Met, (N-Me)Met,
(N-Me)Asp, Glu, (N-Me)Glu, (d)-Glu,
Asn, (N-Me)Asn, (d)-Asn, Gln, (N-Me)Gln, (d)-Gln,
Arg, (N-Me)Arg, (d)-Arg, Cit, (d)-Cit,
a group represented by any of formulas [IV-7], [IV-9], [IV-10], and [IV-13]:

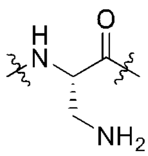 [IV-7], 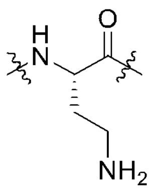 [IV-9], 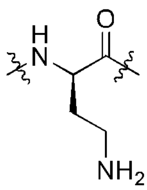 [IV-10],

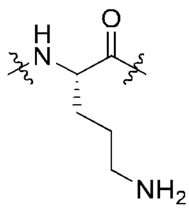 [IV-13]

Lys, (N-Me)Lys, (d)-Lys, a group represented by formula [IV-14]:

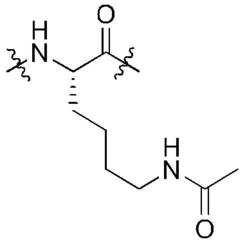 [IV-14]

β-Ala,
β-Asp, β-(d)-Asp, and
a group represented by any of formulas [III-6] and [III-7]:

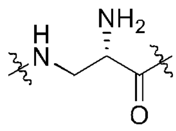 [III-6], 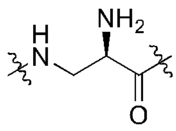 [III-7];

wherein if $L^{1"}$ represents Lys or (d)-Lys, then
the amino in the side chain of the Lys or (d)-Lys is optionally substituted with a group represented by formula [VII-1]:

$FA^{N}-AA^{N5}-AA^{N4}-AA^{N3}-AA^{N2}-AA^{N1}-$ [VII-1]

wherein
$FA^{N}$ represents $C_{2-16}$ alkylcarbonyl terminally-substituted with carboxy;

$AA^{NS}$ represents:

a single bond,
Arg, (d)-Arg,
Lys, (d)-Lys,
γ-Glu, or
a group represented by formula [IV-24]:

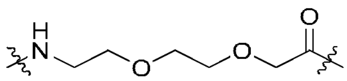 [IV-24];

$AA^{N4}$ represents:

a single bond,
Arg, (d)-Arg,
Lys, (d)-Lys, or
a group represented by formula [IV-24]:

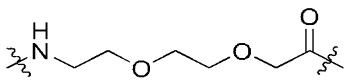 [IV-24];

$AA^{N3}$ represents:

a single bond,
Arg, (d)-Arg,
Lys, (d)-Lys,
γ-Glu, or
a group represented by formula [IV-24]:

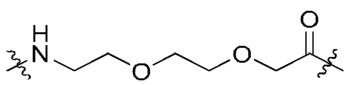 [IV-24];

$AA^{N2}$ represents:

a single bond, or
(d)-Lys; and

$AA^{N1}$ represents:

a single bond, or
(d)-Lys;

wherein if $L^{1''}$ represents Glu and $AA^3$ represents Lys, then the compound represented by formula [I'] may be taken together with $L^3$ attached to each of functional groups in the side chains of the two amino acids to form a cyclic structure, as represented by formula [I'-α]:

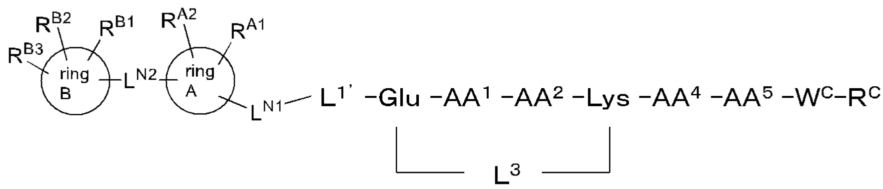

[I'-α]

wherein the $L^3$ represents Gly, β-Ala, or GABA;
$L^{N1}$ represents the formula -C(=O)- or the formula $-S(=O)_2-$;
$L^{N2}$ represents:

a single bond,
$C_{1-3}$ alkanediyl,
$C_{2-3}$ alkenediyl,
ethynediyl,
the formula -O-,
the formula -C(=O)-, the formula -C(=O)-NH-, or
triazolediyl;

$L^2$ represents a single bond;
ring A represents an aromatic ring or a heteroaromatic ring;
$R^{A1}$ and $R^{A2}$ each independently represent:

a hydrogen atom,
a halogen atom,
$C_{1-6}$ alkyl, or
$C_{1-6}$ alkoxy;
ring B represents:
aryl or heteroaryl;

$R^{B1}$, $R^{B2}$, and $R^{B3}$ each independently represent:

a hydrogen atom,
carbamoyl,
cyano,
a halogen atom,
$C_{1-6}$ alkyl optionally substituted with one hydroxy, halo $C_{1-6}$ alkyl,
$C_{1-6}$ alkoxy optionally substituted with one hydroxy, halo $C_{1-6}$ alkoxy,
$C_{1-6}$ alkylcarbonyl,
$C_{1-6}$ alkylcarbonylamino,
mono $C_{1-6}$ alkylaminocarbonyl, di $C_{1-6}$ alkylaminocarbonyl, wherein the alkyl in each of the mono $C_{1-6}$ alkylaminocarbonyl and the di $C_{1-6}$ alkylaminocarbonyl is optionally substituted with one group selected from the group consisting of hydroxy, carboxy, carbamoyl, and amino,
$C_{1-6}$ alkylsulfonyl, or
aryl;

$W^C$ is a single bond or a linker consisting of one to three amino acids, wherein the one to three amino acids forming the linker are same or different and each selected from the group consisting of:

Gly,
Pro,
Arg, (d)-Arg,
Lys, (d)-Lys,
β-Ala, GABA, and Ape,
wherein if Lys or (d)-Lys is included in the group represented by $W^C$, then
the amino in the side chain of the Lys or (d)-Lys is optionally substituted with:

$C_{2-16}$ alkylcarbonyl terminally-substituted with carboxy,
Lys, wherein the amino in the side chain of the Lys is optionally substituted with $C_{2-16}$ alkylcarbonyl terminally-substituted with carboxy, or
(d)-Lys, wherein the amino in the side chain of the (d)-Lys is optionally substituted with $C_{2-16}$ alkylcarbonyl terminally-substituted with carboxy; and

$R^C$ is:

the formula -OH, the formula $-NH_2$,
$C_{1-6}$ alkylamino, wherein the $C_{1-6}$ alkyl of the $C_{1-6}$ alkylamino is optionally substituted with one group selected from the group consisting of hydroxy, amino, $C_{1-6}$ alkoxy, and four- to seven-membered saturated heterocyclyl containing one nitrogen atom and optionally further containing one heteroatom, or
four- to seven-membered saturated heterocyclyl containing one nitrogen atom and optionally further containing one heteroatom, wherein the four- to seven-membered saturated heterocyclyl containing one nitrogen atom and optionally further containing one heteroatom is optionally substituted with one group selected from the group consisting of hydroxy, amino, and $C_{1-6}$ alkyl, wherein the $C_{1-6}$ alkyl is optionally substituted with one carbamoyl; and two carbon atoms in the four- to seven-membered saturated heterocyclyl containing one nitrogen atom and optionally further containing one heteroatom are optionally crosslinked with $C_{1-4}$ alkanediyl.

(2) Provided as another embodiment of the present invention is the substituted polypeptide or pharmaceutically acceptable salt thereof according to (1), wherein

$W^C$ is:

a single bond,
Pro,
Arg, (d)-Arg,
Lys, (d)-Lys,
β-Ala, GABA, Ape,
Gly-(d)-Lys, Gly-(d)-Lys-(d)-Lys, Gly-(d)-Lys-(d)-Arg, Gly-(d)-Arg-(d)-Lys,
Lys-Lys, (d)-Lys-(d)-Lys, (d)-Lys-(d)-Lys-(d)-Lys,
Arg-Arg, (d)-Arg-(d)-Arg, (d)-Arg-(d)-Lys,
Lys-(d)-Lys-(d)-Lys, (d)-Lys-Lys-(d)-Lys, (d)-Lys-(d)-Lys-Lys,
β-Ala-(d)-Lys, β-Ala-(d)-Lys-(d)-Arg, β-Ala-(d)-Arg-(d)-Lys, or β-Ala-(d)-Arg-(d)-Arg

wherein if Lys is contained in the group represented by $W^C$,
then the amino in the side chain of the Lys is optionally substituted with:

$C_{2-16}$ alkylcarbonyl terminally-substituted with carboxy, or
(d)-Lys, wherein the amino in the side chain of the (d)-Lys is optionally substituted with $C_{2-16}$ alkylcarbonyl terminally-substituted with carboxy.

(3) Provided as another embodiment of the present invention is the substituted polypeptide or pharmaceutically acceptable salt thereof according to (1) or (2), wherein

ring A is a benzene ring, a thiophene ring, or a pyridine ring;
$R^{A1}$ and $R^{A2}$ are each independently:

a hydrogen atom, or

a halogen atom;

ring B is:
phenyl, oxazolyl, thiadiazolyl, pyridyl, or benzofuranyl;
$R^{B1}$, $R^{B2}$, and $R^{B3}$ are each independently:

a hydrogen atom,
carbamoyl,
cyano,
a halogen atom,
$C_{1-6}$ alkyl, wherein the $C_{1-6}$ alkyl is optionally substituted with one hydroxy,
halo $C_{1-6}$ alkyl,
$C_{1-6}$ alkoxy, wherein the $C_{1-6}$ alkoxy is optionally substituted with one hydroxy,
halo $C_{1-6}$ alkoxy,
$C_{1-6}$ alkylcarbonyl,
mono $C_{1-6}$ alkylaminocarbonyl, di $C_{1-6}$ alkylaminocarbonyl, wherein the alkyl in each of the mono $C_{1-6}$ alkylaminocarbonyl and the di $C_{1-6}$ alkylaminocarbonyl is optionally substituted with one group selected from the group consisting of hydroxy, carboxy, carbamoyl, and amino, or
$C_{1-6}$ alkylsulfonyl; and

$R^C$ is:

the formula -OH, the formula $-NH_2$,
$C_{1-6}$ alkylamino, wherein the $C_{1-6}$ alkyl of the $C_{1-6}$ alkylamino is optionally substituted with one group selected from the group consisting of hydroxy, amino, $C_{1-6}$ alkoxy, and morpholinyl, or
azetidinyl, pyrrolidinyl, piperidinyl, morpholinyl, or piperazinyl, wherein the azetidinyl, pyrrolidinyl, piperidinyl, morpholinyl, or piperazinyl is optionally substituted with one group selected from the group consisting of hydroxy, amino, and $C_{1-6}$ alkyl, wherein the $C_{1-6}$ alkyl is optionally substituted with one carbamoyl, wherein two carbon atoms in the azetidinyl, pyrrolidinyl, piperidinyl, morpholinyl, or piperazinyl are optionally crosslinked with $C_{1-4}$ alkanediyl.

(4) Provided as another embodiment of the present invention is the substituted polypeptide or pharmaceutically acceptable salt thereof according to any of (1) to (3), wherein in the substituted polypeptide represented by formula [I'],

ring A is a benzene ring;

ring B is phenyl;

$L^{1''}$ is one group selected from the group consisting of:

a single bond,
Gly, (N-Me)Gly,
Ala, (N-Me)Ala, (d)-Ala, Val, (N-Me)Val, (N-Me)Leu, (N-Me)Ile,
a group represented by formula [IV-27]:

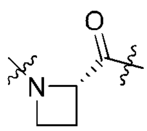
[IV-27]

Pro, (d)-Pro, homoPro, Phe, (N-Me)Phe, (d)-Phe,
His, (d)-His, Trp, (N-Me)Trp, (d)-Trp,
Tyr, (N-Me)Tyr, (d)-Tyr,
(d)-Ser, homoSer, Thr, (N-Me)Thr, (d)-Thr,
Cys, (d)-Cys, Met, (N-Me)Met,
(N-Me)Asp, Glu, (N-Me)Glu, (d)-Glu,
Asn, (N-Me)Asn, (d)-Asn, Gln, (N-Me)Gln, (d)-Gln,

Arg, (N-Me)Arg, (d)-Arg, Cit, (d)-Cit,
a group represented by any of formulas [IV-7], [IV-9], [IV-10], and [IV-13]:

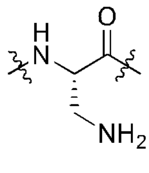 [IV-7], 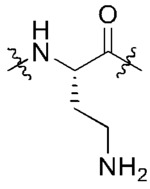 [IV-9], 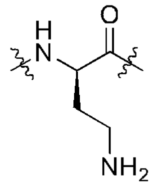 [IV-10],

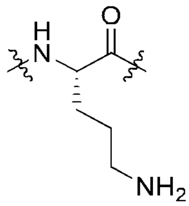 [IV-13]

Lys, (N-Me)Lys, (d)-Lys, a group represented by formula [IV-14]:

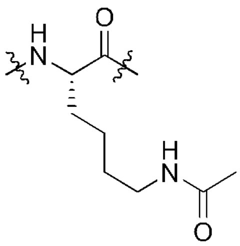 [IV-14]

β-Ala,
β-Asp, β-(d)-Asp, and
a group represented by any of formulas [III-6] and [III-7]:

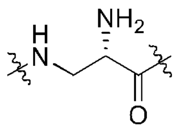 [III-6], 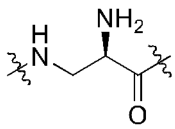 [III-7];

and

$W^C$ is:

a single bond,
Pro,
Arg, (d)-Arg,
Lys, (d)-Lys,
β-Ala, GABA, Ape,
Gly-(d)-Lys, Gly-(d)-Lys-(d)-Lys, Gly-(d)-Lys-(d)-Arg, Gly-(d)-Arg-(d)-Lys,
Lys-Lys, (d)-Lys-(d)-Lys, (d)-Lys-(d)-Lys-(d)-Lys,
Arg-Arg, (d)-Arg-(d)-Arg, (d)-Arg-(d)-Lys,
Lys-(d)-Lys-(d)-Lys, (d)-Lys-Lys-(d)-Lys, (d)-Lys-(d)-Lys-Lys,
β-Ala-(d)-Lys, β-Ala-(d)-Lys-(d)-Arg,
β-Ala-(d)-Arg-(d)-Lys, or β-Ala-(d)-Arg-(d)-Arg.

(5) Provided as another embodiment of the present invention is the substituted polypeptide or pharmaceutically

acceptable salt thereof according to any of (1) to (4), wherein in the substituted polypeptide represented by formula [I'],

$AA^2$ is one group selected from the group consisting of:

a group represented by formula [II-1]:

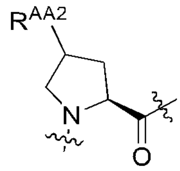

[II-1]

and

a group represented by any of formulas [IV-7], [IV-8], [IV-9], [IV-11], and [IV-12]:

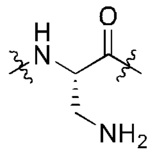
[IV-7],
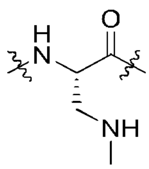
[IV-8],

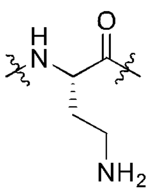
[IV-9],
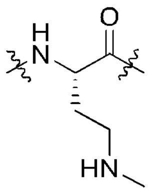
[IV-11],
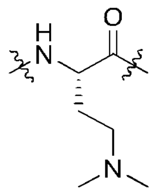
[IV-12];

wherein $R^{AA2}$ is amino;

$AA^3$ is Val, Leu, Ile, Phe, or Trp;
$AA^4$ is (N-Me)Val, (N-Me)Leu, (N-Me)Ile, (N-Me)Asp, or (N-Me)Glu;
$AA^5$ is β-Ala, GABA, Ape, Acp, Pro, (d)-Pro, or β-homoPro;
$W^C$ is a single bond, Arg, (d)-Arg, Lys, or (d)-Lys; and
$R^C$ is the formula -OH or the formula $-NH_2$.

(6) Provided as another embodiment of the present invention is the substituted polypeptide or pharmaceutically acceptable salt thereof according to any of (1) to (5), wherein in the substituted polypeptide represented by formula [I'],

$W^1$ is $-L^{1'}-L^{1''}-$;
$L^2$ is a single bond;
$AA^1$ is Asp;

$L^{1'}$ is one group selected from the group consisting of β-Ala, GABA, Ape, Acp, and a group represented by any of formulas [IV-23] and [IV-24]:

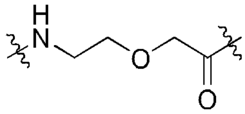
[IV-23],
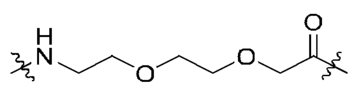
[IV-24];

$L^{1''}$ is a single bond, Asn, (d)-Ser, (d)-Thr, or Glu;

$L^{N1}$ is the formula -C(=O)- or the formula $-S(=O)_2-$;

$L^{N2}$ is the formula -O- or the formula -C(=O)-NH-;
$R^{A1}$ and $R^{A2}$ are each a hydrogen atom; and
$R^{B1}$, $R^{B2}$, and $R^{B3}$ are each independently a hydrogen atom, carbamoyl, a halogen atom, $C_{1-6}$ alkoxy, or halo $C_{1-6}$ alkoxy.

(7) Provided as another embodiment of the present invention is the substituted polypeptide or pharmaceutically acceptable salt thereof according to any of (1) to (5), wherein in the substituted polypeptide represented by formula [I'],

$W^1$ is $-L^1-$, wherein $L^1$ is a single bond;
L2 is a single bond;
$AA^1$ is β-Asp, β-(d)-Asp, γ-Glu, or γ-(d)-Glu;
$AA^2$ is one group selected from the group consisting of:

a group represented by formula [II-1]:

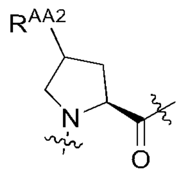 [II-1]

and
a group represented by any of formulas [IV-7], [IV-8], [IV-9], [IV-11], and [IV-12]:

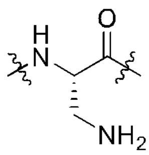 [IV-7], 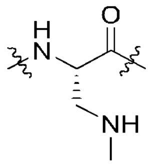 [IV-8],

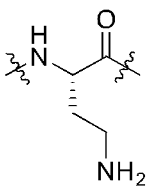 [IV-9], 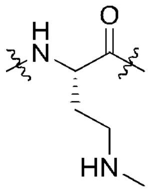 [IV-11], 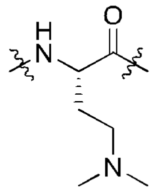 [IV-12];

wherein $R^{AA2}$ is amino;
$AA^3$ is Val, Leu, Ile, Phe, or Trp;
$AA^4$ is (N-Me)Val, (N-Me)Leu, (N-Me)Ile, (N-Me)Asp, or (N-Me)Glu;
$AA^5$ is β-Ala, GABA, Ape, Acp, or β-homoPro;
$L^{N1}$ is the formula -C(=O)- or the formula $-S(=O)_2-$;
$L^{N2}$ is a single bond, the formula -O-, or the formula -C(=O)-NH-;
$R^{A1}$ and $R^{A2}$ are each a hydrogen atom; and
$R^{B1}$, $R^{B2}$, and $R^{B3}$ are each independently a hydrogen atom, carbamoyl, a halogen atom, $C_{1-6}$ alkoxy, or halo $C_{1-6}$ alkoxy.

(8) Provided as another embodiment of the present invention is the substituted polypeptide or pharmaceutically acceptable salt thereof according to any of (1) to (4), wherein the substituted polypeptide represented by formula [1'] is a substituted polypeptide represented by formula [I]:

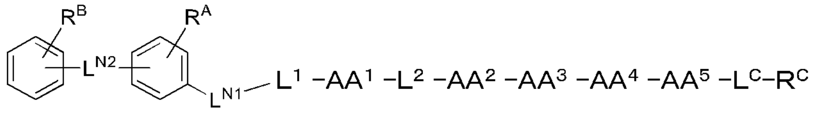 [I]

wherein

AA$^1$ is β-Asp, γ-Glu, or γ-(d)-Glu;
AA$^2$ is a group represented by formula [II-1] or formula [II-2]:

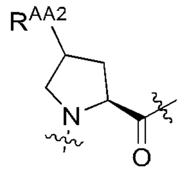 [II-1], 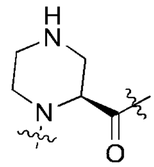 [II-2]

wherein $R^{AA2}$ is hydroxy or amino;
AA$^3$ is Val, Leu, Ile, Phe, or Trp;
AA$^4$ is a single bond, Pro, (N-Me)Ala, (N-Me)Val, (N-Me)Leu, (N-Me)Ile, (N-Me)Phe, (N-Me)Tyr, (N-Me)Ser, (N-Me)Asp, or (N-Me)Glu;
AA$^5$ is a single bond, Pro, (d)-Pro, β-homoPro, Arg, (d)-Arg, Lys, (d)-Lys, β-Ala, GABA, Ape, or Acp;
L$^1$ is a single bond;
L2 is a single bond;
$L^{N1}$ is the formula -C(=O)- or the formula $-S(=O)_2-$;
$L^{N2}$ is a single bond, $C_{1-3}$ alkanediyl, the formula -O-, or the formula -C(=O)-NH-;
$R^A$ is a hydrogen atom, a halogen atom, $C_{1-6}$ alkyl, or $C_{1-6}$ alkoxy;
$R^B$ is a hydrogen atom, carbamoyl, a halogen atom, $C_{1-6}$ alkyl, or $C_{1-6}$ alkoxy;
$L^C$ is a single bond, Pro, Arg, (d)-Arg, Lys, (d)-Lys, or (d)-Lys-(d)-Lys; and
$R^C$ is the formula -OH or the formula $-NH_2$.

(9) Provided as another embodiment of the present invention is the substituted polypeptide or pharmaceutically acceptable salt thereof according to any of (1) to (5), wherein in the substituted polypeptide represented by formula [I'],

AA$^1$ is Asp, β-(d)-Asp, or γ-(d)-Glu;
AA$^2$ is one group selected from the group consisting of:

a group represented by formula [II-1]:

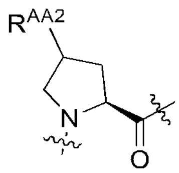 [II-1]

and
a group represented by any of formulas [IV-7] and [IV-9]:

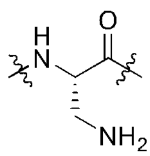 [IV-7], 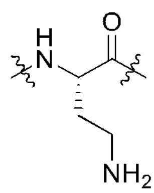 [IV-9]

wherein $R^{AA2}$ is amino;

$AA^3$ is Val, Leu, or Ile;
$AA^4$ is (N-Me)Ile or (N-Me)Glu;
$AA^5$ is Ape or β-homoPro;
$W^1$ is $-L^1-$ or $-L^{1'}-L^{1''}-$,
wherein

$L^1$ is a single bond,
$L^{1'}$ is GABA or Ape, and
$L^{1''}$ is Asn, (d)-Ser, (d)-Thr, or Glu;

$L^{N1}$ is the formula -C(=O)- or the formula $-S(=O)_2-$;
$L^{N2}$ is the formula -O- or the formula -C(=O)-NH-;
$R^{A1}$ and $R^{A2}$ are each a hydrogen atom;
$R^{B1}$, $R^{B2}$, and $R^{B3}$ are each independently a hydrogen atom, carbamoyl, a halogen atom, $C_{1-6}$ alkoxy, or halo $C_{1-6}$ alkoxy;
$W^C$ is a single bond or (d)-Lys; and
$R^C$ is the formula $-NH_2$.

(10) Provided as another embodiment of the present invention is the substituted polypeptide or pharmaceutically acceptable salt thereof according to any one of (1) to (5), wherein the substituted polypeptide is selected from compounds shown in the following:

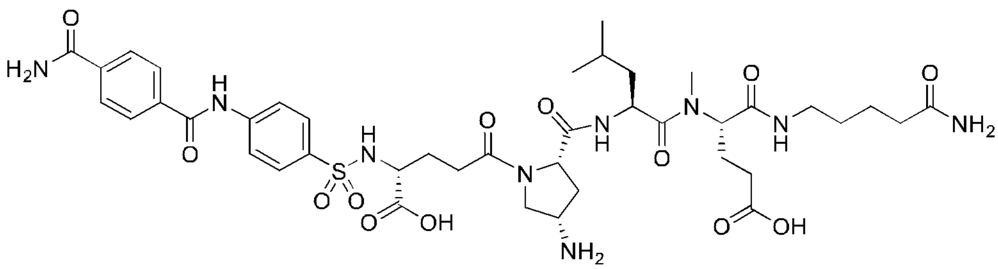
,

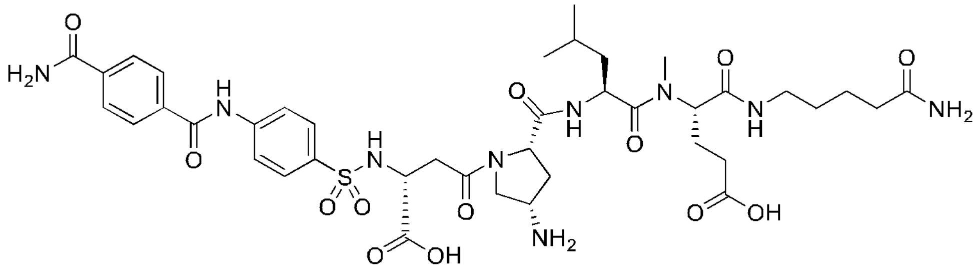
,

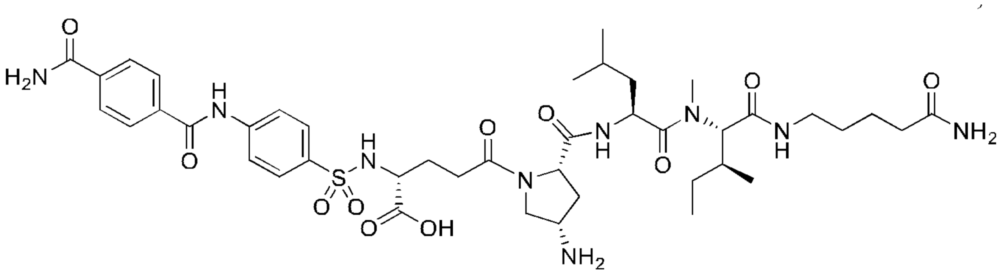
,

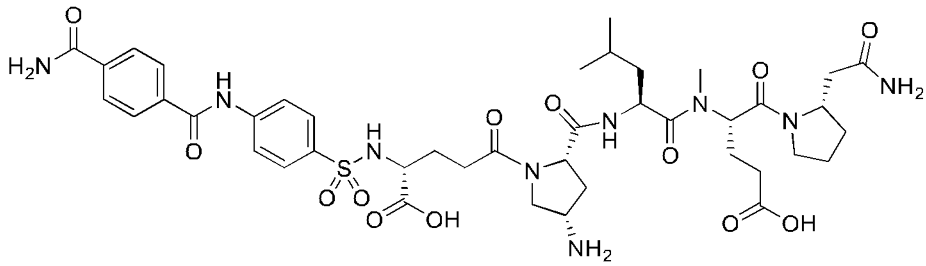

,

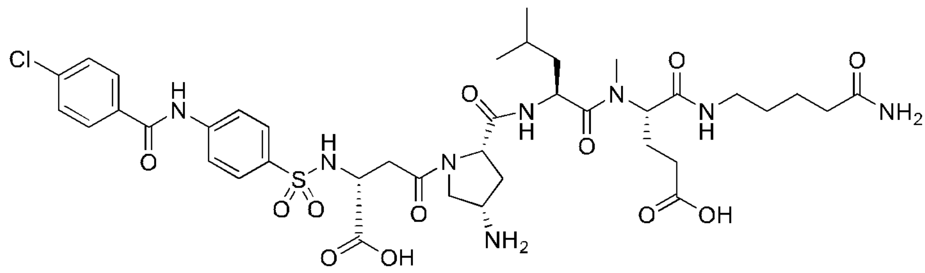

,

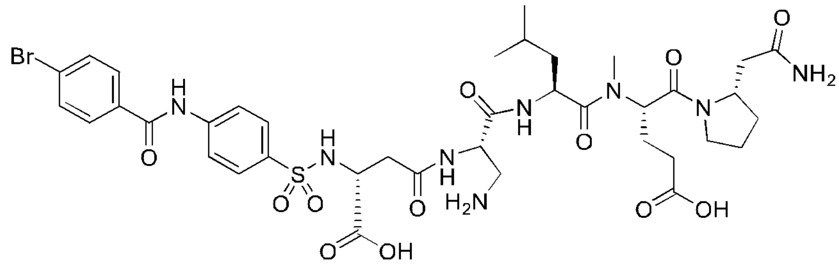

,

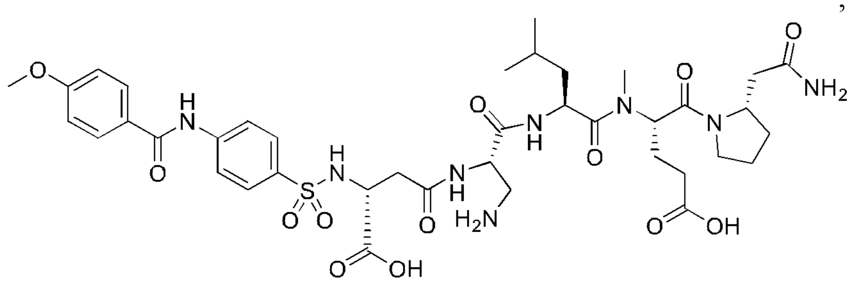

,

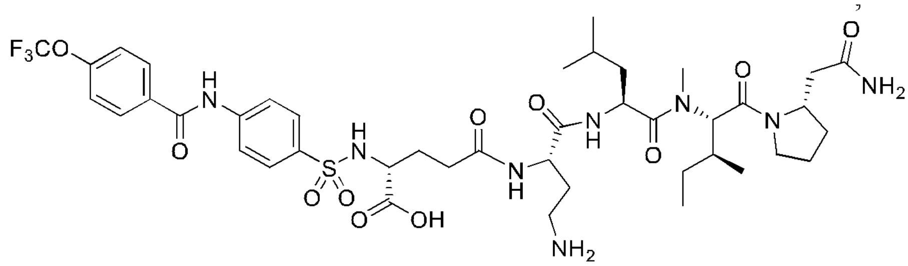

,

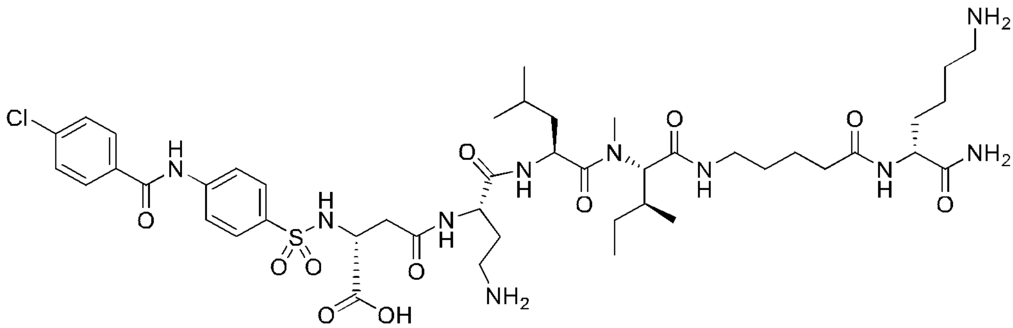

,

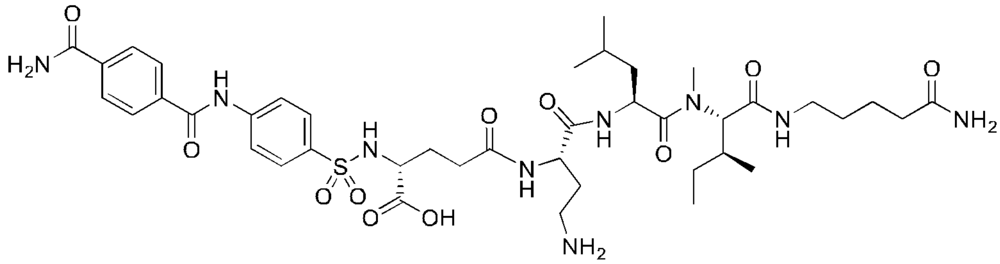

,

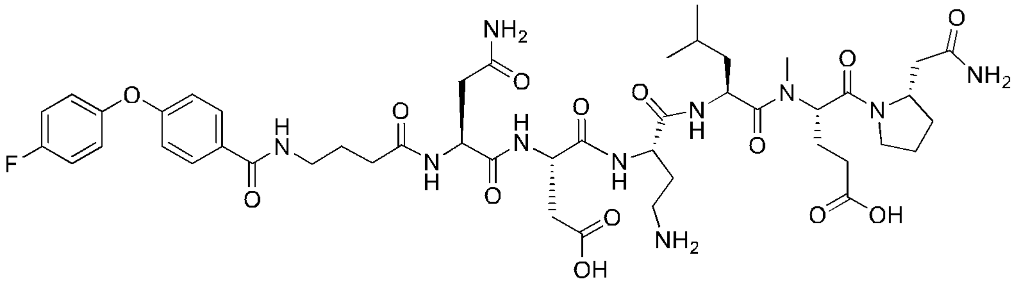

.

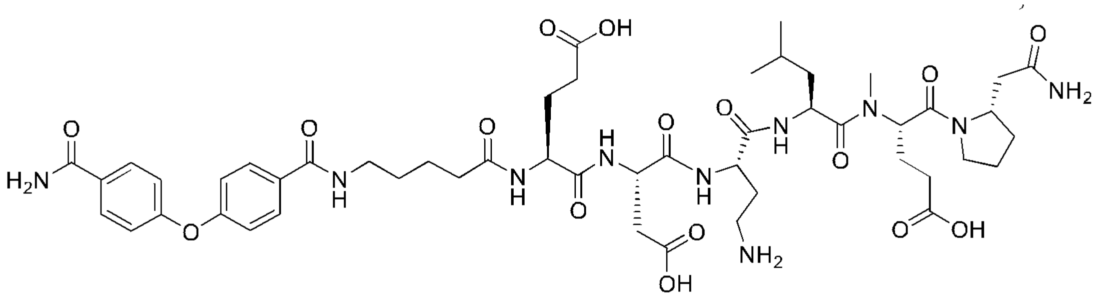

,

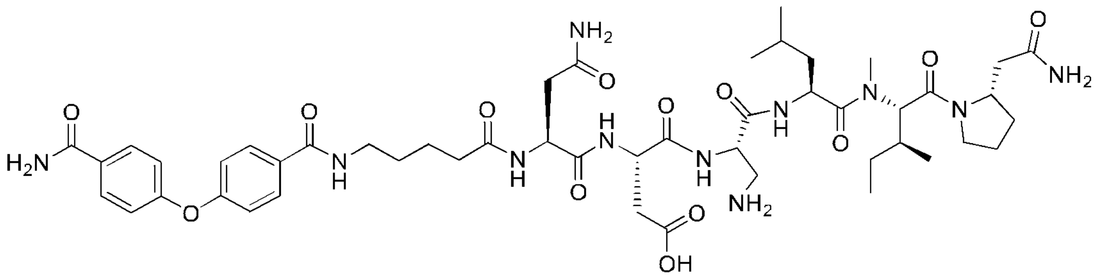

.

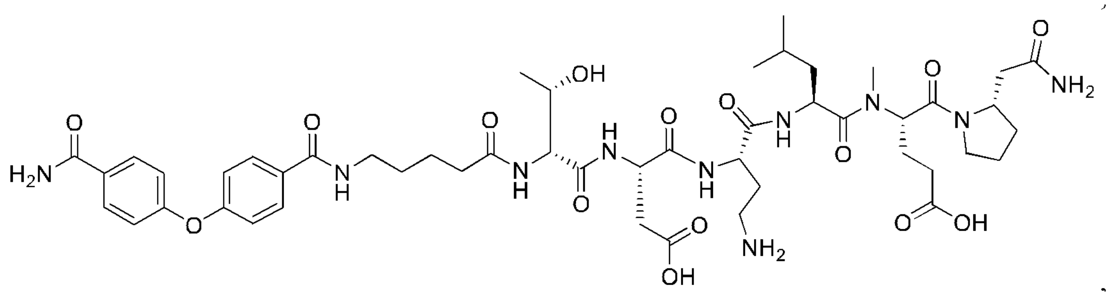

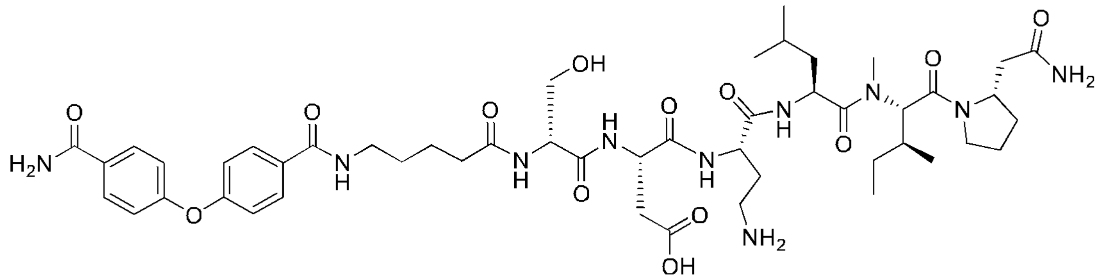

(11) Provided as another embodiment of the present invention is a pharmaceutical comprising the substituted polypeptide or pharmaceutically acceptable salt thereof according to any one of (1) to (10) as an active ingredient.
(12) Provided as another embodiment of the present invention is an MMP2 inhibitor comprising the substituted polypeptide or pharmaceutically acceptable salt thereof according to any one of (1) to (10) as an active ingredient.
(13) Provided as another embodiment of the present invention is an agent for suppressing growth, infiltration, or metastasis of cancer cells, comprising the substituted polypeptide or pharmaceutically acceptable salt thereof according to any one of (1) to (10) as an active ingredient.
(14) Provided as another embodiment of the present invention is an agent for suppressing fibrogenesis comprising the substituted polypeptide or pharmaceutically acceptable salt thereof according to any one of (1) to (10) as an active ingredient.
(15) Provided as another embodiment of the present invention is a drug for preventing or treating cancerous disease or organ fibrosis, or a symptom associated with cancerous disease or organ fibrosis, comprising the substituted polypeptide or pharmaceutically acceptable salt thereof according to any one of (1) to (10) as an active ingredient.

ADVANTAGEOUS EFFECTS OF INVENTION

The compounds of the present invention (hereinafter, occasionally referred to as "the present inventive compounds") have an effect to inhibit MMP2. Some of the present inventive compounds have an effect to selectively inhibit MMP2.

DESCRIPTION OF EMBODIMENTS

The present invention provides a substituted polypeptide represented by formula [I'], or a pharmaceutically acceptable salt thereof, having an effect to inhibit MMP2.
The compounds of the present invention will be described in more detail in the following; however, the present invention is not limited to exemplified ones.
Herein, "amino acid" is, in a broad sense, an organic compound having two functional groups: amino and carboxy. In a narrow sense (in particular, in the field of biochemistry), "amino acid" refers to "α-amino acid" (the α-amino acid is an amino acid in which amino is bonding to the carbon atom to which carboxy is bonding (α carbon)) that serves as a constituent unit of biogenic proteins.
Examples of amino acids in the present specification include natural proteogenic L-amino acids; natural nonproteogenic amino acids; and nonnatural amino acids. Examples of nonnatural amino acids include the D-forms of natural proteogenic L-amino acids; chemically modified amino acids such as amino acid variants and derivatives; and chemically synthesized compounds having properties that are characteristic to amino acids and known in the art.
Herein, when "amino acid" is shown as its name without abbreviating, for example, as a three-letter code or a one-letter code, the name indicates the amino acid in the L-form, D-form, or both.

Herein, when "amino acid" is shown as an abbreviation, for example, in a three-letter code or a one-letter code, the abbreviation indicates the amino acid in the L-form. "L" or "l" is occasionally added immediately before "amino acid" to explicitly show that the amino acid is in the L-form.

Herein, "D" or "d" added immediately before "amino acid" indicates that the amino acid is in the D-form.

Herein, "natural proteogenic L-amino acid" is a naturally occurring amino acid in L-form that constitutes proteins, and examples thereof include Gly, Ala, Val, Leu, Ile, Pro, Phe, His, Trp, Tyr, Ser, Thr, Met, Cys, Asp, Glu, Asn, Gln, Lys, and Arg.

Herein, "D-form of natural proteogenic L-amino acid" refers to an enantiomer of the natural proteogenic L-amino acid. Natural proteogenic L-amino acids except glycine each have at least one asymmetric carbon, thus being optically active. The structures of these amino acids are classified into L-forms and D-forms on the basis of the structures of the L-form and D-form of glyceraldehyde.

Amino acids other than natural proteogenic L-amino acids can also have D-forms.

Herein, "natural nonproteogenic amino acid" is a naturally occurring amino acid that does not constitute proteins, and examples thereof include L-norleucine (hereinafter, also designated as Nle; hereinafter, "code following in parentheses" shows an abbreviation), β-alanine (β-Ala), and L-ornithine (Orn).

If a natural nonproteogenic amino acid has an asymmetric carbon, there exist an L-form and a D-form for the amino acid. There can be L-forms and D-forms also for amino acids other than natural nonproteogenic amino acids.

Herein, "nonnatural amino acid" refers to an amino acid that does not constitute proteins and is primarily artificially produced, being an amino acid not included in the above-described "natural proteogenic L-amino acid and natural nonproteogenic amino acid". Examples of nonnatural amino acids include the D-forms of natural proteogenic L-amino acids (such as D-Cys, D-Ser); α-methylamino acids (such as 2-aminoisobutyric acid (Aib)); amino acids with excessive methylene in the side chain ("homo" amino acids such as L-β-homoproline (β-Hep or β-homoPro), L-homoserine (Hes or homoSer), L-homocysteine (Hec or homoCys), L-homoproline (homoPro), L-homoglutamic acid (homoGlu)); amino acids in which the side chain of an amino acid with a carboxylic acid functional group is substituted with a sulfonic acid group (such as L-cysteic acid); chemically modified amino acids such as amino acid variants and derivatives (such as hydroxyproline, L-2,3-diaminopropionic acid (Dap), L-2,4-diaminobutyric acid (Dab), N-methylglycine); and chemically synthesized compounds having properties that are characteristic to amino acids and known in the art (such as 4-aminobenzoic acid).

If a nonnatural amino acid has an asymmetric carbon, there exist an L-form and a D-form for the amino acid.

Specific examples of "nonnatural amino acid" in the present specification include the followings:

- (d)-Pro, (d)-Ser, (d)-Thr, (d)-Asp, (d)-Glu, (d)-Arg, (d)-Lys
- β-homoPro
- β-Ala, GABA, Ape, Acp
- (N-Me)Ala, (N-Me)Val, (N-Me)Leu, (N-Me)Ile, (N-Me)Phe, (N-Me)Tyr, (N-Me)Ser, (N-Me)Asp, (N-Me)Glu
- a group represented by formula [II-1] or formula [II-2]:

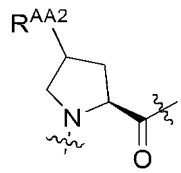 [II-1], 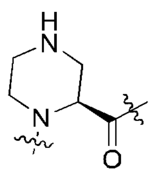 [II-2]

  wherein $R^{AA2}$ is hydroxy or amino; and
- amino acids listed later in Table 1 and Table 2

The nitrogen atom involved in peptide bonding in any amino acid in the present specification may be alkylated. In this case, the amino acid is also called "N-alkyl amino acid". Examples of the alkyl include methyl and ethyl.

Herein, the expression "β-Asp" means aspartic acid involved in amide bonding of the main chain via carboxy in the side chain, as illustrated in the structure represented by formula [III-1]. Likewise, the expressions "β-(d)-Asp", "γ-Glu", and "γ-(d)-Glu" mean the structures represented by formula [III-2] to formula [III-4].

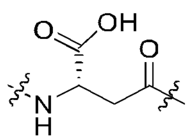 [III-1], 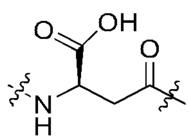 [III-2],

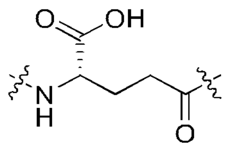 [III-3], 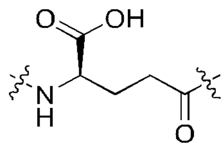 [III-4],

The expression "(N-Me)Glu(OtBu)" means the N-methyl form of the amino acid (Glu(OtBu)), as illustrated in the structure represented by formula [III-5].

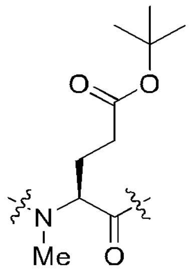 [III-5]

The expression "(2S,4S)-(4-amino)Pro" on the structure corresponding to $AA^2$ means the structure represented by formula [II-3]. Likewise, the expressions "(2S,4R)-(4-amino)Pro", "(2S,4S)-(4-hydroxy)Pro", and "(2S,4R)-(4-hydroxy)Pro" mean the structures represented by formula [II-4] to formula [II-6]. Further, the expression "(S)-piperazine" means the structure represented by formula [II-2].

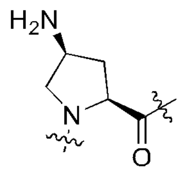 [II-3], 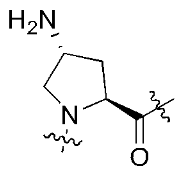 [II-4],

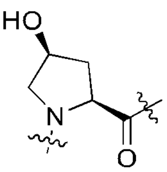 [II-5], 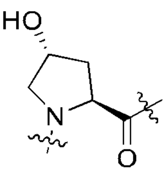 [II-6],

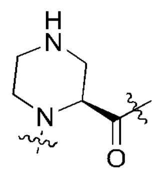 [II-2]

Herein, "n" indicates normal, "i" indicates iso, "s" indicates secondary, "t" and "tert" each indicate tertiary, "c" indicates cyclo, "o" indicates ortho, "m" indicates meta, and "p" indicates para.

**[0037]** "Halogen atom" refers to a fluorine atom, a chlorine atom, a bromine atom, or an iodine atom.

"$C_{1-6}$ alkyl" refers to linear or branched alkyl having one to six carbon atoms. Examples of "$C_{1-6}$ alkyl" include methyl, ethyl, n-propyl, isopropyl, n-butyl, isobutyl, sec-butyl, tert-butyl, n-pentyl, and n-hexyl.

"Halo $C_{1-6}$ alkyl" refers to linear or branched alkyl having one to six carbon atoms and substituted with a halogen atom. A preferred number of halogen atoms as substituents is one to five, and a preferred halogen atom is a fluorine atom. Examples of "halo $C_{1-6}$ alkyl" include monofluoromethyl, difluoromethyl, trifluoromethyl, 1-fluoroethyl, 1,1-difluoroethyl, 1,1,2,2-tetrafluoroethyl, 1,1,2,2,2-pentafluoroethyl, 2-fluoroethyl, 2-fluoro-2-methylpropyl, 2,2-difluoropropyl, 1-fluoro-2-methylpropan-2-yl, 1,1-difluoro-2-methylpropan-2-yl, 1-fluoropentyl, 1-fluorohexyl, and 2,2,2-trifluoro-1-methylethyl.

"Aryl" refers to a monocyclic aromatic hydrocarbon group or fused polycyclic aromatic hydrocarbon group having 6 to 14 carbon atoms. Examples of "aryl" include phenyl, naphthyl, and anthryl.

"Aromatic ring" refers to a monocyclic aromatic hydrocarbon group or fused polycyclic aromatic hydrocarbon group having 6 to 14 carbon atoms. Examples of "aromatic ring" include a benzene ring, a naphthalene ring, and an anthracene ring.

Partially saturated aryl is also included in the scope of "aryl". The same is applied to aromatic rings. "Partially saturated aryl" and the corresponding aromatic ring, "partially saturated aromatic ring", refer to a group wheren a monocyclic aromatic hydrocarbon group or fused polycyclic aromatic hydrocarbon group having 6 to 14 carbon atoms is partially saturated, and a ring having such a structure. Examples thereof include dihydroindenyl and a dihydroindene ring.

"Heteroaryl" refers to a five- to seven-membered monocyclic aromatic heterocyclic group consisting of one to six carbon atoms and one or more atoms that are same or different and selected from the group consisting of an oxygen atom, a sulfur atom, and a nitrogen atom, or a fused polycyclic aromatic heterocyclic group composed of 9 to 14 atoms, specifically, consisting of 1 to 13 carbon atoms and one or more atoms that are same or different and selected from the group consisting of an oxygen atom, a sulfur atom, and a nitrogen atom. Examples of "heteroaryl" include imidazolyl, pyrazolyl, thienyl, thiazolyl, isothiazolyl, thiadiazolyl, oxazolyl, isooxazolyl, oxadiazolyl, pyrrolyl, triazolyl, tetrazolyl, pyridyl, pyrimidinyl, pyrazinyl, pyridazinyl, triazinyl, indolyl, benzopyrazolyl, benzotriazolyl, benzofuranyl, benzothiophenyl, quinolyl, isoquinolyl, and quinoxalyl.

"Heteroaromatic ring" refers to a five- to seven-membered monocyclic aromatic heterocycle consisting of one to six carbon atoms and one or more atoms that are same or different and selected from the group consisting of an oxygen atom, a sulfur atom, and a nitrogen atom, or a fused polycyclic aromatic heterocycle composed of 9 to 14 atoms, specifically, consisting of 1 to 13 carbon atoms and one or more atoms that are same or different and selected from the group consisting of an oxygen atom, a sulfur atom, and a nitrogen atom. Examples of "heteroaromatic ring" include an imidazole ring, a pyrazole ring, a thiophene ring, a thiazole ring, an isothiazole ring, a thiadiazole ring, an oxazole ring, an isoxazole ring, an oxadiazole ring, a pyrrole ring, a triazole ring, a tetrazole ring, a pyridine ring, a pyrimidine ring, a pyrazine ring, a pyridazine ring, a triazine ring, an indole ring, a benzopyrazole ring, a benzotriazole ring, a benzofuran ring, a benzothiophene ring, a quinoline ring, an isoquinoline ring, and a quinoxaline ring.

Partially saturated heteroaryl is also included in the scope of "heteroaryl". The same is applied to heteroaromatic rings. "Partially saturated heteroaryl", and the corresponding heteroaromatic ring, "partially saturated heteroaromatic ring", refer to a five-to seven-membered partially saturated monocyclic heterocyclic group consisting of one to six carbon atoms and one or more atoms that are same or different and selected from the group consisting of an oxygen atom, a sulfur atom, and a nitrogen atom, or a partially saturated fused polycyclic heterocyclic group composed of 9 to 14 atoms, specifically, consisting of 1 to 13 carbon atoms and one or more atoms that are same or different and selected from the group consisting of an oxygen atom, a sulfur atom, and a nitrogen atom, and a ring having such a structure. Examples thereof include oxazolidinyl, thiazolinyl, dihydropyridinyl, dihydrobenzofuranyl, chromanyl, dihydropyranopyridinyl, dihydrofuropyridinyl, tetrahydroquinolyl, dihydrobenzodioxinyl, tetrahydrotriazoloazepinyl, an oxazolidine ring, a thiazoline ring, a dihydropyridine ring, a dihydrobenzofuran ring, a chroman ring, a dihydropyranopyridine ring, a dihydrofuropyridine ring, a tetrahydroquinoline ring, a tetrahydroquinoline ring, a dihydrobenzodioxine ring, and a tetrahydrotriazoloazepine ring.

"Four- to seven-membered saturated heterocyclyl containing nitrogen atom" refers to a four- to seven-membered monocyclic saturated heterocyclic group consisting of one nitrogen atom and three to six carbon atoms, and may further contain, in addition to the nitrogen atom, one atom selected from the group consisting of an oxygen atom, a sulfur atom, and a nitrogen atom. Examples of "four- to seven-membered saturated heterocyclyl containing nitrogen atom" include azetidinyl, pyrrolidinyl, piperidinyl, azepanyl, morpholinyl, thiomorpholinyl, and piperazinyl.

"Four- to seven-membered saturated heterocyclyl containing one nitrogen atom and optionally further containing one heteroatom" refers to a four- to seven-membered monocyclic saturated heterocyclic group consisting of one nitrogen atom and three to six carbon atoms, and may further contain, in addition to the nitrogen atom, one atom selected from the group consisting of an oxygen atom, a sulfur atom, and a nitrogen atom. Examples "four- to seven-membered saturated heterocyclyl containing one nitrogen atom and optionally further containing one heteroatom" include azetidinyl, pyrrolidinyl, piperidinyl, azepanyl, morpholinyl, thiomorpholinyl, and piperazinyl.

Examples of "four- to seven-membered saturated heterocyclyl containing one nitrogen atom and optionally further containing one heteroatom, and optionally crosslinked with $C_{1-4}$ alkanediyl" include 8-oxa-3-azabicyclo[3.2.1]octan-3-yl (the group represented by formula [VI-16]) and 3,8-diazabicyclo[3.2.1]octan-3-yl (the group represented by formula [VI-18]):

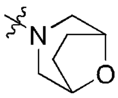 [VI-16], 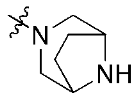 [VI-18]

"$C_{1-6}$ alkoxy" refers to linear or branched alkoxy having one to six carbon atoms. Examples "$C_{1-6}$ alkoxy" include

methoxy, ethoxy, n-propoxy, isopropoxy, n-butoxy, isobutoxy, sec-butoxy, tert-butoxy, n-pentyloxy, and n-hexyloxy.

"Halo $C_{1-6}$ alkoxy" refers to linear or branched alkoxy having one to six carbon atoms and substituted with a halogen atom. A preferred number of halogen atoms as substituents is one to five, and a preferred halogen atom is a fluorine atom. Examples of "halo $C_{1-6}$ alkoxy" include monofluoromethoxy, difluoromethoxy, trifluoromethoxy, 1-fluoroethoxy, 1,1-difluoroethoxy, 1,1,2,2-tetrafluoroethoxy, 2-fluoroethoxy, 2,2,2-trifluoroethoxy, 3,3,3-trifluoropropoxy, 1,3-difluoropropan-2-yloxy, 2-fluoro-2-methylpropoxy, 2,2-difluoropropoxy, 1-fluoro-2-methylpropan-2-yloxy, 1,1-difluoro-2-methylpropan-2-yloxy, and 4,4,4-trifluorobutoxy.

"$C_{1-6}$ alkylcarbonyl" refers to a group in which "$C_{1-6}$ alkyl" described above and carbonyl are bonding together. Examples of "$C_{1-6}$ alkylcarbonyl" include methylcarbonyl, ethylcarbonyl, n-propylcarbonyl, isopropylcarbonyl, n-butylcarbonyl, isobutylcarbonyl, sec-butylcarbonyl, tert-butylcarbonyl, n-pentylcarbonyl, and n-hexylcarbonyl.

"$C_{1-6}$ alkylcarbonylamino" refers to a group in which "$C_{1-6}$ alkylcarbonyl" described above and an amino group are bonding together. Examples of "$C_{1-6}$ alkylcarbonylamino" include methylcarbonylamino, ethylcarbonylamino, n-propylcarbonylamino, isopropylcarbonylamino, n-butylcarbonylamino, isobutylcarbonylamino, sec-butylcarbonylamino, tert-butylcarbonylamino, n-pentylcarbonylamino, and n-hexylcarbonyl amino.

"$C_{1-6}$ alkylamino" refers to amino having one or two "$C_{1-6}$ alkyl" described above, as substituents, that are same or different. Examples of "$C_{1-6}$ alkylamino" include methylamino, ethylamino, n-propylamino, isopropylamino, n-butylamino, isobutylamino, sec-butylamino, tert-butylamino, n-pentylamino, n-hexylamino, dimethylamino, diethylamino, di(n-propyl)amino, di(isopropyl)amino, ethylmethylamino, and methyl(n-propyl)amino.

"Mono $C_{1-6}$ alkylamino" refers to amino having one "$C_{1-6}$ alkyl" described above as a substituent. Examples of "mono $C_{1-6}$ alkylamino" include methylamino, ethylamino, n-propylamino, isopropylamino, n-butylamino, isobutylamino, sec-butylamino, tert-butylamino, n-pentylamino, and n-hexylamino.

"Di $C_{1-6}$ alkylamino" refers to amino having two "$C_{1-6}$ alkyl" described above, as substituents, that are same or different. Examples of "di $C_{1-6}$ alkylamino" include dimethylamino, diethylamino, di(n-propyl)amino, di(isopropyl)amino, ethylmethylamino, and methyl(n-propyl)amino.

"Mono $C_{1-6}$ alkylaminocarbonyl" refers to a group in which "mono $C_{1-6}$ alkylamino" described above and carbonyl are bonding together. Examples of "mono $C_{1-6}$ alkylaminocarbonyl" include methylaminocarbonyl, ethylaminocarbonyl, n-propylaminocarbonyl, isopropylaminocarbonyl, n-butylaminocarbonyl, isobutylaminocarbonyl, n-pentylaminocarbonyl, and n-hexylaminocarbonyl.

"Di $C_{1-6}$ alkylaminocarbonyl" refers to a group in which "di $C_{1-6}$ alkylamino" described above and carbonyl are bonding together. Examples of "di $C_{1-6}$ alkylaminocarbonyl" include dimethylaminocarbonyl, diethylaminocarbonyl, di(n-propyl)aminocarbonyl, di(isopropyl)aminocarbonyl, ethylmethylaminocarbonyl, and methyl (n-propyl)aminocarbonyl.

"$C_{1-6}$ alkylcarbonyl" refers to a group in which "$C_{1-6}$ alkyl" described above and carbonyl are bonding together. Examples of "$C_{1-6}$ alkylcarbonyl" include methylcarbonyl, ethylcarbonyl, n-propylcarbonyl, isopropylcarbonyl, n-butylcarbonyl, isobutylcarbonyl, sec-butylcarbonyl, tert-butylcarbonyl, n-pentylcarbonyl, and n-hexylcarbonyl.

"$C_{2-16}$ alkylcarbonyl" refers to a group in which linear or branched alkyl having 2 to 16 carbon atoms and carbonyl are bonding together. Examples of "$C_{2-16}$ alkylcarbonyl" include ethylcarbonyl, n-propylcarbonyl, isopropylcarbonyl, n-butylcarbonyl, isobutylcarbonyl, sec-butylcarbonyl, tert-butylcarbonyl, n-decylcarbonyl(undecanoyl), n-dodecylcarbonyl(tridecanoyl), and n-tetradecylcarbonyl(pentadecanoyl).

"$C_{2-16}$ alkylcarbonyl terminally-substituted with carboxy" refers to a group in which a terminus of "$C_{2-16}$ alkyl" in "$C_{2-16}$ alkylcarbonyl" described above is substituted with carboxy. Examples of "$C_{2-16}$ alkylcarbonyl terminally-substituted with carboxy" include 11-carboxyundecanoyl, 13-carboxytridecanoyl, and 15-carboxypentadecanoyl.

"$C_{1-6}$ alkylsulfonyl" refers to a group in which "$C_{1-6}$ alkyl" described above and sulfonyl are bonding together. Examples of "$C_{1-6}$ alkylsulfonyl" include methylsulfonyl, ethylsulfonyl, n-propylsulfonyl, isopropylsulfonyl, n-butylsulfonyl, isobutylsulfonyl, sec-butylsulfonyl, tert-butylsulfonyl, n-pentylsulfonyl, and n-hexylsulfonyl.

"$C_{1-3}$ alkanediyl" refers to a divalent hydrocarbon formed by removing one hydrogen atom from alkyl having one to three carbon atoms. Examples of "$C_{1-3}$ alkanediyl" include methanediyl, ethane-1,1-diyl, ethane-1,2-diyl, propane-1,1-diyl, propane-1,3-diyl, and propane-2,2-diyl.

"$C_{1-4}$ alkanediyl" refers to a divalent hydrocarbon formed by removing one hydrogen atom from alkyl having one to four carbon atoms. Examples of "$C_{1-4}$ alkanediyl" include methanediyl, ethane-1,1-diyl, ethane-1,2-diyl, propane-1,1-diyl, propane-1,3-diyl, propane-2,2-diyl, and butane-1,4-diyl.

"$C_{2-3}$ alkenediyl" refers to a divalent unsaturated hydrocarbon formed by removing one hydrogen atom from alkenyl having two or three carbon atoms. Examples of "$C_{2-3}$ alkenediyl" include ethene-1,1-diyl, ethene-1,2-diyl, prop-1-ene-1,1-diyl, prop-2-ene-1,1-diyl, prop-1-ene-1,3-diyl, prop-2-ene-1,3-diyl, and prop-1-ene-2,2-diyl.

"Triazolediyl" refers to divalent triazole formed by removing two hydrogen atoms from a triazole ring. Examples of "triazolediyl" include structures represented by formulas [VIII-1] to [VIII-5]:

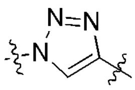 [VIII-1], 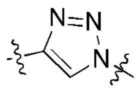 [VIII-2], 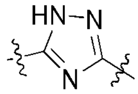 [VIII-3],

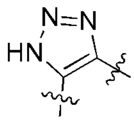 [VIII-4], 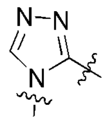 [VIII-5]

Abbreviations used herein indicate structures listed in Table 1 to Table 4.

[Table 1-1]

| # | Formula number | Abbreviation | Structure |
|---|---|---|---|
| 1 | [III-6] | β-Dap | 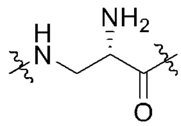 |
| 2 | [III-7] | β-(d)-Dap | 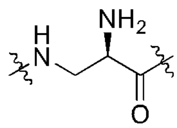 |
| 3 | [III-8] | γ-Dab | 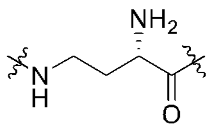 |
| 4 | [III-9] | γ-(d)-Dab | 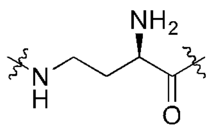 |
| 5 | [III-10] | δ-Orn | 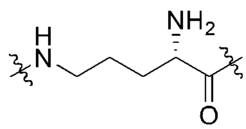 |
| 6 | [III-11] | δ-(d)-Orn | 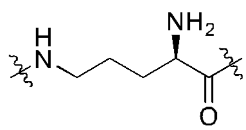 |
| 7 | [III-12] | ε-Lys | 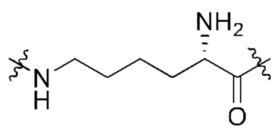 |
| 8 | [III-13] | ε-(d)-Lys | 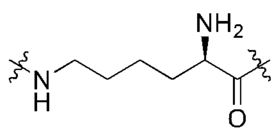 |

[Table 2-1]

| # | Formula number | Abbreviation | Structure |
|---|---|---|---|
| 1 | [IV-1] | Aib | 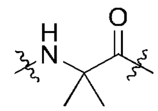 |
| 2 | [IV-2] | Nle | 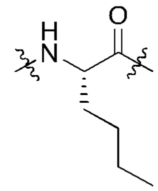 |
| 3 | [IV-3] | Ala(cPropyl) | 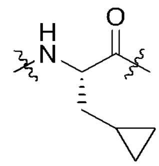 |
| 4 | [IV-4] | Ala(2-Pyr) | 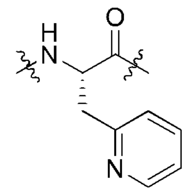 |
| 5 | [IV-5] | Ala(4-Thz) | 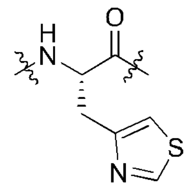 |
| 6 | [IV-6] | homoSer | 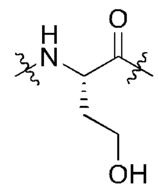 |
| 7 | [IV-7] | Dap | 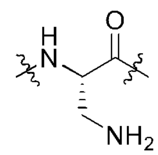 |
| 8 | [IV-8] | Dap(Me) | 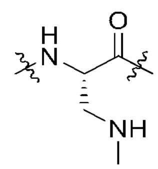 |

[Table 2-2]

| # | Formula number | Abbreviation | Structure |
|---|---|---|---|
| 9 | [IV-9] | Dab | 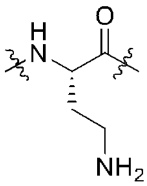 |
| 10 | [IV-10] | (d)-Dab | 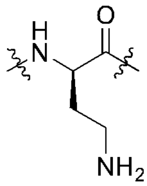 |
| 11 | [IV-11] | Dab(Me) | 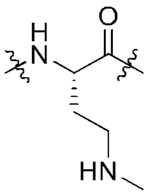 |
| 12 | [IV-12] | $Dab(Me)_2$ | 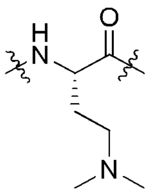 |
| 13 | [IV-13] | Orn | 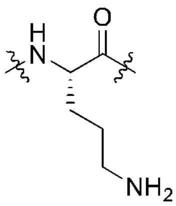 |
| 14 | [IV-14] | Lys(Ac) | 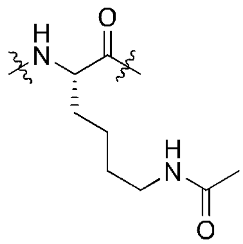 |

[Table 2-3]

| # | Formula number | Abbreviation | Structure |
|---|---|---|---|
| 15 | [IV-15] | Cit | 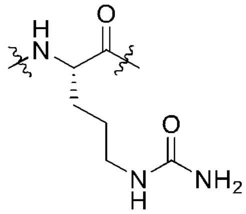 |
| 16 | [IV-16] | (d)-Cit | 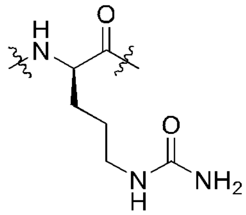 |
| 17 | [IV-17] | β-Ala | 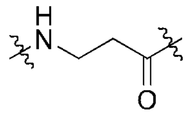 |
| 18 | [IV-18] | (N-Me)-β-Ala | 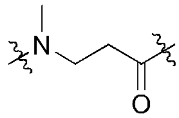 |
| 19 | [IV-19] | GABA | 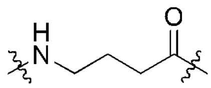 |
| 20 | [IV-20] | (N-Me)GABA | 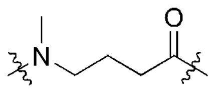 |
| 21 | [IV-21] | Ape | 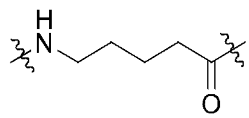 |
| 22 | [IV-22] | Acp | 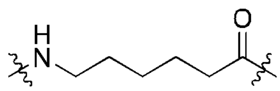 |
| 23 | [IV-23] | -NH-$(CH_2)_2$-O-$CH_2$-CO- | 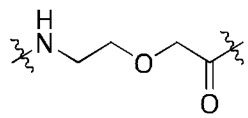 |
| 24 | [IV-24] | Adox | 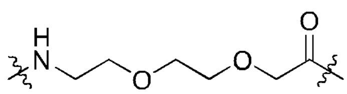 |

[Table 2-4]

| # | Formula number | Abbreviation | Structure |
|---|---|---|---|
| 25 | [IV-25] | cis-NH(3)cPen | 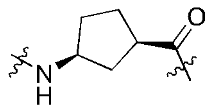 |
| 26 | [IV-26] | (4)Abz | 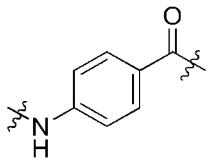 |
| 27 | [IV-27] | Aze(2) | 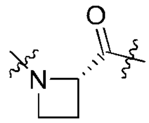 |
| 28 | [IV-28] | (d)-Aze(2) | 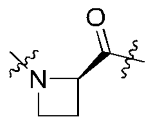 |
| 29 | [IV-29] | Aze(3) | 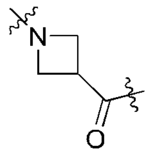 |
| 30 | [IV-30] | β-homoPro | 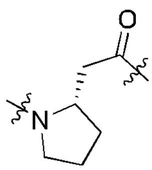 |
| 31 | [IV-31] | homoPro | 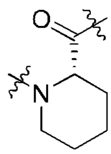 |
| 32 | [IV-32] | aspartimide-Dab | 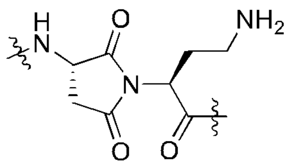 |
| 33 | [IV-33] | homoGlu | 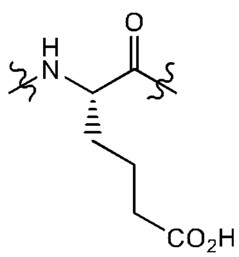 |

[Table 3-1]

| # | Formula number | Abbreviation | Structure |
|---|---|---|---|
| 1 | [V-1] | Lys(CO-$(CH_2)_{10}$-$CO_2H$) | 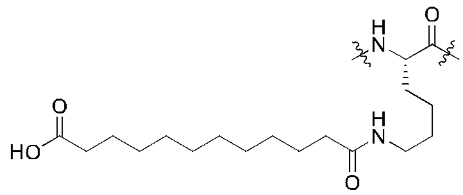 |
| 2 | [V-2] | Lys(CO-$(CH_2)_{12}$-$CO_2H$) | 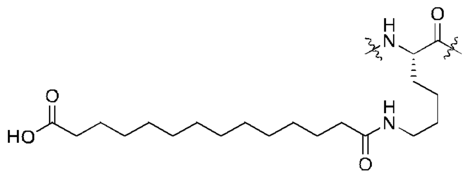 |
| 3 | [V-3] | Lys(CO-$(CH_2)_{14}$-$CO_2H$) | 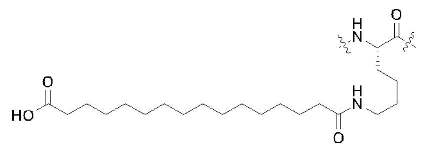 |

[Table 4-1]

| # | Formula number | Abbreviation | Structure |
|---|---|---|---|
| 1 | [VI-1] | -NHMe | 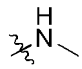 |
| 2 | [VI-2] | -NHEt | 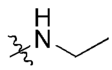 |
| 3 | [VI-3] | -NH-$(CH_2)_2$-OH | 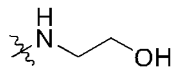 |
| 4 | [VI-4] | -NH-$(CH_2)_2$-$NH_2$ | 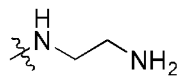 |
| 5 | [VI-5] | -NH-$(CH_2)_3$-$NH_2$ | 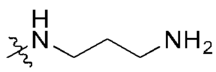 |
| 6 | [VI-6] | -NH-$(CH_2)_4$-$NH_2$ | 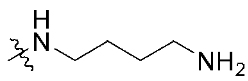 |
| 7 | [VI-7] | -NH-$(CH_2)_5$-$NH_2$ | 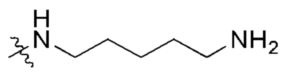 |
| 8 | [VI-8] | the formula [VI-8] | 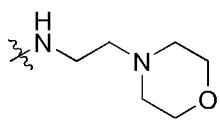 |
| 9 | [VI-9] | azetidin-1-yl | 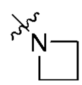 |

(continued)

| # | Formula number | Abbreviation | Structure |
|---|---|---|---|
| 10 | [VI-10] | pyrrolidin-1-yl | 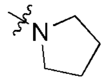 |
| 11 | [VI-11] | piperidin-1-yl | 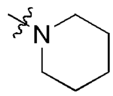 |
| 12 | [VI-12] | (3-OH)azetidin-1-yl | 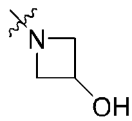 |
| 13 | [VI-13] | (4-OH)piperidin-1-yl | 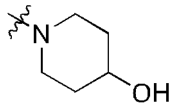 |

[Table 4-2]

| # | Formula number | Abbreviation | Structure |
|---|---|---|---|
| 14 | [VI-14] | the formula [VI-14] | 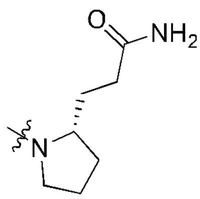 |
| 15 | [VI-15] | morpholin-4-yl | 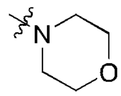 |
| 16 | [VI-16] | the formula [VI-16] | 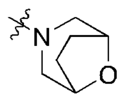 |
| 17 | [VI-17] | (4-Me)piperazin-1-yl | 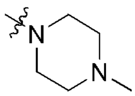 |
| 18 | [VI-18] | the formula [VI-18] | 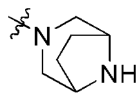 |

Preferable embodiments of the substituted polypeptide represented by formula [I'], or pharmaceutically acceptable salt thereof according to the present invention are as follows.

Preferable $AA^1$ is Asp, β-Asp, β-(d)-Asp, γ-Glu, or γ-(d)-Glu,

one more preferable $AA^1$ is β-Asp, γ-Glu, or γ-(d)-Glu,

wherein further preferable $AA^1$ is γ-(d)-Glu,

another more preferable $AA^1$ is β-(d)-Asp or γ-(d)-Glu,

wherein further preferable $AA^1$ is β-(d)-Asp, and

another more preferable $AA^1$ is Asp.

Preferable $AA^2$ is a group represented by formula [II-1] or formula [II-2]:

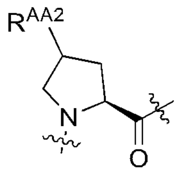 [II-1], 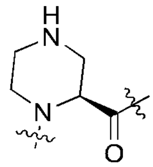 [II-2]

a group represented by formula [IV-7], [IV-8], [IV-9], [IV-11], or [IV-12]:

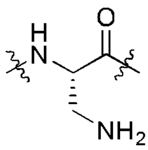 [IV-7], 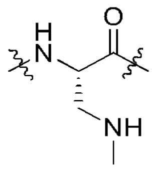 [IV-8],

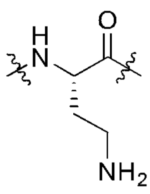 [IV-9], 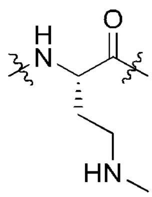 [IV-11], 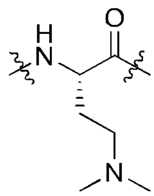 [IV-12]

Ala,
a group represented by formula [IV-27]:

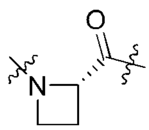 [IV-27]

, or

Pro,
wherein preferable $R^{AA2}$ is hydroxy or amino,
more preferable $AA^2$ is a group represented by formula [II-1]:

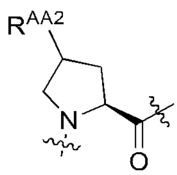 [II-1]

or a group represented by formula [IV-7], [IV-8], [IV-9], [IV-11], or [IV-12]:

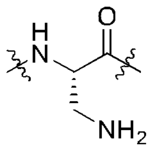 [IV-7], 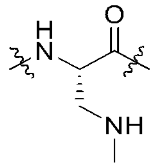 [IV-8],

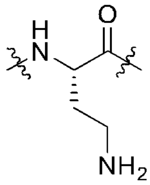 [IV-9], 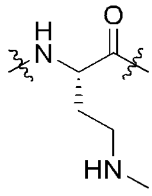 [IV-11], 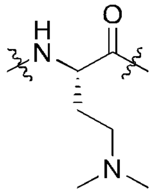 [IV-12]

wherein preferable $R^{AA2}$ is amino,
further preferable $AA^2$ is
a group represented by formula [IV-7] or [IV-9]:

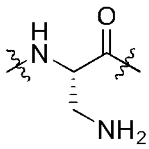 [IV-7], 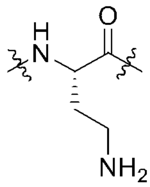 [IV-9]

or a group represented by formula [II-1]:

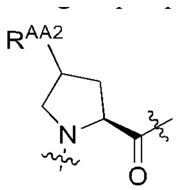 [II-1]

wherein preferable $R^{AA2}$ is amino,
one particularly preferable $AA^2$ is
a group represented by formula [II-1]:

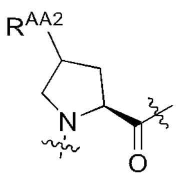 [II-1]

wherein preferable $R^{AA2}$ is amino, and
another particularly preferable $AA^2$ is
a group represented by formula [IV-7] or [IV-9]:

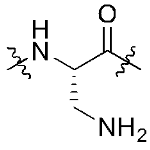 [IV-7], 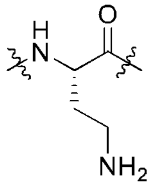 [IV-9].

Preferable $AA^3$ is Val, Leu, Ile, a group represented by formula [IV-2]:

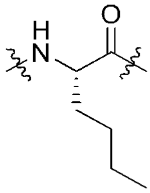 [IV-2]

Phe, a group represented by formula [IV-3], [IV-4], or [IV-5]:

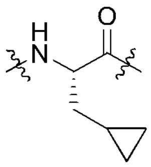 [IV-3], 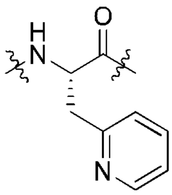 [IV-4], 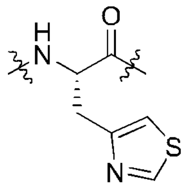 [IV-5]

or Trp,

more preferable $AA^3$ is Val, Leu, Ile, Phe, or Trp,
further preferable $AA^3$ is Val, Leu, or Ile,
one particularly preferable $AA^3$ is Val,
another particularly preferable $AA^3$ is Leu, and
another particularly preferable $AA^3$ is Ile.

Preferable $AA^4$ is a single bond, Pro, Gly, homoSer, Met, Glu, (N-Me)Ala, (N-Me)Val, (N-Me)Leu, (N-Me)Ile, (N-Me)Phe, (N-Me)Tyr, (N-Me)Ser, (N-Me)Met, (N-Me)Asp, (N-Me)Glu, (d)-Pro, (d)-Ala, (d)-Phe, (d)-Tyr, (d)-Ser, (d)-Thr, or (d)-(N-Me)Glu,

more preferable $AA^4$ is (N-Me)Ala, (N-Me)Val, (N-Me)Leu, (N-Me)Ile, (N-Me)Phe, (N-Me)Tyr, (N-Me)Ser, (N-Me)Met, (N-Me)Asp, (N-Me)Glu, (d)-Pro, (d)-Ala, (d)-Phe, (d)-Tyr, (d)-Ser, (d)-Thr, or (d)-(N-Me)Glu,
one further preferable $AA^4$ is (N-Me)Glu or (N-Me)Asp,
wherein particularly preferable $AA^4$ is (N-Me)Glu, and
another further preferable $AA^4$ is (N-Me)Ile, (N-Me)Val, or (N-Me)Leu,
wherein particularly preferable $AA^4$ is (N-Me)Ile.

Preferable $AA^5$ is β-Ala, GABA, Ape, Acp, Pro, (d)-Pro, β-homoPro, a single bond, Arg, (d)-Arg, a group represented by [IV-7], [IV-9], or [IV-13]:

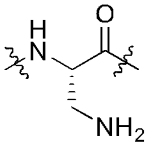 [IV-7], 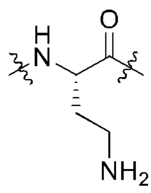 [IV-9], 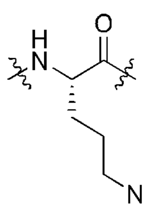 [IV-13]

Lys, (d)-Lys,
Ala, a group represented by formula [IV-1]:

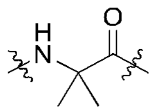 [IV-1]

a group represented by formula [IV-27], [IV-28], or [IV-29]:

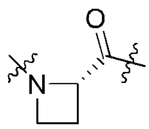 [IV-27], 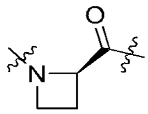 [IV-28], 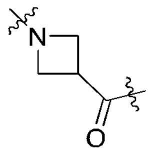 [IV-29]

Phe, His, Thr, or
a group represented by any of formulas [III-6] to [III-13]:

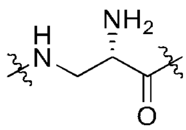 [III-6], 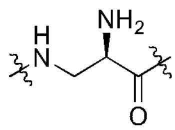 [III-7],

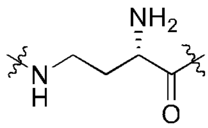 [III-8], 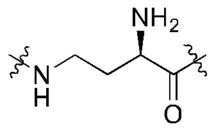 [III-9],

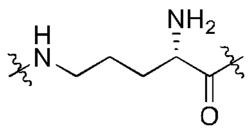 [III-10], 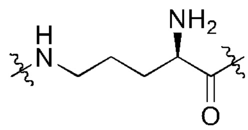 [III-11],

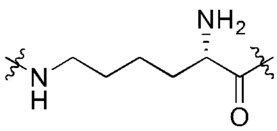 [III-12], 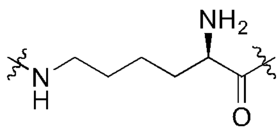 [III-13]

more preferable $AA^5$ is β-Ala, GABA, Ape, Acp, Pro, (d)-Pro, β-homoPro, a single bond, Arg, (d)-Arg, a group represented by formula [IV-7], [IV-9], or [IV-13]:

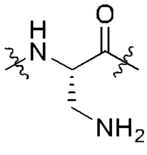 [IV-7], 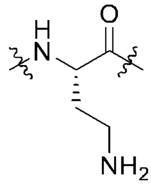 [IV-9], 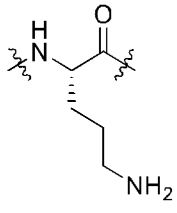 [IV-13]

Lys, or (d)-Lys,
further preferable $AA^5$ is β-Ala, GABA, Ape, Acp, Pro, (d)-Pro, or β-homoPro, and particularly preferable $AA^5$ is β-Ala, GABA, Ape, Acp, or β-homoPro.

Preferable $W^1$ is $-L^1-$ or $-L^{1'}-L^{1''}-$,

wherein $L^1$ is a single bond,
$L^{1'}$ is selected from the group consisting of β-Ala, GABA, Ape, Acp,
a group represented by any of formulas [III-6] to [III-13]:

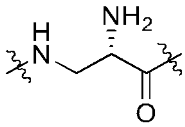 [III-6], 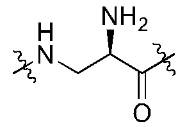 [III-7],

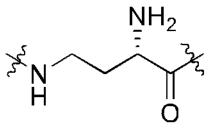 [III-8], 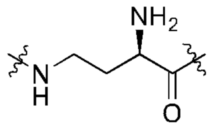 [III-9],

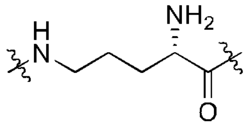 [III-10], 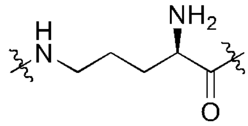 [III-11],

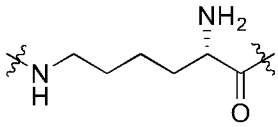 [III-12], 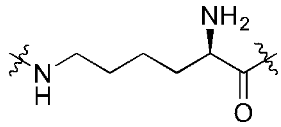 [III-13]

, and a group represented by any of formulas [IV-23] and [IV-24]:

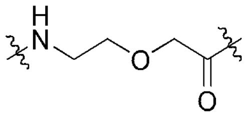 [IV-23], 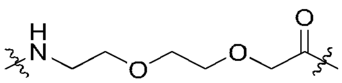 [IV-24];

, and $L^{1''}$ is a single bond, Asn, (d)-Ser, (d)-Thr, Lys, Arg, or Glu, one more preferable $W^1$ is $-L^1-$ or $-L^{1'}-L^{1''}-$,
wherein $L^1$ is a single bond,
$L^{1'}$ is β-Ala, GABA, Ape, Acp, or
a group represented by any of formulas [III-6] to [III-13]:

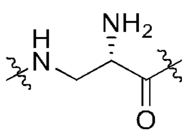 [III-6], 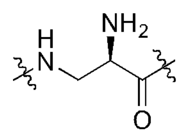 [III-7],

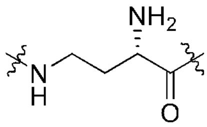 [III-8], 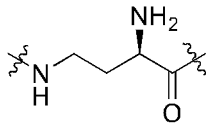 [III-9],

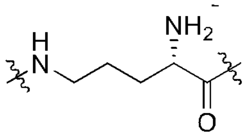 [III-10], 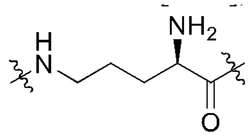 [III-11],

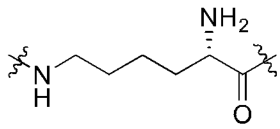 [III-12], 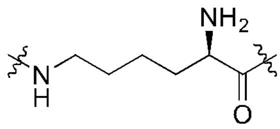 [III-13]

, and $L^{1}$" is Asn, (d)-Ser, (d)-Thr, or Glu,
wherein further preferable $W^1$ is -$L^1$- or -$L^{1}$'-$L^{1}$"-,
wherein $L^1$ is a single bond,
$L^{1}$' is β-Ala, GABA, Ape, or Acp, and
$L^{1}$" is Asn, (d)-Ser, (d)-Thr, or Glu,
wherein particularly preferable $W^1$ is -$L^1$-,
wherein $L^1$ is a single bond, and
another more preferable $W^1$ is -$L^{1}$'-$L^{1}$"-,
wherein $L^{1}$' is a group represented by formula [IV-23] or [IV-24]:

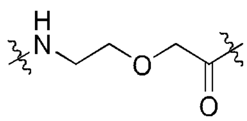 [IV-23], 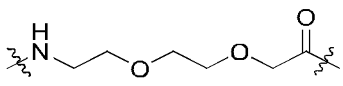 [IV-24];

, and $L^{1}$" is a single bond.

Preferable $L^2$ is a single bond.

Preferable $L^{N1}$ is the formula -C(=O)- or the formula -$S(=O)_2$-,

one more preferable $L^{N1}$ is the formula -C(=O)-, and
another more preferable $L^{N1}$ is the formula -$S(=O)_2$-.

Preferable $L^{N2}$ is a single bond, the formula -O-, or the formula -C(=O)-NH-, more preferable $L^{N2}$ is the formula -O- or the formula -C(=O)-NH-,

one further preferable $L^{N2}$ is the formula -O-, and
another further preferable $L^{N2}$ is the formula -C(=O)-NH-.

Preferable ring A is a benzene ring or a pyridine ring,

more preferable ring A is a benzene ring,
preferable $R^{A1}$ and $R^{A2}$ are each independently a hydrogen atom or a halogen atom, and
more preferable $R^{A1}$ and $R^{A2}$ are each a hydrogen atom.

Preferable ring B is phenyl or pyridyl,

more preferable ring B is phenyl,
preferable $R^{B1}$, $R^{B2}$, and $R^{B3}$ are each independently a hydrogen atom, carbamoyl, a halogen atom, $C_{1-6}$ alkyl, halo $C_{1-6}$ alkyl, $C_{1-6}$ alkoxy, or halo $C_{1-6}$ alkoxy, and more preferable $R^{B1}$, $R^{B2}$, and $R^{B3}$ are each independently a hydrogen atom, carbamoyl, a halogen atom, $C_{1-6}$ alkoxy, or halo $C_{1-6}$ alkoxy.

Preferable $W^C$ is a single bond or a linker consisting of one to three amino acids, wherein

the one to three amino acids forming the linker are not limited, but preferably same or

different and each selected from the group consisting of: Gly, Pro, Arg, (d)-Arg, Lys, (d)-Lys, β-Ala, GABA, and Ape, wherein
if Lys or (d)-Lys is included in the group represented by $W^C$,
then the amino in the side chain of the Lys or (d)-Lys is optionally substituted with:

$C_{2-16}$ alkylcarbonyl terminally-substituted with carboxy,
Lys, wherein the amino in the side chain of the Lys is optionally substituted with $C_{2-16}$ alkylcarbonyl terminally-substituted with carboxy, or
(d)-Lys, wherein the amino in the side chain of the (d)-Lys is optionally substituted with $C_{2-16}$ alkylcarbonyl terminally-substituted with carboxy,

more preferable $W^C$ is a single bond, Pro, Arg, (d)-Arg, Lys, (d)-Lys, or (d)-Lys-(d)-Lys, further preferable $W^C$ is a single bond, Arg, (d)-Arg, Lys, or (d)-Lys,
one particularly preferable $W^C$ is a single bond, and
another particularly preferable $W^C$ is (d)-Lys.

Preferable $R^C$ is:

the formula -OH, the formula $-NH_2$,
$C_{1-6}$ alkylamino, wherein the $C_{1-6}$ alkyl of the $C_{1-6}$ alkylamino is optionally substituted with one group selected from the group consisting of hydroxy, amino, and a four-to seven-membered saturated heterocyclyl containing one nitrogen atom, or
four- to seven-membered saturated heterocyclyl containing one nitrogen atom and optionally further containing one heteroatom, wherein the four- to seven-membered saturated heterocyclyl containing one nitrogen atom and optionally further containing one heteroatom is optionally substituted with one group selected from the group consisting of hydroxy, and $C_{1-6}$ alkyl, wherein the $C_{1-6}$ alkyl is optionally substituted with one carbamoyl; and two carbon atoms in the four- to seven-membered saturated heterocyclyl containing one nitrogen atom and optionally further containing one heteroatom are optionally crosslinked with $C_{1-4}$ alkanediyl,
more preferable $R^C$ is the formula -OH or the formula $-NH_2$, and
further preferable $R^C$ is the formula $-NH_2$.

Another preferable embodiment of the compounds of the present invention is a substituted polypeptide represented by formula [I'], or a pharmaceutically acceptable salt thereof, wherein

$AA^1$ is β-Asp, γ-Glu, or γ-(d)-Glu,
$AA^2$ is a group represented by formula [II-1] or formula [II-2]:

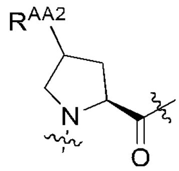
[II-1],
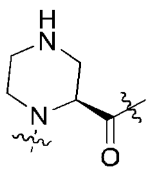
[II-2]

wherein $R^{AA2}$ is hydroxy or amino,
$AA^3$ is Val, Leu, Ile, Phe, or Trp,
$AA^4$ is a single bond, Pro, (N-Me)Ala, (N-Me)Val, (N-Me)Leu, (N-Me)Ile, (N-Me)Phe, (N-Me)Tyr, (N-Me)Ser, (N-Me)Asp, or (N-Me)Glu,
$AA^5$ is a single bond, Pro, (d)-Pro, β-homoPro, Arg, (d)-Arg, Lys, (d)-Lys, β-Ala, GABA, Ape, or Acp,
$W^1$ is $L^1$, and $L^1$ is a single bond,
L2 is a single bond,
$L^{N1}$ is the formula $-S(=O)_2-$,
$L^{N2}$ is a single bond, the formula -O-, or the formula -C(=O)-NH-,
ring A is a benzene ring,
$R^{A1}$ and $R^{A2}$ are each a hydrogen atom,
ring B is phenyl,
$R^{B1}$, $R^{B2}$, and $R^{B3}$ are each independently a hydrogen atom, carbamoyl, a halogen atom, or $C_{1-6}$ alkoxy,
$W^C$ is a single bond, Pro, Arg, (d)-Arg, Lys, (d)-Lys, or (d)-Lys-(d)-Lys, and

$R^C$ is the formula $-NH_2$.

Another preferable embodiment of the present invention is a substituted polypeptide represented by formula [I'],or a pharmaceutically acceptable salt thereof, wherein

$AA^1$ is β-Asp, γ-Glu, or γ-(d)-Glu, wherein further preferable $AA^1$ is γ-(d)-Glu,
$AA^2$ is a group represented by formula [II-1]:

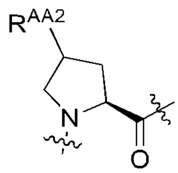

[II-1]

wherein $R^{AA2}$ is amino,
$AA^3$ is Val, Leu, or Ile,

wherein one further preferable $AA^3$ is Val,
another further preferable $AA^3$ is Leu, and
another further preferable $AA^3$ is Ile,

$AA^4$ is (N-Me)Val, (N-Me)Leu, (N-Me)Ile, (N-Me)Asp, or (N-Me)Glu,

wherein one further preferable $AA^4$ is (N-Me)Val, (N-Me)Leu, or (N-Me)Ile,
wherein particularly preferable $AA^4$ is (N-Me)Ile,
another further preferable $AA^4$ is (N-Me)Asp or (N-Me)Glu,
wherein particularly preferable $AA^4$ is (N-Me)Glu,

$AA^5$ is Pro, (d)-Pro, β-homoPro, β-Ala, GABA, Ape, or Acp,

wherein one further preferable $AA^5$ is Pro, (d)-Pro, or β-homoPro,
wherein particularly preferable $AA^5$ is β-homoPro,
another further preferable $AA^5$ is β-Ala, GABA, Ape, or Acp,
wherein particularly preferable $AA^5$ is β-Ala, GABA, or Ape,

$W^1$ is $L^1$,
$L^1$ is a single bond,
$L^2$ is a single bond,
$L^{N1}$ is the formula $-S(=O)_2-$,
$L^{N2}$ is a single bond, the formula -O-, or the formula -C(=O)-NH-,

wherein one further preferable $L^{N1}$ is the formula -C(=O)-, and
another further preferable $L^{N1}$ is the formula $-S(=O)_2-$,

ring A is a benzene ring,
$R^{A1}$ and $R^{A2}$ are each a hydrogen atom,
ring B is phenyl,
$R^{B1}$, $R^{B2}$, and $R^{B3}$ are each independently a hydrogen atom, carbamoyl, a halogen atom, or $C_{1-6}$ alkoxy,
$W^C$ is a single bond or (d)-Lys,

wherein one further preferable $W^C$ is a single bond, and
another further preferable $W^C$ is (d)-Lys, and

$R^C$ is the formula $-NH_2$.

Another preferable embodiment of the compounds of the present invention is the substituted polypeptide or a pharmaceutically acceptable salt thereof, wherein

$W^1$ is $-L^{1'}-L^{1''}-$,
$L^2$ is a single bond, and
the polypeptide is represented by formula [I'-A]:

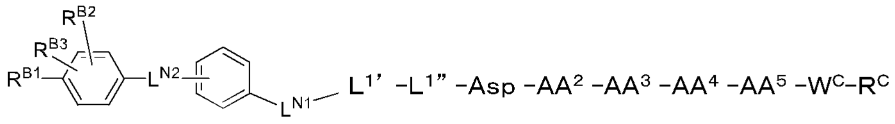
[I'-A],

wherein preferable embodiments of $AA^2$, $AA^3$, $AA^4$, $AA^5$, $L^{1'}$, $L^{1''}$, $L^{N1}$, $L^{N2}$, $R^{B1}$, $R^{B2}$, $R^{B3}$, $W^C$, and $R^C$ are as described above.

In a more preferable embodiment of the polypeptide represented by formula [I'-A] or a pharmaceutically acceptable salt thereof,

$AA^2$ is a group represented by formula [II-1]:

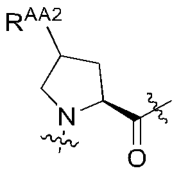
[II-1]

or a group represented by formula [IV-7], [IV-8], [IV-9], [IV-11], or [IV-12]:

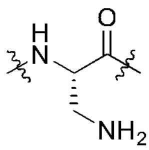
[IV-7],
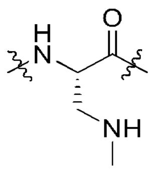
[IV-8],

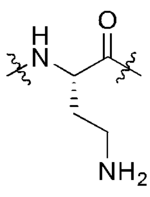
[IV-9],
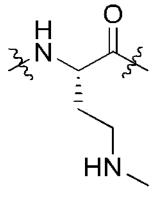
[IV-11],
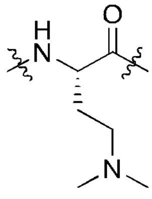
[IV-12]

wherein $R^{AA2}$ is amino,
$AA^3$ is Val, Leu, Ile, Phe, or Trp,
$AA^4$ is (N-Me)Val, (N-Me)Leu, (N-Me)Ile, (N-Me)Asp, or (N-Me)Glu,
$AA^5$ is β-Ala, GABA, Ape, Acp, Pro, (d)-Pro, or β-homoPro,
$L^{1'}$ is β-Ala, GABA, Ape, Acp, or a group represented by formula [IV-23] or [IV-24]:

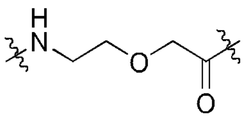
[IV-23],
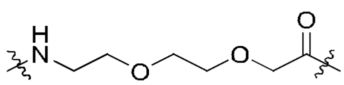
[IV-24];

$L^{1''}$ is a single bond, Asn, (d)-Ser, (d)-Thr, or Glu,
$L^{N1}$ is the formula -C(=O)- or the formula $-S(=O)_2-$,
$L^{N2}$ is the formula -O- or the formula -C(=O)-NH-,
$R^{B1}$, $R^{B2}$, and $R^{B3}$ are each independently a hydrogen atom, carbamoyl, a halogen atom, $C_{1-6}$ alkoxy, or halo $C_{1-6}$

alkoxy,
$W^C$ is a single bond, Arg, (d)-Arg, Lys, or (d)-Lys, and
$R^C$ is the formula -OH or the formula $-NH_2$.

In a further preferable embodiment of the polypeptide represented by formula [I'-A] or a pharmaceutically acceptable salt thereof,

$AA^2$ is a group represented by formula [IV-7] or [IV-9]:

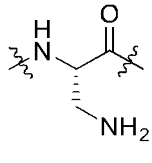 [IV-7], 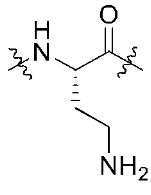 [IV-9]

$AA^3$ is Val, Leu, or Ile,
$AA^4$ is (N-Me)Ile or (N-Me)Glu,
$AA^5$ is β-Ala, GABA, Ape, Acp, or β-homoPro,
$L^{1'}$ is GABA or Ape,
$L^{1''}$ is Asn, Glu, (d)-Ser, or (d)-Thr,
$L^{N1}$ is the formula -C(=O)- or the formula $-S(=O)_2-$,
$L^{N2}$ is the formula -O- or the formula -C(=O)-NH-,
$R^{B1}$ is carbamoyl or fluorine atom,
$R^{B2}$ is a hydrogen atom,
$R^{B3}$ is a hydrogen atom,
$W^C$ is a single bond, and
$R^C$ is the formula $-NH_2$.

One particularly preferable mode of the present embodiment is as follows.
In the polypeptide represented by formula [I'-A] or a pharmaceutically acceptable salt thereof,

$AA^2$ is a group represented by formula [IV-7] or [IV-9]:

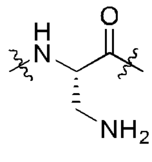 [IV-7], 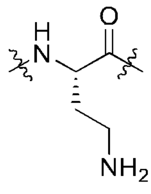 [IV-9]

$AA^3$ is Val,
$AA^3$ may be Leu,
$AA^3$ may be Ile,
$AA^4$ is (N-Me)Ile,
$AA^4$ may be (N-Me)Glu,
$AA^5$ is β-Ala, GABA, Ape, or Acp,
$AA^5$ may be β-homoPro,
$L^{1'}$ is GABA or Ape,
$L^{1''}$ is Asn, Glu, (d)-Ser, or (d)-Thr,
the combination of $L^{1'}$ and $L^{1''}$ is preferably GABA-Asn, Ape-Asn, Ape-Glu, Ape-(d)-Ser, or Ape-(d)-Thr,
$L^{N1}$ is the formula -C(=O)-,
$L^{N1}$ may be the formula $-S(=O)_2-$,
$L^{N2}$ is the formula -O-, and $L^{N2}$ may be the formula -C(=O)-NH-,
$R^{B1}$ is carbamoyl or fluorine atom,
$R^{B2}$ is a hydrogen atom,

$R^{B3}$ is a hydrogen atom,
$W^C$ is a single bond, and
$R^C$ is the formula $-NH_2$.

In another particularly preferable mode of the present embodiment, the substituted polypeptide represented by formula [I'-A] is any of the followings:

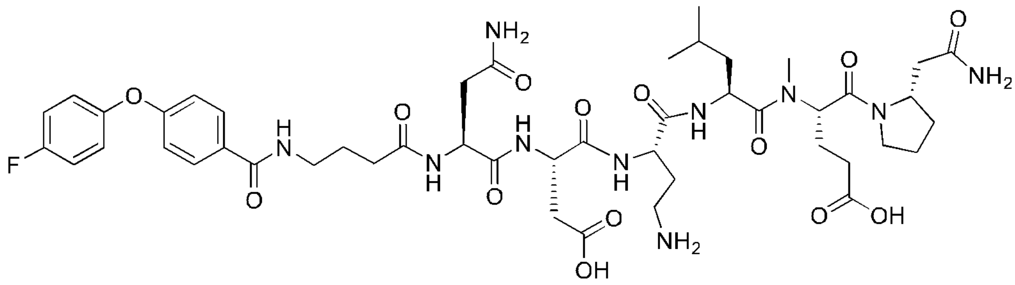

,

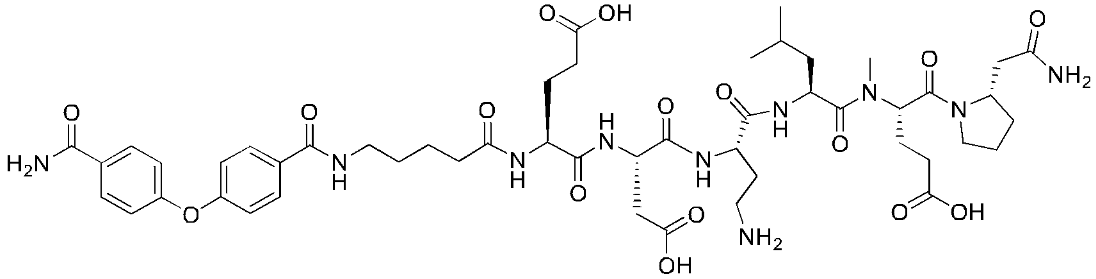

,

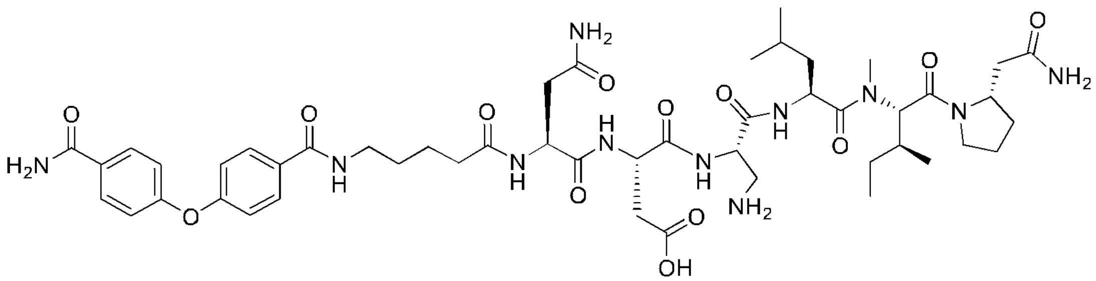

,

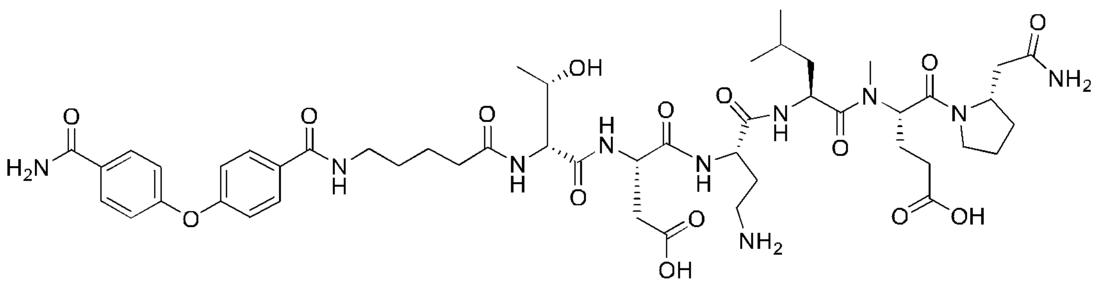

,

and

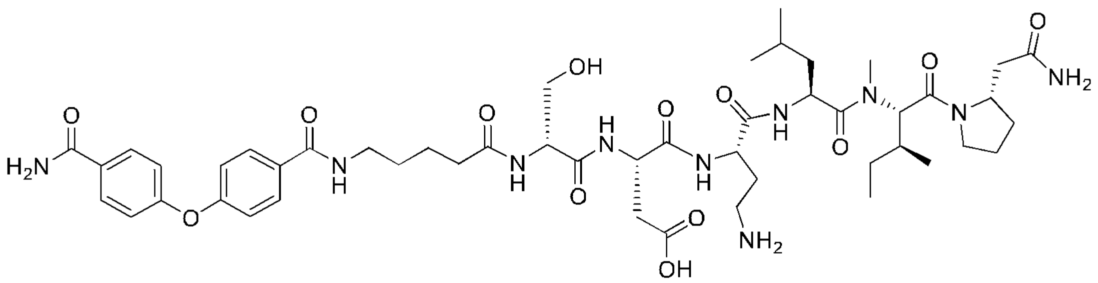

.

Another preferable embodiment of the compounds of the present invention is the substituted polypeptide or a pharmaceutically acceptable salt thereof,

wherein $W^1$ is $-L^1-$, wherein $L^1$ is a single bond,
L2 is a single bond, and
the substituted polypeptide is represented by formula [I'-B]:

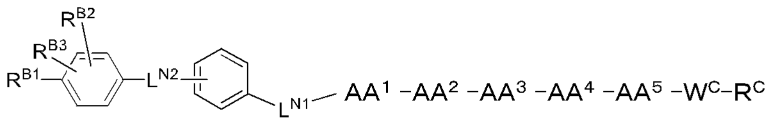
[I'-B],

wherein preferable embodiments of $AA^1$, $AA^2$, $AA^3$, $AA^4$, $AA^5$, $L^{N1}$, $L^{N2}$, $R^{B1}$, $R^{B2}$, $R^{B3}$, $W^C$, and $R^C$ are as described above.

In a more preferable embodiment of the polypeptide represented by formula [I'-B] or a pharmaceutically acceptable salt thereof,

$AA^1$ is β-Asp, β-(d)-Asp, γ-Glu, or γ-(d)-Glu,
$AA^2$ is a group represented by formula [II-1]:

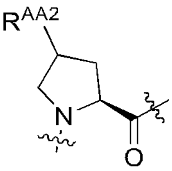
[II-1]

or a group represented by formula [IV-7], [IV-8], [IV-9], [IV-11], or [IV-12]:

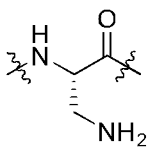
[IV-7],
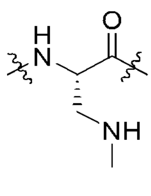
[IV-8],

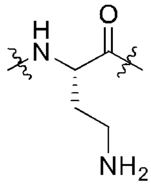
[IV-9],
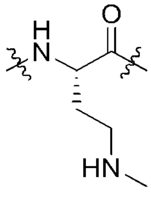
[IV-11],
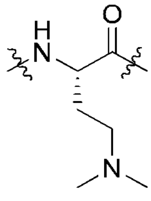
[IV-12]

wherein $R^{AA2}$ is amino,

$AA^3$ is Val, Leu, Ile, Phe, or Trp,
$AA^4$ is (N-Me)Val, (N-Me)Leu, (N-Me)Ile, (N-Me)Asp, or (N-Me)Glu,
$AA^5$ is β-Ala, GABA, Ape, Acp, or β-homoPro,
$L^{N1}$ is the formula -C(=O)- or the formula $-S(=O)_2-$,
$L^{N2}$ is a single bond, the formula -O-, or the formula -C(=O)-NH-,
$R^{B1}$, $R^{B2}$, and $R^{B3}$ are each independently a hydrogen atom, carbamoyl, a halogen atom, $C_{1-6}$ alkoxy, or halo $C_{1-6}$ alkoxy,
$W^C$ is a single bond, Arg, (d)-Arg, Lys, or (d)-Lys, and
$R^C$ is the formula -OH or the formula $-NH_2$.

In a further preferable embodiment of the polypeptide represented by formula [I'-B] or a pharmaceutically acceptable salt thereof,

$AA^1$ is β-(d)-Asp or γ-(d)-Glu,
$AA^2$ is a group represented by formula [II-1]:

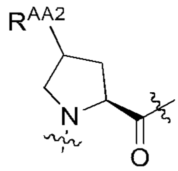
[II-1]

or a group represented by formula [IV-7] or [IV-9]:

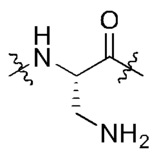
[IV-7],
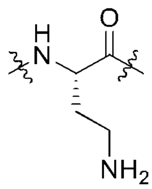
[IV-9]

wherein $R^{AA2}$ is amino,
$AA^3$ is Val, Leu, or Ile,
$AA^4$ is (N-Me)Ile or (N-Me)Glu,
$AA^5$ is β-Ala, GABA, Ape, Acp, or β-homoPro,
$L^{N1}$ is the formula -C(=O)- or the formula $-S(=O)_2-$,
$L^{N2}$ is the formula -O- or the formula -C(=O)-NH-,
$R^{B1}$ is carbamoyl, a chlorine atom, a bromine atom, methoxy, or trifluoromethoxy,
$R^{B2}$ is a hydrogen atom,
$R^{B3}$ is a hydrogen atom,
$W^C$ is a single bond or (d)-Lys, and
$R^C$ is the formula $-NH_2$.

One particularly preferable mode of the present embodiment is as follows.
In the polypeptide represented by formula [I'-B] or a pharmaceutically acceptable salt thereof,

$AA^1$ is β-(d)-Asp,
$AA^1$ may be γ-(d)-Glu,
$AA^2$ is a group represented by formula [II-1]:

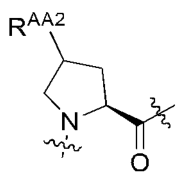
[II-1]

wherein $R^{AA2}$ is amino,
$AA^2$ may be a group represented by formula [IV-7] or [IV-9]:

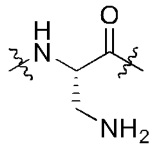 [IV-7], 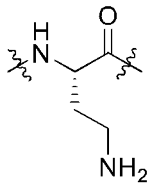 [IV-9]

$AA^3$ is Val,
$AA^3$ may be Leu,
$AA^3$ may be Ile,
$AA^4$ is (N-Me)Ile,
$AA^4$ may be (N-Me)Glu,
$AA^5$ is β-Ala, GABA, Ape, or Acp,
$AA^5$ may be β-homoPro,
$L^{N1}$ is the formula -C(=O)-,
$L^{N1}$ may be the formula $-S(=O)_2-$,
$L^{N2}$ is the formula -O-,
$L^{N2}$ may be the formula -C(=O)-NH-,
$R^{B1}$ is carbamoyl, a chlorine atom, a bromine atom, methoxy, or trifluoromethoxy,
$R^{B2}$ is a hydrogen atom,
$R^{B3}$ is a hydrogen atom,
$W^C$ is a single bond,
$W^C$ may be (d)-Lys, and
$R^C$ is the formula $-NH_2$.

In another particularly preferable mode of the present embodiment, the substituted polypeptide represented by formula [I'-B] is any of the followings:

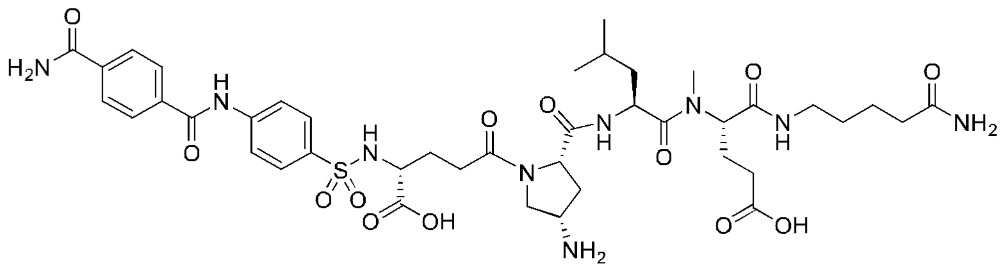

,

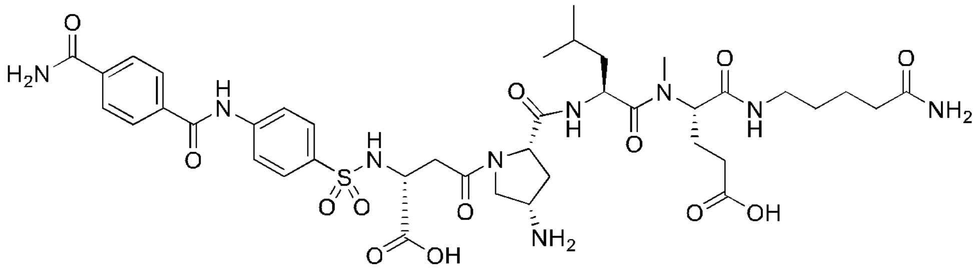

.

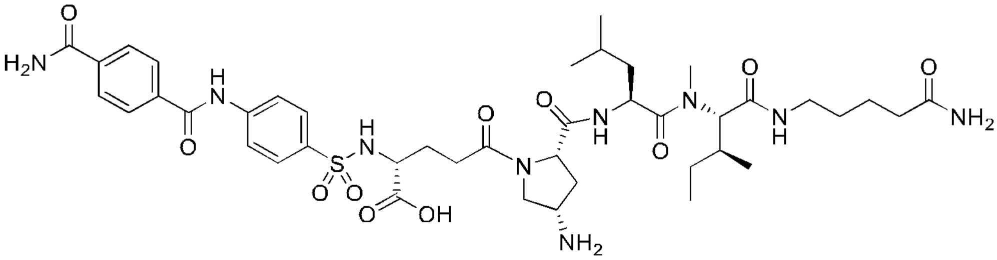

,

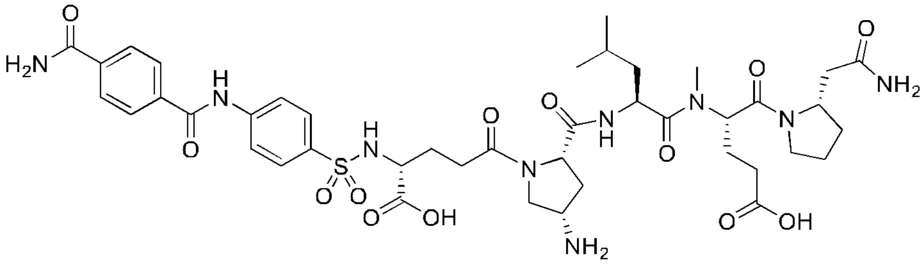

,

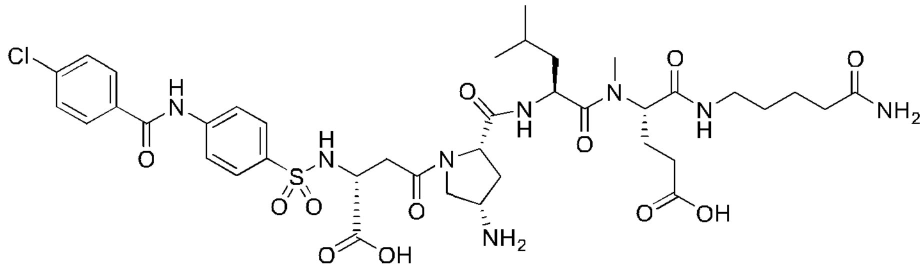

,

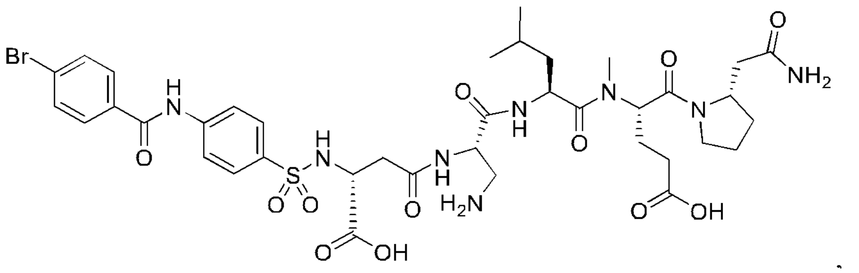

,

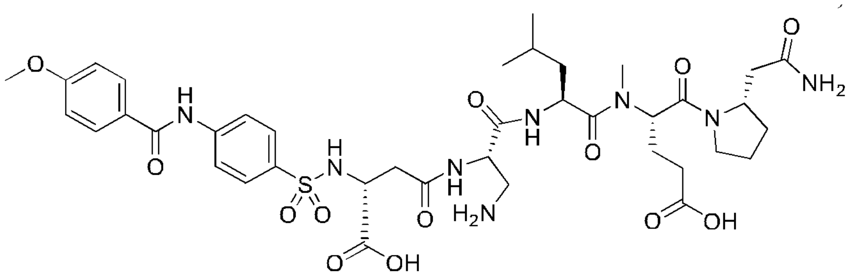

,

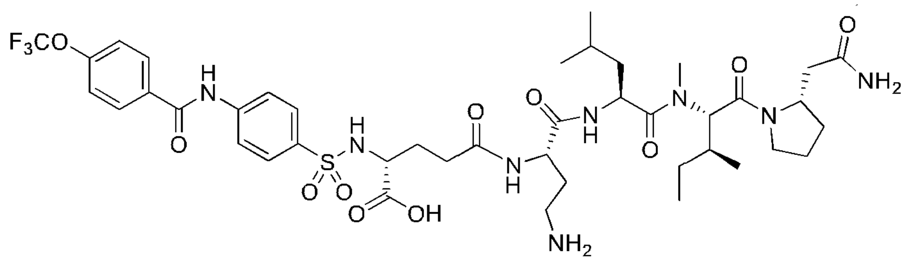

,

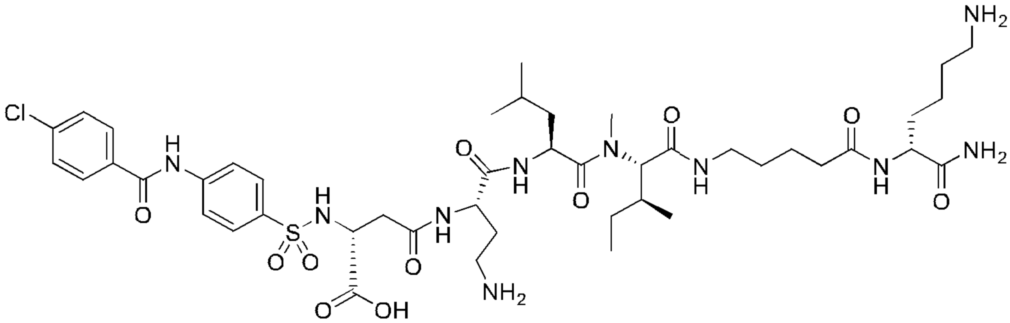

,

and

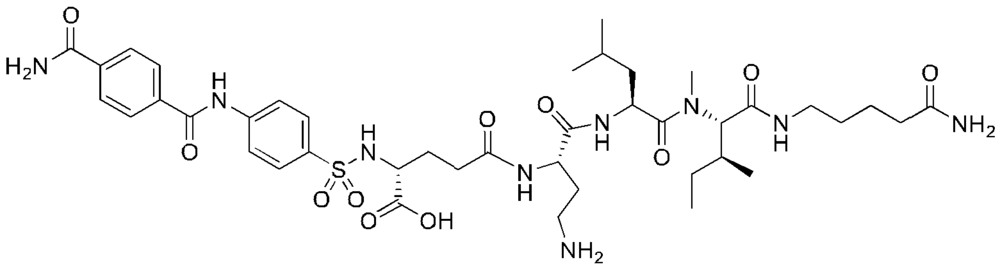

.

Another preferable embodiment of the compounds of the present invention is the substituted polypeptide or a pharmaceutically acceptable salt thereof,

wherein $L^2$ is a single bond, and
the substituted polypeptid is represented by formula [I'-C]:

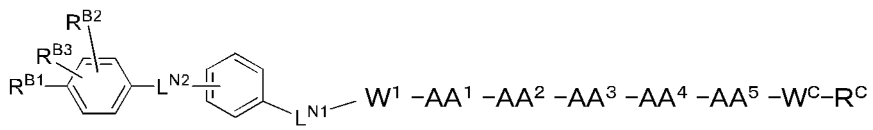

[I'-C],

wherein

preferable embodiments of $AA^1$, $AA^2$, $AA^3$, $AA^4$, $AA^5$, $W^1$, $L^{N1}$, $L^{N2}$, $R^{B1}$, $R^{B2}$, $R^{B3}$, $W^C$, and $R^C$ are as described above.

In a more preferable embodiment of the polypeptide represented by formula [I'-C] or a pharmaceutically acceptable salt thereof,

$AA^1$ is Asp, β-(d)-Asp, or γ-(d)-Glu,
$AA^2$ is a group represented by formula [IV-7] or [IV-9]:

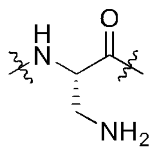 [IV-7], 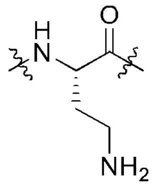 [IV-9]

or a group represented by formula [II-1]:

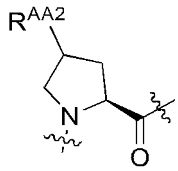 [II-1]

wherein $R^{AA2}$ is amino,
$AA^3$ is Val, Leu, or Ile,
$AA^4$ is (N-Me)Ile or (N-Me)Glu,
$AA^5$ is Ape or β-homoPro,
$W^1$ is $-L^1-$ or $-L^{1'}-L^{1''}-$,
wherein $L^1$ is a single bond,
$L^{1'}$ is GABA or Ape, and
$L^{1''}$ is Asn, (d)-Ser, (d)-Thr, or Glu,
$L^{N1}$ is the formula -C(=O)- or the formula $-S(=O)_2-$,
$L^{N2}$ is the formula -O- or the formula -C(=O)-NH-,
$R^{B1}$, $R^{B2}$, and $R^{B3}$ are each independently a hydrogen atom, carbamoyl, a halogen atom, $C_{1-6}$ alkoxy, or halo $C_{1-6}$ alkoxy,
$W^C$ is a single bond or (d)-Lys, and
$R^C$ is the formula $-NH_2$.

In a further preferable embodiment of the polypeptide represented by formula [I'-C] or a pharmaceutically acceptable salt thereof,

$AA^1$ is Asp,
$AA^1$ may be β-(d)-Asp,
$AA^1$ may be γ-(d)-Glu,
$AA^2$ is a group represented by formula [IV-7] or [IV-9]:

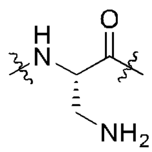 [IV-7], 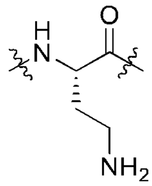 [IV-9]

$AA^2$ may be a group represented by formula [II-1']:

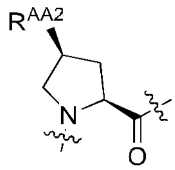 [II-1']

wherein $R^{AA2}$ is amino,

$AA^3$ is Val,
$AA^3$ may be Leu,
$AA^3$ may be Ile,
$AA^4$ is (N-Me)Ile,
$AA^4$ may be (N-Me)Glu,
$AA^5$ is Ape,
$AA^5$ may be β-homoPro,
$W^1$ is $-L^1-$ or $-L^{1'}-L^{1''}-$,
$L^1$ is a single bond,
the combination of $L^{1'}$ and $L^{1''}$ ($-L^{1'}-L^{1''}-$) is GABA-Asn, Ape-Asn, Ape-Glu, Ape-(d)-Ser, or Ape-(d)-Thr,
$L^{N1}$ is the formula -C(=O)-,
$L^{N1}$ may be the formula $-S(=O)_2-$,
$L^{N2}$ is the formula -O-,
$L^{N2}$ may be the formula -C(=O)-NH-,
$R^{B1}$ is carbamoyl, a chlorine atom, a bromine atom, methoxy, or trifluoromethoxy,
$R^{B2}$ is a hydrogen atom,
$R^{B3}$ is a hydrogen atom,
$W^C$ is a single bond,
$W^C$ may be (d)-Lys, and
$R^C$ is the formula $-NH_2$.

In one particularly preferable mode of the present embodiment,
the substituted polypeptide represented by formula [I'-C] is any of the followings:

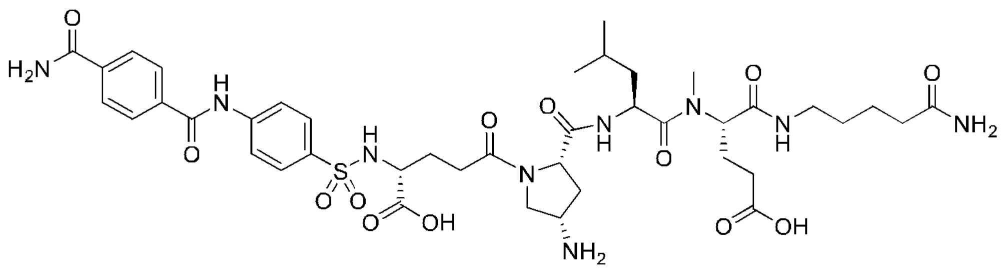

,

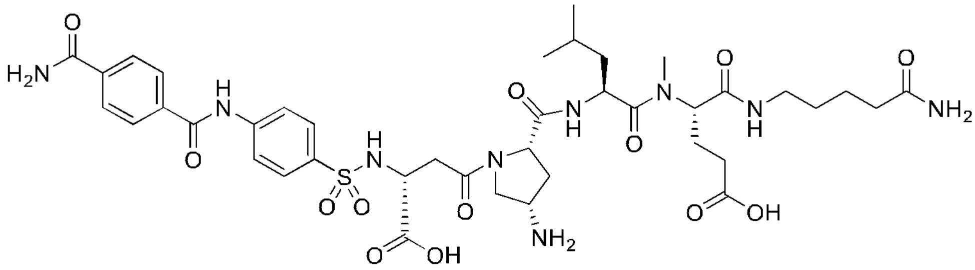

,

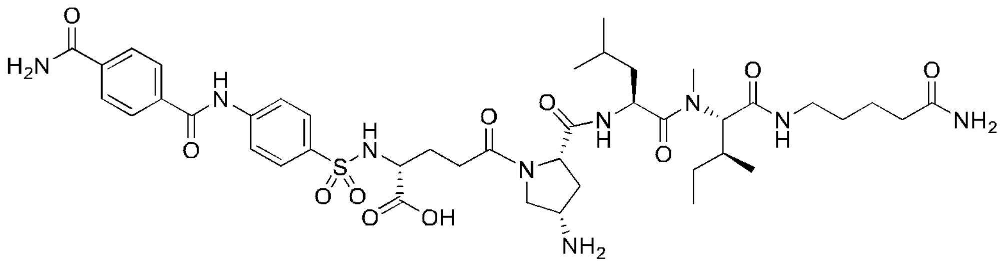

,

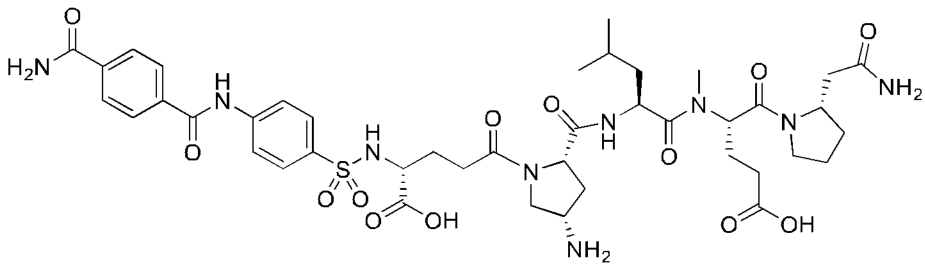

,

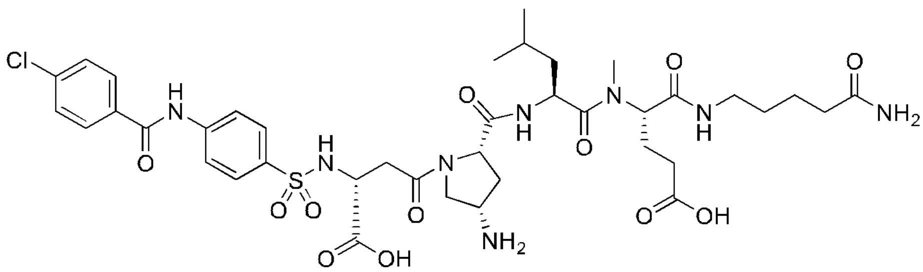

,

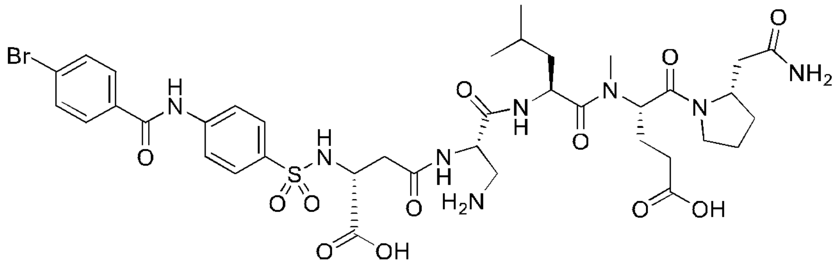

,

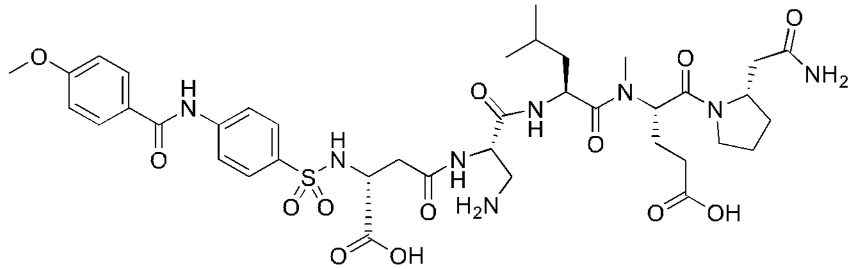

,

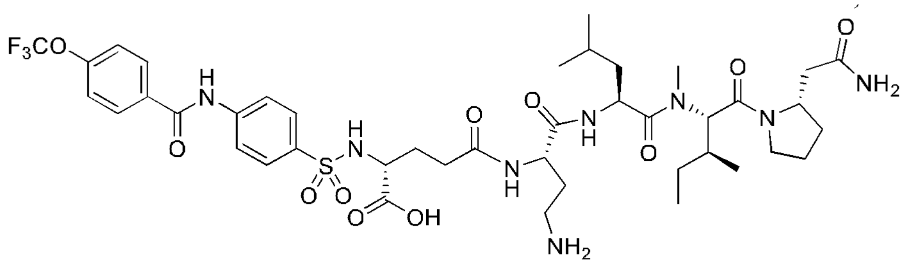

,

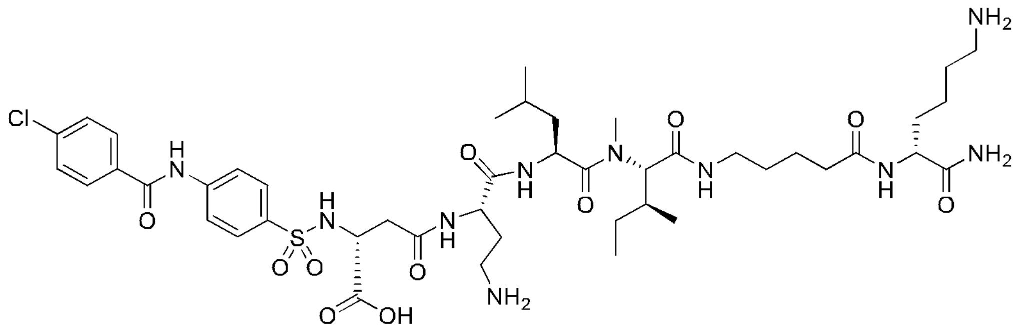

,

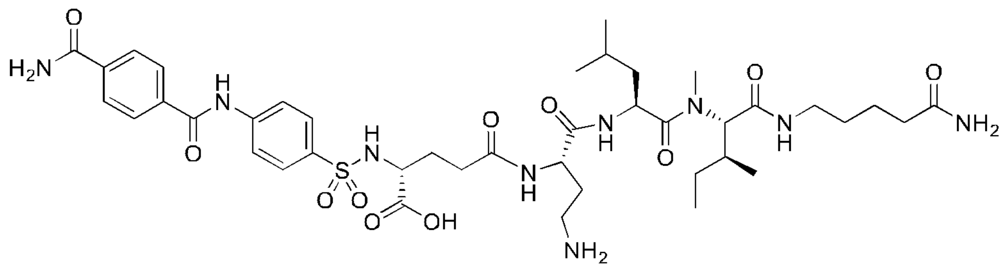

,

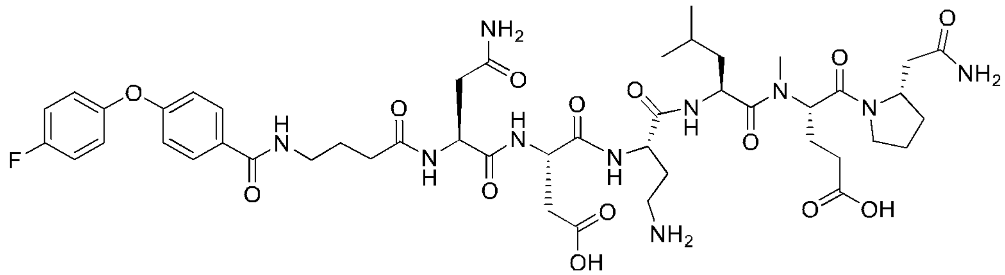

,

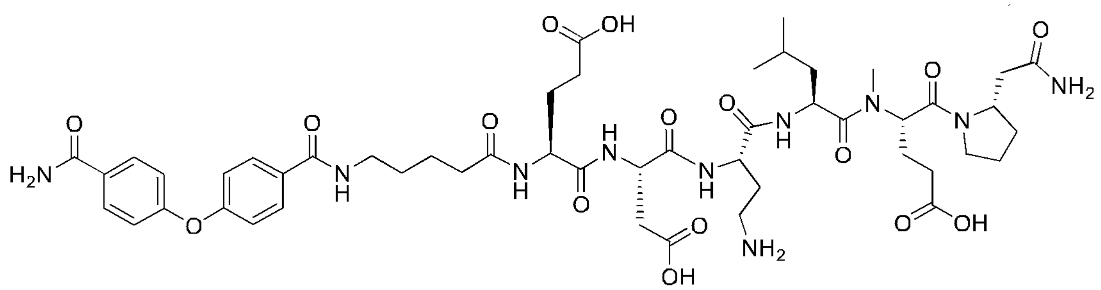

,

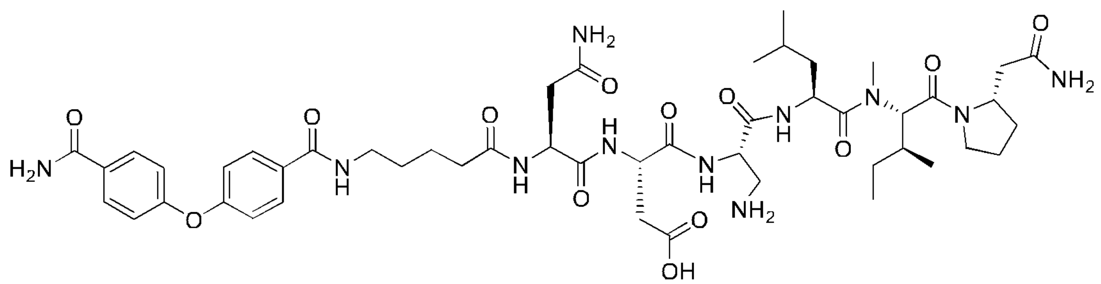

,

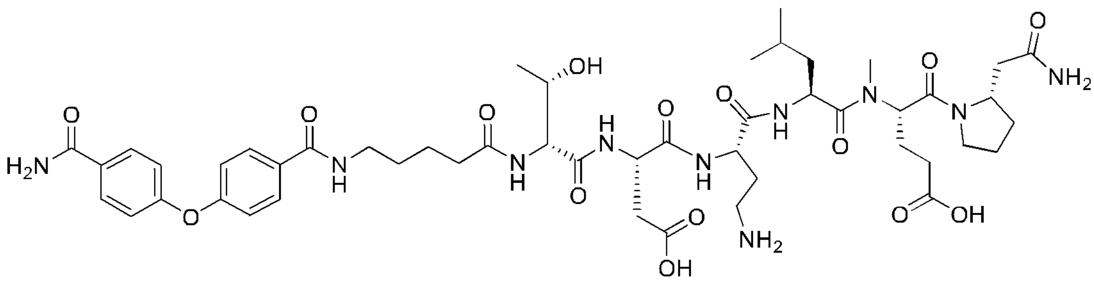

,

and

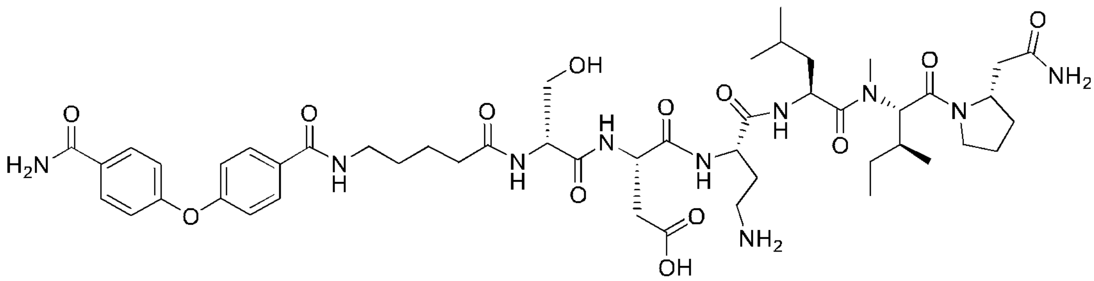

.

In another particularly preferable mode of the present embodiment, the substituted polypeptide represented by formula [I'-C] is the following:

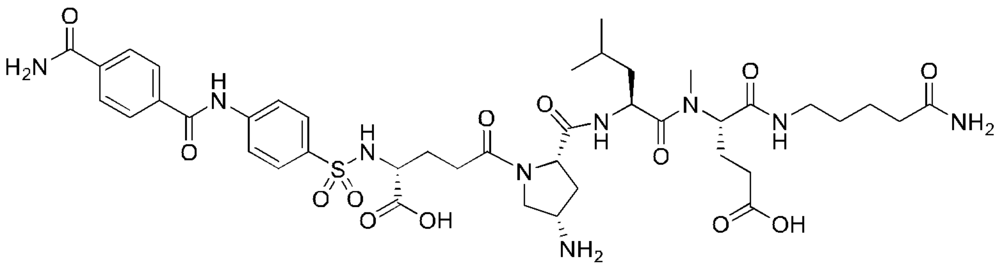

.

In another particularly preferable mode of the present embodiment, the substituted polypeptide represented by formula [I'-C] is the following:

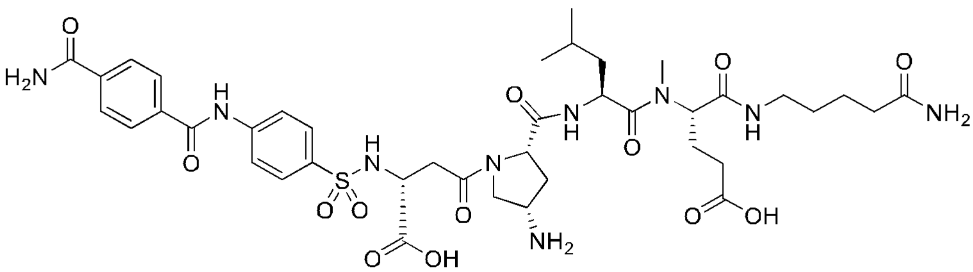

.

In another particularly preferable mode of the present embodiment, the substituted polypeptide represented by formula [I'-C] is the following:

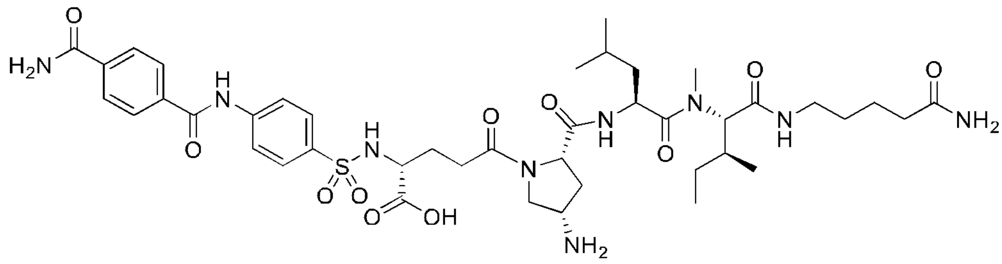

.

In another particularly preferable mode of the present embodiment, the substituted polypeptide represented by formula [I'-C] is the following:

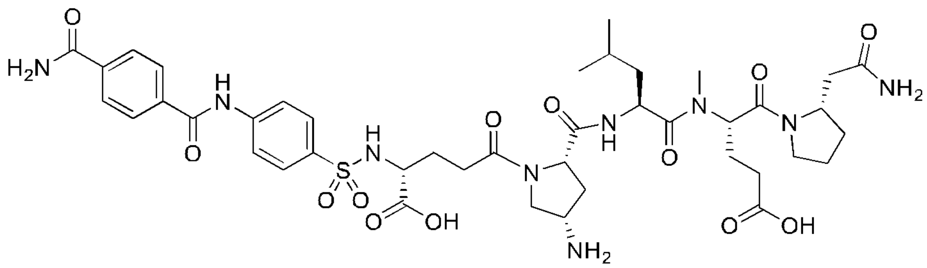

.

In another particularly preferable mode of the present embodiment, the substituted polypeptide represented by formula [I'-C] is the following:

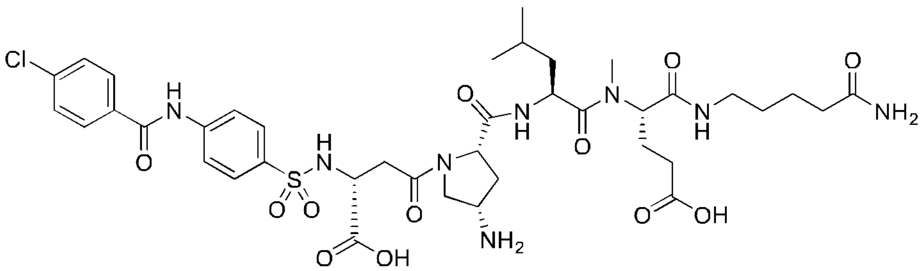

.

In another particularly preferable mode of the present embodiment, the substituted polypeptide represented by formula [I'-C] is the following:

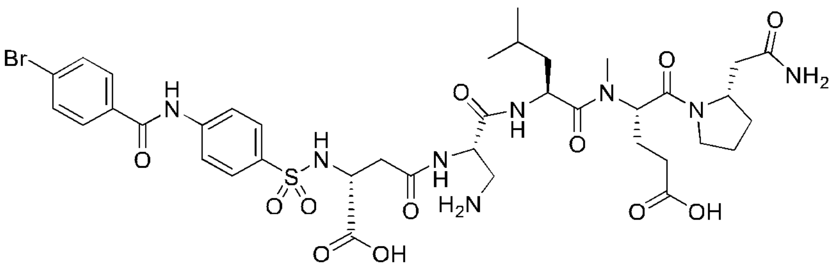

.

In another particularly preferable mode of the present embodiment, the substituted polypeptide represented by formula [I'-C] is the following:

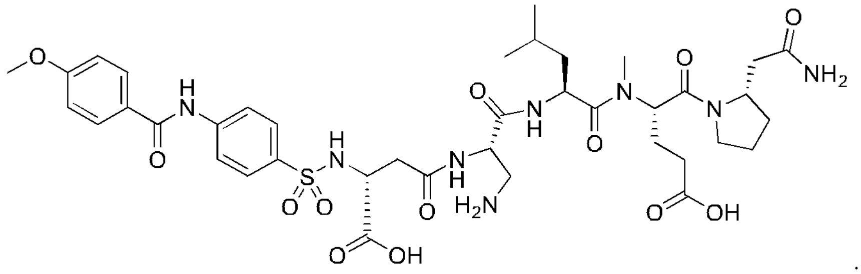

In another particularly preferable mode of the present embodiment, the substituted polypeptide represented by formula [I'-C] is the following:

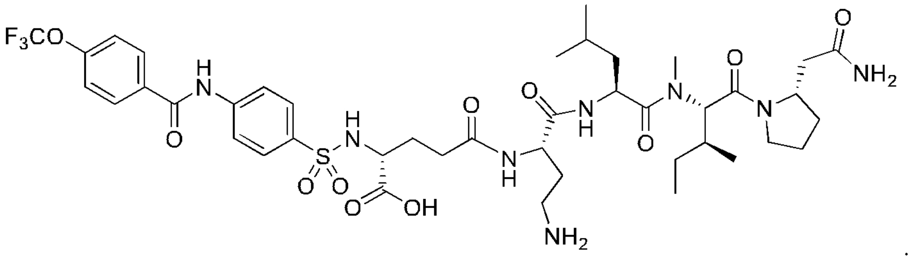

In another particularly preferable mode of the present embodiment, the substituted polypeptide represented by formula [I'-C] is the following:

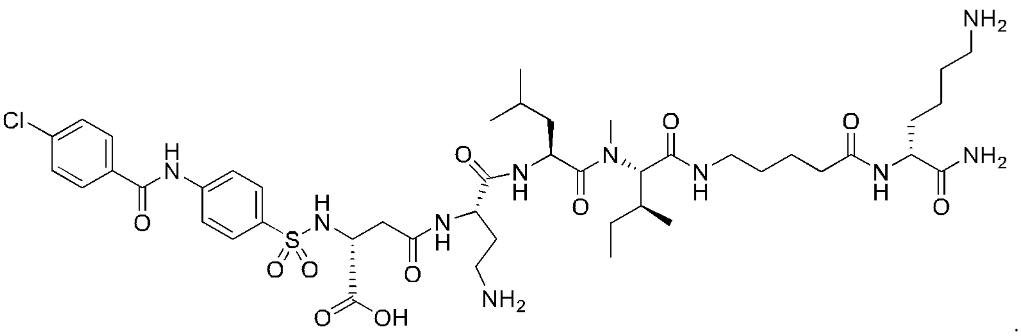

In another particularly preferable mode of the present embodiment, the substituted polypeptide represented by formula [I'-C] is the following:

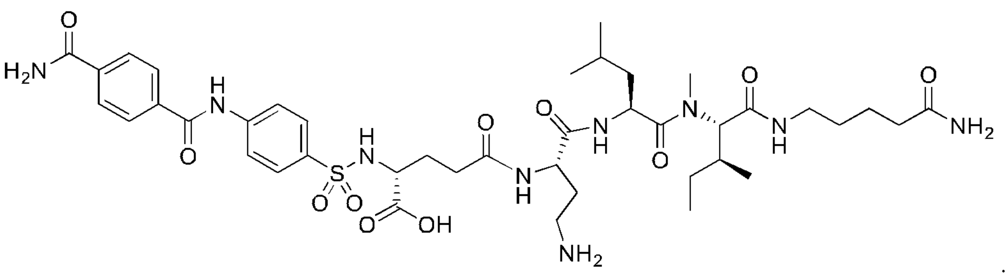

In another particularly preferable mode of the present embodiment, the substituted polypeptide represented by formula [I'-C] is the following:

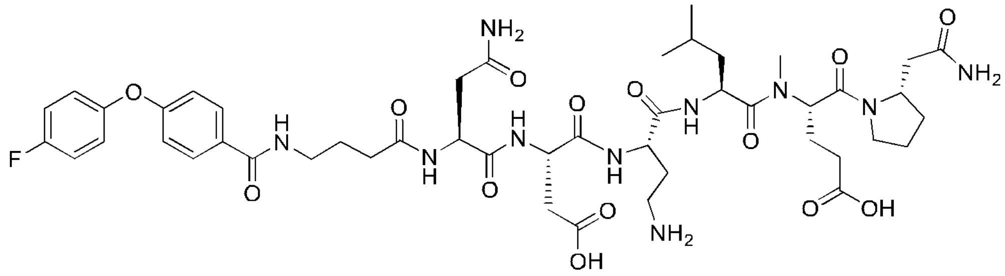

.

In another particularly preferable mode of the present embodiment, the substituted polypeptide represented by formula [I'-C] is the following:

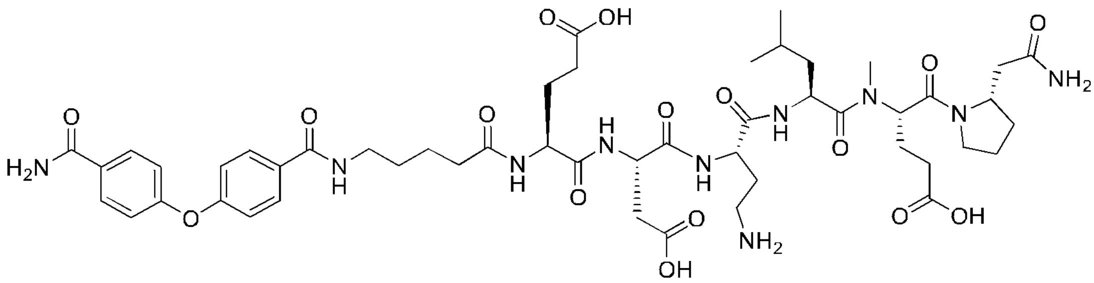

.

In another particularly preferable mode of the present embodiment, the substituted polypeptide represented by formula [I'-C] is the following:

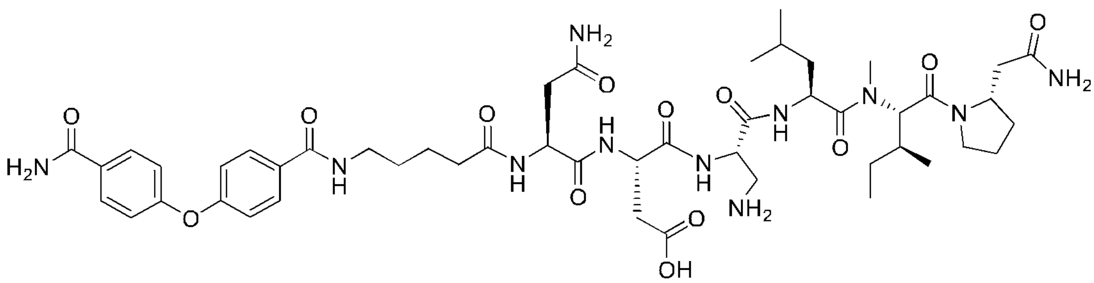

.

In another particularly preferable mode of the present embodiment, the substituted polypeptide represented by formula [I'-C] is the following:

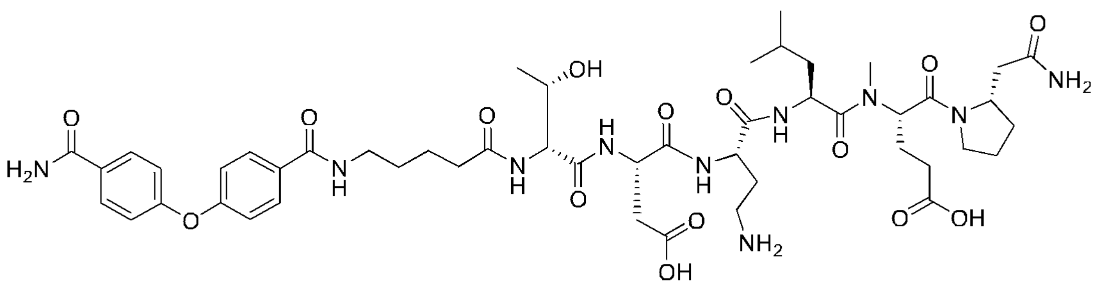

.

In another particularly preferable mode of the present embodiment, the substituted polypeptide represented by formula [I'-C] is the following:

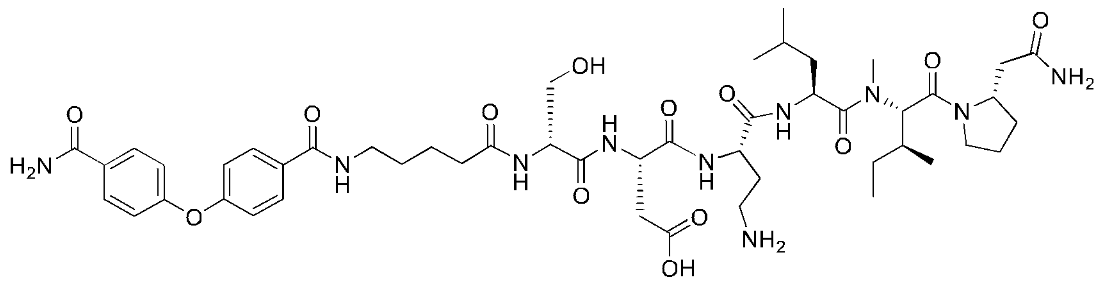
.

Another preferable embodiment of the compounds of the present invention is a substituted polypeptide represented by formula [I], or a pharmaceutically acceptable salt thereof, wherein

$AA^1$ is β-Asp, γ-Glu, or γ-(d)-Glu,
$AA^2$ is a group represented by formula [II-1] or formula [II-2]:

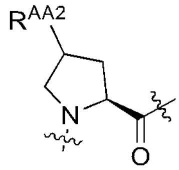 [II-1], 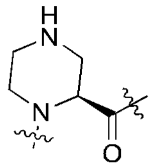 [II-2]

wherein $R^{AA2}$ is hydroxy or amino,
$AA^3$ is Val, Leu, Ile, Phe, or Trp,
$AA^4$ is a single bond, Pro, (N-Me)Ala, (N-Me)Val, (N-Me)Leu, (N-Me)Ile, (N-Me)Phe, (N-Me)Tyr, (N-Me)Ser, (N-Me)Asp, or (N-Me)Glu,
$AA^5$ is a single bond, Pro, (d)-Pro, β-homoPro, Arg, (d)-Arg, Lys, (d)-Lys, β-Ala, GABA, Ape, or Acp,
$L^1$ is a single bond,
$L^2$ is a single bond,
$L^{N1}$ is the formula $-S(=O)_2-$,
$L^{N2}$ is a single bond, the formula -O-, or the formula -C(=O)-NH-,
$R^A$ is a hydrogen atom,
$R^B$ is a hydrogen atom, carbamoyl, a halogen atom, or $C_{1-6}$ alkoxy,
$L^C$ is a single bond, Pro, Arg, (d)-Arg, Lys, (d)-Lys, or (d)-Lys-(d)-Lys, and
$R^C$ is the formula $-NH_2$.

The compounds of the present invention are each a compound having a basic backbone of polypeptide consisting of three to seven amino acids and further having substituted benzoyl or substituted phenylsulfonyl, and may be in the form of a pharmaceutically acceptable salt thereof.

Examples of the pharmaceutically acceptable salt include: mineral acid salts such as hydrochlorides, hydrobromides, hydroiodides, phosphates, sulfates, and nitrates; acid addition salts such as sulfonates including methanesulfonates, ethanesulfonates, benzenesulfonates, p-toluenesulfonates, and trifluoromethanesulfonates, and organic acid salts including oxalates, tartrates, citrates, maleates, succinates, acetates, trifluoroacetates, benzoates, mandelates, ascorbates, lactates, gluconates, and malates; amino acid salts such as glycinates, lysinates, argininates, ornithinates, glutamates, and aspartates; inorganic salts such as lithium salts, sodium salts, potassium salts, calcium salts, and magnesium salts; and salts with organic base such as ammonium salts, triethylamine salts, diisopropylamine salts, and cyclohexylamine salts. Salts include hydrate salts.

Each of the compounds of the present invention may have an asymmetric center, and in this case various optical isomers exist therefor. Thus, each of the compounds of the present invention may exist as an individual optically active substance in any of the (R)-form and the (S)-form, or as a racemate or an (RS) mixture. For a compound having two or more asymmetric centers, diastereomers due to the optical isomerism of them further exist. The scope of the compounds of the present invention also includes mixtures containing all the forms at any ratio. For example, diastereomers can be separated with a method well known to those skilled in the art such as a fractional crystallization method, and optically active substances can be obtained with a technique of organic chemistry well known to those skilled in the art for that purpose. In addition, geometric isomers such as a cis form and a trans form may exist for each of the compounds of the present invention. Moreover, the compounds of the present invention each exhibit tautomerism, and

there exist various tautomers therefor. The scope of the compounds of the present invention also includes those isomers and mixtures containing them at any ratio.

Further, if any of the compounds of the present invention or a salt thereof forms a hydrate or solvate, the hydrate or solvate is also included in the scope of the compounds of the present invention or a salt thereof.

"Matrix metalloprotease 2 (MMP2)" is one of endopeptidases having an active center of zinc.

As described above, MMP2 degrades the extracellular matrix including collagen and gelatin, and hence is involved in cell infiltration and migration, metastasis, and so on, thus being involved in pathological condition of cancerous disease, organ fibrosis, and so on.

Therefore, cancerous disease and organ fibrosis, and symptoms relating to cancerous disease and organ fibrosis can be prevented or treated by inhibiting MMP2.

The compounds of the present invention have an effect to inhibit MMP2. Accordingly, the compounds of the present invention can be used as an MMP2 inhibitor, or an active ingredient of a drug for preventing or treating cancerous disease and organ fibrosis.

In addition, the compounds of the present invention can be used as an active ingredient of a drug for preventing or treating symptoms relating to cancerous disease and organ fibrosis.

Examples of "cancerous disease" include breast cancer, pancreatic cancer, bladder cancer, colorectal cancer, ovarian cancer, prostate cancer, brain tumor, gastric cancer, hepatocellular carcinoma, head and neck cancer, melanoma, uterine cancer, esophageal cancer, renal cell carcinoma, lung cancer, and glioma. Examples of "symptoms relating to cancerous disease" include pain associated with neoplastic cell multiplication or tumor growth, loss of body weight, and paraneoplastic syndrome.

Examples of "organ fibrosis" include chronic kidney disease, interstitial pneumonia, and idiopathic pulmonary fibrosis. Examples of "symptoms relating to organ fibrosis" include proteinuria, kidney dysfunction, and so on in chronic kidney disease, and exertional dyspnea, dry cough, and so on in interstitial pneumonia and idiopathic pulmonary fibrosis.

Evaluation of the effect of any of the compounds of the present invention to inhibit MMP2 can be carried out with a known procedure such as a method described later in Test Examples in the present specification.

For the pharmaceutical according to the present invention, a compound having an effect to inhibit MMP2, being any of the compounds of the present invention contained in the pharmaceutical, or a pharmaceutically acceptable salt thereof may be administered singly, or together with a pharmaceutically acceptable additive.

A common excipient or diluent, and, as necessary, a binder, a disintegrant, a lubricating agent, a coating agent, a sugar-coating agent, a pH adjuster, and a solubilizer or aqueous or nonaqueous solvent which is commonly used can be used as the additive. Specific examples of the additives may include water, lactose, dextrose, fructose, sucrose, sorbitol, mannitol, polyethylene glycol, propylene glycol, starch, cornstarch, gum, gelatin, arginate, calcium silicate, calcium phosphate, cellulose, water syrup, methylcellulose, polyvinylpyrrolidone, alkyl para-hydroxybenzoate, talc, stearic acid, magnesium stearate, agar, pectin, gum arabic, glycerin, sesame oil, olive oil, soybean oil cocoa butter, ethylene glycol, low-viscosity hydroxypropylcellulose (HPC-L), microcrystalline cellulose, carboxymethylcellulose (CMC), carboxymethyl cellulose sodium (CMC-Na), and other common additives.

The pharmaceutical according to the present invention may be in any form of solid compositions, liquid compositions, and other compositions, and an optimum form is selected according to necessity.

The pharmaceutical according to the present invention can be prepared, for example, as a tablet, a pill, a capsule, a granule, a powdered agent, a powder, a solution, an emulsion, a suspension, or an injection through a common formulation technique with addition of the aforementioned additive to any of the compounds of the present invention.

The pharmaceutical according to the present invention can be formulated by forming a clathrate compound with any of the compounds of the present invention and $\alpha$-, $\beta$-, or $\gamma$-cyclodextrin or methylated cyclodextrin.

With respect to compounds that can be used in combination with any of the compounds of the present invention, the pharmaceutical according to the present invention can be prepared as a single formulation (combination drug) or two or more formulations (concomitant drugs) obtained by separately formulating.

If those compounds are separately formulated into two or more formulations, the individual formulations can be administered simultaneously, or separately at specific time intervals. In the latter case, any formulation may be first administered. The two or more formulations may be administered in different numbers of doses per day. The two or more formulations may be administered through different routes.

If those compounds are separately formulated into two or more formulations, they may be administered simultaneously, or separately at very short intervals, and it is preferable to instruct to use them in combination by means of a document such as an accompanying document or sales brochure of a commercially available pharmaceutical.

It is also preferable to separately formulate those active ingredients into the form of a kit consisting of two formulations.

The form of administration when any of the compounds of the present invention is used as an MMP2 inhibitor or the like is not limited, and any of the compounds of the present invention can be directly administered orally or parenterally. Alternatively, an agent containing any of the compounds of the present invention as an active ingredient

may be orally or parenterally administered.

Also in the case that any of the compounds of the present invention is used as an agent for preventing or treating cancerous disease and organ fibrosis, and symptoms relating to cancerous disease and organ fibrosis, any of the compounds of the present invention can be directly administered orally or parenterally. Alternatively, an agent containing any of the compounds of the present invention as an active ingredient may be orally or parenterally administered.

In the parenteral administration, it is preferable to perform intravenous administration, subcutaneous administration, or transdermal administration.

Examples of the dosage form for parenteral administration include an injection, an infusion drip, an implant drug, a transdermal drug, a transmucosal drug, and a patch, and the dosage form may be a microsphere formulation.

The dose of any of the compounds of the present invention differs among subjects, routes of administration, target diseases, symptoms, and so on, and it is typically desirable in oral administration or parenteral administration to an adult patient to administer a single dose of 0.1 mg to 1000 mg, preferably of 1 mg to 200 mg, once to three times per day, or once every 2 to 3 days.

Production Examples for formulations of the compounds of the present invention will be shown in the following.

Production Example 1

A granule containing the following components is produced.

Components: compound represented by general formula [I'], lactose, and cornstarch, HPC-L.

The compound represented by general formula [I'] and lactose are sieved. Cornstarch is sieved. These are mixed by using a mixer. HPC-L aqueous solution is added to the mixed powder, and the resultant is kneaded, granulated (extrusion granulation), and then dried. The resulting dry granule is sieved through a vibrating screen to obtain a granule.

Production Example 2

A powder for encapsulation containing the following components is produced.

Components: compound represented by general formula [I'], lactose, and cornstarch, magnesium stearate.

The compound represented by general formula [I'] and lactose are sieved. Cornstarch is sieved. These together with magnesium stearate are mixed by using a mixer to obtain a powder. The powder obtained can be encapsulated.

Production Example 3

A granule for encapsulation containing the following components is produced.

Components: compound represented by general formula [I'], lactose, and cornstarch, HPC-L.

The compound represented by general formula [I'] and lactose are sieved. Cornstarch is sieved. These are mixed by using a mixer. HPC-L aqueous solution is added to the mixed powder, and the resultant is kneaded, granulated, and then dried. The resulting dry granule is sieved through a vibrating screen to regulate the particle seize, and a granule is obtaened. The granule obtained can be encapsulated.

Production Example 4

Tablets containing the following components are produced.

Components: compound represented by general formula [I'], lactose, microcrystalline cellulose, magnesium stearate, and CMC-Na.

The compound represented by general formula [I'], lactose, microcrystalline cellulose, and CMC-Na are sieved, and mixed together. Magnesium stearate is added to the mixed powder to obtain a mixed powder for formulation. This mixed powder is subjected to direct compression to obtain tablets.

Production Example 5

An injection containing the following compositions is produced.

Components: compound represented by general formula [I'], purified egg yolk lecithin, oleic acid, purified soybean oil, glycerin, and water for injection.

The compound represented by general formula [I'], purified egg yolk lecithin, and oleic acid are added to purified soybean oil to dissolve therein; water for injection, mixed with glycerin, is then added; and the resultant is emulsified by using an emulsifying machine. Thereto, water for injection is added, and the resultant is aliquoted into ampules, which are sealed and sterilized to form an injection.

Production methods for compound [I'] according to the present invention will be described in detail in the following, but the production is not limited to the exemplified production methods. Solvent to be used for the production may be any solvent that does not inhibit individual reactions, and is not limited by the following description.

In the production of compound [I'], the order of steps in the production can be appropriately changed.

In the production, each raw material compound may be used as a salt thereof, and examples of such salts include "pharmaceutically acceptable salt" presented above.

Compound [I'] can be produced through solid-phase synthesis, liquid-phase synthesis, or a combination of them. Production examples with solid-phase synthesis will be shown in the following production methods.

Compound [I'] can be produced through methods shown in any of Schemes 1 to 9 or any combination of them.

The side chain of each amino acid to form the polypeptide moiety of compound [I'] may have a functional group, and the functional group may be protected with a protecting group. Examples of the protecting group include tBu for carboxy, Trt for carbamoyl, tBu for hydroxy, tBu for phenolic hydroxy, tBu for thiol (sulfanyl), Boc for amino, Trt for imidazolyl, Boc for indolyl, and Pbf for guanidino. These protecting groups can be each subjected to deprotection by treating with an acid.

Each of the amino acids can be used for the production even if the functional group in the side chain is not protected.

Method A: a method for producing compound [1-f], a compound in which the amino of the N-terminal amino acid of the polypeptide moiety is sulfonylated and the carboxy of the C-terminal amino acid is amidated ($-CONH_2$)

Compound [1-f] can be produced, for example, through solid-phase synthesis the summary of which is described in the following (Scheme 1).

Scheme 1

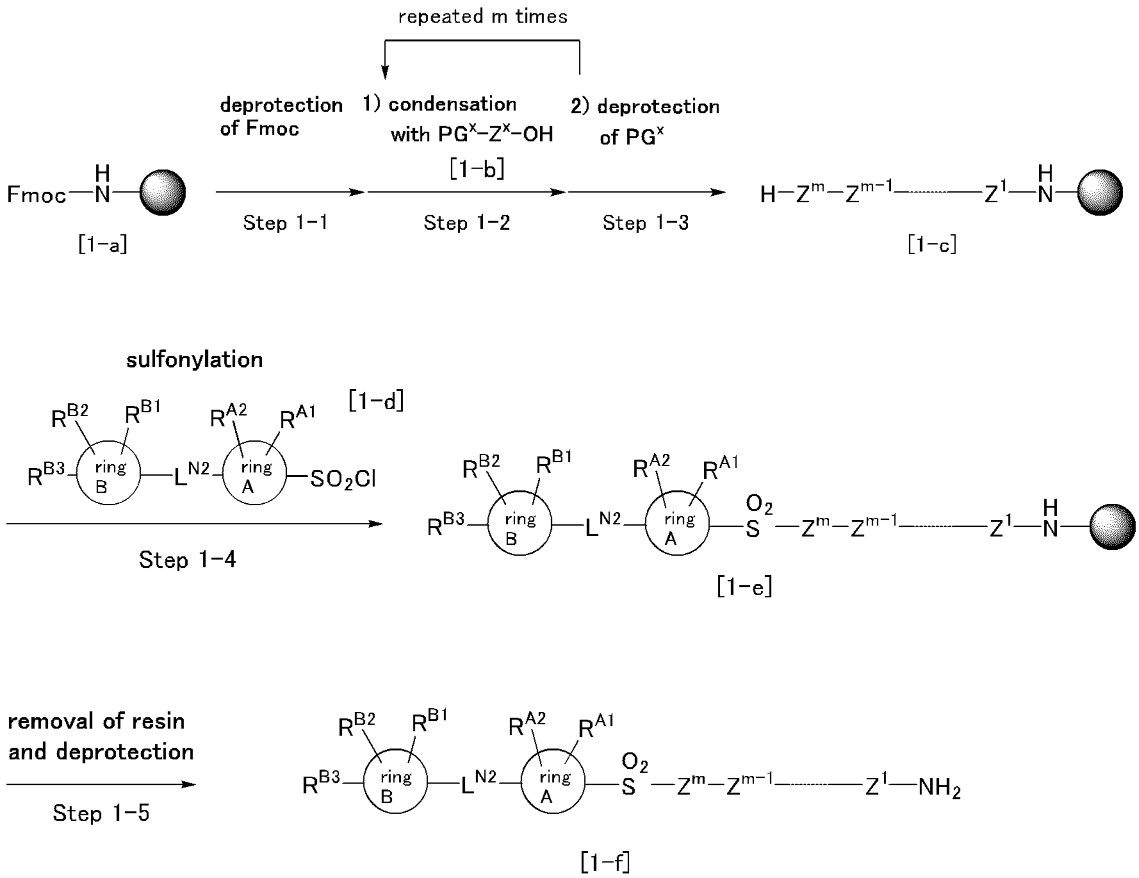


wherein

$R^{A1}$, $R^{A2}$, $R^{B1}$, $R^{B2}$, $R^{B3}$, $L^{N2}$, ring A, and ring B have the same definitions as those described above,
$H-Z^x-OH$ represents an amino acid,

x represents an integer of 1 to m,
m represents an integer of 2 to 15,
wherein $Z^x$ corresponds to $AA^x$, $L^2$, $W^1$, or $W^c$ in the compound represented by formula [I'], and may represent a single bond, and
$PG^x$ represents a liposoluble protecting group protecting the amino of the amino acid (H-$Z^x$-OH).

(1) [Step 1-1]

A resin having amino protected with Fmoc (compound [1-a]) is subjected to deFmoc by using a base.

(2) [Step 1-2]

The resin obtained in (1) is reacted with an amino acid whose amino has been protected with a liposoluble protecting group ($PG^x$) (compound [1-b]) for amidation reaction between the free amino in the resin and the carboxy in compound [1-b].

(3) [Step 1-3]

The liposoluble protecting group of the amino of the N-terminal amino acid of the polypeptide moiety in the compound obtained in (2) is subjected to deprotection.
(4) By repeating steps (2) and (3) twice or more times, compound [1-c] can be produced in which the carboxy of the C-terminal amino acid of the polypeptide moiety is bonding to the resin and the amino of the N-terminal amino acid is free.

(5) [Step 1-4]

Compound [1-e] can be produced by reacting compound [1-c] obtained in (4) with compound [1-d] to sulfonylate the free amino in compound [1-c].

(6) [Step 1-5]

Compound [1-f], one of the present inventive compounds, can be produced by using an acid on compound [1-e] obtained in (5) to cleave the bond to the resin. Examples of the acid applicable in the present step may include TFA.
At that time, if a protected functional group is present in the side chain of an amino acid forming the polypeptide moiety, the protecting group of the functional group can be simultaneously deprotected.
Compound [1-f] obtained in the described manner can be isolate/purified by a known separation/purification means such as concentration, concentration under reduced pressure, reprecipitation, solvent extraction, crystallization, and chromatography.
In the method described in Scheme 1, resin [1-a], compound [1-b], and compound [1-d] to be used as raw material compounds can be obtained by producing with a method known per se, or purchasing commercially available products of them.

Method B: a method for producing compound [2-c], a compound in which the amino of the N-terminal amino acid of the polypeptide moiety is acylated and the carboxy of the C-terminal amino acid is amidated ($-CONH_2$)

Compound [2-c] can be produced, for example, through solid-phase synthesis the summary of which is described in the following (Scheme 2) with use of compound [1-c] as a starting material.

Scheme 2

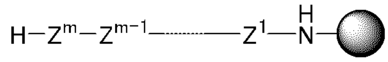

[1-c]

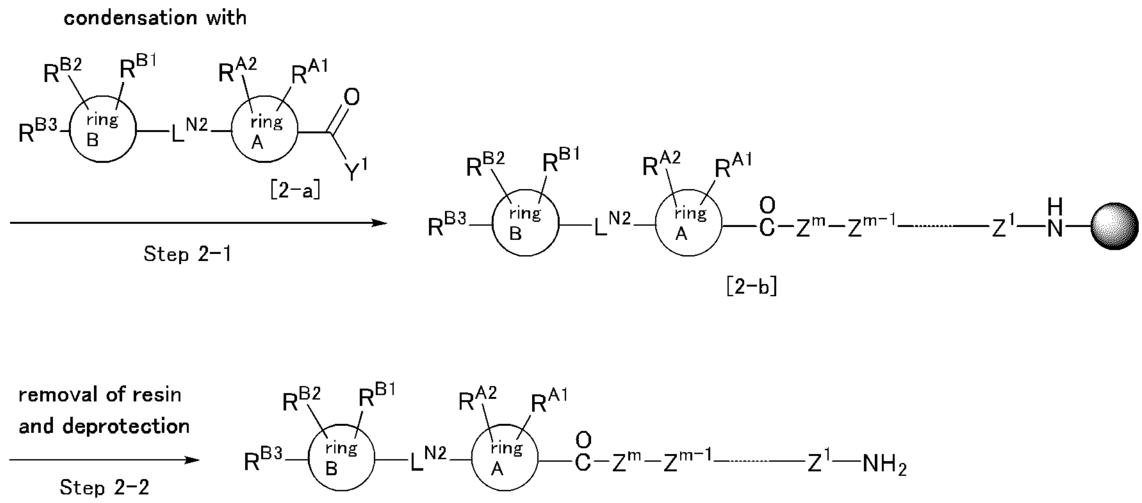


[2-a]

[2-b]

[2-c]

wherein

$R^{A1}$, $R^{A2}$, $R^{B1}$, $R^{B2}$, $R^{B3}$, $L^{N2}$, ring A, ring B, H-$Z^x$-OH, x, m, and $Z^x$ have the same definitions as those described above, and
$Y^1$ represents hydroxy or a chlorine atom.

(1) [Step 2-1]

Compound [2-b] can be produced by allowing compound [2-a] to act on compound [1-c] for amidation reaction.

(2) [Step 2-2]

Compound [2-c], one of the present inventive compounds, can be produced by using an acid on compound [2-b] obtained in (1) to cleave the bond to the resin. Examples of the acid applicable in the present step may include TFA.
At that time, if a protected functional group is present in the side chain of an amino acid forming the polypeptide moiety, the protecting group of the functional group can be simultaneously deprotected.
Compound [2-c] obtained in the described manner can be isolate/purified by a known separation/purification means such as concentration, concentration under reduced pressure, reprecipitation, solvent extraction, crystallization, and chromatography.
In the method described in Scheme 2, compound [2-a] to be used as a raw material compound can be obtained by producing with a method known per se, or purchasing a commercially available product of it. Compound [1-c] can be produced with the method described in Scheme 1.

Method C-1: a method for producing compound [3-h], a compound in which the amino of the N-terminal amino acid of the polypeptide moiety is sulfonylated or acylated and the carboxy of the C-terminal amino acid is secondary- or tertiary-amidated

Compound [3-h] can be produced, for example, through solid-phase synthesis the summary of which is described in the following (Scheme 3).
Examples of the resin (resin [3-a]) applicable in the present production method may include chlorotrityl chloride resin and Wang resin.

Scheme 3

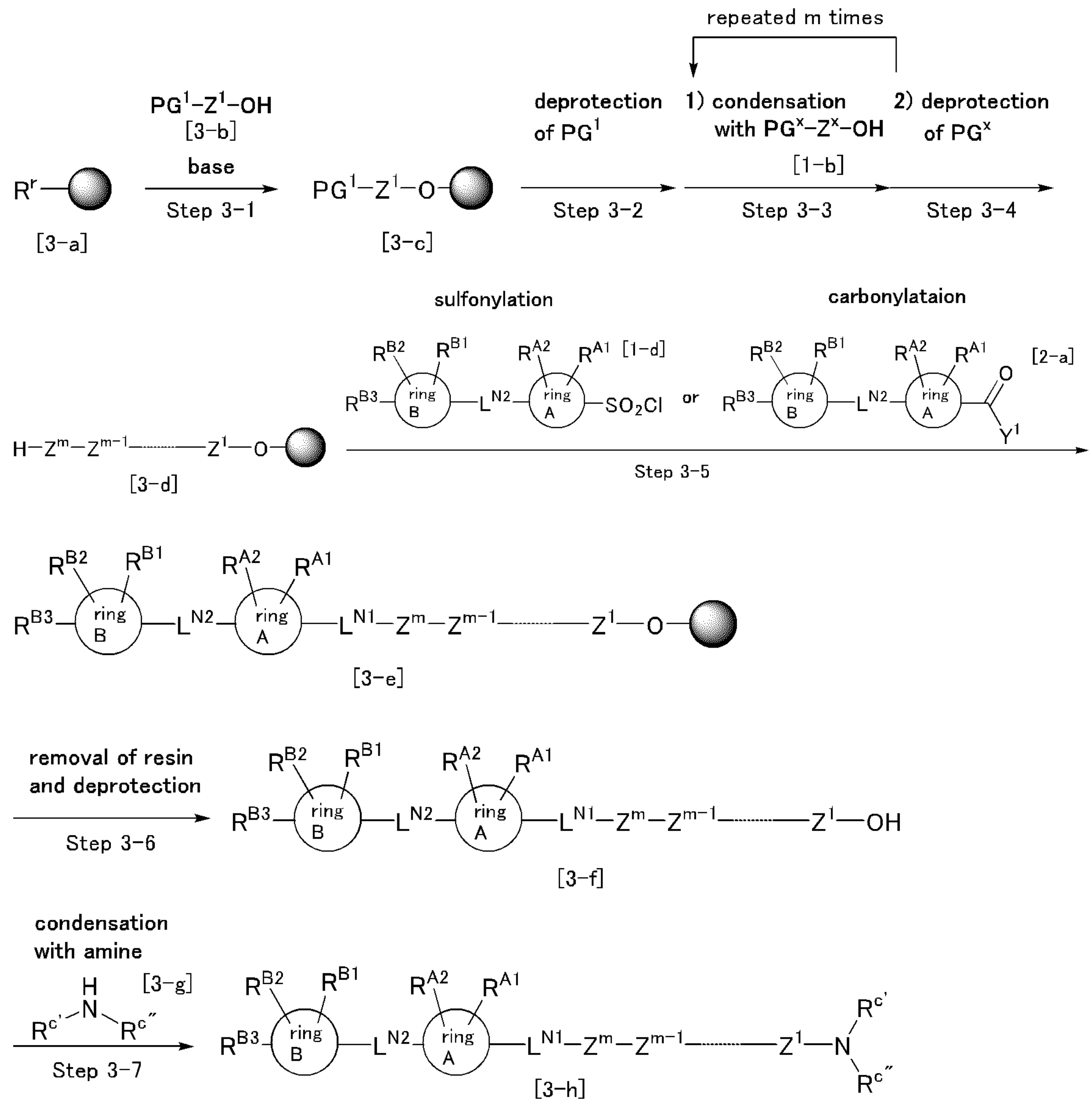


wherein

$R^{A1}$, $R^{A2}$, $R^{B1}$, $R^{B2}$, $R^{B3}$, $L^{N1}$, $L^{N2}$, ring A, ring B, H-$Z^x$-OH, x, m, $Z^x$, $PG^x$, and $Y^1$ have the same definitions as those described above,
$R^r$ represents hydroxy or a chlorine atom,
$R^{c'}$ and $R^{c''}$ are same or different and each represent a hydrogen atom or $C_{1-6}$ alkyl, wherein the $C_{1-6}$ alkyl is optionally substituted with one group selected from the group consisting of hydroxy, amino, and $C_{1-6}$ alkoxy,
$R^{c'}$ and $R^{c''}$ may be taken together with the neighboring nitrogen atom to form a four- to seven-membered saturated heterocycle containing a nitrogen atom, wherein
the four- to seven-membered saturated heterocycle containing a nitrogen atom, formed by $R^{c'}$ and $R^{c''}$ taken together with the neighboring nitrogen atom, is optionally substituted with one group selected from the group consisting of hydroxy, amino, $C_{1-6}$ alkyl, and $C_{1-6}$ alkoxy, and
two carbon atoms in the four- to seven-membered saturated heterocycle containing a nitrogen atom, formed by $R^{c'}$ and $R^{c''}$ taken together with the neighboring nitrogen atom, are optionally crosslinked with $C_{1-4}$ alkanediyl.

(1) [Step 3-1]

Compound [3-c] can be produced by reacting the resin (compound [3-a]) with an amino acid whose amino has been protected with a liposoluble protecting group ($PG^1$) (compound [3-b]).

(2) [Step 3-2]

The liposoluble protecting group ($PG^1$) in compound [3-c] obtained in (1) is subjected to deprotection.

(3) [Step 3-3]

The compound obtained in (2) is reacted with an amino acid whose amino has been protected with a liposoluble protecting group ($PG^X$) (compound [1-b]) for amidation reaction between the free amino in the compound obtained in (1) and the carboxy in compound [1-b].

(4) [Step 3-4]

The liposoluble protecting group of the amino of the N-terminal amino acid of the polypeptide moiety in the compound obtained in (3) is subjected to deprotection.
(5) By repeating steps (3) and (4) twice or more times, compound [3-d] can be produced in which the carboxy of the C-terminal amino acid of the polypeptide moiety is bonding to the resin and the amino of the N-terminal amino acid is free.

(6) [Step 3-5]

Compound [3-e] can be produced by reacting compound [3-d] obtained in (5) with compound [1-d] or compound [2-a] to sulfonylate or acylate the free amino in compound [3-d].

(7) [Step 3-6]

Compound [3-f], in which the carboxy of the C-terminal amino acid of the polypeptide moiety is free, can be produced by using an acid on compound [3-e] obtained in (6) to cleave the bond to the resin. Examples of the acid applicable in the present step may include TFA.
At that time, if a protected functional group is present in the side chain of an amino acid forming the polypeptide moiety, the protecting group of the functional group can be simultaneously deprotected.

(8) [Step 3-7]

Compound [3-h], one of the present inventive compounds, can be produced by allowing compound [3-g] to act on compound [3-f] obtained in (7) for amidation reaction.
Compound [3-h] obtained in the described manner can be isolate/purified by a known separation/purification means such as concentration, concentration under reduced pressure, reprecipitation, solvent extraction, crystallization, and chromatography.
In the method described in Scheme 3, compound [3-a], compound [3-b], compound [1-b], compound [1-d], compound [2-a], and [3-g] to be used as raw material compounds can be obtained by producing with a method known per se, or purchasing commercially available products of them.

Method C-2: a method for producing compound [4-e], a compound in which the amino of the N-terminal amino acid of the polypeptide moiety is sulfonylated or acylated and the carboxy of the C-terminal amino acid is secondary- or tertiary-amidated

Alternatively, compound [4-e] can be produced, for example, through solid-phase synthesis the summary of which is described in the following (Scheme 4).
In Scheme 4, a method for producing a compound wherein a functional group is present in the side chain of the m'th amino acid counted from the C-terminal amino acid of the polypeptide moiety in compound [4-e] is presented as an example.
Examples of the resin applicable in the present production method may include, as in Scheme 3, chlorotrityl chloride resin and Wang resin.

Scheme 4

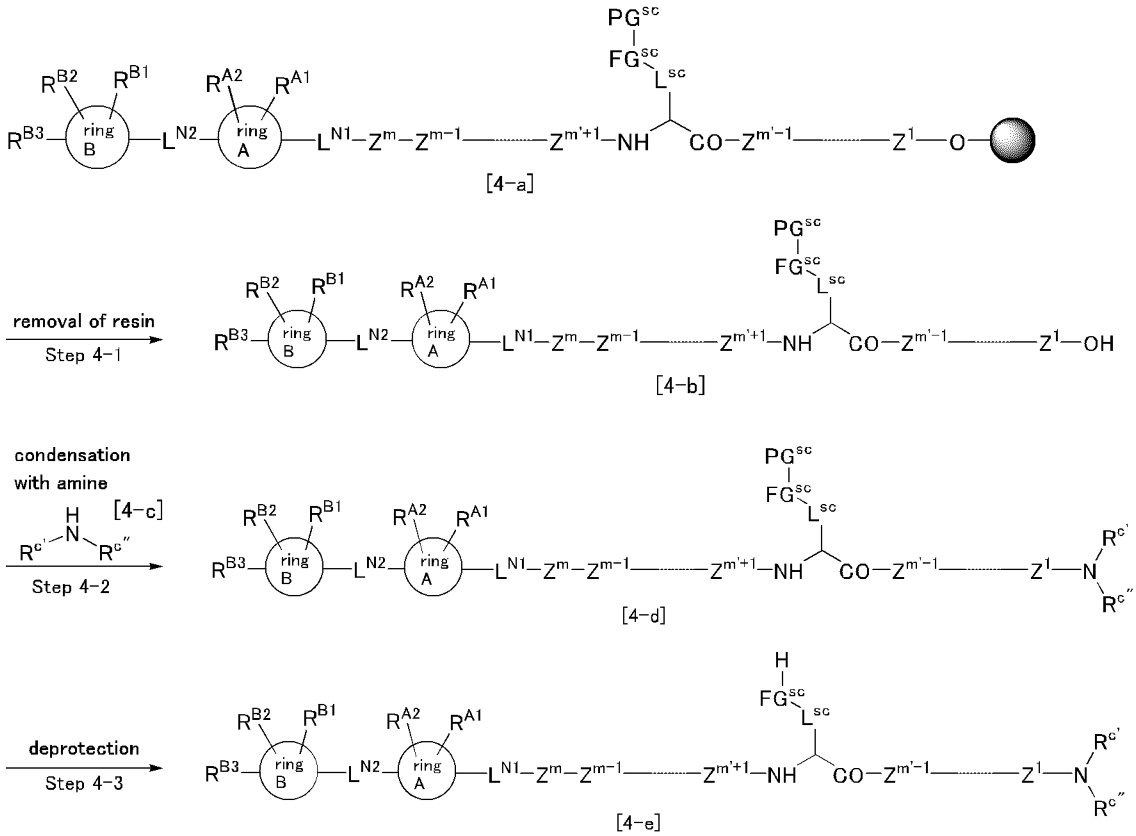


wherein

$R^{A1}$, $R^{A2}$, $R^{B1}$, $R^{B2}$, $R^{B3}$, $L^{N1}$, $L^{N2}$, ring A, ring B, H-$Z^x$-OH, x, m, $Z^x$, $R^{c'}$, and $R^{c''}$ have the same definitions as those described above,
m' represents an integer of 1 to 15, provided that the relationship "m' ≤ m" is satisfied,
$L^{sc}$ represents $C_{1-4}$ alkanediyl,
$FG^{sc}$ represents the aforementioned functional group present in the side chain of the m'th amino acid counted from the C-terminal amino acid of the polypeptide moiety,
wherein examples of the functional group may include the formula -O- (the formula -OH), the formula -S- (the formula -SH), the formula -NH- (the formula $-NH_2$), the formula -C(=O)O- (the formula -C(=O)OH), the formula -C(=O)NH- (the formula $-C(=O)NH_2$), and the formula -NHC(=NH)NH- ($-NHC(=NH)NH_2$), and
$PG^{sc}$ represents a protecting group for the functional group, wherein examples of the functional group may include, as described above, tBu, Trt, Boc, and Pbf.

(1) [Step 4-1]

Compound [4-b], in which the carboxy of the C-terminal amino acid of the polypeptide moiety is free, can be produced by using an acid on compound [4-a] to cleave the bond to the resin.

At that time, the deprotection for the protecting group ($PG^{sc}$) present in the side chain of the m'th amino acid counted from the C-terminal amino acid can be prevented by using a weak acid as the acid. Examples of the weak acid may include HFIP, acetic acid, and diluted TFA.

(2) [Step 4-2]

Compound [4-d] can be produced by allowing compound "4-c" to act on compound [4-b] obtained in (1) for amidation reaction between the free carboxy in compound [4-b] and the amino in compound [4-c].

(3) [Step 4-3]

Compound [4-e], one of the present inventive compounds, can be produced by using an acid for deprotection for the aforementioned protecting group ($PG^{sc}$) present in the side chain of the m'th amino acid counted from the C-terminal amino acid. Examples of the acid applicable in the present step may include TFA.

Compound [4-e] obtained in the described manner can be isolate/purified by a known separation/purification means such as concentration, concentration under reduced pressure, reprecipitation, solvent extraction, crystallization, and chromatography.

In the method described in Scheme 4, compound [4-c] to be used as a raw material compound can be obtained by producing with a method known per se, or purchasing a commercially available product of it. Compound [4-a] can be produced with the method described in Scheme 3.

Method D: a method for producing compound [5-f], in which a functional group is present in the side chain of an amino acid forming the polypeptide moiety, the functional group is substituted with a polypeptide linker and the amino of the N-terminal amino acid of the polypeptide linker is substituted with "$C_{1-20}$ alkylcarbonyl substituted with carboxy"

Compound [5-f] can be produced through solid-phase synthesis the summary of which is described in the following (Scheme 5).

Here, the polypeptide linker consists of one to five amino acids.

In Scheme 5, a method for producing a compound wherein a functional group is present in the side chain of the m'th amino acid counted from the C-terminal amino acid of the polypeptide moiety in compound [5-f], the functional group is substituted with a polypeptide linker, and the amino of the N-terminal amino acid of the polypeptide linker is substituted with "$C_{1-20}$ alkylcarbonyl substituted with carboxy" is presented as an example.

Scheme 5

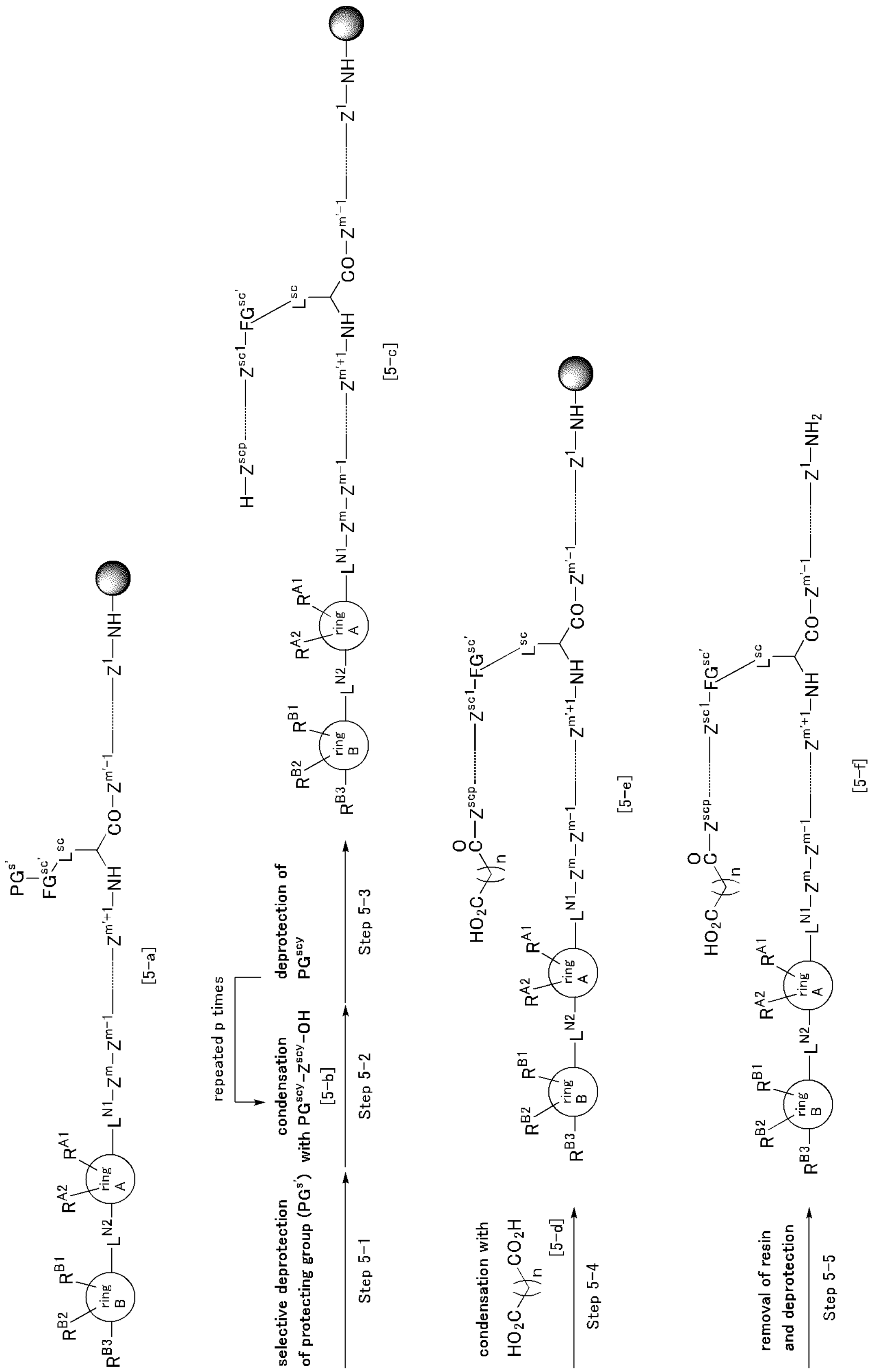


wherein

$R^{A1}$, $R^{A2}$, $R^{B1}$, $R^{B2}$, $R^{B3}$, $L^{N1}$, $L^{N2}$, ring A, ring B, H-$Z^x$-OH, x, m, m', $Z^x$, and $L^{sc}$ have the same definitions as those

described above,
$PG^{s'}$ represents a protecting group for the aforementioned functional group ($FG^{sc'}$) present in the side chain of an amino acid, wherein examples of the protecting group include Dde, ivDde, Alloc, and Mtt for amino,
H-$Z^{sc\ y}$-OH represents an amino acid forming the polypeptide linker attached to the functional group ($FG^{sc'}$),
y represents an integer of 1 to p,
p represents an integer of 0 to 5,
$PG^{sc\ y}$ represents a liposoluble protecting group protecting the amino of the amino acid (H-$Z^{sc\ y}$-OH),
$FG^{sc'}$ represents a functional group present in the side chain of an amino acid; examples of the functional group may include the formula -NH- (the formula $-NH_2$) and the formula -COO-(the formula -COOH); Scheme 5 shows the case that $FG^{sc'}$ is the formula -NH- (the formula $-NH_2$), and
n represents an integer of 1 to 20.

(1) [Step 5-1]

The protecting group ($PG^{S'}$) for the functional group present in the side chain of the m'th amino acid counted from the C-terminal amino acid of the polypeptide moiety in compound [5-a] is subjected to selective deprotection.

(2) [Step 5-2]

The compound obtained in (1) is reacted with an amino acid whose amino has been protected with a liposoluble protecting group ($PG^{sc\ y}$) (compound [5-b]) for amidation reaction between the free functional group ($FG^{sc'}$) in the compound obtained in (1) and the carboxy in compound [5-b].

(3) [Step 5-3]

The liposoluble protecting group ($PG^{sc\ y}$) is subjected to deprotection.
(4) By repeating steps (2) and (3) once or more times, compound [5-c], in which the carboxy of the C-terminal amino acid of the polypeptide moiety is bonding to the resin, the functional group present in the side chain of the m'th amino acid counted from the C-terminal amino acid is substituted with a polypeptide linker, and the amino of the N-terminal amino acid of the polypeptide linker is free, can be produced.

(5) [Step 5-4]

Compound [5-e] can be produced by reacting compound [5-c] obtained in (4) with compound [5-d], which is an alkanedicarboxylic acid, for amidation reaction of the free amino in compound [5-c] with one carboxy in compound [5-d].

(6) [Step 5-5]

Compound [5-f], one of the present inventive compounds, can be produced by using an acid on compound [5-e] obtained in (5) to cleave the bond to the resin. Examples of the acid applicable in the present step may include TFA.
At that time, if a protected functional group is present in the side chain of an amino acid forming the polypeptide moiety, the protecting group of the functional group can be simultaneously deprotected.
Compound [5-f] obtained in the described manner can be isolate/purified by a known separation/purification means such as concentration, concentration under reduced pressure, reprecipitation, solvent extraction, crystallization, and chromatography.
In the method described in Scheme 5, compound [5-b] and compound [5-d] to be used as raw material compounds can be obtained by producing with a method known per se, or purchasing commercially available products of them. Compound [5-a] can be produced with the method described in Scheme 1 or Scheme 2, or a method similar to any of these.

Method E: a method for producing compound [6-c], in which a functional group is present in the side chain of each of two amino acids among the amino acids forming the polypeptide moiety, and the functional groups are substituted with the same polypeptide linker (-$Z^{sc\ 1}$-$Z^{sc\ 2}$-...-$Z^{sc\ y'}$-) to form a ring

Compound [6-c] can be produced, for example, through solid-phase synthesis the summary of which is described in the following (Scheme 6).
Here, the polypeptide linker consists of one to four amino acids.
In Scheme 6, a method for producing a compound wherein a functional group is present in the side chain of

each of the m'th and m"th amino acids counted from the C-terminal amino acid of the polypeptide moiety, and the functional groups are substituted with the same polypeptide linker to form a ring is presented as an example.

Scheme 6

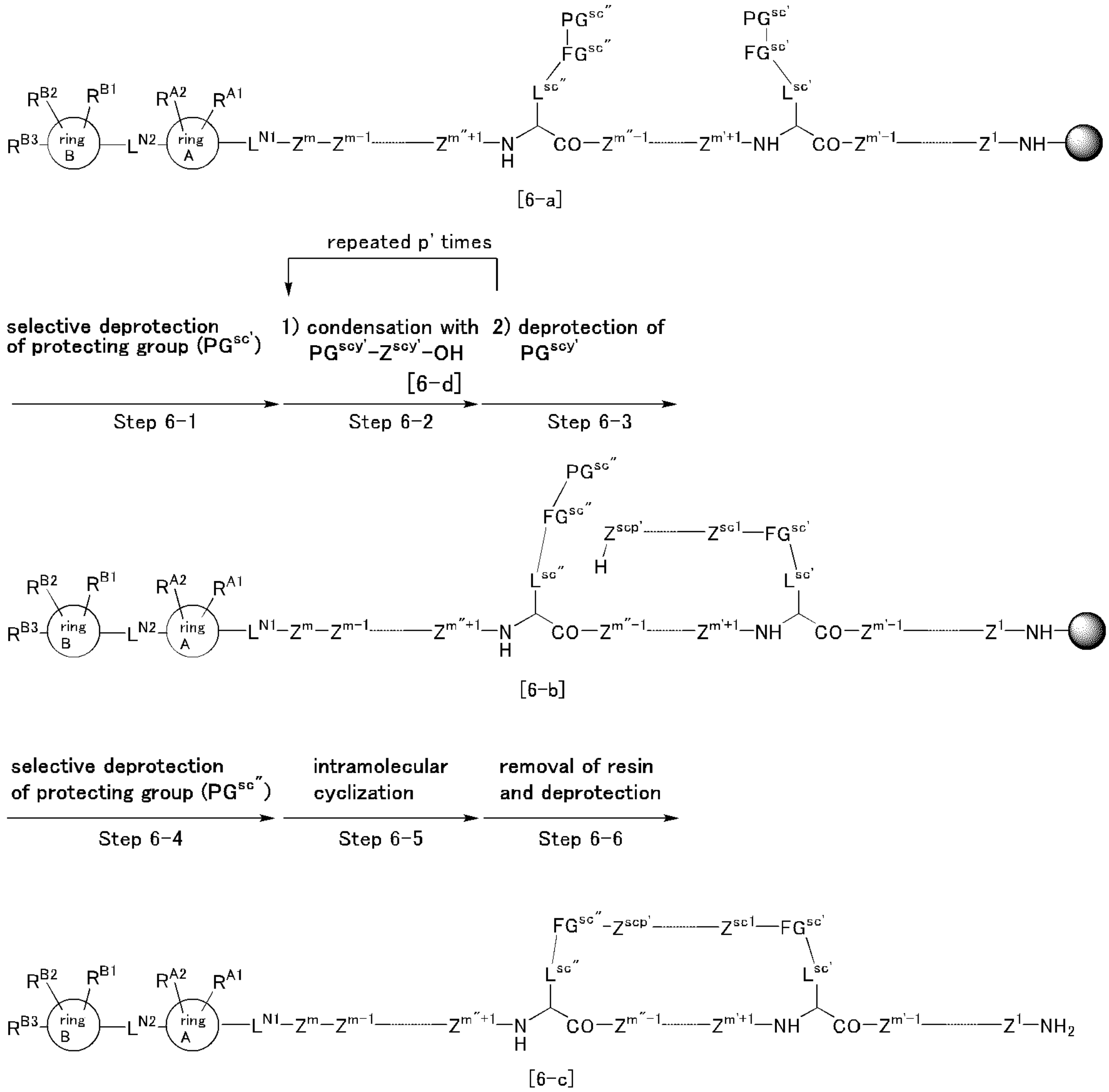


wherein

$R^{A1}$, $R^{A2}$, $R^{B1}$, $R^{B2}$, $R^{B3}$, $L^{N1}$, $L^{N2}$, ring A, ring B, H-$Z^x$-OH, x, m, m', $Z^x$, and $L^{sc}$ have the same definitions as those described above,

m" represents an integer of 2 to 15, provided that the relationship "m' < m" ≤ m" is satisfied, the polypeptide linker (-$Z^{sc\,1}$-$Z^{sc\,2}$-...-$Z^{sc\,y'}$-) in compound [6-c] corresponds to $L^3$ in the compound represented by formula [I'], wherein $L^3$ represents Gly, β-Ala, or GABA,

y' represents an integer of 1 to p',

p' represents an integer of 1 to 4,

$L^{sc'}$ and $L^{sc''}$ each represent $C_{1-4}$ alkanediyl,

$FG^{sc'}$ and $FG^{sc''}$ each represent a functional group present in the side chain of an amino acid, wherein the functional group represents the formula -O- (the formula -OH), the formula -S-(the formula -SH), the formula -NH- (the formula $-NH_2$), the formula -C(=O)O- (the formula -C(=O)OH), or the formula -C(=O)NH- (the formula $-C(=O)NH_2$), and $PG^{sc'}$ and $PG^{sc''}$ each represent a protecting group for a functional group present in the side chain of an amino acid, wherein the functional group is, for example, Dde, ivDde, Alloc, or allyl.

(1) [Step 6-1]

The protecting group ($PG^{sc'}$) for the functional group present in the side chain of the m'th amino acid counted from the C-terminal amino acid of the polypeptide moiety in compound [6-a] is subjected to selective deprotection.

(2) [Step 6-2]

The compound obtained in (1) is reacted with an amino acid whose amino has been protected with a liposoluble protecting group ($PG^{sc\,y'}$) (compound [6-d]) for amidation reaction between the free functional group ($FG^{sc'}$) in the compound obtained in (1) and the carboxy in compound [6-d], the amino of which has been protected with the liposoluble protecting group.

(3) [Step 6-3]

The liposoluble protecting group is subjected to deprotection.

(4) By repeating steps (2) and (3) once or more times, compound [6-b], in which the functional group present in the side chain of the m'th amino acid counted from the C-terminal amino acid of the polypeptide moiety is substituted with a polypeptide linker, the amino of the N-terminal amino acid of the polypeptide linker is free, and the functional group ($FG^{sc''}$) present in the side chain of the m"th amino acid counted from the C-terminal amino acid is protected with a protecting group ($PG^{sc''}$), can be produced.

(5) [Step 6-4]

The protecting group ($PG^{sc''}$) present in the side chain of the m"th amino acid counted from the C-terminal amino acid of the polypeptide moiety in compound [6-b] is subjected to selective deprotection.

(6) [Step 6-5]

"Free functional group ($FG^{sc''}$)" and "free amino in $Z^{scp'}$" in the compound obtained in (5) are reacted for intramolecular cyclization reaction.

(7) [Step 6-6]

Compound [6-c], one of the present inventive compounds, can be produced by using an acid on the compound obtained in (6) to cleave the bond to the resin. Examples of the acid applicable in the present step may include TFA.

At that time, if a protected functional group is present in the side chain of an amino acid forming the polypeptide moiety, the protecting group of the functional group can be simultaneously deprotected.

Compound [6-c] obtained in the described manner can be isolate/purified by a known separation/purification means such as concentration, concentration under reduced pressure, reprecipitation, solvent extraction, crystallization, and chromatography.

In the method described in Scheme 6, compound [6-d] to be used as a raw material compound can be obtained by producing with a method known per se, or purchasing a commercially available product of it. Compound [6-a] can be produced with the method described in Scheme 1 or Scheme 2, or a method similar to any of these.

Method F: a method for producing compound [7-f], which has a substituent, "alkylaminocarbonyl substituted with functional group", on ring B

Compound [7-f] can be produced, for example, through solid-phase synthesis the summary of which is described in the following (Scheme 7).

Scheme 7

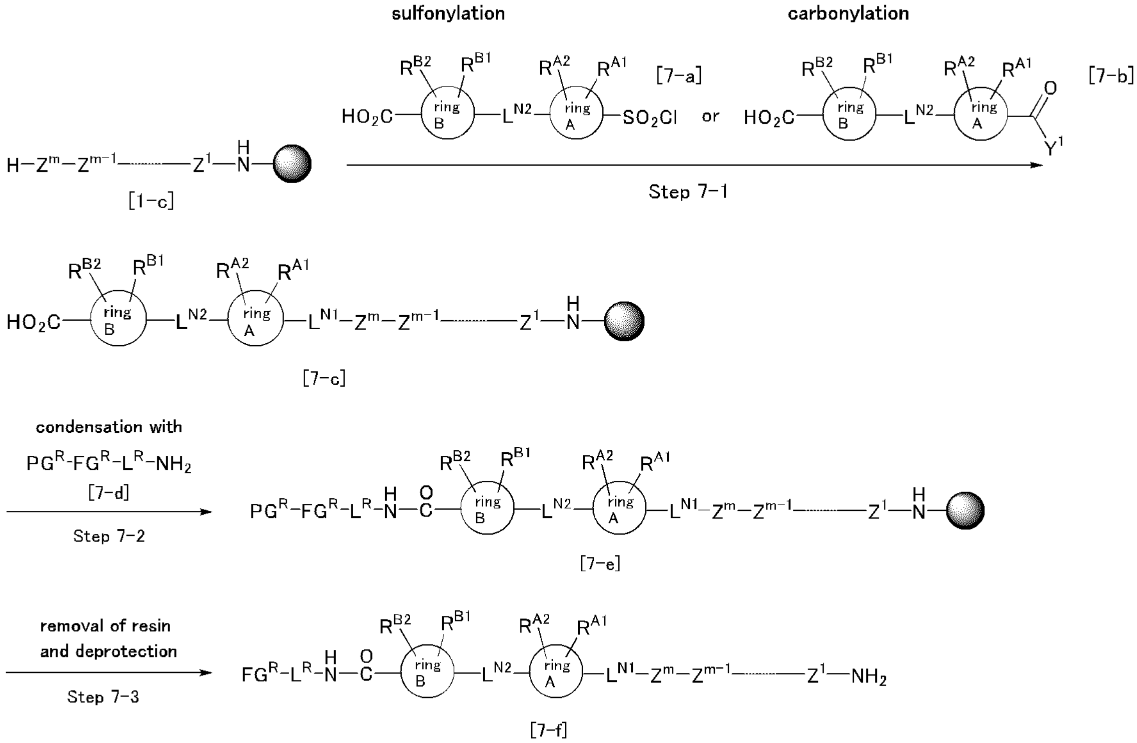


wherein

$R^{A1}$, $R^{A2}$, $R^{B1}$, $R^{B2}$, $L^{N1}$, $L^{N2}$, ring A, ring B, H-$Z^x$-OH, x, m, $Z^x$, and $Y^1$ have the same definitions as those described above,

$L^R$ represents $C_{1-4}$ alkanediyl,

$FG^R$ represents a functional group attached to "alkyl ($L^R$)" of "alkylaminocarbonyl ($L^R$-NHC(=O)-)", a substituent present on ring B, and examples thereof may include the formula -O- (the formula -OH), the formula -S- (the formula -SH), the formula -NH- (the formula $-NH_2$), the formula -C(=O)O- (the formula -C(=O)OH), and the formula -C(=O)NH-(the formula $-C(=O)NH_2$),

$PG^R$ represents a protecting group for the functional group ($FG^R$), wherein examples of the protecting group include tBu for carboxy, Trt for carbamoyl, tBu for hydroxy, tBu for thiol (sulfanyl), and Boc for amino, and these protecting groups can be each deprotected by treating with an acid.

(1) [Step 7-1]

Compound [7-c], which has carboxy on ring B, can be produced by reacting compound [1-c] with compound [7-a] or compound [7-b] to sulfonylate or acylate the free amino of the N-terminal amino acid of the polypeptide moiety in compound [1-c].

(2) [Step 7-2]

Compound [7-e] can be produced by reacting compound [7-c] with compound [7-d] for amidation reaction between the carboxy in compound [7-c] and the amino in compound [7-d].

(3) [Step 7-3]

Compound [7-f], one of the present inventive compounds, can be produced by using an acid on compound [7-e] obtained in (2) to cleave the bond to the resin and simultaneously deprotect the protecting group ($PG^R$) of the functional group of "alkylaminocarbonyl substituted with functional group ($FG^R$)" present on ring B. Examples of the acid applicable in the present step may include TFA. At that time, if a protected functional group is present in the side chain of an amino acid forming the polypeptide moiety, the protecting group of the functional group can be simultaneously deprotected.

Compound [7-f] obtained in the described manner can be isolate/purified by a known separation/purification means such as concentration, concentration under reduced pressure, reprecipitation, solvent extraction, crystallization, and chromatography.

In the method described in Scheme 7, compound [7-a], compound [7-b], and compound [7-d] to be used as raw material compounds can be obtained by producing with a method known per se, or purchasing commercially available products of them. Compound [1-c] can be produced with the method described in Scheme 1, or a method similar to it.

Method G: a method for producing compound [8-f], in which ring A is directly substituted with ring B

Compound [8-f] can be produced, for example, through solid-phase synthesis the summary of which is described in the following (Scheme 8).

Scheme 8

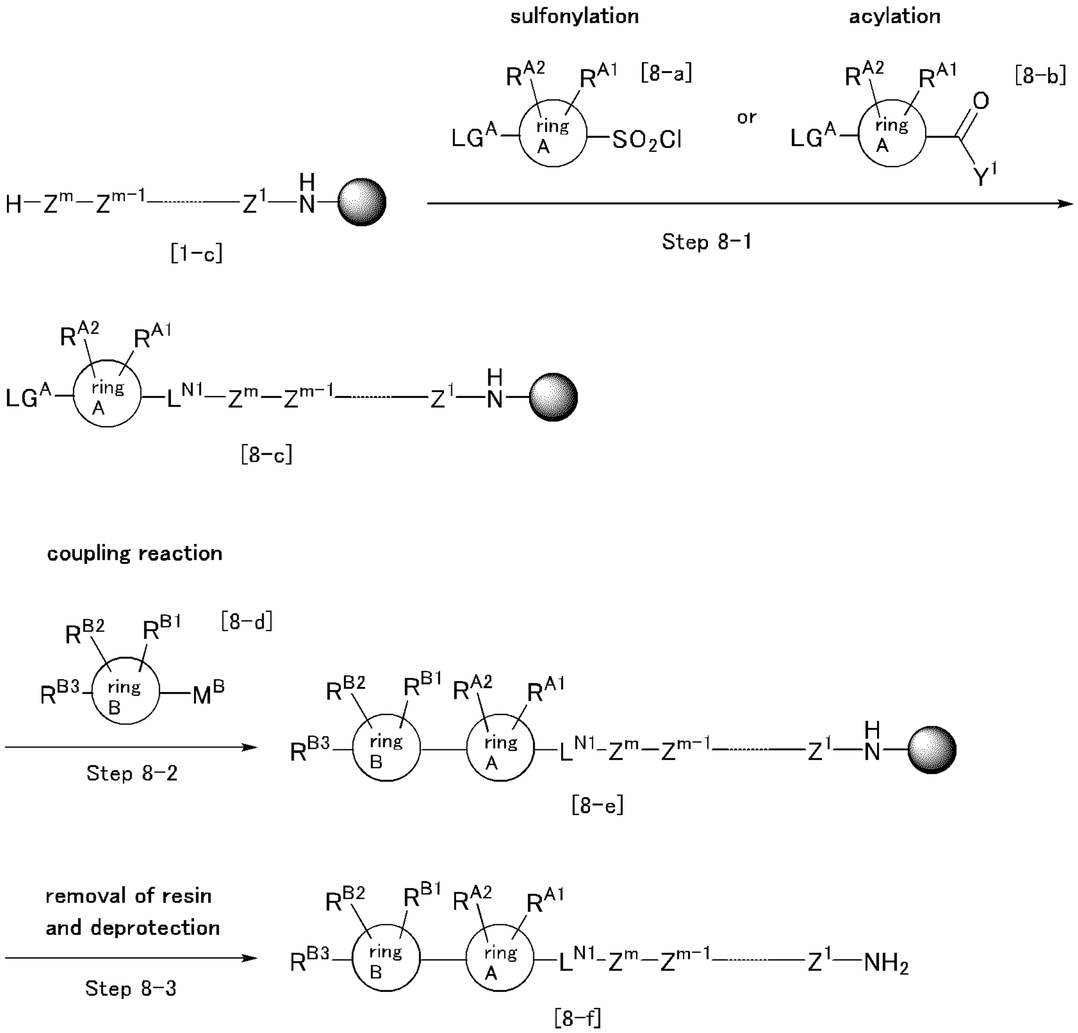


wherein

$R^{A1}$, $R^{A2}$, $R^{B1}$, $R^{B2}$, $R^{B3}$, $L^{N1}$, ring A, ring B, H-$Z^x$-OH, x, m, $Z^x$, and $Y^1$ have the same definitions as those described above,

$LG^A$ represents a leaving group, wherein examples of the leaving group may include a chlorine atom, a bromine atom, an iodine atom, and halo $C_{1-6}$ alkylsulfonyloxy, and $M^B$ represents boronic acid, boronic acid ester, alkyltin, alkylsilane, or alkoxysilane.

(1) [Step 8-1]

Compound [8-c] can be produced by reacting compound [1-c] with compound [8-a] or compound [8-b] to sulfonylate or acylate the free amino of the N-terminal amino acid of the polypeptide moiety in compound [1-c].

(2) [Step 8-2]

Compound [8-e], in which ring A is directly substituted with ring B, can be produced by subjecting compound [8-c] to coupling reaction with compound [8-d] in the presence of a metal catalyst and a base. Examples of the metal catalyst may include tetrakis(triphenylphosphine)palladium, palladium acetate, and tris(dibenzylideneacetone)dipalladium. Examples of the base may include metal carbonates such as cesium carbonate, potassium carbonate, and sodium carbonate, and potassium phosphate, cesium fluoride, potassium fluoride, and tetrabutylammonium fluoride.

(3) [Step 8-3]

Compound [8-f], one of the present inventive compounds, can be produced by using an acid on compound [8-e] obtained in (2) to cleave the bond to the resin. Examples of the acid applicable in the present step may include TFA.
At that time, if a protected functional group is present in the side chain of an amino acid forming the polypeptide moiety, the protecting group of the functional group can be simultaneously deprotected.
Compound [8-f] obtained in the described manner can be isolate/purified by a known separation/purification means such as concentration, concentration under reduced pressure, reprecipitation, solvent extraction, crystallization, and chromatography.
In the method described in Scheme 8, compound [8-a], compound [8-b], and compound [8-d] to be used as raw material compounds can be obtained by producing with a method known per se, or purchasing commercially available products of them. Compound [1-c] can be produced with the method described in Scheme 1, or a method similar to it.

Method H: a method for producing compound [9-f], which has the substituent "arylcarbonylamino (the formula ring B-C(=O)NH-)" on ring A

Compound [9-f] can be produced, for example, through solid-phase synthesis the summary of which is described in the following (Scheme 9).

Scheme 9

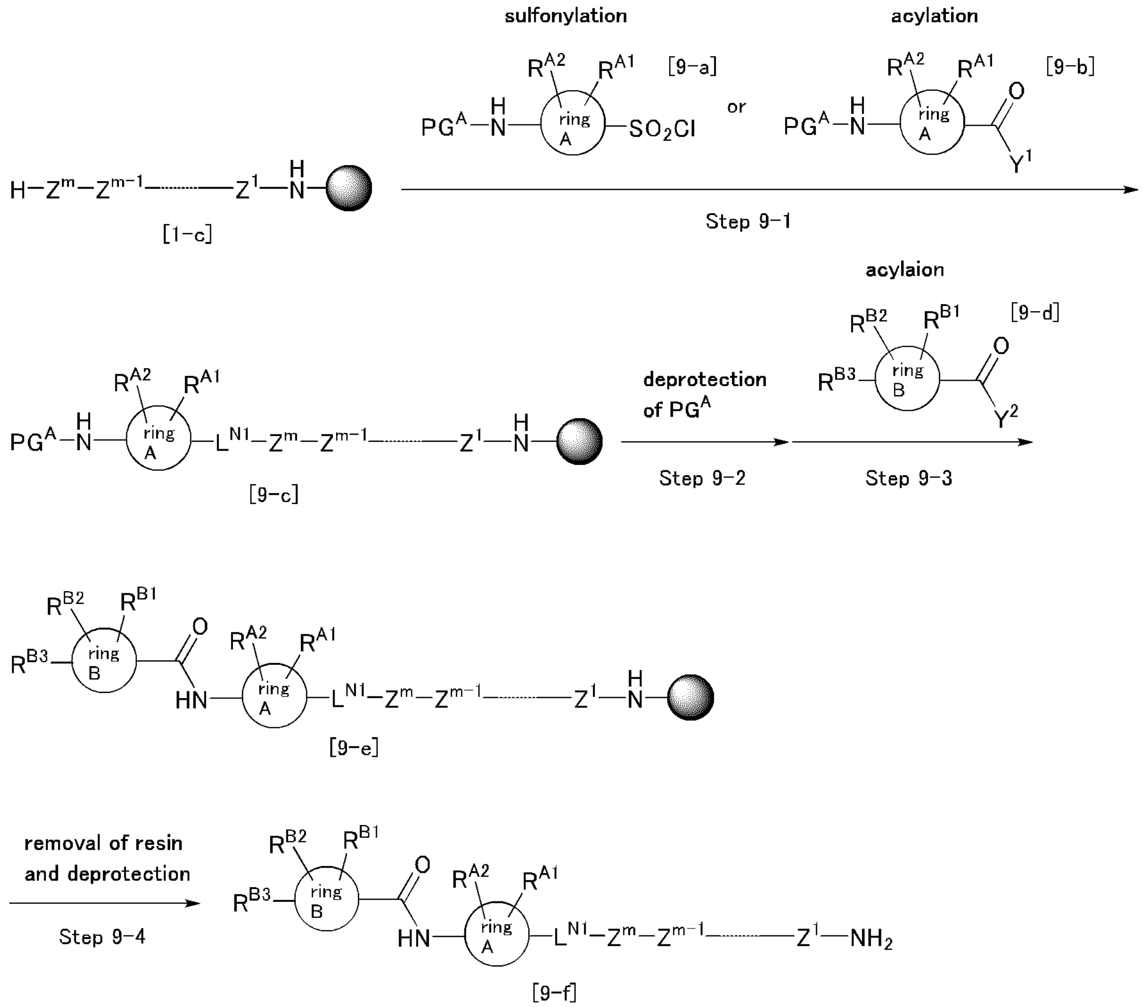


wherein

$R^{A1}$, $R^{A2}$, $R^{B1}$, $R^{B2}$, $R^{B3}$, $L^{N1}$, ring A, ring B, H-$Z^x$-OH, x, m, $Z^x$, and $Y^1$ have the same definitions as those described above,
$Y^2$ represents hydroxy or a chlorine atom, and
$PG^A$ represents a liposoluble protecting group protecting amino present on ring A,
wherein examples of the liposoluble protecting group may include Fmoc.

(1) [Step 9-1]

As in step 8-1 of Scheme 8, compound [9-c] can be produced by reacting compound [1-c] with compound [9-a] or compound [9-b] to sulfonylate or acylate the free amino of the N-terminal amino acid of the polypeptide moiety in compound [1-c].

(2) [Step 9-2]

The liposoluble protecting group ($PG^A$) in compound [9-c] obtained in (1) is subjected to deprotection.

(3) [Step 9-3]

Compound [9-e] can be produced by reacting the compound obtained in (2) with compound [9-d] for amidation (acylation) reaction between the free amino in the compound obtained in (2) and the carboxy in compound [9-d].

(4) [Step 9-4]

Compound [9-f], one of the present inventive compounds, can be produced by using an acid on compound [9-e] obtained in (3) to cleave the bond to the resin. Examples of the acid applicable in the present step may include TFA.
At that time, if a protected functional group is present in the side chain of an amino acid forming the polypeptide moiety, the protecting group of the functional group can be simultaneously deprotected.
Compound [9-f] obtained in the described manner can be isolate/purified by a known separation/purification means such as concentration, concentration under reduced pressure, reprecipitation, solvent extraction, crystallization, and chromatography.
In the method described in Scheme 9, compound [9-a], compound [9-b], and compound [9-d] to be used as raw material compounds can be obtained by producing with a method known per se, or purchasing commercially available products of them. Compound [1-c] can be produced with the method described in Scheme 1, or a method similar to it.
All kinds of amino acids can be used for the amino acids in the methods shown in Schemes 1 to 9 for producing compound [I'] according to the present invention, and examples of the amino acids include natural proteogenic L-amino acids: Gly, Ala, Val, Leu, Ile, Pro, Phe, His, Trp, Tyr, Ser, Thr, Met, Cys, Asp, Glu, Asn, Gln, Lys, and Arg.
In addition, nonnatural amino acids including natural nonproteogenic amino acids and the D-forms of the natural proteogenic L-amino acids described above may be used in the present production methods.
Here, examples of nonnatural amino acids applicable in the present production methods include the following:

- (d)-Pro, (d)-Ser, (d)-Thr, (d)-Asp, (d)-Glu, (d)-Arg, (d)-Lys
- β-homoPro
- β-Ala, GABA, Ape, Acp
- (N-Me)Ala, (N-Me)Val, (N-Me)Leu, (N-Me)Ile, (N-Me)Phe, (N-Me)Tyr, (N-Me)Ser, (N-Me)Asp, (N-Me)Glu
- a group represented by formula [II-1] or formula [II-2]:

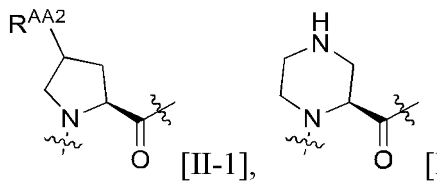
[II-1], [II-2]

  wherein $R^{AA2}$ is hydroxy or amino; and
- the amino acids listed in Table 1 and Table 2

Examples of liposoluble protecting groups may include carbonate protecting groups, amide protecting groups, and alkyl protecting groups such as 9-fluorenylmethoxycarbonyl (Fmoc), tert-butyloxycarbonyl (Boc), benzyl (Bn), allyl (Allyl), allyloxycarbonyl (Alloc), and acetyl (Ac). Introduction of a liposoluble protecting group to an amino acid, for example, introduction of Fmoc, can be achieved by adding 9-fluorenylmethyl-N-succinidyl carbonate and sodium hydrogen carbonate for reaction. It is recommended to perform the reaction at 0 to 50°C, preferably at room temperature, for about 1 to 5 hours.
Commercially available amino acids protected with a liposoluble protecting group may be used. Examples thereof may include Fmoc-Ser-OH, Fmoc-Asn-OH, Fmoc-Val-OH, Fmoc-Leu-OH, Fmoc-Ile-OH, Fmoc-Ala-OH, Fmoc-Tyr-OH, Fmoc-Gly-OH, Fmoc-Lys-OH, Fmoc-Arg-OH, Fmoc-His-OH, Fmoc-Asp-OH, Fmoc-Glu-OH, Fmoc-Gln-OH, Fmoc-Thr-OH, Fmoc-Cys-OH, Fmoc-Met-OH, Fmoc-Phe-OH, Fmoc-Trp-OH, and Fmoc-Pro-OH.
Examples of amino acids protected with a liposoluble protecting group and having a protecting group introduced into the side chain may include Fmoc-Arg(Pbf)-OH, Fmoc-Asn(Trt)-OH, Fmoc-Asp(OtBu)-OH, Fmoc-Cys(tBu)-OH, Fmoc-Glu(OtBu)-OH, Fmoc-Gln(Trt)-OH, Fmoc-His(Trt)-OH, Fmoc-Lys(Boc)-OH, Fmoc-Ser(tBu)-OH, Fmoc-Thr(tBu)-OH, Fmoc-Trp(Boc)-OH, and Fmoc-Tyr(tBu)-OH.
The resin may be any resin commonly used for solid-phase synthesis, and, for example, 2-chlorotrityl chloride resin, which is functionalized with a chlorine atom, Wang resin, HMPA-PEGA resin, and Amino-PEGA resin, which is functionalized with amino, are applicable.
Solid-phase synthesis using resin for amide synthesis is recommended as a method for obtaining an amide formed at the C terminus of peptide. For example, Rink-Amide-AM resin, SAL-PEG resin, SAL-MBHA resin, or Rink-Amide-PEGA resin can be used. An amide formed at the C terminus of peptide can be obtained by cleaving off peptide from the resin.
To bond an amino acid whose amino has been protected with a liposoluble protecting group to resin, for example, in the case that resin having hydroxy or resin functionalized with a chlorine atom is used, the bonding can be achieved

by allowing the carboxy of the amino acid to form an ester bond to the resin.

In the case that resin functionalized with amino is used, the bonding can be achieved by allowing the carboxy of the amino acid to form an amide bond to the resin.

In the case that 2-chlorotrityl chloride resin is used, esterification can be performed by using a base such as diisopropylethylamine (DIPEA), triethylamine, pyridine, and 2,4,6-collidine.

In the case that resin having hydroxy is used, a known dehydrating/condensing agent such as 1-(mesitylene-2-sulfonyl)-3-nitro-1,2,4,-triazole (MSNT), dicyclohexylcarbodiimide (DCC), and diisopropylcarbodiimide (DIC) can be used as an esterification catalyst. Regarding the usage ratio between an amino acid and a dehydrating/condensing agent, the amount of the latter is typically 1 to 10 equivalents, and preferably 2 to 5 equivalents per equivalent of the former.

For example, it is preferable to perform esterification reaction in such a manner that a resin is put in a solid-phase column, this resin is washed with a solvent, and a solution of an amino acid is then added thereto. Examples of the washing solvent may include chloroform, dimethylformamide (DMF), 2-propanol, and dichloromethane. Examples of the solvent to dissolve an amino acid therein include chloroform, dimethyl sulfoxide (DMSO), DMF, and dichloromethane. It is recommended to perform esterification reaction at 0 to 50°C, preferably at room temperature, for about 10 minutes to 30 hours, preferably for about 15 minutes to 24 hours.

At that time, it is also preferable to acetylate unreacted hydroxy on the solid phase by using acetic anhydride or the like for capping.

Elimination of a liposoluble protecting group can be performed, for example, by treating with a base. Examples of the base may include piperidine and morpholine. At that time, it is preferable that the elimination be performed in the presence of a solvent. Examples of the solvent may include DMF, DMSO, and methanol.

It is preferable to perform amidation reaction between the liberated amino and the carboxy of any amino acid whose amino has been protected with a liposoluble protecting group in the presence of an activator and a solvent.

Examples of the activator may include dicyclohexylcarbodiimide (DCC), 1-ethyl-3-(dimethylaminopropyl)carbodiimide·hydrochloride (WSC/HCl), diphenylphosphorylazide (DPPA), carbonyldiimidazole (CDI), diethylcyanophosphonate (DEPC), benzotriazol-1-yloxy-trispyrrolidinophosphonium (DIPCI), benzotriazol-1-yloxy-trispyrrolidinophosphonium hexafluorophosphate (PyBOP), 1-hydroxybenzotriazole (HOBt), hydroxysuccinimide (HOSu), dimethylaminopyridine (DMAP), 1-hydroxy-7-azabenzotriazole (HOAt), hydroxyphthalimide (HOPht), pentafluorophenol (Pfp-OH), 2-(1H-benzotriazol-1-yl)-1,1,3,3,-tetramethyluronium hexafluorophosphate (HBTU), 1-[bis(dimethylamino)methylene]-5-chloro-1H-benzotriazolium 3-oxide hexafluorophosphate (HCTU), O-(7-azabenzotriazol-1-yl)-1,1,3,3-tetramethyluronium hexafluorophosphonate (HATU), O-benzotriazol-1-yl-1,1,3,3-tetramethyluronium tetrafluoroborate (TBTU), 3,4-dihydro-3-hydrodi-4-oxa-1,2,3-benzotriazine (Dhbt), N-[1-(cyano-2-ethoxy-2-oxoethylideneaminooxy)dimethylamino (morpholino)]uronium hexafluorophosphate (COMU), and ethyl cyano(hydroxyimino)acetate (Oxyma).

It is preferable to set the amount of usage of the activator to 1 to 20 equivalents, preferably to 1 to 10 equivalents, more preferably to 1 to 5 equivalents, with respect to any amino acid whose amino has been protected with a protecting group having liposolubility.

Examples of the solvent may include DMF, DMSO, and dichloromethane. It is recommended to perform the reaction at 0 to 50°C, preferably at room temperature, for about 10 minutes to 30 hours, preferably for about 15 minutes to 2 hours.

Cleaving-off of a peptide chain from resin can be performed through treatment with an acid. Examples of the acid may include trifluoroacetic acid (TFA) and hydrogen fluoride (HF). It is recommended to perform the reaction for separation from resin at 0 to 50°C, preferably at room temperature, for about 10 minutes to 10 hours, preferably for about 30 minutes to 4 hours.

The present invention is described in more detail with reference to Examples, Reference Examples, and Test Examples below; however, these do not limit the present invention, and any modification may be made without departing from the scope of the present invention.

Abbreviations in the present specification are shown in the following.

APCI: atmospheric pressure chemical ionization
Arg (Pbf): $N^{\omega}$-2,2,4,6,7-pentamethyldihydrobenzofuransulfonylarginine
Bu: butyl
BuOH: butanol
Dde: 1-(4,4-dimethyl-2,6-dioxocyclohexylidene)-3-ethyl
DMSO-$d_6$: hexadeutrated dimethyl sulfoxide
ELSD: evaporative light scattering detector
ESI: electrospray ionization
HPLC: high-performance liquid chromatography
ivDde: 1-(4,4-dimethyl-2,6-dioxocyclohexylidene)-3-methylbutyl
LCMS: liquid chromatography/mass spectrometry

NMP: 1-methyl-2-pyrrolidone
Trt: trityl
UV: ultraviolet ray

Herein, "room temperature" refers to 20 to 30°C, unless otherwise specified.
"Under ice-cooling" refers to 0 to 5°C, unless otherwise specified.
Herein, Biotage (registered trademark) SNAP Ultra manufactured by Biotage AB or REVELERIS (registered trademark) Silica 40 μm manufactured by BUCHI Labortechnik AG was used for "silica gel cartridge" in purification with column chromatography.

In purification with reversed-phase column chromatography (hereinafter, occasionally referred to as preparative LCMS), an appropriate condition was selected from two conditions shown in the following, and purification was performed.
Separation apparatus: used was Agilent 1260 Infinity and Agilent 6130 (ionization method: Electron Spray Ionization: ESI), or Agilent 385-ELSD when an ELSD detector was involved, each apparatus being from Agilent Technologies
Solvent: solution A; water with 0.1% formic acid, solution B; acetonitrile with 0.1% formic acid

Flow rate: 50 mL/min
One of the following columns was used.
Waters XBridge Prep C18, 5 μm, 30 × 50 mm
Waters XSelect CSH C18, 5 μm, 30 × 50 mm
(Separation condition A)

0.0-0.5 min (solution A/solution B = 90/10)
0.5-7.5 min (solution A/solution B = 90/10 to 20/80)
7.5-7.95 min (solution A/solution B = 20/80)
7.95-8.0 min (solution A/solution B = 20/80 to 5/95)
8.0-9.0 min (solution A/solution B = 5/95)

(Separation condition B)

0.0-0.5 min (solution A/solution B = 95/5)
0.5-7.5 min (solution A/solution B = 95/5 to 50/50)
7.5-7.95 min (solution A/solution B = 50/50)
7.95-8.0 min (solution A/solution B = 50/50 to 5/95)
8.0-9.0 min (solution A/solution B = 5/95)

Instrumental data shown herein were determined with the following instruments.

Microwave reactor: Initiator (Biotage AB)
NMR spectra: [1H-NMR] 600 MHz: JNM-ECA600 (JEOL Ltd.), 400 MHz: AVANCE III HD 400 (Bruker)

Mass spectra of high-performance liquid chromatography (LCMS) and retention time (RT) in the present specification were determined under conditions shown in the following.

Measurement apparatus: LCMS-2010EV, Shimadzu Corporation
Column: Shimadzu XR-ODS, 2.2 μm, 2.0 × 30 mm
Ionization method: ESI/APCI dual source
Solvent: solution A; water with 0.1% formic acid, solution B; acetonitrile with 0.1% formic acid
Flow rate: 0.6 mL/min, detection method: UV 210 nm, 254 nm
(Analysis condition A)

0.0-1.0 min (solution A/solution B = 90/10 to 60/40)
1.0-2.0 min (solution A/solution B = 60/40 to 1/99)
2.0-2.6 min (solution A/solution B = 1/99)

(Analysis condition B)

0.0-0.5 min (solution A/solution B = 90/10)
0.5-1.5 min (solution A/solution B = 90/10 to 70/30)
1.5-2.5 min (solution A/solution B = 70/30 to 1/99)
2.5-5.0 min (solution A/solution B = 1/99)

Compound names in Production Examples, Reference Examples, and Examples were given in accordance with "ACD/Name 2019.1.2 (ACD Labs 2019.1.2, Advanced Chemistry Development Inc.)".

Reference Example 1 4-(4-Carbamoylbenzamide)benzenesulfonyl chloride

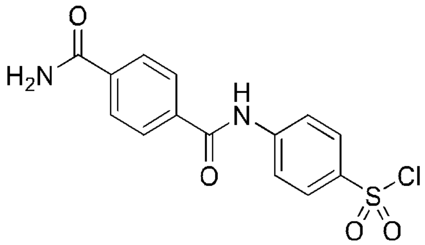

To a solution of 4-carbamoylbenzoic acid (150 g) in DMF (180 mL), WSC monohydrochloride (209 g), HOBt monohydrate (167 g), and DIPEA (380 mL) were added. After stirring at room temperature for 5 minutes, aniline (99 mL) was added, and the resultant was stirred at room temperature for 72 hours. Water was added to the reaction solution, and a solid precipitated was collected through filtration to afford N-phenylterephthalamide (147 g) as a yellow amorphous.
To N-phenylterephthalamide obtained, chlorosulfonic acid (407 mL) was added, and the resultant was stirred at 60°C for 1 hour with heating. The reaction mixture was ice-cooled, and then added to iced water. A solid precipitated was collected through filtration, washed with water, and then dried to afford the title compound (198 g) as a light-yellow solid.
$^1$H NMR (400 MHz, DMSO-$d_6$) $\delta$ ppm 7.54 (bs, 1 H), 7.62 (d, J=8.2 Hz, 2 H), 7.79 (d, J=8.2 Hz, 2 H), 7.96-8.09 (m, 4 H), 8.14 (bs, 1 H), 10.46 (s, 1 H)

Reference Example 2 4-(4-(Trifluoromethoxy)benzamide)benzenesulfonyl chloride

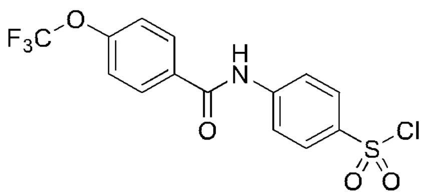

With the same procedure as in Reference Example 1, the title compound was obtained as a yellow solid from the corresponding raw material.
$^1$H NMR (400 MHz, DMSO-$d_6$) $\delta$ ppm 7.52 (d, J=8.3 Hz, 2 H), 7.58 (d, J=8.3 Hz, 2 H), 7.72 (d, J=8.3 Hz, 2 H), 8.08 (d, J=8.3 Hz, 2 H), 10.42 (s, 1 H)

Reference Example 3 4-(4-(Methylcarbamoyl)benzamide)benzenesulfonyl chloride

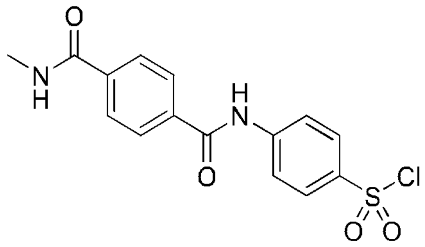

With the same procedure as in Reference Example 1, the title compound was obtained as a gray solid from the corresponding raw material.

$^1H$ NMR (600 MHz, DMSO-$d_6$) $\delta$ ppm 2.81 (d, J=4.1 Hz, 3 H), 7.57-7.61 (m, 2 H), 7.72-7.78 (m, 2 H), 7.96 (d, J=8.7 Hz, 2 H), 8.03 (d, J=8.7 Hz, 2 H), 8.62 (q, J=4.1, 1 H), 10.42 (s, 1 H)

Reference Example 4 4-(4-(Dimethylcarbamoyl)benzamide)benzenesulfonyl chloride

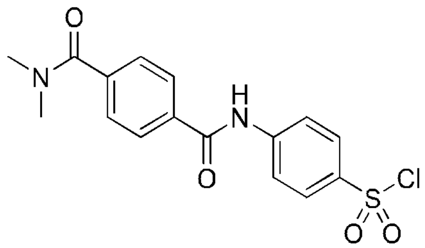

With the same procedure as in Reference Example 1, the title compound was obtained as a brown solid from the corresponding raw material.

$^1H$ NMR (600 MHz, DMSO-$d_6$) $\delta$ ppm 2.91 (s, 3 H), 3.01 (s, 3 H), 7.54 (d, J=8.3 Hz, 2 H), 7.58 (d, J=8.3 Hz, 2 H), 7.74 (d, J=8.3 Hz, 2 H), 8.01 (d, J=8.3 Hz, 2 H), 10.41 (s, 1 H)

Reference Example 5 4-(4-(Trifluoromethyl)benzamide)benzenesulfonyl chloride

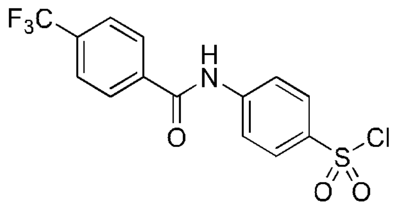

With the same procedure as in Reference Example 1, the title compound was obtained as a white solid from the corresponding raw material.

$^1H$ NMR (400 MHz, CHLOROFORM-d) $\delta$ ppm 7.81 (d, J=8.2 Hz, 2 H), 7.93 (d, J=8.1 Hz, 2 H), 8.01 (d, J=8.2 Hz, 2 H), 8.08 (d, J=8.1 Hz, 3 H)

Reference Example 6 4-(4-Cyanobenzamide)benzenesulfonyl chloride

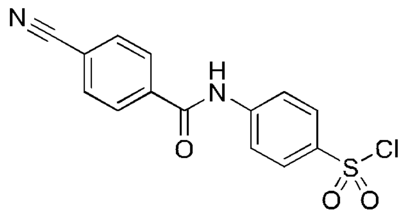

With the same procedure as in Reference Example 1, the title compound was obtained as a light brown solid from the corresponding raw material.

$^1H$ NMR (600 MHz, DMSO-$d_6$) $\delta$ ppm 7.58 (d, J=8.3 Hz, 2 H), 7.74 (d, J=8.3 Hz, 2 H), 7.99 (d, J=8.3 Hz, 2 H), 8.01 (d, J=8.3 Hz, 2 H), 10.42 (s, 1 H)

Reference Example 7 4-(4-(1,1,2,2-Tetrafluoroethoxy)benzamide)benzenesulfonyl chloride

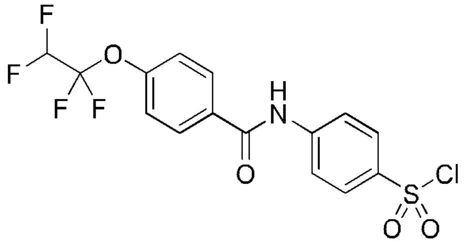

With the same procedure as in Reference Example 1, the title compound was obtained as a yellow solid from the corresponding raw material.

$^1H$ NMR (400 MHz, CHLOROFORM-d) $\delta$ ppm 5.95 (tt, J=53.2 Hz, 2.8 Hz, 1 H), 7.37 (d, J=8,6 Hz, 2 H), 7.92 (d, J=7.2 Hz, 2 H), 7.94 (d, J=7.2 Hz, 2 H), 8.05 (d, J=8.6 Hz, 2 H), 8.12 (s, 1 H)

Reference Example 8 4-(2-Fluorobenzamide)benzenesulfonyl chloride

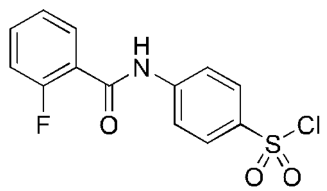

With the same procedure as in Reference Example 1, the title compound was obtained as a white solid from the corresponding raw material.

$^1H$ NMR (400 MHz, CHLOROFORM-d) $\delta$ ppm 7.20-7.27 (m, 1 H), 7.37 (dd, J=7.6 Hz, 7.6 Hz, 1 H), 7.56-7.63 (m, 1 H), 7.95 (d, J=9.1 Hz, 2 H), 8.06 (d, J=9.1 Hz, 2 H), 8.20 (td, J=8.0 Hz, 1.8 Hz, 1 H), 8.75 (d, J=16.9 Hz, 1 H)

Reference Example 9 2-Fluoro-4-phenoxybenzoic acid

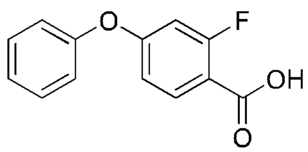

To a solution of 2,4-difluorobenzaldehyde (250 mg) and phenol (199 mg) in DMF (10 mL), potassium carbonate (535 mg) was added, and the resultant was stirred at 120°C for 4 hours with heating. After allowing to cool to room temperature, the reaction solution was diluted with ethyl acetate, and washed with water. The organic layer was dried over anhydrous magnesium sulfate, and concentrated under reduced pressure to afford 2-fluoro-4-phenoxybenzaldehyde.

In tetrahydrofuran (5 mL) and water (5 mL), 2-fluoro-4-phenoxybenzaldehyde obtained was dissolved, to which sodium dihydrogen phosphate (844 mg), 2-methyl-2-butene (1.49 mL), and sodium chlorite (636 mg) were added, and the resultant was stirred at room temperature for 3 hours. Chloroform was added to the reaction solution, which was washed with water. The organic layer was allowed to pass through a Phase separator, and concentrated under reduced pressure to afford the title compound (182 mg) as a white solid.

$^1$H NMR (400 MHz, DMSO-$d_6$) δ ppm 6.75-6.92 (m, 2 H), 7.17 (d, J=7.5 Hz, 2 H), 7.24-7.33 (m, 1 H), 7.43-7.55 (m, 2 H), 7.83-7.97 (m, 1 H), 13.0 (br s, 1 H)

Reference Example 10 4-(4-Carbamoyl-2-fluorophenoxy)benzoic acid

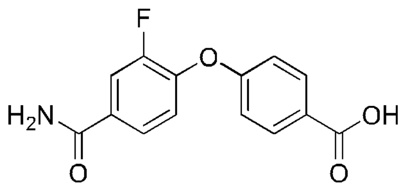

With the same procedure as in Reference Example 9, the title compound was obtained as a white solid from the corresponding raw material.

$^1$H NMR (400 MHz, DMSO-$d_6$) δ ppm 7.12 (d, J=8.4 Hz, 1 H), 7.19 (d, J=8.4 Hz, 2 H), 7.40 (t, J=8.3 Hz, 1 H), 7.53 (br s, 1 H), 7.82 (t, J=8.3 Hz, 1 H), 7.90-8.00 (m, 2 H), 8.07 (br s, 1 H), 9.95 (s, 1 H)

Reference Example 11 3-Fluoro-4-(2-fluorophenoxy)benzoic acid

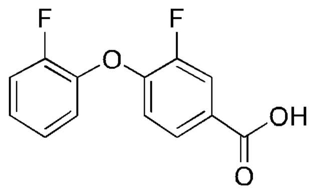

With the same procedure as in Reference Example 9, the title compound was obtained as a white solid from the corresponding raw material.

$^1$H NMR (400 MHz, CHLOROFORM-d) δ ppm 6.89 (t, J=8.1 Hz, 1 H), 7.10-7.25 (m, 4H), 7.80 (d, J=8.4 Hz, 1 H), 7.89 (d, J=11.3 Hz, 1 H)

Reference Example 12 5-((4-(4-Carbamoylphenoxy)phenyl)sulfonamide)pentanoic acid

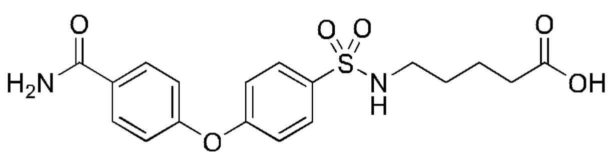

(1)

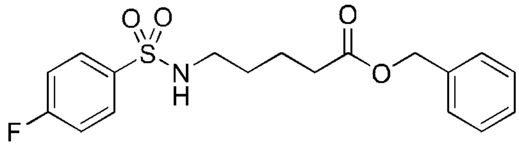

To a solution of benzyl 5-aminopentanoate·tosilate (1.48 g) in chloroform (20 mL), triethylamine (2.3 mL) and 4-fluorobenzenesulfonyl chloride (800 mg) were added under ice-cooling, and the resultant was stirred under ice-cooling for 1 hour. After the completion of the reaction, saturated sodium hydrogen carbonate aqueous solution was added to the reaction mixture, and extraction was performed with chloroform. The organic layer was allowed to pass through a Phase separator, and concentrated under reduced pressure. The residue was purified by column chromatography (silica gel cartridge, hexane:ethyl acetate = 80:20 to 50:50) to afford benzyl 5-((4-fluorophenyl)sulfonamide)pentanoate (1.24 g) as a colorless oily substance.

$^1$H NMR (400 MHz, CHLOROFORM-d) δ ppm 1.46-1.56 (m, 2 H), 1.58-1.68 (m, 2 H), 2.33 (t, J=7.0 Hz, 2 H), 2.96 (q, J=6.5 Hz, 2 H), 4.40-4.47 (m, 1 H), 5.09 (s, 2 H), 7.18 (t, J=8.2 Hz, 2 H), 7.31-7.39 (m, 5 H), 7.86 (dd, J=7.6 Hz, 5.3 Hz, 2 H)

(2)

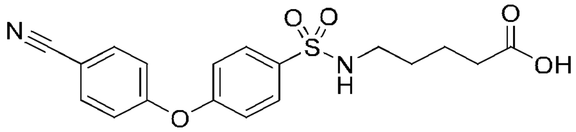

To a solution of benzyl 5-((4-fluorophenyl)sulfonamide)pentanoate (500 mg) obtained in (1) in DMF (13 mL), 4-hydroxybenzonitrile (179 mg) and potassium carbonate (567 mg) were added, and the resultant was stirred under microwave irradiation at 180°C for 1 hour. After the reaction solution was allowed to cool, ethyl acetate and water were added to the reaction mixture for liquid separation. After the aqueous layer was washed with ethyl acetate, the pH was adjusted to 1 with 1 M hydrochloric acid aqueous solution, and extraction was performed with ethyl acetate/toluene mixed solvent. The organic layer was washed with water, then dried over anhydrous magnesium sulfate, and concentrated under reduced pressure to afford 5-((4-(4-cyanophenoxy)phenyl)sulfonamide)pentanoic acid (285 mg) as a white solid.

$^1$H NMR (400 MHz, DMSO-$d_6$) δ ppm 1.32-1.49 (m, 4 H), 2.10-2.17 (m, 2 H), 2.69-2.78 (m, 2 H), 7.23-7.31 (m, 2 H), 7.43 (t, J=8.7 Hz, 2 H), 7.59-7.66 (m, 1 H), 7.81-7.87 (m, 3 H), 7.91 (d, J=8.3 Hz, 1 H)

(3) To a solution of 5-((4-(4-cyanophenoxy)phenyl)sulfonamide)pentanoic acid (100 mg) obtained in (2) in DMSO (2 mL), potassium carbonate (81 mg) and 30% hydrogen peroxide solution (0.15 mL) were added, and the resultant was stirred at room temperature for 2 hours. After the completion of the reaction, sodium thiosulfate aqueous solution and 1 M hydrochloric acid aqueous solution were added, and extraction was performed with ethyl acetate. The organic layer was washed with water, then dried over anhydrous magnesium sulfate, and concentrated under reduced pressure to afford the title compound (89 mg) as a white solid.

$^1$H NMR (400 MHz, DMSO-$d_6$) δ ppm 1.32-1.50 (m, 4 H), 2.10-2.18 (m, 2 H), 2.69-2.78 (m, 2 H), 7.16 (d, J= 8.2 Hz, 1 H), 7.19 (d, J= 8.6 Hz, 1 H), 7.34 (br s, 1 H), 7.43 (t, J= 8.6 Hz, 2 H), 7.52-7.58 (m, 1 H), 7.59-7.66 (m, 1 H), 7.86- 7.79 (m 3 H), 7.96 (d, J=7.7 Hz, 1 H), 12.0 (br s, 1 H)

Example 1 Synthesis of compound of compound No. 1:

(*N*-[4-(4-carbamoylbenzamido)benzene-1-sulfonyl]-D-γ-glutamyl-(4*S*)-4-amino-L-prolyl-L-l eucyl-*N*-(5-amino-5-oxo-pentyl)-*N*$^2$-methyl-L-α-glutamine)

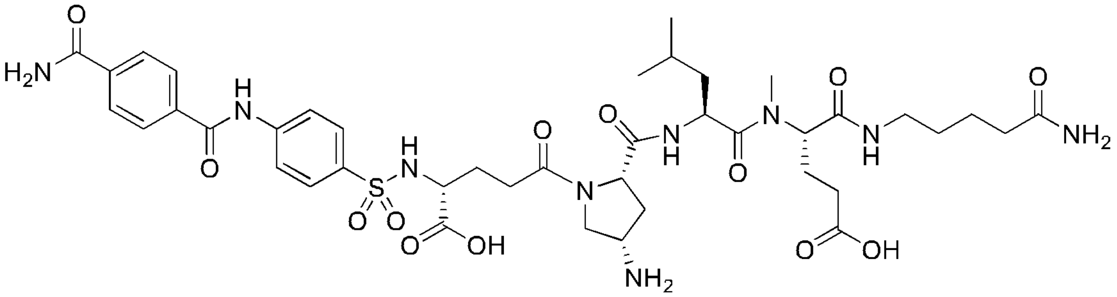

(1) Fmoc-Rink Amide AM resin (0.10 mmol) was treated with DMF solution of piperidine (concentration: 40%, 1.6 mL) for 3 minutes, and then treated with DMF solution of piperidine (concentration: 20%, 1.6 mL) for 12 minutes to deprotect the Fmoc on the resin. Subsequently, a solution of Fmoc-Ape-OH (0.40 mmol) in DMF (0.8 mL), a solution of COMU (0.40 mmol) and Oxyma (0.40 mmol) in DMF (0.8 mL), and a solution of DIPEA (0.80 mmol) in NMP (0.4 mL) were added, and the mixture was shaken at room temperature for 40 minutes to introduce an Ape residue. In the same manner, deprotection for Fmoc and condensation were repeated: specifically, Fmoc-(N-Me)Glu(OtBu)-OH, Fmoc-Leu-OH, Fmoc-(2S,4S)-(4-NHBoc)Pro-OH, and Fmoc-(d)-Glu-OtBu were sequentially condensed, wherein deprotection was performed for the N-terminal Fmoc formed in the resin after each condensation by piperidine/DMF treatment in the above manner, and

H-γ-(d)-Glu(OtBu)-(2S,4S)-(4-NHBoc)Pro-Leu-(N-Me)Glu(OtBu)-Ape-Rink Amide AM resin was synthesized.

(2) To the resin obtained in (1), a solution of 4-(4-carbamoylbenzamide)benzenesulfonyl chloride (0.30 mmol) obtained in Reference Example 1 and DIPEA (0.60 mmol) in DMF (3 mL) was added, and the mixture was shaken at room temperature for 1 hour. After the completion of the reaction, the resulting resin was washed with DMF (3 mL × three times) and chloroform (3 mL × three times).

(3) To the resin obtained in (2), TFA:water:triisopropylsilane (92.5:2.5:5, 4 mL) was added, the mixture was shaken at room temperature for 1.5 hours, and the resin was removed through filtration. An operation in which cooled diethyl ether was added to the filtrate, a resulting white powder was precipitated by centrifugation, and diethyl ether was removed by decantation was repeated three times to afford a crude product of peptide. The crude product obtained was purified by preparative LCMS (separation condition B). The eluate was fractionated using test tubes, and eluted fractions containing the target product were collected and lyophilized to afford the title compound (46 mg) as a white powder.

Example 2 Synthesis of compound of compound No. 106:

(*N*2-[4-(4-phenoxybenzamido)butanoyl]-L-asparaginyl-*N*-[(2*S*)-4-amino-1-({(2*S*)-1-[{(2*S*,3*S*) -1-[(2*S*)-2-(2-amino-2-oxoethyl)pyrrolidin-1-yl]-3-methyl-1-oxopentan-2-yl}(methyl)amino] -4-methyl-1-oxopentan-2-yl}amino)-1-oxobutan-2-yl]-L-α-asparagine)

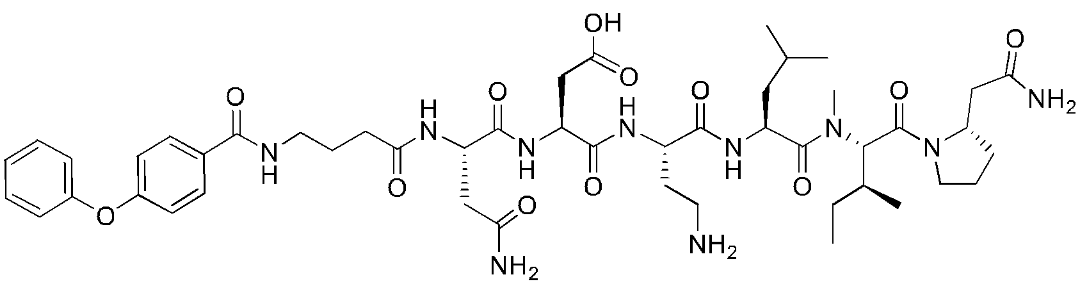

(1) Fmoc-NH-SAL-PEG resin (0.12 mmol) was treated with DMF solution of piperidine (concentration: 40%, 1.9 mL) for 3 minutes, and then treated with DMF solution of piperidine (concentration: 20%, 1.9 mL) for 12 minutes to deprotect the Fmoc on the resin. Subsequently, a solution of Fmoc-β-homoPro-OH (0.48 mmol) in DMF (1.0 mL), a solution of COMU (0.48 mmol) and Oxyma (0.48 mmol) in DMF (1.0 mL), and a solution of DIPEA (0.96 mmol) in NMP (0.48 mL) were added, and the mixture was shaken at room temperature for 40 minutes to introduce a β-homoPro residue. In the same manner, deprotection for Fmoc and condensation were repeated: specifically, Fmoc-(N-Me)Ile-OH, Fmoc-Leu-OH, Fmoc-Dab(Boc)-OH, Fmoc-Asp(OtBu)-OH, Fmoc-Asn(Trt)-OH, Fmoc-GABA-OH, and 4-phenoxybenzoic acid were sequentially condensed, and Ph-O-Ph-CO-GABA-Asn(Trt)-Asp(OtBu)-Dab(Boc)-Leu-(N-Me)Ile-β-homoPro-NH-SAL-P EG resin was synthesized. After the completion of the reaction, the resulting resin was washed with DMF (3 mL × three times) and chloroform (3 mL × three times).

(2) To the resin obtained in (1), TFA:water:triisopropylsilane:dithiothreitol (90:2.5:5:2.5, 4 mL) was added, the mixture was shaken at room temperature for 2 hours, and the resin was removed through filtration. An operation in which cooled diethyl ether was added to the filtrate, a resulting white powder was precipitated by centrifugation, and diethyl ether was removed by decantation was repeated three times to afford a crude product of peptide. The crude product obtained was purified by preparative LCMS (separation condition A). The eluate was fractionated using test tubes, and eluted fractions containing the target product were collected and lyophilized to afford the title compound (14 mg) as a white powder.

Example 3 Synthesis of compound of compound No. 177:

(*N*2-(4-{4-[4-(methylcarbamoyl)phenoxy]benzamido}butanoyl)-L-asparaginyl-L-α-aspartyl-L-alanyl-L-leucyl-L-methionyl-L-prolinamide)

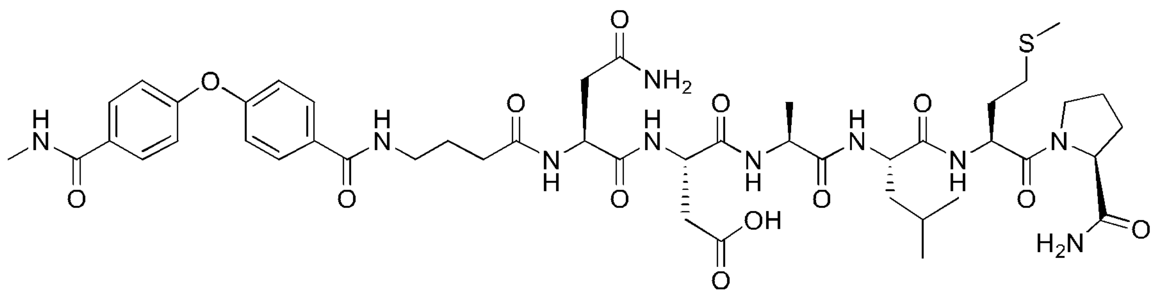

(1) Fmoc-NH-SAL-PEG resin (0.10 mmol) was treated with DMF solution of piperidine (concentration: 40%, 1.6 mL) for 3 minutes, and then treated with DMF solution of piperidine (concentration: 20%, 1.6 mL) for 12 minutes to deprotect the Fmoc on the resin. Subsequently, a solution of Fmoc-Pro-OH (0.40 mmol) in DMF (0.8 mL), a solution of COMU (0.40 mmol) and Oxyma (0.40 mmol) in DMF (0.8 mL), and a solution of DIPEA (0.80 mmol) in NMP (0.4 mL) were added, and the mixture was shaken at room temperature for 40 minutes to introduce a Pro residue. In the same manner, deprotection for Fmoc and condensation were repeated: specifically, Fmoc-Met-OH, Fmoc-Leu-OH, Fmoc-Ala-OH, Fmoc-Asp(OtBu)-OH, Fmoc-Asn(Trt)-OH, and Fmoc-GABA-OH were sequentially condensed, wherein deprotection was performed for the N-terminal Fmoc formed in the resin after each condensation by piperidine/DMF treatment in the above manner, and H-GABA-Asn(Trt)-Asp(OtBu)-Ala-Leu-Met-Pro-NH-SAL-PEG resin was synthesized.

(2) To the resin obtained in (1), a solution of 4,4'-oxybisbenzoic acid (0.75 mmol), HATU (0.15 mmol), and DIPEA (2.0 mmol) in DMF (3 mL) was added, and the mixture was shaken at room temperature for 2 hours. After the completion of the reaction, the resulting resin was washed with DMF (3 mL × three times).

(3) To the resin obtained in (2), a solution of HATU (0.30 mmol), DIPEA (0.60 mmol), and methanol solution of methylamine (concentration: 9.8 mol/L, 0.30 mmol) in DMF (3 mL) was added, and the mixture was stirred at room temperature for 4 hours. After the completion of the reaction, the resulting resin was washed with DMF (3 mL × three times) and chloroform (3 mL × three times).

(4) To the resin obtained in (3), TFA:water:triisopropylsilane:dithiothreitol (90:2.5:5:2.5, 4 mL) was added, the mixture was shaken at room temperature for 2 hours, and the resin was removed through filtration. An operation in which cooled diethyl ether was added to the filtrate, a resulting white powder was precipitated by centrifugation, and diethyl ether was removed by decantation was repeated three times to afford a crude product of peptide. The crude product obtained was purified by preparative LCMS (separation condition A). The eluate was fractionated using test tubes, and eluted fractions containing the target product were collected and lyophilized to afford the title compound (30 mg) as a white powder.

Example 4 Synthesis of compound of compound No. 587:

(*N*-{5-[(4-methylphenyl)ethynyl]thiophene-2-sulfonyl}-β-alanyl-L-asparaginyl-L-α-aspartyl-L-alanyl-L-leucyl-*N*-methyl-L-methionyl-L-prolinamide)

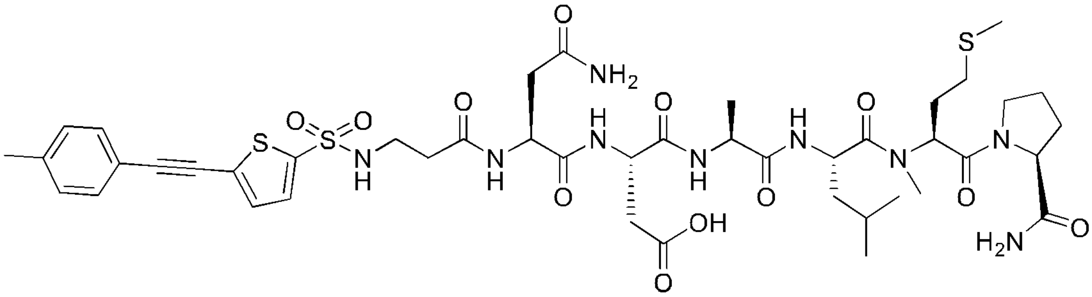

(1) Fmoc-NH-SAL-PEG resin (0.10 mmol) was treated with DMF solution of piperidine (concentration: 40%, 1.6 mL) for 3 minutes, and then treated with DMF solution of piperidine (concentration: 20%, 1.6 mL) for 12 minutes to deprotect the Fmoc on the resin. Subsequently, a solution of Fmoc-Pro-OH (0.40 mmol) in DMF (0.8 mL) solution, a solution of COMU (0.40 mmol) and Oxyma (0.40 mmol) in DMF (0.8 mL), and a solution of DIPEA (0.80 mmol) in NMP (0.4 mL) were added, and the mixture was shaken at room temperature for 40 minutes to introduce a Pro residue. In the same manner, deprotection for Fmoc and condensation were repeated: specifically, Fmoc-(N-Me)Met-OH, Fmoc-Leu-OH, Fmoc-Ala-OH, Fmoc-Asp(OtBu)-OH, Fmoc-Asn(Trt)-OH, and Fmoc-β-Ala-OH were sequentially condensed, wherein deprotection was performed for the N-terminal Fmoc formed in the resin after each condensation by piperidine/DMF treatment in the above manner, and H-β-Ala-Asn(Trt)-Asp(OtBu)-Ala-Leu-(N-Me)Met-Pro-NH-SAL-PEG resin was synthesized.

(2) To the resin obtained in (1), a solution of 5-bromothiophene-2-sulfonyl chloride (0.20 mmol) and DIPEA (0.40 mmol) in DMF (3 mL) was added, and the mixture was shaken at room temperature for 2 hours. After the completion of the reaction, the resulting resin was washed with DMF (3 mL × three times) and chloroform (3 mL × three times).

(3) To the resin obtained in (2), TFA:water:triisopropylsilane:dithiothreitol (90:2.5:5:2.5, 4 mL) was added, the mixture was shaken at room temperature for 2 hours, and the resin was removed through filtration. An operation in which cooled diethyl ether was added to the filtrate, a resulting white powder was precipitated by centrifugation, and diethyl ether was removed by decantation was repeated three times to afford a crude product of peptide. The crude product obtained was purified by preparative LCMS (separation condition B). The eluate was fractionated using test tubes, and eluted fractions containing the target product were collected and lyophilized to afford
5-Br-thiophene-2-$SO_2$-β-Ala-Asn-Asp-Ala-Leu-(N-Me)Met-Pro-$NH_2$ (27 mg) as a white powder.

(4) The compound obtained in (3) was dissolved in DMF (0.5 mL), 4-ethinyltoluene (0.30 mmol), dichlorobis(triphenylphosphine)palladium(II) (0.01 mmol), triethylamine (0.18 mmol), and copper iodide (0.01 mmol) were added thereto, and the mixture was stirred under nitrogen atmosphere at 50°C for 5 hours with heating. After allowing to cool to room temperature, the reaction solution was diluted with DMSO, filtered, and purified by preparative LCMS (separation condition A). The eluate was fractionated using test tubes, and eluted fractions containing the target product were collected and lyophilized to afford the title compound (4 mg) as a white powder.

Example 5 Synthesis of compound of compound No. 608:

(*N*-[(2*S*)-4-amino-1-({(2*S*)-1-[{(2*S*,3*S*)-1-[(2*S*)-2-(2-amino-2-oxoethyl)pyrrolidin-1-yl]-3-met hyl-1-oxopentan-2-yl}(methyl)amino]-4-methyl-1-oxopentan-2-yl}amino)-1-oxobutan-2-yl]-*N*[2]-[4-(1-phenyl-1*H*-1,2,3-triazol-4-yl)benzene-1-sulfonyl]-D-glutamine)

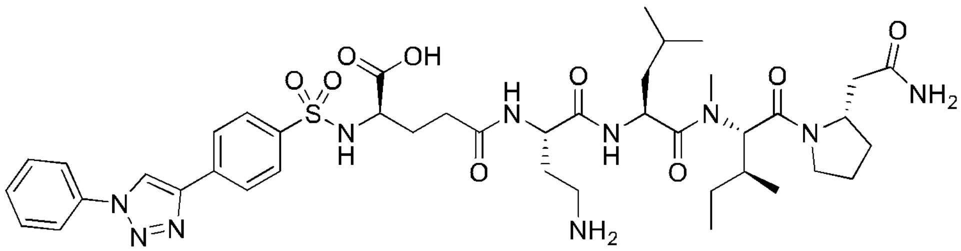

(1) Fmoc-Rink Amide AM resin (0.10 mmol) was treated with DMF solution of piperidine (concentration: 40%, 1.6 mL) for 3 minutes, and then treated with DMF solution of piperidine (concentration: 20%, 1.6 mL) for 12 minutes to deprotect the Fmoc on the resin. Subsequently, a solution of Fmoc-β-homoPro-OH (0.40 mmol) in DMF (0.8 mL), a solution of COMU (0.40 mmol) and Oxyma (0.40 mmol) in DMF (0.8 mL), and a solution of DIPEA (0.80 mmol) in NMP (0.4 mL) were added, and the mixture was shaken at room temperature for 40 minutes to introduce a β-homoPro residue. In the same manner, deprotection for Fmoc and condensation were repeated: specifically, Fmoc-(N-Me)Ile-OH, Fmoc-Leu-OH, Fmoc-Dab(Boc)-OH, and Fmoc-(d)-Glu-OtBu were sequentially condensed, wherein deprotection was performed for the N-terminal Fmoc formed in the resin after each condensation by piperidine/DMF treatment in the above manner, and H-γ-(d)-Glu(OtBu)-Dab(Boc)-Leu-(N-Me)Ile-β-homoPro-Rink Amide AM resin was synthesized.

(2) To the resin obtained in (1), a solution of 4-bromobenzenesulfonyl chloride (0.30 mmol) and DIPEA (0.60 mmol) in DMF (2 mL) was added, and the mixture was shaken at room temperature for 2 hours. After the completion of the reaction, the resulting resin was washed with DMF (2 mL × three times).

(3) To the resin obtained in (2), a solution of trimethylsilylacetylene (0.30 mmol), dichlorobis(triphenylphosphine)palladium(II) (0.03 mmol), and copper iodide (0.03 mmol) in DMF (2 mL) was added, and the mixture was stirred under microwave irradiation at 80°C for 30 minutes with heating. After the completion of the reaction, the resulting resin was washed with DMF (2 mL × three times) and chloroform (2 mL × three times).

To the resin obtained, tetrahydrofuran solution of tetrabutylammonium fluoride (concentration: 0.33 mol/L, 0.50 mmol) was added, and the mixture was shaken at room temperature for 1 hour. After the completion of the reaction, the resulting resin was washed with DMF (2 mL × three times) and chloroform (2 mL × three times).

(4) To the resin obtained in (3), a solution of azidobenzene (0.20 mmol), copper(II) sulfate pentahydrate (0.40 mmol), and ascorbic acid (0.40 mmol) in water (2 mL) and tBuOH (1 mL) was added, and the mixture was stirred under microwave irradiation at 60°C for 1 hour with heating. After the completion of the reaction, the resulting resin was washed with DMF (2 mL × three times) and chloroform (2 mL × three times).

(5) To the resin obtained in (4), TFA:water:triisopropylsilane (92.5:2.5:5, 4 mL) was added, the mixture was shaken at room temperature for 2 hours, and the resin was removed through filtration. An operation in which cooled diethyl ether was added to the filtrate, a resulting white powder was precipitated by centrifugation, and diethyl ether was removed by decantation was repeated three times to afford a crude product of peptide. The crude product obtained was purified by preparative LCMS (separation condition A). The eluate was fractionated using test tubes, and eluted fractions containing the target product were collected and lyophilized to afford the title compound (12 mg) as a white powder.

Example 6 Synthesis of compound of compound No. 634:

(*N*2-(4'-acetyl[1,1'-biphenyl]-4-sulfonyl)--*N*-[(2*S*)-1-({(2*S*)-1-[{(2*S*,3*S*)-1-[(2*S*)-2-(2-amino-2-oxoethyl)pyrrolidin-1-yl]-3-methyl-1-oxopentan-2-yl}(methyl)amino]-4-methyl-1-oxopentan -2-yl}amino)-1-oxopropan-2-yl]-D-asparagine)

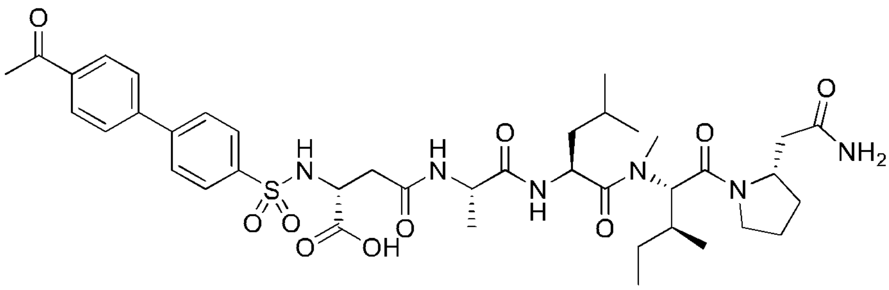

(1) Fmoc-NH-SAL-PEG resin (0.10 mmol) was treated with DMF solution of piperidine (concentration: 40%, 1.6 mL) for 3 minutes, and then treated with DMF solution of piperidine (concentration: 20%, 1.6 mL) for 12 minutes to deprotect the Fmoc on the resin. Subsequently, a solution of Fmoc-β-homoPro-OH (0.40 mmol) in DMF (0.8 mL), a solution of COMU (0.40 mmol) and Oxyma (0.40 mmol) in DMF (0.8 mL), and a solution of DIPEA (0.80 mmol) in NMP (0.4 mL) were added, and the mixture was shaken at room temperature for 40 minutes to introduce a β-homoPro residue. In the same manner, deprotection for Fmoc and condensation were repeated: specifically, Fmoc-(N-Me)Ile-OH, Fmoc-Leu-OH, Fmoc-Ala-OH, and Fmoc-(d)-Asp-OtBu were sequentially condensed, wherein deprotection was performed for the N-terminal Fmoc formed in the resin after each condensation by piperidine/DMF treatment in the above manner, and H-β-(d)-Asp(OtBu)-Ala-Leu-(N-Me)Ile-β-homoPro-NH-SAL-PEG resin was synthesized.

(2) To the resin obtained in (1), a solution of 4-iodobenzenesulfonyl chloride (0.30 mmol) and DIPEA (0.60 mmol) in DMF (2 mL) was added, and the mixture was shaken at room temperature for 2 hours. After the completion of the reaction, the resulting resin was washed with DMF (2 mL × three times) and chloroform (2 mL × three times).

(3) To the resin obtained in (2), a solution of 4-acetylphenylboronic acid (0.40 mmol), tetrakis(triphenylphosphine)palladium(0) (0.03 mmol), and potassium phosphate (0.50 mmol) in 1,4-dioxane (1.5 mL) and water (1.5 mL) was added, and the mixture was stirred under microwave irradiation at 100°C for 30 minutes with heating. After the completion of the reaction, the resulting resin was washed with DMF (2 mL × three times) and chloroform (2 mL × three times).

(4) To the resin obtained in (3), TFA:water:triisopropylsilane (92.5:2.5:5, 4 mL) was added, the mixture was shaken at room temperature for 2 hours, and the resin was removed through filtration. An operation in which cooled diethyl ether was added to the filtrate, a resulting white powder was precipitated by centrifugation, and diethyl ether was removed by decantation was repeated three times to afford a crude product of peptide. The crude product obtained was purified by preparative LCMS (separation condition A). The eluate was fractionated using test tubes, and eluted fractions containing the target product were collected and lyophilized to afford the title compound (8 mg) as a white powder.

Example 7 Synthesis of compound of compound No. 692:

(*N*-[(2*S*)-3-amino-1-({(2*S*)-1-[{(2*S*,3*S*)-1-[(2*S*)-2-(2-amino-2-oxoethyl)pyrrolidin-1-yl]-3-met hyl-1-oxopentan-2-yl}(methyl)amino]-4-methyl-1-oxopentan-2-yl}amino)-1-oxopropan-2-yl ]-$N^2$-[4-(4-methoxybenzamido)benzene-1-sulfonyl]-D-asparagine)

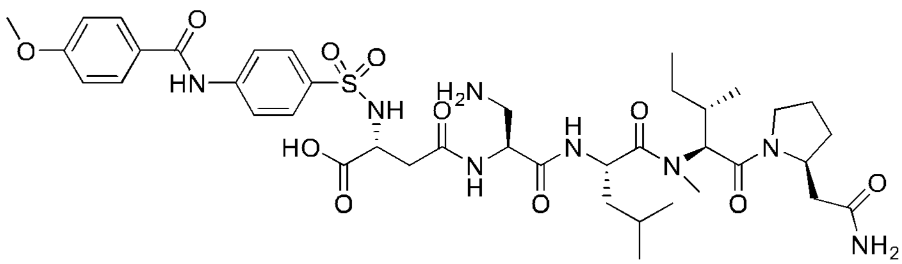

(1) Fmoc-Rink Amide AM resin (0.10 mmol) was treated with DMF solution of piperidine (concentration: 40%, 1.6 mL) for 3 minutes, and then treated with DMF solution of piperidine (concentration: 20%, 1.6 mL) for 12 minutes to deprotect the Fmoc on the resin. Subsequently, a solution of Fmoc-β-homoPro-OH (0.40 mmol) in DMF (0.8 mL), a solution of COMU (0.40 mmol) and Oxyma (0.40 mmol) in DMF (0.8 mL), and a solution of DIPEA (0.80 mmol) in NMP (0.4 mL) were added, and the mixture was shaken at room temperature for 40 minutes to introduce a β-homoPro residue. In the same manner, deprotection for Fmoc and condensation were repeated: specifically, Fmoc-(N-Me)Ile-OH, Fmoc-Leu-OH, Fmoc-Dap(Boc)-OH, and Fmoc-(d)-Asp-OtBu were sequentially condensed, wherein deprotection was performed for the N-terminal Fmoc formed in the resin after each condensation by piperidine/DMF treatment in the above manner, and H-γ-(d)-Asp(OtBu)-Dap(Boc)-Leu-(N-Me)Ile-β-homoPro-Rink Amide AM resin, was synthesized.

(2) To the resin obtained in (1), a solution of (9H-fluoren-9-yl)methyl (4-(chlorosulfonyl)phenyl)carbamate (0.30 mmol) and DIPEA (0.60 mmol) in 1,4-dioxane (3 mL) was added, and the mixture was shaken at room temperature for 1 hour. After the completion of the reaction, the resulting resin was washed with DMF (3 mL × three times). Subsequently, DMF solution of piperidine (concentration: 20%, 3 mL) and Oxyma (0.01 mmol) were added, and the resultant was shaken at room temperature for 10 minutes.

(3) To the resin obtained in (2), a solution of 4-methoxybenzoyl chloride (0.30 mmol) and DIPEA (0.60 mmol) in 1,4-dioxane (2 mL) was added, and the mixture was shaken at room temperature for 3 hours. After the completion of the reaction, the resulting resin was washed with chloroform (3 mL × three times).

(4)To the resin obtained in (3), TFA:water:triisopropylsilane:dithiothreitol (90:2.5:5:2.5, 4 mL) was added, the mixture was shaken at room temperature for 2 hours, and the resin was removed through filtration. An operation in which cooled diethyl ether was added to the filtrate, a resulting white powder was precipitated by centrifugation, and diethyl ether was removed by decantation was repeated three times to afford a crude product of peptide. The crude product obtained was purified by preparative LCMS (separation condition A). The eluate was fractionated using test tubes, and eluted fractions containing the target product were collected and lyophilized to afford the title compound (12 mg) as a white powder.

Example 8 Synthesis of compound of compound No. 892:

([(2*S*,5*S*,8*S*,20*S*)-8-[{(2*S*,3*S*)-1-[(2*S*)-2-(2-amino-2-oxoethyl)pyrrolidin-1-yl]-3-methyl-1-oxo pentan-2-yl}(methyl)carbamoyl]-5-methyl-3,6,14,17,21-pentaoxo-20-{4-[(4-phenoxybenzene -1-sulfonyl)amino]butanamido}-1,4,7,13,16-pentaazacyclohenicosan-2-yl]acetic acid)

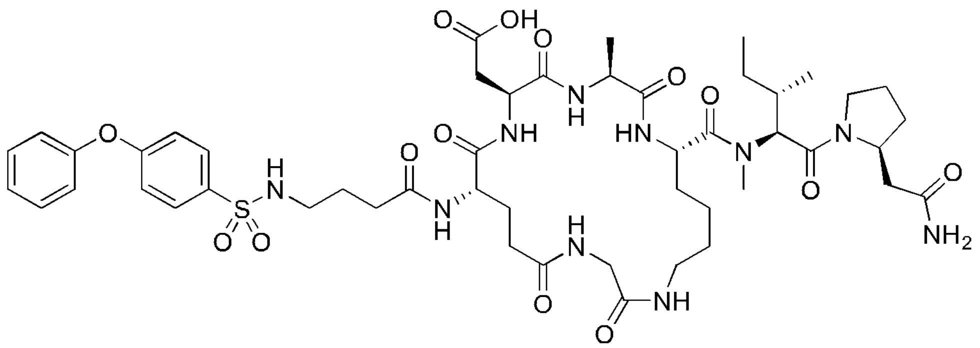

(1) Fmoc-NH-SAL-PEG resin (0.12 mmol) was treated with DMF solution of piperidine (concentration: 40%, 1.9 mL) for 3 minutes, and then treated with DMF solution of piperidine (concentration: 20%, 1.9 mL) for 12 minutes to deprotect the Fmoc on the resin. Subsequently, a solution of Fmoc-β-homoPro-OH (0.48 mmol) in DMF (1.0 mL), a solution of COMU (0.48 mmol) and Oxyma (0.48 mmol) in DMF (1.0 mL), and a solution of DIPEA (0.96 mmol) in NMP (0.48 mL) were added, and the mixture was shaken at room temperature for 40 minutes to introduce a β-homoPro residue. In the same manner, deprotection for Fmoc and condensation were repeated: specifically, Fmoc-(N-Me)Ile-OH, Fmoc-Lys(Dde)-OH, Fmoc-Ala-OH, Fmoc-Asp(OtBu)-OH, Fmoc-Glu(OAllyl)-OH, and Fmoc-GABA-OH were sequentially condensed, wherein deprotection was performed for the N-terminal Fmoc formed in the resin after each condensation by piperidine/DMF treatment in the above manner, andH-GABA-Glu(OAllyl)-Asp(OtBu)-Ala-Lys(Dde)-(N-Me)Ile-β-homoPro-SAL-PEG resin was synthesized.

(2) To the resin obtained in (1), a solution of 4-phenoxybenzenesulfonyl chloride (0.36 mmol) and DIPEA (0.72 mmol) in DMF (3 mL) was added, and the mixture was shaken at room temperature for 1.5 hours. After the completion of the reaction, the resulting resin was washed with DMF (3 mL × three times).

(3) To the resin obtained in (2), DMF solution of hydrazine monohydrate (concentration: 5%, 3 mL) and allyl alcohol (3.1 mmol) were added, and the mixture was shaken at room temperature for 30 minutes to deprotect the Dde of the Lys side chain. Subsequently, a solution of Fmoc-Gly-OH (0.48 mmol), COMU (0.48 mmol), Oxyma (0.48 mmol), and DIPEA (0.96 mmol) in DMF (3 mL) was added, and the mixture was shaken at room temperature for 30 minutes to introduce a Gly residue. After the completion of the reaction, the resulting resin was washed with DMF (3 mL × three times) and chloroform (3 mL × three times).

(4) To the resin obtained in (3), a solution of tetrakis(triphenylphosphine)palladium(0) (0.12 mmol) and phenylsilane (0.60 mmol) in chloroform (4 mL) was added, and the mixture was shaken at room temperature for 1.5 hours to deprotect the Allyl of the Glu side chain. Subsequently, DMF solution of piperidine (concentration: 20%, 3 mL) was added, and the mixture was shaken at room temperature for 30 minutes to deprotect the Fmoc. After the completion of the reaction, the resulting residue was washed with DMF (3 mL × five times).

(5) To the resin obtained in (4), a solution of PyBOP (0.36 mmol) and DIPEA (0.12 mmol) in DMF (4 mL) was added, and the mixture was shaken at room temperature for 2 hours. After the completion of the reaction, the resulting residue was washed with DMF (3 mL × three times) and chloroform (3 mL × three times).

(6) To the resin obtained in (5), TFA:water:triisopropylsilane:dithiothreitol (90:2.5:5:2.5, 4 mL) was added, the mixture was shaken at room temperature for 2 hours, and the resin was removed through filtration. An operation in which

cooled diethyl ether was added to the filtrate, a resulting white powder was precipitated by centrifugation, and diethyl ether was removed by decantation was repeated three times to afford a crude product of peptide. The crude product obtained was purified by preparative LCMS (separation condition A). The eluate was fractionated using test tubes, and eluted fractions containing the target product were collected and lyophilized to afford the title compound (10 mg) as a white powder.

Example 9 Synthesis of compound of compound No. 903:

((2*S*,5*S*,32*R*)-2-{[(2*S*)-1-({(2*S*)-1-[{(2*S*,3*S*)-1-[(2*S*)-2-(2-amino-2-oxoethyl)pyrrolidin-1-yl]-3 -methyl-1-oxopentan-2-yl}(methyl)amino]-4-methyl-1-oxopentan-2-yl}amino)-1-oxopropan-2-yl]carbamoyl}-4,11,20,29,34-pentaoxo-5-{4-[(4-phenoxybenzene-1-sulfonyl)amino]butana mido}-13,16,22,25-tetraoxa-3,10,19,28,33-pentaazaoctatetracontane-1,32,48-tricarboxylic acid)

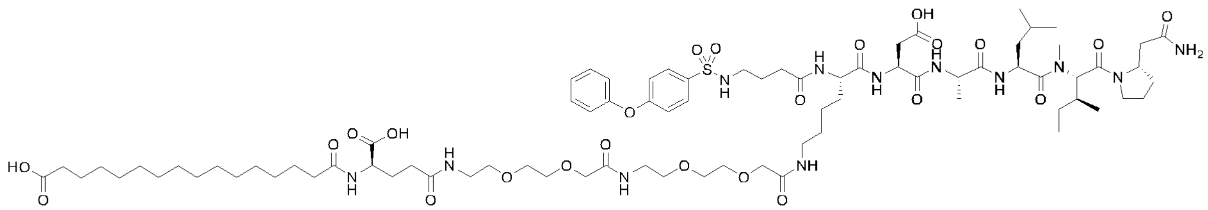

(1) Fmoc-Rink Amide AM resin (0.12 mmol) was treated with DMF solution of piperidine (concentration: 40%, 1.9 mL) for 3 minutes, and then treated with DMF solution of piperidine (concentration: 20%, 1.9 mL) for 12 minutes to deprotect the Fmoc on the resin. Subsequently, a solution of Fmoc-β-homoPro-OH (0.48 mmol) in DMF (1.0 mL), a solution of COMU (0.48 mmol) and Oxyma (0.48 mmol) in DMF (1.0 mL), and a solution of DIPEA (0.96 mmol) in NMP (0.48 mL) were added, and the mixture was shaken at room temperature for 40 minutes to introduce a β-homoPro residue. In the same manner, deprotection for Fmoc and condensation were repeated: specifically, Fmoc-(N-Me)Ile-OH, Fmoc-Leu-OH, Fmoc-Ala-OH, Fmoc-Asp(OtBu)-OH, Fmoc-Lys(Dde)-OH, and Fmoc-GABA-OH were sequentially condensed, wherein deprotection was performed for the N-terminal Fmoc formed in the resin after each condensation by piperidine/DMF treatment in the above manner, and H-GABA-Lys(Dde)-Asp(OtBu)-Ala-Leu-(N-Me)Ile-β-homoPro-Rink Amide AM resin was synthesized.

(2) To the resin obtained in (1), a solution of 4-phenoxybenzenesulfonyl chloride (0.36 mmol) and DIPEA (0.72 mmol) in DMF (3 mL) was added, and the mixture was shaken at room temperature for 2 hours. After the completion of the reaction, the resulting resin was washed with DMF (3 mL × three times).

(3) To the resin obtained in (2), DMF solution of hydrazine monohydrate (concentration: 5%, 3 mL) was added, and the mixture was shaken at room temperature for 30 minutes. This operation was repeated four times to deprotect the Dde of the Lys side chain. For the resulting resin, condensation and deFmoc were repeatedly performed: specifically, Fmoc-Adox-OH, Fmoc-Adox-OH, and Fmoc-Glu-OtBu were sequentially condensed to extend the Lys side chain with a peptide. Deprotection was performed for the N-terminal Fmoc of the Lys side chain by piperidine/DMF treatment.

(4)To the resin obtained in (3), a solution of hexadecanedioic acid (0.96 mmol), COMU (0.18 mmol), Oxyma (0.18 mmol), and DIPEA (0.96 mmol) in DMF (3 mL) was added, and the mixture was shaken at room temperature for 1 hour. After the completion of the reaction, the resulting resin was washed with DMF (3 mL × four times) and chloroform (3 mL × three times).

(5) To the resin obtained in (4), TFA:water:triisopropylsilane:dithiothreitol (90:2.5:5:2.5, 4 mL) was added, the mixture was shaken at room temperature for 2 hours, and the resin was removed through filtration. An operation in which cooled diethyl ether was added to the filtrate, a resulting white powder was precipitated by centrifugation, and diethyl ether was removed by decantation was repeated three times to afford a crude product of peptide. The crude product obtained was purified by preparative LCMS (separation condition A). The eluate was fractionated using test tubes, and eluted fractions containing the target product were collected and lyophilized to afford the title compound (29 mg) as a white powder.

The structures of compounds represented by formula [I'-1], which were synthesized in Example 1 or with the same method as in Example 1, are shown in the following table.

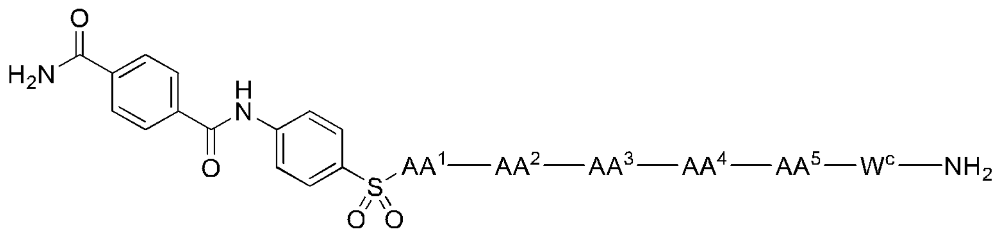

[I'-1]

[Table 5]

| Compound No. | AA1 | AA2 | AA3 | AA4 | AA5 | W6 |
|---|---|---|---|---|---|---|
| 1 | γ-(d)-Glu | (2S, 4S) - (4-anino)Pro | Leu | (N-Me)Glu | Ape | single bond |
| 2 | β-Asp | (2S, 4S)-(4-amino)Pro | Leu | (N-Me)Glu | Ape | single bond |
| 3 | β-(d)-Asp | (2S, 4S)-(4-amino)Pro | Lett | (N-Me)Glu | Ape | single bond |
| 4 | γ-Glu | (2S, 4S)-(4-amino)Pro | Leu | (N-Me)Glu | Ape | single bond |
| 5 | γ-(d)-Glu | (2S, 4S)-(4-amino)Pro | Val | (N-Me)Glu | Ape | single bond |
| 6 | γ-(d)-Glu | (2S, 4S)-(4-amino)Pro | Ile | (N-Me)Glu | Ape | single bond |
| 7 | γ-(d)-Glu | (2S, 4S)-(4-amino)Pro | Phe | (N-Me)Glu | Ape | single bond |
| 8 | γ-(d)-Glu | (2S, 4S)-(4-amino)Pro | Trp | (N-Me)Glu | Ape | single bond |
| 9 | γ-(d)-Glu | (2S, 4S)-(4-amino)Pro | Leu | (N-Me)Ile | Ape | single bond |
| 10 | γ-(d)-Glu | (2S, 4S)-(4-amino)Pro | Leu | (N-Me)Val | Ape | single bond |
| 11 | γ-(d)-Glu | (2S, 4S)-(4-amino)Pro | Leu | (N-Me)Leu | Ape | single bond |
| 12 | γ-(d)-Glu | (2S, 4S)-(4-amino)Pro | Leu | (N-Me)LeU | Ape | single bond |
| 13 | γ-(d)-Glu | (2S, 4S)-(4-amino)Pro | Leu | (N-Me)Phe | Ape | single bond |
| 14 | γ-(d)-Glu | (2S, 4S)-(4-amino)Pro | Leu | (N-Me)Tyr | Ape | single bond |
| 16 | γ-(d)-Glu | (2S, 4S)-(4-amino)Pro | Leu | (N-Me)Ser | Ape | single bond |
| 16 | γ-(d)-Glu | (2S, 4S)-(4-amino)Pro | Leu | Pro | Ape | single bond |
| 17 | γ-(d)-Glu | (2S, 4S)-(4-amino)Pro | Leu | (N-Me)Asp | β-homoPro | single bond |
| 18 | γ-(d)-Glu | (2S, 4S)-(4-amino)Pro | Leu | (N-Me)Asp | Pro | single bond |
| 19 | γ-(d)-Glu | (2S, 4S)-(4-amino)Pro | LeU | (N-Me)Asp | (d)-Pro | single bond |
| 20 | γ-(d)-Glu | (2S, 4S)-(4-amino)Pro | Leu | (N-Me)Glu | β-homoPro | single bond |
| 21 | γ-(d)-Glu | (2S, 4S)-(4-amino)Pro | Leu | (N-Me)Glu | Pro | single bond |
| 22 | γ-(d)-Glu | (2S, 4S)-(4-amino)Pro | Leu | (N-Me)Glu | β-Ala | single bond |
| 23 | γ -(d)-Glu | (2S, 4S)-(4-amino)Pro | Leu | (N-Me)Glu | GABA | single bond |
| 24 | γ-(d)-Glu | (2S, 4S)-(4-amino)Pro | Leu | (N-Me)Glu | Acp | single bond |
| 25 | γ-(4)-Glu | (2S, 4S)-(4-amino)Pro | Leu | (N-Me)Glu | (d)-Pro | single bond |
| 26 | γ-(d)-Glu | (2S, 4S)-(4-amino)Pro | Leu | (N-Me)Glu | Lys | single bond |
| 27 | γ-(d)-Glu | (2S, 4S)-(4-amino)Pro | Leu | (N-Me)Glu | (d)-Lys | single bond |
| 28 | γ-(d)-Glu | -(2S, 4S) - (4-amino)Pro | Leu | (N-Me)Glu | Arg | single bond |
| 29 | γ-(d)-Glu | (2S, 4S)-(4-amino)Pro | Leu | (N-Me)Glu | (d)-Arg | single bond |
| 30 | γ-(d)-Glu | (2S, 4S)-(4-amino)Pro | Leu | (N-Me)Glu | single bond | single bond |

(continued)

| Compound No. | AA1 | AA2 | AA3 | AA4 | AA5 | W6 |
|---|---|---|---|---|---|---|
| **31** | **γ-(d)-Glu** | **(2S, 4S)-(4-amino)Pro** | **Leu** | single bond | single bond | single bond |
| **32** | **γ-(d)-Glu** | **(2S, 4S)-(4-amino)Pro** | **Leu** | **(N-Me)Glu** | **Ape** | **Pro** |
| **33** | **γ-(d)-Glu** | **(2S, 4S)-(4-amino)Pro** | **Leu** | **(N-Me)Glu** | **Ape** | **Lys** |
| **34** | **γ-(d)-Glu** | **(2S, 4S)-(4-amino)Pro** | **Leu** | **(N-Me)Glu** | **Ape** | **(d)-Lys** |
| **35** | **γ-(d)-Glu** | **(2S, 4S)-(4-amino)Pro** | **Leu** | **(N-Me)Glu** | **Ape** | **Arg** |
| **36** | **γ-(d)-Glu** | **(2S, 4S)-(4-amino)Pro** | **Leu** | **(N-Me)Glu** | **Ape** | **(d)-Arg** |
| **37** | **γ-(d)-Glu** | **(2S, 4S)-(4-amino)Pro** | **Leu** | **(N-Me)Glu** | **Ape** | **(d)-Lys-(d)-Lys** |
| **38** | **β-(d)-Asp** | **(2S, 4S)-(4-amino)Pro** | **Leu** | **(N-Me)Ile** | **β-homoPro** | single bond |
| **39** | **γ-(d)-Glu** | **(2S, 4S) - (4-amino) Pro** | **Leu** | **(N-Me)Ile** | **β-homoPro** | single bond |

The structure of a compound represented by formula [I'-2], which was synthesized with the same method as in Example 1, is shown in the following table.

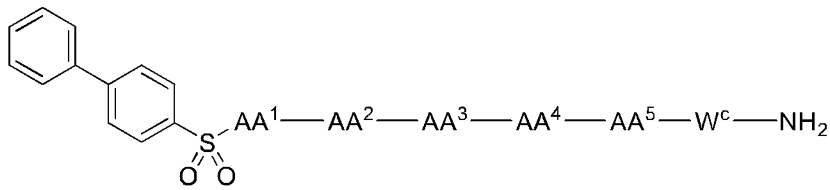

[I'-2]

[Table 6]

| Compound No. | AA1 | AA2 | AA3 | AA4 | AA5 | Wc |
|---|---|---|---|---|---|---|
| **40** | **γ-(d)-Glu** | (2S, 4S)-(4-amino)Pro | Leu | (N-Me)Glu | **Ape** | single bond |

The structure of a compound represented by formula [I'-3], which was synthesized with the same method as in Example 1, is shown in the following table.

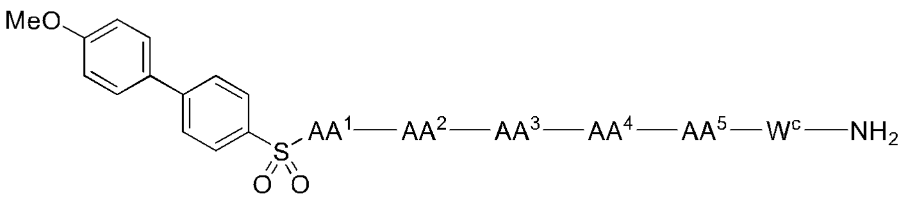

[I'-3]

[Table 7]

| Compound No. | AA1 | AA2 | AA3 | AA4 | AA5 | Wc |
|---|---|---|---|---|---|---|
| **41** | **γ-(d)-Glu** | **(2S, 4S)-(4-amino) Pro** | **Leu** | **(N-Me)Glu** | **Ape** | single bond |

The structure of a compound represented by formula [I'-4], which was synthesized with the same method as in Example 1, is shown in the following table.

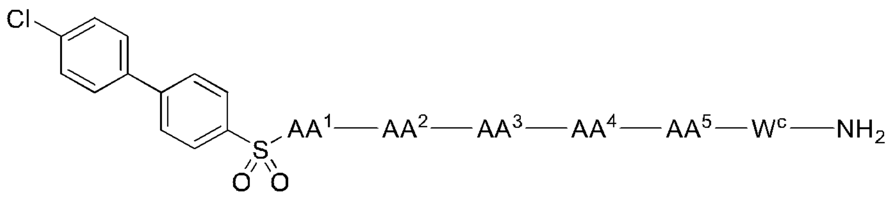

[I'-4]

[Table 8]

| Compound No. | **AA$^1$** | **AA$^2$** | **AA$^3$** | **AA$^1$** | **AA$^5$** | **W$^e$** |
|---|---|---|---|---|---|---|
| **42** | **γ-(d)-Glu** | **(2S, 4S)-(4-amino)Pro** | **Leu** | **(N-Me)Glu** | **Ape** | single bond |

The structure of a compound represented by formula [I'-5], which was synthesized with the same method as in Example 1, is shown in the following table.

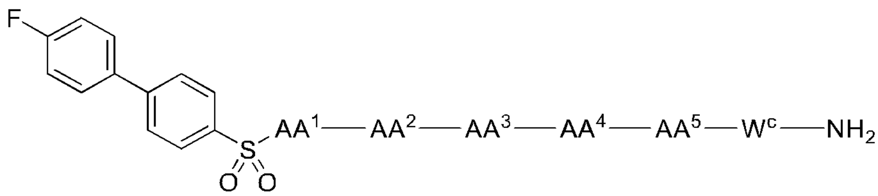

[I'-5]

[Table 9]

| Compound No. | **AA$^1$** | **AA$^2$** | **AA$^3$** | **AA$^4$** | **AA$^5$** | **W$^c$** |
|---|---|---|---|---|---|---|
| **43** | **γ-(d)-Glu** | **(2S, 4S)-(4-amino)Pro** | **Leu** | **(N-Me)Glu** | **Ape** | single bond |

The structure of a compound represented by formula [I'-6], which was synthesized with the same method as in Example 1 is shown in the following table.

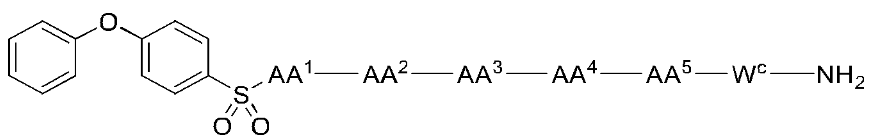

[I'-6]

[Table 10]

| Compound No. | **AA$^1$** | **AA$^2$** | **AA$^3$** | **AA$^4$** | **AA$^5$** | **W$^c$** |
|---|---|---|---|---|---|---|
| **44** | **γ-(d)-Glu** | **(2S, 4S)-(4-amino)Pro** | **Leu** | **(N-Me)Glu** | **Ape** | single bond |

The structures of compounds represented by formula [I'-7], which weer synthesized with the same method as in Example 1, are shown in the following table.

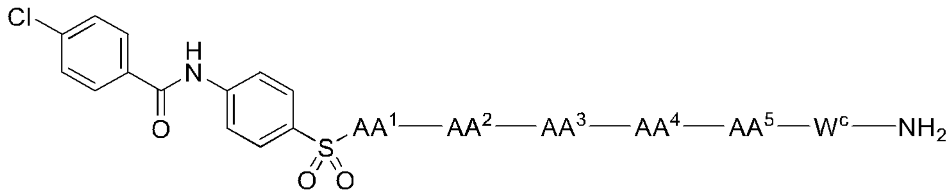

[I'-7]

[Table 11]

| Compound No. | $AA^1$ | $AA^2$ | $AA^3$ | $AA^4$ | $AA^5$ | $W^c$ |
|---|---|---|---|---|---|---|
| **45** | **β-(d)-Asp** | **(2S, 4S)-(4-amino)Pro** | **Leu** | **(N-Me)Ile** | **Ape** | single bond |
| **46** | **β-(d)-Asp** | **(S)-piperazine** | **Leu** | **(N-Me)Ile** | **Ape** | single bond |
| **47** | **β-(d)-Asp** | **(2S, 4R)-(4-amino)Pro** | **Leu** | **(N-Me)Ile** | **Ape** | single bond |
| **48** | **β-(d)-Asp** | **(2S, 4S)-(4-hydroxy)Pro** | **Leu** | **(N-Me)Ile** | **β-homoPro** | single bond |
| **49** | **β-(d)-Asp** | **(2S, 4R)-(4-hydroxy)Pro** | **Leu** | **(N-Me)Ile** | **Ape** | single bond |
| **50** | **β-(d)-Asp** | **(2S, 4S)-(4-amino)Pro** | **Leu** | **(N-Me)Glu** | **β-homoPro** | single bond |
| **51** | **β-(d)-Asp** | **(2S, 4S)-(4-amino)Pro** | **Leu** | **(N-Me)Ile** | **β-homoPro** | single bond |
| **52** | **β-(d)-Asp** | **(2S, 4S)-(4-amino)Pro** | **Leu** | **(N-Me)Glu** | **Ape** | single bond |
| **53** | **γ-(d)-Glu** | **(2S, 4S) - (4-amino)Pro** | **Leu** | **(N-Me) Glu** | **Ape** | single bond |
| **64** | **γ-Glu** | **(2S, 4S)-(4-amino)Pro** | **Leu** | **(N-Me)Ile** | **β-homoPro** | single bond |
| **55** | **γ-(d)-Glu** | **(2S, 4S)-(4-amino)Pro** | **Leu** | **(N-Me)Ile** | **β-homoPro** | single bond |

The structures of compounds represented by formula [I'-8], which were synthesized with the same method as in Example 1, are shown in the following table.

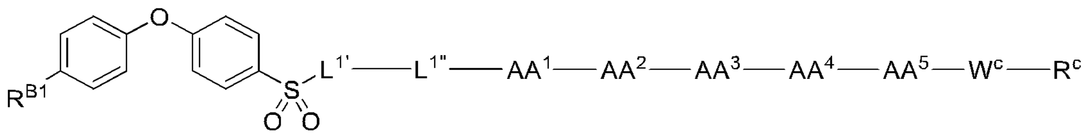

[I'-8]

[Table 12]

| Compound No. | $R^{B1}$ | $L^{1*}$ | $L^{1*}$ | $AA^1$ | $AA^2$ | $AA^3$ | $AA^4$ | $AA^5$ | $W^c$ | $R^c$ |
|---|---|---|---|---|---|---|---|---|---|---|
| **56** | **H** | **GABA** | **Asn** | **Asp** | **Ala** | **Leu** | **Met** | **Pro** | single bond | $NH_2$ |
| **57** | **H** | **GABA** | **Asn** | **Asp** | **Ala** | **Leu** | **(N-Me) Val** | **Pro** | single bond | $NH_2$ |
| **58** | **H** | **GABA** | **Asn** | **Asp** | **Ala** | **Leu** | **(N-Me) Ile** | **Pro** | single bond | $NH_2$ |
| **59** | **H** | **GABA** | **Asn** | **Asp** | **Ala** | **Leu** | **(N-Me)Val** | **β-homo Pro** | single bond | $NH_2$ |
| **60** | **H** | **(N-Me) GABA** | **Asn** | **Asp** | **Ala** | **Leu** | **(N-Me) Ile** | **Pro** | single bond | $NH_2$ |

(continued)

| Compound No. | $R^{B1}$ | $L^{1*}$ | $L^{1*}$ | $AA^1$ | $AA^2$ | $AA^3$ | $AA^4$ | $AA^5$ | $W^c$ | $R^c$ |
|---|---|---|---|---|---|---|---|---|---|---|
| **61** | **H** | **GABA** | **Asn** | **Asp** | **Dap** | **Leu** | **(N-Me) Ile** | **β-homo Pro** | single bond | **$NH_2$** |
| **62** | **H** | **GABA** | **Asn** | **Asp** | **Dab** | **Leu** | **(N-Me) Ile** | **β-homo Pro** | single bond | **$NH_2$** |
| **63** | **H** | **GABA** | **Asn** | **Asp** | **Orn** | **Leu** | **(N-Me) Ile** | **β-homo Pro** | single bond | **$NH_2$** |
| **64** | **H** | **GABA** | **Asn** | **Asp** | **Ala** | **Leu** | **Glu** | **β-homo Pro** | single bond | **$NH_2$** |
| **65** | **H** | **GABA** | **(N-Me) Asn** | **Asp** | **Ala** | **Leu** | **(N-He) Ile** | **Pro** | single bond | **$NH_2$** |
| **66** | **H** | **GABA** | **Asn** | **Asp** | **Ala** | **Leu** | **(N-Me) Glu** | **β-homo Pro** | single bond | **$NH_2$** |
| **67** | **H** | **GABA** | **Asn** | **Asp** | **Dab** | **Leu** | **(N-Me) Ile** | **Pro** | single bond | **$NH_2$** |
| **68** | **H** | **GABA** | **Asn** | **Asp** | **Dab** | **Leu** | **(N-Me) Glu** | **β-homo Pro** | single bond | **$NH_2$** |
| **69** | **H** | **GABA** | **Asn** | **Asp** | **Dab** | **Leu** | **(N-Me) Glu** | **Pro** | single bond | **$NH_2$** |
| **70** | **H** | **GABA** | **(N-Me) Asn** | **Asp** | **Dab** | **Leu** | **(N-Me) Ile** | **β-homo Pro** | single bond | **$NH_2$** |
| **71** | **H** | **GABA** | **(N-Me) Asn** | **Asp** | **Dab** | **Leu** | **(N-Me) Ile** | **Pro** | single bond | **$NH_2$** |
| **72** | **H** | **GABA** | **(N-Me) Asn** | **Asp** | **Dab** | **Leu** | **(N-Me) Glu** | **β-homo Pro** | single bond | **$NH_2$** |
| **73** | **H** | **GABA** | **(N-Me) Asn** | **Asp** | **Dab** | **Leu** | **(N-Me) Glu** | **Pro** | single bond | **$NH_2$** |
| **74** | **H** | **GABA** | **Asn** | **Asp** | **Ala** | **Dab** | **(N-Me) Ile** | **β-homo Pro** | single bond | **$NH_2$** |
| **75** | **H** | **GABA** | **Asn** | **Asp** | **Ala** | **Leu** | **homoGlu** | **β-homo Pro** | single bond | **$NH_2$** |
| **76** | **H** | **GABA** | **Asn** | **Asp** | **Dab** | **Leu** | **(N-Me) Glu** | **β-homo Pro** | single bond | **OH** |
| **77** | **F** | **Ape** | **Glu** | **Asp** | **Dab** | **Leu** | **(N-Me) Glu** | **β-homo Pro** | single bond | **$NH_2$** |

(continued)

| Compound No. | $R^{B1}$ | $L^{1*}$ | $L^{1*}$ | $AA^1$ | $AA^2$ | $AA^3$ | $AA^4$ | $AA^5$ | $W^c$ | $R^c$ |
|---|---|---|---|---|---|---|---|---|---|---|
| **78** | **F** | **Ape** | **Asn** | **Asp** | **Dab** | **Leu** | **(N-Me) Ile** | **β-homo Pro** | single bond | **$NH_2$** |
| **79** | **F** | **Ape** | **Asn** | **Asp** | **Dab** | **Leu** | **(N-Me) Glu** | **β-homo Pro** | single bond | **$NH_2$** |
| **80** | **$H_2NCO$** | **Ape** | **Asn** | **Asp** | **Dab** | **Leu** | **(N-Me) Glu** | **β-homo Pro** | single bond | **$NH_2$** |
| **81** | **$H_2NCO$** | **Ape** | **Gln** | **Asp** | **Dab** | **Leu** | **(N-Me) Glu** | **β-homo Pro** | single bond | **$NH_2$** |
| **82** | **$H_2NCO$** | **Ape** | **(d)-Ser** | **Asp** | **Dab** | **Leu** | **(N-Me) Glu** | **β-homo Pro** | single bond | **$NH_2$** |
| **83** | **$H_2NCO$** | **Ape** | **homo Ser** | **Asp** | **Dab** | **Leu** | **(N-Me) Glu** | **β-homo Pro** | single bond | **$NH_2$** |
| **84** | **H** | **Ape** | **(d)-Thr** | **Asp** | **Dab** | **Leu** | **(N-Me) Glu** | **β-homo Pro** | single bond | **$NH_2$** |
| **85** | **H** | **Ape** | **(d)-Thr** | **Asp** | **Dab** | **Leu** | **(N-Me) Ile** | **β-homo Pro** | single bond | **$NH_2$** |
| **86** | **F** | **Ape** | **(d)-Thr** | **Asp** | **Dab** | **Leu** | **(N-Me) Glu** | **β-homo Pro** | single bond | **$NH_2$** |
| **87** | **F** | **Ape** | **(d)-Thr** | **Asp** | **Dab** | **Leu** | **(N-Me) Ile** | **β-homo Pro** | single bond | **$NH_2$** |
| **88** | **F** | **Ape** | **Arg** | **Asp** | **Dab** | **Leu** | **(N-Me) Glu** | **β-homo Pro** | single bond | **$NH_2$** |
| **89** | **F** | **Ape** | **Arg** | **Asp** | **Dab** | **Leu** | **(N-Me) Ile** | **β-homo Pro** | single bond | **$NH_2$** |
| **90** | **F** | **Ape** | **Gln** | **Asp** | **Dab** | **Leu** | **(N-Me) Ile** | **β-homo Pro** | **(d)-Lys** | **$NH_2$** |
| **91** | **F** | **Ape** | **Gln** | **Asp** | **Dab** | **Leu** | **(N-Me) Ile** | **β-homo Pro** | **(d)-Arg** | **$NH_2$** |

The structures of compounds represented by formula [I'-9], which were synthesized in Example 2 or with the same method as in Example 2, are shown in the following table.

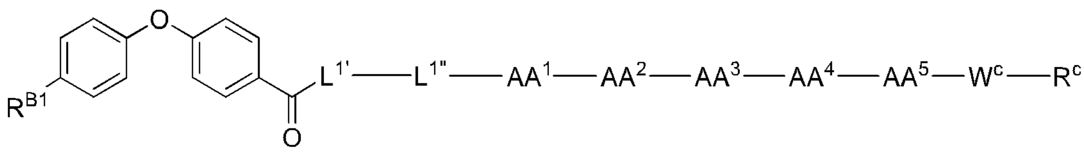

[I'-9]

[Table 13]

| Compound No. | R$^{B1}$ | L$^{1*}$ | L$^{1*}$ | AA$^1$ | AA$^2$ | AA$^3$ | AA$^4$ | AA$^5$ | W$^c$ | R$^c$ |
|---|---|---|---|---|---|---|---|---|---|---|
| **92** | **H** | **GABA** | **(N-Me) Ala** | **Asp** | **Ala** | **Leu** | **(N-Me) Ile** | **Pro** | single bond | **NH$_2$** |
| **93** | **F** | **GABA** | **(N-Me) Ala** | **Asp** | **Ala** | **Leu** | **(N-Me) Ile** | **Pro** | single bond | **NH$_2$** |
| **94** | **H** | **GABA** | **(N-Me) Asn** | **Asp** | **Ala** | **Leu** | **(N-Me) Ile** | **Pro** | single bond | **NH$_2$** |
| **95** | **F** | **GABA** | **(N-Me) Asn** | **Asp** | **Ala** | **Leu** | **(N-Me) Ile** | **Pro** | single bond | **NH$_2$** |
| **96** | **H** | **GABA** | **(N-Me) Ala** | **Asp** | **Ala** | **Leu** | **(N-Me) Ile** | **Aib** | single bond | **NH$_2$** |
| **97** | **F** | **GABA** | **(N-Me) Ala** | **Asp** | **Ala** | **Leu** | **(N-Me) Ile** | **Aib** | single bond | **NH$_2$** |
| **98** | **H** | **GABA** | **(N-Me) Asn** | **Asp** | **Ala** | **Leu** | **(N-Me) Ile** | **Aib** | single bond | **NH$_2$** |
| **99** | **F** | **GABA** | **(N-Me) Asn** | **Asp** | **Ala** | **Leu** | **(N-Me) Ile** | **Aib** | single bond | **NH$_2$** |
| **100** | **H** | **(N-Me) GABA** | **(N-Me) Ala** | **Asp** | **Ala** | **Leu** | **(N-Me) Ile** | **Pro** | single bond | **NH$_2$** |
| **101** | **F** | **(N-Me) GABA** | **(N-Me) Ala** | **Asp** | **Ala** | **Leu** | **(N-Me) Ile** | **Pro** | single bond | **NH$_2$** |
| **102** | **Me** | **(N-Me) GABA** | **(N-Me) Ala** | **Asp** | **Ala** | **Leu** | **(N-Me) Ile** | **Pro** | single bond | **NH$_2$** |
| **103** | **H** | **(N-Me) GABA** | **(N-Me) Asn** | **Asp** | **Ala** | **Leu** | **(N-Me) Ile** | **Pro** | single bond | **NH$_2$** |
| **104** | **F** | **(N-Me) GABA** | **(N-Me) Asn** | **Asp** | **Ala** | **Leu** | **(N-Me) Ile** | **Pro** | single bond | **NH$_2$** |

(continued)

| Compound No. | $R^{B1}$ | $L^{1*}$ | $L^{1*}$ | $AA^1$ | $AA^2$ | $AA^3$ | $AA^4$ | $AA^5$ | $W^c$ | $R^c$ |
|---|---|---|---|---|---|---|---|---|---|---|
| **105** | **Me** | **(N-Me) GABA** | **(N-Me) Asn** | **Asp** | **Ala** | **Leu** | **(N-Me) Ile** | **Pro** | single bond | $NH_2$ |
| **106** | **H** | **GABA** | **Asn** | **Asp** | **Dab** | **Leu** | **(N-Me) Ile** | **$\beta$-homo Pro** | single bond | $NH_2$ |
| **107** | **H** | **GABA** | **Asn** | **Asp** | **Dab** | **Leu** | **(N-Me) Ile** | **Pro** | single bond | $NH_2$ |
| **108** | **H** | **GABA** | **Asn** | **Asp** | **Dab** | **Leu** | **(N-Me) Glu** | **$\beta$-homo Pro** | single bond | $NH_2$ |
| **109** | **H** | **GABA** | **Asn** | **Asp** | **Dab** | **Leu** | **(N-Me) Glu** | **Pro** | single bond | $NH_2$ |
| **110** | **H** | **GABA** | **(N-Me) Asn** | **Asp** | **Dab** | **Leu** | **(N-Me) Ile** | **$\beta$-homo Pro** | single bond | $NH_2$ |
| **111** | **H** | **GABA** | **(N-Me) Asn** | **Asp** | **Dab** | **Leu** | **(N-Me) Ile** | **Pro** | single bond | $NH_2$ |
| **112** | **H** | **GABA** | **(N-Me) Asn** | **Asp** | **Dab** | **Leu** | **(N-Me) Glu** | **$\beta$-homo Pro** | single bond | $NH_2$ |
| **113** | **H** | **GABA** | **(N-Me) Asn** | **Asp** | **Dab** | **Leu** | **(N-Me) Glu** | **Pro** | single bond | $NH_2$ |
| **114** | **H** | **GABA** | **(d) - Asn** | **Asp** | **Ala** | **Leu** | **(N-Me) Ile** | **Pro** | single bond | $NH_2$ |
| **115** | **H** | **GABA** | **(N-Me) Gly** | **Asp** | **Ala** | **Leu** | **(N-Me) Ile** | **Pro** | single bond | $NH_2$ |
| **116** | **H** | **GABA** | **Aze (2)** | **Asp** | **Ala** | **Leu** | **(N-Me) Ile** | **Pro** | single bond | $NH_2$ |
| **117** | **H** | **GABA** | **Pro** | **Asp** | **Ala** | **Leu** | **(N-Me) Ile** | **Pro** | single bond | $NH_2$ |
| **118** | **H** | **GABA** | **homo Pro** | **Asp** | **Ala** | **Leu** | **(N-Me) Ile** | **Pro** | single bond | $NH_2$ |
| **119** | **H** | **GABA** | **(N-Me) Val** | **Asp** | **Ala** | **Lou** | **(N-Me) Ile** | **Pro** | single bond | $NH_2$ |

(continued)

| Compound No. | $R^{B1}$ | $L^{1*}$ | $L^{1*}$ | $AA^1$ | $AA^2$ | $AA^3$ | $AA^4$ | $AA^5$ | $W^c$ | $R^c$ |
|---|---|---|---|---|---|---|---|---|---|---|
| **120** | **H** | **GABA** | **(N-Me) Leu** | **Asp** | **Ala** | **Leu** | **(N-Me) Ile** | **Pro** | single bond | **$NH_2$** |
| **121** | **H** | **GABA** | **(N-Me) Ile** | **Asp** | **Ala** | **Leu** | **(N-Me) Ile** | **Pro** | single bond | **$NH_2$** |
| **122** | **H** | **GABA** | **(N-Me) Met** | **Asp** | **Ala** | **Leu** | **(N-Me) Ile** | **Pro** | single bond | **$NH_2$** |
| **123** | **H** | **GABA** | **(d)-Lys** | **Asp** | **Ala** | **Leu** | **(N-Me) Ile** | **Pro** | single bond | **$NH_2$** |
| **124** | **H** | **GABA** | **(N-Me) Phe** | **Asp** | **Ala** | **Leu** | **(N-Me) Ile** | **Pro** | single bond | **$NH_2$** |
| **126** | **H** | **GABA** | **(N-Me) Asp** | **Asp** | **Ala** | **Leu** | **(N-Me) Ile** | **Pro** | single bond | **$NH_2$** |
| **126** | **H** | **GABA** | **(N-Me) Glu** | **Asp** | **Ala** | **Leu** | **(N-Me) Ile** | **Pro** | single bond | **$NH_2$** |
| **127** | **H** | **GABA** | **(N-Me) Lys** | **Asp** | **Ala** | **Leu** | **(N-Me) Ile** | **Pro** | single bond | **$NH_2$** |
| **128** | **H** | **GABA** | **Gln** | **Asp** | **Ala** | **Leu** | **(N-Me) Ile** | **Pro** | single bond | **$NH_2$** |
| **129** | **H** | **GABA** | **(N-Me) Tyr** | **Asp** | **Ala** | **Leu** | **(N-Me) Ile** | **Pro** | single bond | **$NH_2$** |
| **130** | **H** | **GABA** | **(N-Me) Trp** | **Asp** | **Ala** | **Leu** | **(N-Me) Ile** | **Pro** | single bond | **$NH_2$** |
| **131** | **H** | **GABA** | **(N-Me) Thr** | **Asp** | **Ala** | **Leu** | **(N-Me) Ile** | **Pro** | single bond | **$NH_2$** |
| **132** | **H** | **GABA** | **Aze(2)** | **Asp** | **Dap** | **Leu** | **(N-Me) Ile** | **$\beta$- homo Pro** | single bond | **$NH_2$** |
| **133** | **H** | **GABA** | **Aze (2)** | **Asp** | **Dab** | **Leu** | **(N-Me) Ile** | **$\beta$- homo Pro** | single bond | **$NH_2$** |
| **134** | **H** | **GABA** | **(N-Me) Met** | **Asp** | **Dab** | **Leu** | **(N-Me) Ile** | **Pro** | single bond | **$NH_2$** |

(continued)

| Compound No. | $R^{B1}$ | $L^{1*}$ | $L^{1*}$ | $AA^1$ | $AA^2$ | $AA^3$ | $AA^4$ | $AA^5$ | $W^c$ | $R^c$ |
|---|---|---|---|---|---|---|---|---|---|---|
| **135** | **H** | **GABA** | **(N-Me) Val** | **Asp** | **Dab** | **Leu** | **(N-Me) Ile** | **Pro** | single bond | **$NH_2$** |
| **136** | **H** | **GABA** | **(N-Me) Leu** | **Asp** | **Dab** | **Leu** | **(N-Me) Ile** | **Pro** | single bond | **$NH_2$** |
| **137** | **F** | **GABA** | **(N-Me) Asn** | **Asp** | **Dab** | **Leu** | **(N-Me) Ile** | **Pro** | single bond | **$NH_2$** |
| **138** | **F** | **GABA** | **(N-Me) Asn** | **Asp** | **Dab** | **Leu** | **(N-Me) Ile** | **$\beta$ - homo Pro** | single bond | **$NH_2$** |
| **139** | **F** | **GABA** | **(N-Me) Asn** | **Asp** | **Dab** | **Leu** | **(N-Me) Asp** | **$\beta$ - homo Pro** | single bond | **$NH_2$** |
| **140** | **F** | **GABA** | **(N-Me) Asn** | **Asp** | **Dab** | **Leu** | **(N-Me) Glu** | **$\beta$ - homo Pro** | single bond | **$NH_2$** |
| 141 | F | GABA | (N-Me) Asn | Asp | Dab | Leu | (N-Me) Asp | Pro | single bond | $NH_2$ |
| 142 | F | GABA | (N-Me) Asn | Asp | Dab | Leu | (N-Me) Glu | Pro | single bond | $NH_2$ |
| 143 | H | GABA | Val | Asp | Dab | Leu | (N-Me) Glu | $\beta$ - homo Pro | single bond | $NH_2$ |
| 144 | H | GABA | Glu | Asp | Dab | Leu | (N-Me) Glu | $\beta$ - homo Pro | single bond | $NH_2$ |
| 145 | H | GABA | Orn | Asp | Dab | Leu | (N-Me) Glu | $\beta$ - homo Pro | single bond | $NH_2$ |
| 146 | H | GABA | Val | Asp | Dab | Leu | (N-Me) Ile | $\beta$ - homo Pro | single bond | $NH_2$ |
| 147 | H | GABA | Glu | Asp | Dab | Leu | (N-Me) Ile | $\beta$ - homo Pro | single bond | $NH_2$ |
| 148 | H | GABA | Orn | Asp | Dab | Leu | (N-Me) Ile | $\beta$ - homo Pro | single bond | $NH_2$ |
| 149 | F | GABA | Asn | Asp | Dab | Leu | (N-Me) Glu | $\beta$ - homo Pro | single bond | $NH_2$ |

(continued)

| Compound No. | $R^{B1}$ | $L^{1*}$ | $L^{1*}$ | $AA^1$ | $AA^2$ | $AA^3$ | $AA^4$ | $AA^5$ | $W^c$ | $R^c$ |
|---|---|---|---|---|---|---|---|---|---|---|
| 150 | F | GABA | Val | Asp | Dab | Leu | (N-Me) Glu | β- homo Pro | single bond | $NH_2$ |
| 161 | F | GABA | Glu | Asp | Dab | Leu | (N-Me) Glu | β- homo Pro | single bond | $NH_2$ |
| 152 | F | GABA | Orn | Asp | Dab | Leu | (N-Me) Glu | β- homo Pro | single bond | $NH_2$ |
| 153 | F | GABA | Asn | Asp | Dab | Leu | (N-Me) Ile | β- homo Pro | single bond | $NH_2$ |
| 154 | H | GABA | (N-Me) Asn | Asp | Dap (Me) | Leu | (N-Me) Ile | β- homo Pro | single bond | $NH_2$ |
| 155 | H | GABA | Asn | Asp | Dab | Leu | (N-Me) Glu | β- homo Pro | single bond | $NH_2$ |
| 156 | H | GABA | Asn | Asp | Dab | Leu | (N-Me) Ile | β- homo Pro | single bond | $NH_2$ |

The structures of compounds represented by formula [I'-10], which were synthesized with the same method as in Example 9, are shown in the following table.

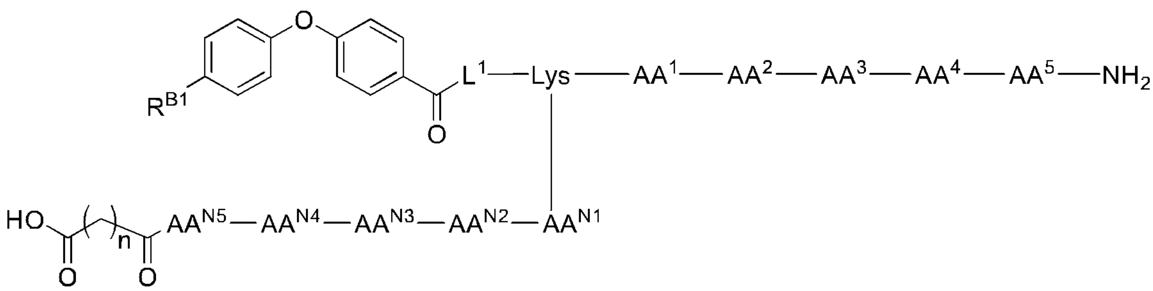

[I'-10]

[Table 14]

| Compound No. | $R^{B1}$ | $L^1$ | $AA^{N5}$ | $AA^{N4}$ | $AA^{N3}$ | $AA^{N2}$ | $AA^{N1}$ | n | $AA^1$ | $AA^2$ | $AA^3$ | $AA^4$ | $AA^5$ |
|---|---|---|---|---|---|---|---|---|---|---|---|---|---|
| 157 | $H_2NCO$ | GABA | $\gamma$ - Glu | Ado x | Ado x | single bond | single bond | 12 | Asp | Dab | Leu | (N-Me) Glu | $\beta$- homoPro |
| 158 | $H_2NCO$ | GABA | $\gamma$ Glu | Ado x | Ado x | single bond | single bond | 14 | Asp | Dab | Leu | (N-Me) Glu | $\beta$- homoPro |
| 159 | $H_2NCO$ | GABA | (d) - Lys | (d) - Lys | (d) - Lys | single bond | single bond | 12 | Asp | Dab | Leu | (N-Me) Glu | $\beta$- homoPro |
| 160 | $H_2NCO$ | GABA | (d) Lys | (d) - Lys | (d) - Lys | single bond | single bond | 14 | Asp | Dab | Leu | (N-Me) Glu | $\beta$- homoPro |

The structures of compounds represented by formula [I'-9], which were synthesized in Example 2 or with the same method as in Example 2, are shown in the following table.

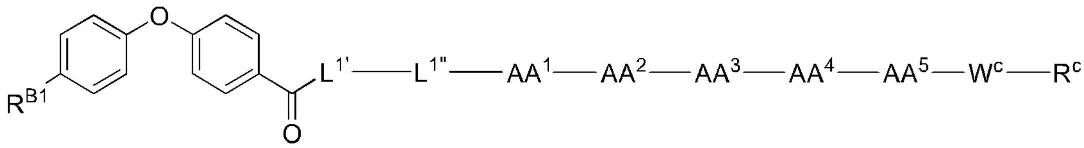

[I'-9]

[Table 15]

| Compound No. | $R^{B1}$ | $L^{1'}$ | $L^{1''}$ | $AA^1$ | $AA^2$ | $AA^3$ | $AA^4$ | $AA^5$ | $W^c$ | $R^c$ |
|---|---|---|---|---|---|---|---|---|---|---|
| 161 | F | GABA | Asn | Asp | Dab | Leu | (N-Me) Glu | $\beta$- homoPro | single bond | OH |
| 162 | F | GABA | Gln | Asp | Dab | Leu | (N-Me) Glu | $\beta$- homoPro | single bond | $NH_2$ |
| 163 | F | Ape | Asn | Asp | Dab | Leu | (N-Me) Glu | $\beta$- homoPro | single bond | $NH_2$ |
| 164 | F | Ape | Gln | Asp | Dab | Leu | (N-Me) Glu | $\beta$- homoPro | single bond | $NH_2$ |
| 165 | F | Ape | Gln | Asp | Dab | Leu | (N-Me) Ile | $\beta$- homoPro | single bond | $NH_2$ |
| 166 | H | Adox | Asn | Asp | Dab | Leu | (N-Me) Ile | $\beta$- homoPro | single bond | $NH_2$ |
| 167 | H | Adox | Asn | Asp | Dab | Leu | (N-Me) Glu | $\beta$- homoPro | single bond | $NH_2$ |
| 168 | $MeSO_2$ | Ape | Asn | Asp | Dab | Leu | (N-Me) Glu | $\beta$- homoPro | single bond | $NH_2$ |
| 169 | $MeSO_2$ | Ape | (d) - Ser | Asp | Dab | Leu | (N-Me) Glu | $\beta$- homoPro | single bond | $NH_2$ |
| 170 | F | Ape | Gln | Asp | Dab | Leu | (N-Me) Ile | $\beta$- homoPro | single bond | $NH_2$ |
| 171 | F | Ape | Glu | Asp | Dab | Leu | (N-Me) Ile | $\beta$- homoPro | single bond | $NH_2$ |
| 172 | F | Ape | (d) - Ser | Asp | Dab | Leu | (N-Me) Ile | $\beta$- homoPro | single bond | $NH_2$ |
| 173 | H | Ape | (d) - Thr | Asp | Dab | Leu | (N-Me) Glu | $\beta$- homoPro | single bond | $NH_2$ |
| 174 | H | Ape | (d) - Thr | Asp | Dab | Leu | (N-Me) Ile | $\beta$- homoPro | single bond | $NH_2$ |
| 175 | F | Ape | Gln | Asp | Dab | Leu | (N-Me) Ile | $\beta$- homoPro | (d) - Lys | NH2 |
| 176 | F | Ape | Gln | Asp | Dab | Leu | (N-Me) Ile | $\beta$- homoPro | (d) - Arg | $NH_2$ |

The structures of compounds represented by formula [I'-9], which were synthesized in Example 3 or with the same method as in Example 3, are shown in the following table.

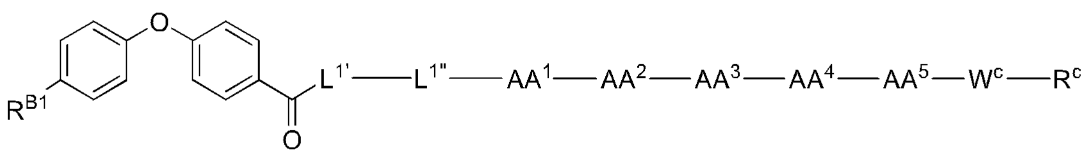

[I'-9]

[Table 16]

| Compound No. | $R^{B1}$ | $L^{1'}$ | $L^{1''}$ | $AA^1$ | $AA^2$ | $AA^3$ | $AA^4$ | $AA^5$ | $W^c$ | $R^c$ |
|---|---|---|---|---|---|---|---|---|---|---|
| 177 | MeHNC O | GABA | Asn | Asp | Ala | Leu | Met | Pro | single bond | $NH_2$ |

(continued)

| Compound No. | $R^{B1}$ | $L^{1'}$ | $L^{1''}$ | $AA^1$ | $AA^2$ | $AA^3$ | $AA^4$ | $AA^5$ | $W^c$ | $R^c$ |
|---|---|---|---|---|---|---|---|---|---|---|
| 178 | $HO_2C-H_2CHN$ CO | Ape | Asn | Asp | Ala | Leu | Met | Pro | single bond | $NH_2$ |
| 179 | $H_2N-(CH_2)_2-NH$ CO | Ape | Asn | Asp | Dab | Leu | (N-Me) Glu | $\beta$-homo Pro | single bond | $HH_2$ |
| 180 | $H_2N-(CH_2)_a-NH$ CO | Ape | Asn | Asp | Dab | Leu | (N-Me) Glu | $\beta$-homo Pro | single bond | $NH_2$ |
| 181 | $H_2N-(CH_2)_4-NH$ CO | Ape | Asn | Asp | Dab | Leu | (N-Me) Glu | $\beta$-homo Pro | single bond | $NH_2$ |
| 182 | $HO-(CH_2)_3-N$ HCO | Ape | Asn | Asp | Dab | Leu | (N-Me) Glu | $\beta$-homo Pro | single bond | $NH_2$ |
| 183 | $HO_2C-(CH_2)_2$ -NHCO | Ape | Asn | Asp | Dab | Leu | (N-Me) Glu | $\beta$-homo Pro | single bond | $NH_2$ |
| 184 | MeNHC O | Ape | Lys | Asp | Dab | Leu | (N-Me) Glu | $\beta$-homo Pro | single bond | $NH_2$ |
| 185 | MeNHC O | Ape | Lys | Asp | Dab | Leu | (N-Me) Ile | $\beta$-homo Pro | single bond | $HH_2$ |
| 186 | MeNHC O | Acp | Lys | Asp | Dab | Leu | (N-Me) Glu | $\beta$-homo Pro | single bond | $NH_2$ |
| 187 | MeNHC O | Acp | Lys | Asp | Dab | Leu | (N-Me) Ile | $\beta$-homo Pro | single bond | $NH_2$ |
| 188 | MeNHC O | Ape | Arg | Asp | Dab | Leu | (N-Me) Glu | $\beta$-homo Pro | single bond | $NH_2$ |
| 189 | MeNHC O | Ape | Arg | Asp | Dab | Leu | (N-Me) Ile | $\beta$-homo Pro | single bond | $NH_2$ |
| 190 | MeNHC O | Acp | Arg | Asp | Dab | Leu | (N-Me) Glu | $\beta$-homo Pro | single bond | $NH_2$ |
| 191 | MeNHC O | Acp | Arg | Asp | Dab | Leu | (N-Me) Ile | $\beta$-homo Pro | single bond | $NH_2$ |
| 192 | EtNHC O | Ape | Lys | Asp | Dab | Leu | (N-Me) Glu | $\beta$-homo Pro | single bond | $NH_2$ |
| 193 | EtNHC O) | Ape | Lys | Asp | Dab | Leu | Of-Me) Ile | $\beta$-homo Pro | single bond | $NH_2$ |
| 194 | EtNHC O | Acp | Lys | Asp | Dab | Leu | (N-Me) Glu | $\beta$-homo Pro | single bond | $NH_2$ |

(continued)

| Compound No. | $R^{B1}$ | $L^{1'}$ | $L^{1''}$ | $AA^1$ | $AA^2$ | $AA^3$ | $AA^4$ | $AA^5$ | $W^c$ | $R^c$ |
|---|---|---|---|---|---|---|---|---|---|---|
| 195 | EtNHC O | Acp | Lys | Asp | Dab | Leu | (N-Me) Ile | β-homo Pro | single bond | $NH_2$ |
| 196 | ETNHC O | Ape | Arg | Asp | Dab | Leu | (N-Me) Glu | β-homo Pro | single bond | $NH_2$ |
| 197 | EtNHC O | Ape | Arg | Asp | Dab | Leu | (N-Me) Lie | βPro | single bond | $NH_2$ |
| 198 | ETNHC O | Acp | Arg | Asp | Dab | Leu | (N-Me) Ile | β-homo Pro | single bond | $NH_2$ |
| 199 | $H_2NCO$ $-(CH_2)_4$-NH CO | Ape | Glu | Asp | Dab | Leu | (N-Me) Glu | β-homo Pro | single bond | $NH_2$ |
| 200 | $HO_2C-(CH_2)_4$ -NHCO | Ape | Glu | Asp | Dab | Leu | (N-Me) Ile | β-homo Pro | single bond | $NH_2$ |
| 201 | $H_2NCO$ $-(CH_2)_4$-NH CO | Ape | Glu | Asp | Dab | Leu | (N-Me) Ile | β-homo **Pro** | single bond | $NH_2$ |

The structure of a compound represented by formula [I'-11], which was synthesized with the same method as in Example 2, is shown in the following table.

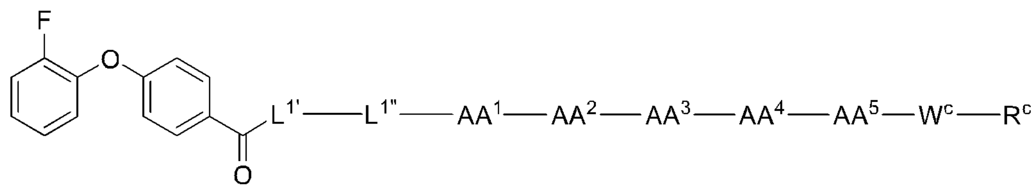

[I'-11]

[Table 17]

| Compound No. | $L^{1'}$ | $L^{1''}$ | $AA^1$ | $AA^2$ | $AA^3$ | $AA^4$ | $AA^5$ | $W^c$ | $R^c$ |
|---|---|---|---|---|---|---|---|---|---|
| 202 | GABA | Asn | Asp | Ala | Leu | Met | Pro | single bond | $NH_2$ |

The structures of compounds represented by formula [I'-12], which were synthesized with the same method as in Example 2, are shown in the following table.

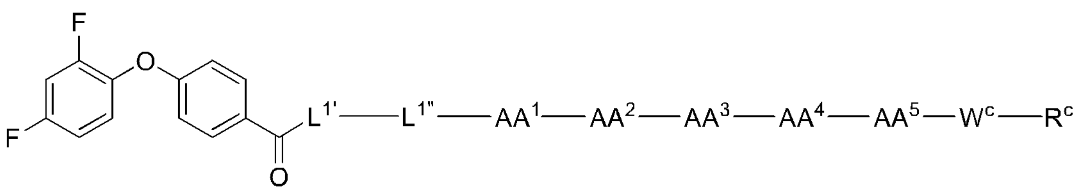

[I'-12]

[Table 18]

| Compound No. | L1' | L1" | AA1 | AA2 | AA3 | AA4 | AA5 | Wc | Rc |
|---|---|---|---|---|---|---|---|---|---|
| 203 | Acp | Asn | Asp | Dab | Leu | (N-Me) Glu | β-homo Pro | single bond | $NH_2$ |
| 204 | Acp | Gln | Asp | Dab | Leu | (N-Me) Glu | β-homo Pro | single bond | $NH_2$ |
| 205 | Acp | Ala | Asp | Dab | Leu | (N-Me) Glu | β-homo Pro | single bond | $NH_2$ |

The structures of compounds represented by formula [I'-13], which were synthesized with the same method as in Example 2, are shown in the following table.

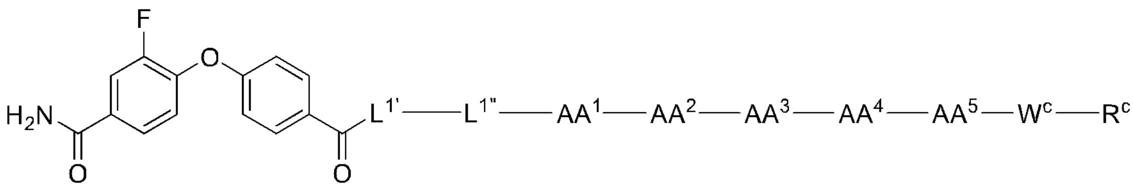

[I'-13]

[Table 19]

| Compound No. | L1' | L1" | AA1 | AA2 | AA3 | AA4 | AA5 | Wc | Rc |
|---|---|---|---|---|---|---|---|---|---|
| 206 | Acp | Asn | Asp | Dab | Leu | (N-Me) Glu | β-homo Pro | single bond | $NH_2$ |
| 207 | Acp | Gln | Asp | Dab | Leu | (N-Me) Glu | β-homo Pro | single bond | $NH_2$ |
| 208 | Acp | Ala | Asp | Dab | Leu | (N-Me) Glu | β-homo Pro | single bond | $NH_2$ |
| 209 | Ape | Asn | Asp | Dab | Leu | (N-Me) Glu | β-homo Pro | single bond | $NH_2$ |

The structures of compounds represented by formula [I'-14], which were synthesized with the same method as in Example 2, are shown in the following table.

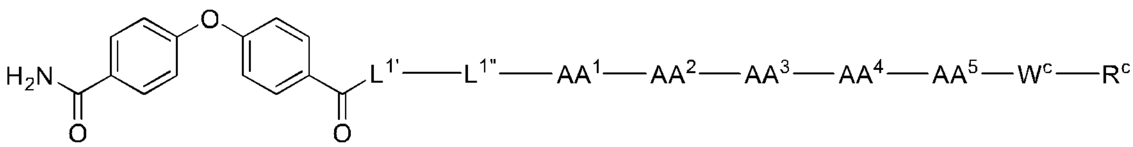

[I'-14]

[Table 20]

| Compound No. | L1' | L1" | AA1 | AA2 | AA3 | AA4 | AA5 | Wc | Rc |
|---|---|---|---|---|---|---|---|---|---|
| 210 | GABA | Asn | Asp | Dab | Leu | (N-Me) Glu | β-homoPro | single bond | $NH_2$ |
| 211 | GABA | Val | Asp | Dab | Leu | (N-Me) Glu | β-homoPro | single bond | $NH_2$ |
| 212 | GABA | Glu | Asp | Dab | Leu | (N-Me) Glu | β-homoPro | single bond | $NH_2$ |

(continued)

| Compound No. | $L^{1'}$ | $L^{1''}$ | $AA^1$ | $AA^2$ | $AA^3$ | $AA^4$ | $AA^5$ | $W^c$ | $R^c$ |
|---|---|---|---|---|---|---|---|---|---|
| 213 | GABA | Orn | Asp | Dab | Leu | (N-Me) Glu | $\beta$-homoPro | single bond | $NH_2$ |
| 214 | GABA | Asn | Asp | Dab | Leu | (N-Me) Ile | $\beta$-homoPro | single bond | $NH_2$ |
| 215 | GABA | Val | Asp | Dab | Leu | (N-Me) Ile | $\beta$-homoPro | single bond | $NH_2$ |
| 216 | GABA | Glu | Asp | Dab | Leu | (N-Me) Ile | $\beta$-homoPro | single bond | $NH_2$ |
| 217 | GABA | Orn | Asp | Dab | Leu | (N-Me) Ile | $\beta$-homoPro | single bond | $NH_2$ |
| 218 | GABA | (N-Me) Asn | Asp | Dap (Me) | Leu | (N-Me) Ile | $\beta$ -homoPro | single bond | $NH_2$ |
| 219 | GABA | (N-Me) Asn | Asp | Dap (Me) | Leu | (N-Me) Glu | $\beta$ -homoPro | single bond | $NH_2$ |
| 220 | GABA | (N-Me) Asn | Asp | Dab (Me) | Leu | (N-Me) Glu | $\beta$ | single bond | $NH_2$ |
| 221 | $\beta$-Ala | Asn | Asp | Dab | Leu | (N-Me) Glu | $\beta$ -homoPro | single bond | $NH_2$ |
| 222 | Ape | Asn | Asp | Dab | Leu | (N-Me) Glu | $\beta$ -homoPro | single bond | $NH_2$ |
| 223 | Ape | Glu | Asp | Dab | Leu | (N-Me) Glu | $\beta$ -homoPro | single bond | $NH_2$ |
| 224 | $\beta$-Ala | Asn | Asp | Dap | Leu | (N-Me) Glu | $\beta$ | single bond | $NH_2$ |
| 225 | GABA | Asn | Asp | Dap | Leu | (N-Me) Glu | $\beta$ | single bond | $NH_2$ |
| 226 | Ape | Asn | Asp | Dap | Leu | (N-Me) Glu | $\beta$ -homoPro | single bond | $NH_2$ |
| 227 | GABA | Glu | Asp | Dap | Leu | (N-Me) Glu | $\beta$ -homoPro | single bond | $NH_2$ |

(continued)

| Compound No. | $L^{1'}$ | $L^{1''}$ | $AA^1$ | $AA^2$ | $AA^3$ | $AA^4$ | $AA^5$ | $W^c$ | $R^c$ |
|---|---|---|---|---|---|---|---|---|---|
| 228 | Ape | Glu | Asp | Dap | Leu | (N-Me) Glu | β -homoPro | single bond | $NH_2$ |
| 229 | β-Ala | Asn | Asp | Dab | Leu | (N-Me) Ile | β -homoPro | single bond | $NH_2$ |
| 230 | Ape | Asn | Asp | Dab | Leu | (N-Me) Ile | | single bond | $NH_2$ |
| 231 | β-Ala | Glu | As p | Dab | Leu | (N-Me) Ile | β -homoPro | single bond | $NH_2$ |
| 232 | Ape | Glu | Asp | Dab | Leu | (N-Me) Ile | β -homoPro | single bond | $NH_2$ |
| 233 | Ape | Gln | Asp | Dab | Leu | (N-Me) Glu | β | single bond | $NH_2$ |
| 234 | Ape | Gln | Asp | Dab | Leu | (N-Me) Ile | β -homoPro | single bond | $NH_2$ |
| 235 | GABA | Asn | Asp | Dab | Leu | (N-Me) Glu | β -homoPro | single bond | OH |
| 236 | GABA | Asn | Asp | Dab | Leu | (N-Me) Ile | β | single bond | OH |
| 237 | GABA | Gln | Asp | Dab | Leu | (N-Me) Glu | β -homoPro | single bond | OH |
| 238 | GABA | (d) - Asn | Asp | Dab | Leu | (N-Me) Glu | homoPro | single bond | OH |
| 239 | GABA | (d)-Asn | Asp | Dab | Leu | (N-Me) Ile | β -homoPro | single bond | OH |
| 240 | GABA | Asn | Asp | Dab | Leu | (N-Me) Asp | β -homoPro | single bond | OH |
| 241 | GABA | Asn | Asp | Dab | Leu | (N-Me) Asn | β -homoPro | single bond | OH |
| 242 | GABA | (d) - Gln | Asp | Dab | Leu | (N-Me) Glu | β -homoPro | single bond | OH |

(continued)

| Compound No. | $L^{1'}$ | $L^{1''}$ | $AA^1$ | $AA^2$ | $AA^3$ | $AA^4$ | $AA^5$ | $W^c$ | $R^c$ |
|---|---|---|---|---|---|---|---|---|---|
| 243 | GABA | Gln | Asp | Dab | Leu | (N-Me) Glu | $\beta$ -homoPro | single bond | $NH_2$ |
| 244 | GABA | Gln | Asp | Dab | Leu | (N-Me) Ile | $\beta$ -homoPro | single bond | $NH_2$ |
| 245 | ε -Lys | Asn | Asp | Dab | Leu | (N-Me) Ile | $\beta$ | single bond | $NH_2$ |
| 246 | ε -Lys | Gln | Asp | Dab | Leu | (N-Me) Ile | $\beta$ | single bond | $NH_2$ |
| 247 | ε - (d) - Lys | Asn | Asp | Dab | Leu | (N-Me) Ile | $\beta$ -homoPro | single bond | $NH_2$ |
| 248 | GABA | Asn | Asp | Dab (Me $)_2$ | Leu | (N-Me) Ile | $\beta$ -homoPro | single bond | $NH_2$ |
| 249 | GABA | Gln | Asp | Dab (Me $)_2$ | Leu | (N-Me) Ile | $\beta$ -homoPro | single bond | $NH_2$ |
| 250 | GABA | (d) - Asn | Asp | Dab | Leu | (N-Me) Glu | $\beta$ -homoPro | single bond | $NH_2$ |
| 251 | GABA | (d)-Asn | Asp | Dab | Leu | (N-Me) Ile | $\beta$ | single bond | $NH_2$ |
| 252 | GABA | Asn | Asp | Dab | Leu | (N-Me) Asp | $\beta$ -homoPro | single bond | $NH_2$ |
| 253 | GABA | (d)-Gln | Asp | Dab | Leu | (N-Me) Glu | $\beta$ -homoPro | single bond | $NH_2$ |
| 254 | Ape | Asn | Asp | Dab | Leu | (N-Me) Ile | $\beta$ | single bond | NHEt |
| 255 | Ape | Asn | Asp | Dab | Leu | (N-Me) Ile | $\beta$ -homoPro | single bond | pipe ridi n-1-yl |
| 256 | Acp | Asn | Asp | Dab | Leu | (N-Me) Glu | $\beta$ -homoPro | single bond | $NH_2$ |
| 257 | Aep | Gln | Asp | Dab | Leu | (N-Me) Glu | $\beta$ | single bond | $NH_2$ |

(continued)

| Compound No. | L1' | L1" | $AA^1$ | $AA^2$ | $AA^3$ | $AA^4$ | $AA^5$ | $W^c$ | $R^c$ |
|---|---|---|---|---|---|---|---|---|---|
| 258 | Acp | Glu | Asp | Dab | Leu | (N-Me) Glu | β -homoPro | single bond | $NH_2$ |
| 259 | Ape | Asn | Asp | Dab | Leu | (N-Me) Glu | β -homoPro | single bond | OH |
| 260 | Ape | Gln | Asp | Dab | Leu | (N-Me) Glu | β -homoPro | single bond | OH |
| 261 | Ape | Gln | Asp | Dab | Leu | (N-Me) Ile | β -homoPro | single bond | OH |
| 262 | Adox | Asn | Asp | Dab | Leu | (N-Me) Ile | β -homoPro | single bond | $NH_2$ |
| 263 | Adox | Asn | Asp | Dab | Leu | (N-Me) Glu | β -homoPro | single bond | $NH_2$ |
| 264 | Adox | Glu | Asp | Dab | Leu | (N-Me) Ile | β | single bond | $NH_2$ |
| 265 | Adox | Glu | Asp | Dab | Leu | (N-Me) Glu | β - honoPro | single bond | $NH_2$ |
| 266 | Adox | Gln | Asp | Dab | Leu | (N-Me) Ile | β - homoPro | single bond | $NH_2$ |
| 267 | Adox | Gln | As p | Dab | Leu | (N-Me) Glu | β -homoPro | single bond | $NH_2$ |
| 268 | Ape | Asn | Asp | Dab | Leu | (N-Me) Glu | β -homoPro | single bond | NHMe |
| 269 | Ape | Asn | Asp | Dab | Leu | (N-Me) Glu | β -homoPro | single bond | azet idin -1-yl |
| 270 | Ape | Asn | Asp | Dab | Leu | (N-Me) Glu | β -homoPro | single bond | pyrr olid in-1-yl |
| 271 | Ape | Asn | Asp | Dab | Leu | (N-Me) Glu | β -homoPro | single bond | $(4\text{-}OH)_p$ iper idin -1-yl |
| 272 | Ape | Asn | Asp | Dab | Leu | (N-Me) Glu | β -homoPro | single bond | $NH\text{-}(CH_2)_2\text{-}OH$ |

(continued)

| Compound No. | L1' | L1" | AA1 | AA2 | AA3 | AA4 | AA5 | Wc | Rc |
|---|---|---|---|---|---|---|---|---|---|
| 273 | Ape | Asn | Asp | Dab | Leu | (N-Me) Ile | β-homoPro | single bond | azet idin -1-yl |
| 274 | Ape | Asn | Asp | Dab | Leu | (N-Me) Ile | β-homoPro | single bond | $(3\text{-OH})_3$ zeti din-1-yl |
| 275 | Ape | Asn | Asp | Dab | Leu | (N-Me) Ile | β-homoPro | single bond | pyrr olid in-1-yl |
| 276 | Ape | Asn | Asp | Dab | Leu | (N-Me) Ile | β-homoPro | single bond | (4-0H) p iper idin -1-yl |
| 277 | Ape | Asn | Asp | Dab | Leu | (N-Me) Ile | β-homoPro | single bond | $NH\text{-}(CH_2)_2\text{-}OH$ |
| 278 | Ape | Glu | Asp | Dab | Leu | (N-Me) Glu | β-homoPro | single bond | $NH_2$ |
| 279 | GABA | (N-Me) Asn | Asp | Dab | Leu | (N-Me) Ile | β-homoPro | single bond | $NH_2$ |
| 280 | GABA | (N-Me) Asn | Asp | Dab | Leu | (N-Me) Glu | β-homoPro | single bond | $NH_2$ |
| 281 | Ape | (N-Me) Asn | Asp | Dab | Leu | (N-Me) Ile | β-homoPro | single bond | $NH_2$ |
| 282 | Ape | (N-Me) Asn | Asp | Dab | Leu | (N-Me) Glu | β-homoPro | single bond | $NH_2$ |
| 283 | Ape | (d) - Asn | Asp | Dab | Leu | (N-Me) Ile | β-homoPro | single bond | $NH_2$ |
| 284 | Ape | (d) - Asn | Asp | Dab | Leu | (N-Me) Glu | β-homoPro | single bond | $NH_2$ |
| 285 | Ape | (d) - Ser | Asp | Dab | Leu | (N-Me) Glu | β-homoPro | single bond | $NH_2$ |
| 286 | Ape | (d) - Ala | Asp | Dab | Leu | (N-Me) Glu | β-homoPro | single bond | $NH_2$ |
| 287 | Ape | Phe | Asp | Dab | Leu | (N-Me) Glu | β-homoPro | single bond | $NH_2$ |

(continued)

| Compound No. | $L^{1'}$ | $L^{1''}$ | $AA^1$ | $AA^2$ | $AA^3$ | $AA^4$ | $AA^5$ | $W^c$ | $R^c$ |
|---|---|---|---|---|---|---|---|---|---|
| 288 | Ape | (d) - Phe | Asp | Dab | Leu | (N-Me) Glu | $\beta$ - homoPro | single bond | $NH_2$ |
| 289 | Ape | Tyr | Asp | Dab | Leu | (N-Me) Glu | $\beta$ - homoPro | single bond | $NH_2$ |
| 290 | Ape | (d) - Tyr | Asp | Dab | Leu | (N-Me) Glu | $\beta$ - homoPro | single bond | $NH_2$ |
| 291 | $\gamma$-Dab | Asn | Asp | Dab | Leu | (N-Me) Glu | $\beta$ - homoPro | single bond | $NH_2$ |
| 292 | $\delta$ - (d) - 0rn | Asn | Asp | Dab | Leu | (N-Me) Glu | $\beta$ - homoPro | single bond | $NH_2$ |
| 293 | GABA | $\beta$-Dap | Asp | Dab | Leu | (N-Me) Glu | $\beta$ - homoPro | single bond | $NH_2$ |
| 294 | GABA | $\beta$ - (d)-Dap | Asp | Dab | Leu | (N-Me) Glu | $\beta$ - homoPro | single bond | $NH_2$ |
| 295 | GABA | $\beta$-Ala | Asp | Dab | Leu | (N-Me) Glu | $\beta$ - homoPro | single bond | $NH_2$ |
| 296 | Ape | Dab | Asp | Dab | Leu | (N-Me) Glu | $\beta$ - homoPro | single bond | $NH_2$ |
| 297 | Ape | Dap | Asp | Dab | Leu | (N-Me) Glu | $\beta$ - homoPro | single bond | $NH_2$ |
| 298 | Ape | (d) - Dab | Asp | Dab | Leu | (N-Me) Glu | $\beta$ - homoPro | single bond | $NH_2$ |
| 299 | $\gamma$ - (d) - Dab | Asn | Asp | Dab | Leu | (N-Me) Glu | $\beta$ - homoPro | single bond | $NH_2$ |
| 300 | GABA | Aze (2) | Asp | Dab | Leu | (N-Me) Ile | $\beta$ - homoPro | single bond | $NH_2$ |
| 301 | GABA | Aze (2) | Asp | Dab | Leu | (N-Me) Glu | $\beta$ - homoPro | single bond | $NH_2$ |
| 302 | Ape | Aze (2) | Asp | Dab | Leu | (N-Me) Ile | $\beta$ - homoPro | single bond | $NH_2$ |

(continued)

| Compound No. | $L^{1'}$ | $L^{1''}$ | $AA^1$ | $AA^2$ | $AA^3$ | $AA^4$ | $AA^5$ | $W^c$ | $R^c$ |
|---|---|---|---|---|---|---|---|---|---|
| 303 | Ape | Aze (2) | Asp | Dab | Leu | (N-Me) Glu | β-homoPro | single bond | $NH_2$ |
| 304 | GABA | (N-Me) Glu | Asp | Dab | Leu | (N-Me) Ile | β-homoPro | single bond | $NH_2$ |
| 305 | GABA | (N-Me) Glu | Asp | Dab | Leu | (N-Me) Glu | β-homoPro | single bond | $NH_2$ |
| 306 | β-Dap | Asn | Asp | Dab | Leu | (N-Me) Glu | β-homoPro | single bond | $NH_2$ |
| 307 | β - (d) - Dap | Asn | Asp | Dab | Leu | (N-Me) Glu | β-homoPro | single bond | $NH_2$ |
| 308 | Ape | (N-Me) Glu | Asp | Dab | Leu | (N-Me) Glu | β-bomoPro | single bond | $NH_2$ |
| 309 | GABA | (N-Me) Ile | Asp | Dab | Leu | (N-Me) Ile | β-homoPro | single bond | $NH_2$ |
| 310 | GABA | (N-Me) Lys | Asp | Dab | Leu | (N-Me) Ile | β-homoPro | single bond | $NH_2$ |
| 311 | GABA | (N-Me) Lys | Asp | Dab | Leu | (N-Me) Glu | β-homoPro | single bond | $NH_2$ |
| 312 | Ape | (N-Me ) Lys | Asp | Dab | Leu | (N-Me) Ile | β-homoPro | single bond | $NH_2$ |
| 313 | Ape | (N-Me) Lys | Asp | Dab | Leu | (N-Me) Glu | β-homoPro | single bond | $NH_2$ |
| 314 | $-NH-(CH_2)_2-0-CH_2-C0-$ | Asn | Asp | Dab | Leu | (N-Me) Ile | β-homoPro | single bond | $NH_2$ |
| 315 | $-NH-(CH_2)_2-0-CH_2-C0-$ | (d) - Ser | Asp | Dab | Leu | (N-Me) Ile | β-homoPro | single bond | $NH_2$ |

The structures of compounds represented by formula [I'-15], which were synthesized with the same method as in Example 2, are shown in the following table.

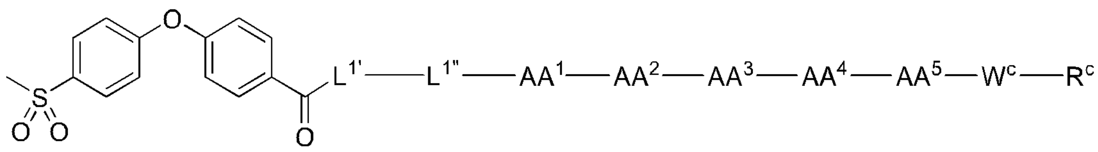

[I'-15]

[Table 21]

| Compound No. | L1- | L1- | AA1 | AA2 | AA3 | AA4 | AA5 | Wc | Rc |
|---|---|---|---|---|---|---|---|---|---|
| 316 | Ape | Asn | Asp | Dab | Leu | (N-Me) Ile | $\beta$- homo Pro | single bond | $NH_2$ |
| 317 | Ape | (d) - Ser | Asp | Dab | Leu | (N-Me) Ile | $\beta$- homo Pro | single bond | $NH_2$ |

The structures of compounds represented by formula [I'-14], which were synthesized with the same method as in Example 2, are shown in the following table.

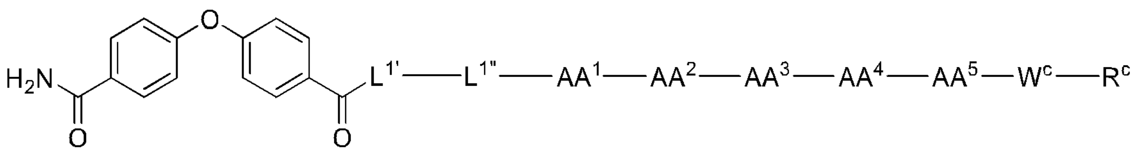

[I'-14]

[Table 22]

| Compound No. | $L^{1"}$ | $L^{1-}$ | $AA^1$ | $AA^2$ | $AA^3$ | $AA^4$ | $AA^5$ | $W^c$ | $R^c$ |
|---|---|---|---|---|---|---|---|---|---|
| 318 | Ape | (N-Me) Glu | Asp | Dab | Leu | (N-Me) lie | β - homoPro | single bond | $NH_2$ |
| 319 | Ape | Thr | Asp | Dab | Leu | (N-Me) Glu | β - homoPro | single bond | $NH_2$ |
| 320 | Ape | (d) - Thr | Asp | Dab | Leu | (N-Me) Glu | β - homoPro | single bond | $NH_2$ |
| 321 | Ape | (d) - Pro | Asp | Dab | Leu | (N-Me) Glu | β - homoPro | single bond | $NH_2$ |
| 322 | Ape | Trp | Asp | Dab | Leu | (N-Me) Glu | β - homoPro | single bond | $NH_2$ |
| 323 | Ape | (N-Me) Leu | Asp | Dab | Leu | (N-Me) Ile | β - homoPro | single bond | $NH_2$ |
| 324 | Ape | (N-Me) Leu | As p | Dab | Leu | (N-Me) Glu | β - homoPro | single bond | $NH_2$ |
| 325 | Ape | (N-Me) Met | As p | Dab | Leu | (N-Me) Ile | β - homoPro | single bond | $NH_2$ |
| 326 | Ape | (N-Me) Met | Asp | Dab | Leu | (N-Me) Glu | β - homoPro | single bond | $NH_2$ |
| 327 | Ape | (d) - Trp | Asp | Dab | Leu | (N-Me) Glu | β - homoPro | single bond | $NH_2$ |
| 328 | Ape | His | Asp | Dab | Leu | (N-Me) Glu | β - homoPro | single bond | $NH_2$ |
| 329 | Ape | (d) - His | Asp | Dab | Leu | (N-Me) Glu | β - homoPro | single bond | $NH_2$ |
| 330 | Ape | Cys | Asp | Dab | Leu | (N-Me) Glu | β - homoPro | single bond | $NH_2$ |
| 331 | Ape | (d) - Cys | Asp | Dab | Leu | (N-Me) Glu | β - homoPro | single bond | $NH_2$ |
| 332 | Ape | Arg | Asp | Dab | Leu | (N-Me) Glu | β - homoPro | single bond | $NH_2$ |
| 333 | Ape | (d) - Arg | Asp | Dab | Leu | (N-Me) Glu | β - homoPro | single bond | $NH_2$ |
| 334 | Ape | (d) - Glu | Asp | Dab | Leu | (N-Me) Glu | β - homoPro | single bond | $NH_2$ |
| 335 | $-NH-(CH_2)_2-0-CH_2-C0-$ | (d) - Ser | Asp | Dab | Leu | (N-Me) Glu | β - homoPro | single bond | $NH_2$ |
| 336 | Ape | Lys (Ac ) | As p | Dab | Leu | (N-Me) Ile | β - homoPro | single bond | $NH_2$ |
| 337 | Ape | Lys (Ac ) | Asp | Dab | Leu | (N-Me) Glu | β - homoPro | single bond | $NH_2$ |
| 338 | Ape | Cit | Asp | Dab | Leu | (N-Me) Ile | β - homoPro | single bond | $NH_2$ |
| 339 | Ape | (d) - Cit | Asp | Dab | Leu | (N-Me) Ile | β - homoPro | single bond | $NH_2$ |
| 340 | Ape | Cit | Asp | Dab | Leu | (N-Me) Glu | β - homoPro | single bond | $NH_2$ |
| 341 | Ape | (d) - Cit | Asp | Dab | Leu | (N-Me) Glu | β - homoPro | single bond | $NH_2$ |
| 342 | Ape | β -Asp | As p | Dab | Leu | (N-Me) Glu | β - homoPro | single bond | $NH_2$ |

(continued)

| Compound No. | $L^{1''}$ | $L^{1-}$ | $AA^1$ | $AA^2$ | $AA^3$ | $AA^4$ | $AA^5$ | $W^c$ | $R^c$ |
|---|---|---|---|---|---|---|---|---|---|
| 343 | Ape | $\beta$ - (d) - Asp | As p | Dab | Leu | (N-Me) Glu | $\beta$ - homoPro | single bond | $NH_2$ |
| 344 | Ape | Lys | Asp | Dab | Leu | (N-Me) Ile | $\beta$ - homoPro | single bond | $NH_2$ |
| 345 | Ape | Met | Asp | Dab | Leu | (N-Me) Ile | $\beta$ - homoPro | single bond | $NH_2$ |
| 346 | Ape | Met | Asp | Dab | Leu | (N-Me) Glu | $\beta$ - homoPro | single bond | $NH_2$ |
| 347 | Ape | (d) - Ser | Asp | Dab | Leu | (N-Me) Ile | $\beta$ - homoPro | single bond | $NH_2$ |
| 348 | Ape | (d) - Thr | Asp | Dab | Leu | (N-Me) Ile | $\beta$ - homoPro | single bond | $NH_2$ |
| 349 | Adox | 単結合 | Asp | Dab | Leu | (N-Me) Glu | $\beta$ - homoPro | single bond | $NH_2$ |
| 350 | Ape | (d) - Thr | Asp | Dab | Leu | (N-Me) Glu | $\beta$ - homoPro | (d) - Lys-(d) - Lys-(d) - Lys | $NH_2$ |
| 351 | Ape | (d) - Thr | As p | Dab | Leu | (N-Me) Ile | $\beta$ - homoPro | (d) - Lys-(d) - Lys-(d) - Lys | $NH_2$ |
| 352 | GABA | (d) - Thr | Asp | Dab | Leu | (N-Me) Glu | $\beta$ - homoPro | single bond | $NH_2$ |
| 353 | GABA | (d) - Thr | Asp | Dab | Leu | (N-Me) Ile | $\beta$ - homoPro | single bond | $NH_2$ |
| 354 | Acp | (d) - Thr | Asp | Dab | Leu | (N-Me) Glu | $\beta$ - homoPro | single bond | $NH_2$ |
| 355 | Acp | (d) - Thr | Asp | Dab | Leu | (N-Me) Ile | $\beta$ - homoPro | single bond | $NH_2$ |
| 356 | Ape | Arg | Asp | Dab | Leu | (N-Me) Ile | $\beta$ - homoPro | single bond | $NH_2$ |
| 357 | Ape | (N-Me) Gln | Asp | Dab | Leu | (N-Me) Ile | $\beta$ - homoPro | single bond | $NH_2$ |
| 358 | Ape | (N-Me) Gln | Asp | Dab | Leu | (N-Me) Glu | $\beta$ - homoPro | single bond | $NH_2$ |
| 359 | Ape | Asn | Asp | Dab | Phe | (N-Me) Ile | $\beta$ - homoPro | single bond | $NH_2$ |
| 360 | Ape | Asn | Asp | Dab | Phe | (N-Me) Glu | $\beta$ - homoPro | single bond | $NH_2$ |
| 361 | $\delta$ -0rn | (d) - Ser | As p | Dab | Leu | (N-Me) Glu | $\beta$ - homoPro | single bond | $NH_2$ |
| 362 | $\delta$ -0rn | (d) - Thr | Asp | Dab | Leu | (N-Me) Glu | $\beta$ - homoPro | single bond | $NH_2$ |
| 363 | $\delta$ -Orn | Lys | Asp | Dab | Leu | (N-Me) Glu | $\beta$ - homoPro | single bond | $NH_2$ |
| 364 | $\delta$ -0rn | (d) - Ser | As p | Dab | Leu | (N-Me) Ile | $\beta$ - homoPro | single bond | $NH_2$ |
| 365 | $\delta$ -0rn | (d) - Thr | Asp | Dab | Leu | (N-Me) Ile | $\beta$ - homoPro | single bond | $NH_2$ |
| 366 | $\delta$ -0rn | Lys | Asp | Dab | Leu | (N-Me) Ile | $\beta$ - homoPro | single bond | $NH_2$ |
| 367 | Ape | (N-Me) Arg | Asp | Dab | Leu | (N-Me) Glu | $\beta$ - homoPro | single bond | $NH_2$ |

(continued)

| Compound No. | $L^{1''}$ | $L^{1-}$ | $AA^1$ | $AA^2$ | $AA^3$ | $AA^4$ | $AA^5$ | $W^c$ | $R^c$ |
|---|---|---|---|---|---|---|---|---|---|
| 368 | Ape | (N-Me) Arg | Asp | Dab | Leu | (N-Me) Ile | $\beta$ - homoPro | . single bond | $NH_2$ |
| 369 | Ape | (d)-Lys | Asp | Dab | Leu | (N-Me) Glu | $\beta$ - homoPro | single bond | $NH_2$ |
| 370 | Ape | (d) - Lys | Asp | Dab | Leu | (N-Me) Ile | $\beta$ - homoPro | single bond | $NH_2$ |
| 371 | Ape | Lys | Asp | Dab | Leu | Dap | $\beta$ - homoPro | single bond | $NH_2$ |
| 372 | Ape | Lys | Asp | Dab | Leu | Dab | $\beta$ - homoPro | single bond | $NH_2$ |
| 373 | Ape | Lys | Asp | Dab | Leu | 0rn | $\beta$ - homoPro | single bond | $NH_2$ |
| 374 | Ape | Lys | Asp | Dab | Leu | (N-Me) Arg | $\beta$ - homoPro | single bond | $NH_2$ |
| 375 | Ape | Arg | Asp | Dab | Leu | Dap | $\beta$ - homoPro | single bond | $NH_2$ |
| 376 | Ape | Arg | Asp | Dab | Leu | Dab | $\beta$ - homoPro | single bond | $NH_2$ |
| 377 | Ape | Lys | Asp | Dab | Leu | 0rn | $\beta$ - homoPro | single bond | $NH_2$ |
| 378 | Ape | Arg | As p | Dab | Leu | (N-Me) Lys | $\beta$ - homoPro | single bond | $NH_2$ |
| 379 | Ape | Gln | Asp | Dap | Leu | (N-Me) lie | single bond | single bond | $NH_2$ |
| 380 | Ape | Gln | Asp | Dab | Leu | (N-Me) Glu | single bond | single bond | $NH_2$ |
| 331 | Ape | Gln | Asp | Dab | Leu | (N-Me) Ile | single bond | single bond | $NH_2$ |
| 382 | Ape | Gln | Asp | Dab | Leu | 単結合 | single bond | single bond | $NH_2$ |
| 383 | Ape | Gln | aspart imide | Dab | Leu | (N-Me) Ile | $\beta$ - homoPro | single bond | $NH_2$ |
| 384 | Ape | Gln | Asp | Dab | Leu | (N-Me) Glu | $\beta$ - homoPro | (d)-Lys | $NH_2$ |
| 385 | Ape | Gln | Asp | Dab | Leu | (N-Me) Ile | $\beta$ - homoPro | (d) - Lys | $NH_2$ |
| 386 | Ape | Gln | Asp | Dab | Leu | (N-Me) Glu | $\beta$ - homoPro | (d)-Arg | $NH_2$ |
| 387 | Ape | Gln | Asp | Dab | Leu | (N-Me) Ile | $\beta$ - homoPro | (d)-Arg | $NH_2$ |
| 388 | Ape | Arg | Asp | Dab | Leu | (N-Me) Glu | $\beta$ - homoPro | (d) - Lys | $NH_2$ |
| 389 | Ape | Arg | Asp | Dab | Leu | (N-Me) Ile | $\beta$ - homoPro | (d) - Lys | $NH_2$ |
| 390 | Ape | Arg | Asp | Dab | Leu | (N-Me) Glu | $\beta$ - homoPro | (d)-Arg | $NH_2$ |
| 391 | Ape | Arg | Asp | Dab | Leu | (N-Me) Ile | $\beta$ - homoPro | (d) - Arg | $NH_2$ |
| 392 | Ape | Gln | Asp | Dab | Leu | (N-Me) Ile | Pro | single bond | $NH_2$ |

(continued)

| Compound No. | $L^{1''}$ | $L^{1-}$ | $AA^1$ | $AA^2$ | $AA^3$ | $AA^4$ | $AA^5$ | $W^c$ | $R^c$ |
|---|---|---|---|---|---|---|---|---|---|
| 393 | Ape | Gln | Asp | Dab | Leu | (N-Me) Glu | Pro | single bond | $NH_2$ |
| 394 | Ape | Glu | Asp | Dab | Leu | (N-Me) Ile | Pro | single bond | $NH_2$ |
| 395 | Ape | Glu | Asp | Dab | Leu | (N-Me) Glu | Pro | single bond | $NH_2$ |
| 396 | Ape | Arg | Asp | Dab | Leu | (N-Me) Ile | Pro | single bond | $NH_2$ |
| 397 | Ape | Arg | Asp | Dab | Leu | (N-Me) Glu | Pro | single bond | $NH_2$ |
| 398 | Ape | (d) - Thr | Asp | Dab | Leu | (N-Me) Ile | Pro | single bond | $NH_2$ |
| 399 | Ape | (d) - Thr | Asp | Dab | Leu | (N-Me) Glu | Pro | single bond | $NH_2$ |
| 400 | Ape | Gln | Asp | Dab | Leu | (N-Me) Glu | Pro | (d) - Arg | $NH_2$ |
| 401 | Ape | Gln | Asp | Dab | Leu | (N-Me) Ile | Pro | (d) - Arg | $NH_2$ |
| 402 | Ape | Gln | Asp | Dab | Leu | (N-Me) Glu | Pro | (d) - Lys | $NH_2$ |
| 403 | Ape | Gln | Asp | Dab | Leu | (N-Me) Ile | Pro | (d) - Lys | $NH_2$ |
| 404 | Ape | Gln | Asp | Dab | Leu | (N-Me) Glu | Pro | Lys | $NH_2$ |
| 405 | Ape | Gln | Asp | Dab | Leu | (N-Me) Ile | Pro | Lys | $NH_2$ |
| 406 | Ape | Gln | Asp | Dab | Leu | (N-Me) Glu | Pro | Arg | $NH_2$ |
| 407 | Ape | Gln | Asp | Dab | Leu | (N-Me) Ile | Pro | Arg | $NH_2$ |
| 408 | Ape | Gln | Asp | Dab | Leu | (N-Me) Glu | Arg | single bond | $NH_2$ |
| 409 | Ape | Gln | Asp | Dab | Leu | (N-Me) Ile | Arg | single bond | $NH_2$ |
| 410 | Ape | Gln | Asp | Dab | Leu | (N-Me) Glu | Lys | single bond | $NH_2$ |
| 411 | Ape | Gln | Asp | Dab | Leu | (N-Me) Ile | Lys | single bond | $NH_2$ |
| 412 | Ape | Gln | Asp | Dab | Leu | (N-Me) Glu | $\beta$-Ala | single bond | $NH_2$ |
| 413 | Ape | Gln | Asp | Dab | Leu | (N-Me) Ile | $\beta$-Ala | single bond | $NH_2$ |
| 414 | Ape | Gln | Asp | Dab | Leu | (N-Me) Glu | GABA | single bond | $NH_2$ |
| 415 | Ape | Gln | Asp | Dab | Leu | (N-Me) Ile | GABA | single bond | $NH_2$ |
| 416 | Ape | Gln | Asp | Dab | Leu | (N-Me) Glu | Ape | single bond | $NH_2$ |
| 417 | Ape | Gln | Asp | Dab | Leu | (N-Me) Glu | His | single bond | $NH_2$ |

(continued)

| Compound No. | $L^{1"}$ | $L^{1-}$ | $AA^1$ | $AA^2$ | $AA^3$ | $AA^4$ | $AA^5$ | $W^c$ | $R^c$ |
|---|---|---|---|---|---|---|---|---|---|
| 418 | Ape | Gln | Asp | Dab | Leu | (N-Me) Ile | (d) -Arg | single bond | $NH_2$ |
| 419 | Ape | Gln | Asp | Dab | Leu | (N-Me) Glu | (d) -Arg | single bond | $NH_2$ |
| 420 | Ape | Gin | Asp | Dab | Leu | (N-Me) Ile | (d) -Lys | single bond | $NH_2$ |
| 42 1 | Ape | Gln | Asp | Dab | Leu | (N-Me) Glu | (d) -Lys | single bond | $NH_2$ |
| 422 | Ape | Gln | Asp | Dab | Leu | (N-Me) Glu | Da p | single bond | $NH_2$ |
| 423 | Ape | Gln | Asp | Dab | Leu | (N-Me) Glu | Dab | single bond | $NH_2$ |
| 424 | Ape | Gln | As p | Dab | Leu | (N-Me) Ile | 0rn | single bond | $NH_2$ |
| 425 | Ape | Gln | Asp | Dab | Leu | (N-Me) Glu | 0rn | single bond | $NH_2$ |
| 426 | Ape | Gin | Asp | Dab | Leu | (N-Me) Glu | homoPro | single bond | $NH_2$ |
| 427 | Ape | Gln | Asp | Dab | Leu | (N-Me) Ile | Ape | single bond | $NH_2$ |
| 428 | Ape | Gln | Asp | Dab | Leu | (N-Me) Ile | (2S, 4R) -(4-amino)P ro | single bond | $NH_2$ |
| 429 | Ape | Gln | Asp | Dab | Leu | (N-Me) Glu | $\beta$ - homoPro | Arg-Arg | $NH_2$ |
| 430 | Ape | Gln | Asp | Dab | Leu | (N-Me) Ile | $\beta$ - homoPro | Arg-Arg | $NH_2$ |
| 431 | Ape | Gln | Asp | Dab | Leu | (N-Me) Glu | Pro | Arg-Arg | $NH_2$ |
| 432 | Ape | Gln | Asp | Dab | Leu | (N-Me) Ile | Pro | Arg-Arg | $NH_2$ |
| 433 | Ape | Gln | Asp | Dab | Leu | (N-Me) Glu | $\beta$ - homoPro | (d) - Arg-(d) - Arg | $NH_2$ |
| 434 | Ape | Gln | Asp | Dab | Leu | (N-Me) Ile | $\beta$ - homoPro | (d) - Arg-(d) - Arg | $NH_2$ |
| 435 | Ape | Gln | Asp | Dab | Leu | (N-Me) Glu | Pro | (d)-Arg-(d) - Arg | $NH_2$ |
| 436 | Ape | Gln | Asp | Dab | Leu | (N-Me) Ile | Pro | (d) - Arg-(d) - Arg | $NH_2$ |
| 437 | Ape | Gln | Asp | Dab | Leu | (N-Me) Ile | $\beta$ -Dap | single bond | $NH_2$ |
| 438 | Ape | Gln | As p | Dab | Leu | (N-Me) Ile | $\beta$ -(d)-Dap | single bond | $NH_2$ |
| 439 | Ape | Gln | Asp | Dab | Leu | (N-Me) He | $\gamma$ -Dab | single bond | $NH_2$ |
| 440 | Ape | Gln | Asp | Dab | Leu | (N-Me) Glu | $\beta$ - homoPro | Lys-Lys | $NH_2$ |
| 441 | Ape | Gln | Asp | Dab | Leu | (N-Me) Ile | $\beta$ - homoPro | Lys-Lys | $NH_2$ |
| 442 | Ape | Gln | As p | Dab | Leu | (N-Me) Glu | Pro | Lys-Lys | $NH_2$ |

(continued)

| Compound No. | $L^{1"}$ | $L^{1-}$ | $AA^1$ | $AA^2$ | $AA^3$ | $AA^4$ | $AA^5$ | $W^c$ | $R^c$ |
|---|---|---|---|---|---|---|---|---|---|
| 443 | Ape | Gln | Asp | Dab | Leu | (N-Me) Ile | Pro | Lys-Lys | $NH_2$ |
| 444 | Ape | Gln | As p | Dab | Leu | (N-Me) Glu | $\beta$ - homoPro | (d)-Lys-(d) - Lys | $NH_2$ |
| 445 | Ape | Gln | Asp | Dab | Leu | (N-Me) Ile | $\beta$ - homoPro | (d) - Lys-(d) - Lys | $NH_2$ |
| 446 | Ape | Gln | Asp | Dab | Leu | (N-Me) Glu | Pro | (d) - Lys-(d) - Lys | $NH_2$ |
| 447 | Ape | Gln | Asp | Dab | Leu | (N-Me) Ile | Pro | (d) - Lys-(d) - Lys | $NH_2$ |
| 448 | Ape | Gln | Asp | Dab | Leu | (N-Me) Ile | $\beta$ - (d) - Dap | single bond | $NH_2$ |
| 449 | Ape | Gln | As p | Dab | Leu | (N-Me) Ile | $\delta$ -Orn | single bond | $NH_2$ |
| 450 | Ape | Gln | Asp | Dab | Leu | (N-Me) Ile | $\delta$ - (d) - Orn | single bond | $NH_2$ |
| 451 | Ape | Gln | As p | Dab | Leu | (N-Me) Ile | $\varepsilon$ -Lys | single bond | $NH_2$ |
| 452 | Ape | Gln | Asp | Dab | Leu | (N-Me) Ile | $\varepsilon$ -(d)- Lys | single bond | $NH_2$ |
| 453 | Ape | Gln | $\beta$ -Asp | Dab | Leu | (N-Me) Ile | $\beta$ - homoPro | single bond | $NH_2$ |
| 454 | Ape | Gln | Asp | Dab | Leu | (N-Me) Ile | Aze(2) | single bond | $NH_2$ |
| 455 | Ape | Gln | Asp | Dab | Leu | (N-Me) Ile | (d)-Aze (2) | single bond | $NH_2$ |
| 456 | Ape | Gin | Asp | Dab | Leu | (N-Me) Ile | (N-Me) - $\beta$ -Ala | single bond | $NH_2$ |
| 457 | Ape | Glu | Asp | Dab | Leu | (N-Me) Ile | GABA | Arg | $NH_2$ |
| 458 | Ape | Gln | Asp | Dab | Leu | (N-Me) Ile | GABA | (d)-Arg | $NH_2$ |
| 459 | Ape | Gin | Asp | Dab | Leu | (N-Me) Ile | GABA | Lys | $NH_2$ |
| 460 | Ape | Gln | Asp | Dab | Leu | (N-Me) Ile | GABA | (d) - Lys | $NH_2$ |
| 461 | Ape | Gln | Asp | Dab | Leu | (N-Me) Ile | GABA | Arg-Arg | $NH_2$ |
| 462 | Ape | Gln | Asp | Dab | Leu | (N-Me) Ile | GABA | (d) - Arg-(d) - Arg | $NH_2$ |
| 453 | Ape | Gln | Asp | Dab | Leu | (N-Me) Ile | GABA | (d) - Lys-(d) - Lys | $NH_2$ |
| 464 | Ape | Gln | Asp | Dab | Leu | (N-Me) Glu | GABA | Arg | $NH_2$ |
| 465 | Ape | Gln | Asp | Dab | Leu | (N-Me) Glu | GABA | (d) - Arg | $NH_2$ |
| 466 | Ape | Gln | Asp | Dab | Leu | (N-Me) Glu | GABA | Lys | $NH_2$ |
| 467 | Ape | Gln | Asp | Dab | Leu | (N-Me) Glu | GABA | (d)-Lys | $NH_2$ |

(continued)

| Compound No. | L'' | L1- | $AA^1$ | $AA^2$ | $AA^3$ | $AA^4$ | $AA^5$ | $W^c$ | $R^c$ |
|---|---|---|---|---|---|---|---|---|---|
| 468 | Ape | Gln | Asp | Dab | Leu | (N-Me) Glu | GABA | Arg-Arg | $NH_2$ |
| 469 | Ape | Gln | As p | Dab | Leu | (N-Me) Glu | GABA | (d) - Arg-(d) - Arg | $NH_2$ |
| 470 | Ape | Gln | Asp | Dab | Leu | (N-Me) Glu | GABA | Lys-Lys | $NH_2$ |
| 471 | Ape | Gln | Asp | Dab | Leu | (N-Me) Glu | GABA | (d) - Lys-(d) - Lys | $NH_2$ |
| 472 | Ape | Gln | Asp | Dab | Leu | (N-Me) Ile | Ape | Arg | $NH_2$ |
| 473 | Ape | Gln | Asp | Dab | Leu | (N-Me) Ile | Ape | (d) - Arg | $NH_2$ |
| 474 | Ape | Gln | Asp | Dab | Leu | (N-Me) Ile | Ape | Lys | $NH_2$ |
| 476 | Ape | Gln | Asp | Dab | Leu | (N-Me) Ile | Ape | (d) - Lys | $NH_2$ |
| 476 | Ape | Gln | Asp | Dab | Leu | (N-Me) Ile | Ape | Arg-Arg | $NH_2$ |
| 477 | Ape | Gln | Asp | Dab | Leu | (N-Me) Ile | Ape | (d) - Arg-(d) - Arg | $NH_2$ |
| 478 | Ape | Gln | Asp | Dab | Leu | (N-Me) Ile | Ape | Lys-Lys | $NH_2$ |
| 479 | Ape | Gln | Asp | Dab | Leu | (N-Me) Ile | Ape | (d) - Lys-(d) - Lys | $NH_2$ |
| 480 | Ape | Gln | Asp | Dab | Leu | (N-Me) Ile | Acp | single bond | $NH_2$ |
| 481 | Ape | Gln | Asp | Dab | Ile | (N-Me) Glu | Ape | single bond | $NH_2$ |
| 482 | Ape | Gln | Asp | Dab | Ile | (N-Me) Ile | Ape | single bond | $NH_2$ |
| 483 | Ape | Gln | Asp | Dab | Leu | (N-Me) lie | $\beta$ - homoPro | Arg | $NH_2$ |
| 484 | Ape | Gln | Asp | Dab | Leu | (N-Me) Ile | $\beta$ - homoPro | Lys | $NH_2$ |
| 485 | Ape | Gln | Asp | Dab | Leu | (N-Me) Val | Ape | single bond | $NH_2$ |
| 486 | Ape | Gln | Asp | Dab | Leu | (N-Me) Leu | Ape | single bond | $NH_2$ |
| 487 | Ape | Gln | Asp | Dab | Ile | (N-Me) Ile | Ape | (d) - Lys | $NH_2$ |
| 488 | Ape | Gln | Asp | Dab | Ile | (N-Me) Ile | Ape | (d) - Arg | $NH_2$ |
| 489 | Ape | Gln | Asp | Dab | Leu | (N-Me) Val | Ape | (d) - Arg | $NH_2$ |
| 490 | Ape | Gln | Asp | Dab | Leu | (N-Me) Leu | Ape | (d) - Lys | $NH_2$ |
| 491 | Ape | Gln | Asp | Dab | Leu | (N-Me) Leu | Ape | (d) - Arg | $NH_2$ |
| 492 | Ape | Gln | Asp | Dab | Leu | (N-Me) Ile | single bond | single bond | $NH-(CH_2)_2-NH_2$ |

(continued)

| Compound No. | L1" | L1- | $AA^1$ | $AA^2$ | $AA^3$ | $AA^4$ | $AA^5$ | $W^c$ | $R^c$ |
|---|---|---|---|---|---|---|---|---|---|
| 493 | Ape | Gln | Asp | Dab | Leu | (N-Me) Ile | Ape | single bond | MH-$(CH_2)_2$ -$NH_2$ |
| 494 | Ape | Gln | Asp | Dab | Leu | (N-Me) Ile | single bond | single bond | NH-$(CH_2)_4$ -$NM_2$ |
| 495 | Ape | Gln | Asp | Dab | Leu | (N-Me) Ile | single bond | single bond | NH-$(CH_2)_5$ -$NH_2$ |
| 496 | δ -Orn | Gln | Asp | Dab | Leu | (N-Me) Ile | β - homoPro | single bond | $NH_2$ |
| 497 | δ -Orn | Gln | Asp | Dab | Leu | (N-Me) Ile | Ape | single bond | $NH_2$ |
| 498 | δ -Orn | Ala | As p | Dab | Leu | (N-Me) Ile | β - homoPro | single bond | $NH_2$ |
| 499 | δ -Orn | Ala | Asp | Dab | Leu | (N-Me) Ile | Ape | single bond | $NH_2$ |
| 500 | δ -Orn | Lys(Ac) | Asp | Dab | Leu | (N-Me) Ile | β - homoPro | single bond | $NH_2$ |
| 501 | δ -Orn | Lys(Ac ) | Asp | Dab | Leu | (N-Me) Ile | Ape | single bond | $NH_2$ |
| 502 | δ -Orn | Dap | Asp | Dab | Leu | (N-Me) Ile | β - homoPro | single bond | $NH_2$ |
| 503 | δ -Orn | Dab | Asp | Dab | Leu | (N-Me) Ile | Ape | single bond | $NH_2$ |
| 504 | δ -Orn | Gly | Asp | Dab | Leu | (N-Me) Ile | β - homoPro | single bond | $NH_2$ |
| 505 | δ -Orn | Gly | Asp | Dab | Leu | (N-Me) Ile | Ape | single bond | $NH_2$ |
| 506 | δ -Orn | Gln | Asp | Dab | Leu | (N-Me) Val | β - homoPro | single bond | $NH_2$ |
| 507 | δ -Orn | Gln | Asp | Dab | Leu | (N-Me) Val | Ape | single bond | $NH_2$ |
| 508 | Ape | (d) - Thr | Asp | Dab | Leu | (N-Me) Val | Pro | single bond | $NH_2$ |
| 509 | Ape | Gln | Asp | Dab | Leu | (N-Me) Val | β - homoPro | (d) - Arg | $NH_2$ |
| 510 | Ape | Gln | Asp | Dab | Leu | (N-Me) Val | β - homoPro | (d) - Lys | $NH_2$ |
| 511 | Ape | Gln | Asp | Dab | Leu | (N-Me) Val | β -Ala | single bond | $NH_2$ |
| 512 | Ape | Gln | As p | Dab | Leu | (N-Me) Val | GABA | single bond | $NH_2$ |
| 513 | Ape | Gln | Asp | Dab | Leu | (N-Me) Val | ε - (d) - Lys | single bond | $NH_2$ |
| 514 | Ape | Gln | Asp | Dab | Leu | (N-Me) Val | Ape | (d) - Arg | $NH_2$ |
| 515 | Ape | Gln | Asp | Dab | Leu | (N-Me) Val | Ape | (d) - Ly s | $NH_2$ |
| 516 | δ -Orn | Dap | Asp | Dab | Leu | (N-Me) Val | β - homoPro | single bond | $NH_2$ |
| 517 | δ -Orn | Dap | Asp | Dab | Leu | (N-Me) Val | Ape | single bond | $NH_2$ |

(continued)

| Compound No. | L1" | L1- | $AA^1$ | $AA^2$ | $AA^3$ | $AA^4$ | $AA^5$ | $W^c$ | $R^c$ |
|---|---|---|---|---|---|---|---|---|---|
| 518 | Ape | Gln | Asp | Dab | Leu | (N-Me) Ile | ε -(d)-Lys | (d) - **Arg** | $NH_2$ |
| 519 | Ape | Gln | Asp | Dab | Leu | (N-Me) Ile | ε -(d)-Lys | (d) - Lys | $NH_2$ |
| 520 | Ape | Gln | Asp | Dab | Leu | (N-Me) Ile | γ-Dab | β-Ala | $NH_2$ |
| 521 | Ape | Gln | Asp | Dab | Leu | (N-Me) Ile | γ-Dab | GABA | $NH_2$ |
| 522 | ε - (d) - Lys | single bond | Asp | Dab | Leu | (N-Me) Ile | Ape | single bond | $NH_2$ |
| 523 | ε -Lys | single bond | Asp | Dab | Leu | (N-Me) Ile | Ape | single bond | $NH_2$ |
| 524 | δ -Orn | Gln | Asp | Dab | Leu | (N-Me) Val | ε - (d) - Lys | single bond | $NH_2$ |
| 525 | δ -Orn | Gln | Asp | Dab | Leu | (N-Me) Val | ε -Lys | single bond | $NH_2$ |
| 526 | δ -Orn | Gln | Asp | Dab | Leu | (N-Me) Ile | ε - (d) - Lys | single bond | $NH_2$ |
| 527 | δ -Orn | Gln | Asp | Dab | Leu | (N-Me) Ile | ε -Lys | single bond | $NH_2$ |
| 528 | δ -Orn | Arg | Asp | Dab | Leu | (N-Me) Ile | ε - (d) - Lys | single bond | $NH_2$ |
| 529 | Ape | Gln | Asp | Dab | Leu | (N-Me) Val | β-Dap | single bond | $NH_2$ |
| 530 | Ape | Gln | Asp | Dab | Leu | (N-Me) Val | β- (d) - Dap | single bond | $NEt_i$ |
| 531 | Ape | Gln | Asp | Dab | Leu | (N-Me) Val | γ-Dab | single bond | $NH_2$ |
| 532 | Ape | Gln | Asp | Dab | Leu | (N-Me) Val | γ- (d) - Dab | single bond | $NH_2$ |
| 533 | Ape | Gln | Asp | Dab | Leu | (N-Me) Val | δ - (d) - Orn | single bond | $tm_{:2}$ |
| 534 | Ape | Gln | Asp | Dab | Leu | (N-Me) Val | ε -Lys | single bond | $NH_2$ |
| 535 | δ -Orn | Gln | Asp | Dab | Leu | (N-Me) Val | β-Dap | single bond | $NH_2$ |
| 536 | δ -Orn | Gln | Asp | Dab | Leu | (N-Me) Val | β- (d) - Dap | single bond | $NH_2$ |
| 537 | δ -Orn | Gln | Asp | Dab | Leu | (N-Me) Val | γ-Dab | single bond | $NH_2$ |
| 538 | δ -Orn | Gln | Asp | Dab | Leu | (N-Me) Val | δ -Orn | single bond | HM: |
| 539 | δ -Orn | Gln | Asp | Dab | Leu | (N-Me) Val | δ - (d) - Orn | single bond | $NH_2$ |
| 540 | Ape | Gln | Asp | Dab | Leu | (N-Me) Val | γ-Dab | -Ala | $NR_2$ |
| 541 | Ape | Gln | Asp | Dab | Leu | (N-Me) Val | γ-Dab | GABA | $NH_2$ |
| 542 | Ape | Gln | Asp | Dab | Leu | (N-Me) Val | δ-Orn | single bond | $NH_2$ |

(continued)

| Compound No. | $L^{1''}$ | $L^{1-}$ | $AA^1$ | $AA^2$ | $AA^3$ | $AA^4$ | $AA^5$ | $W^c$ | $R^c$ |
|---|---|---|---|---|---|---|---|---|---|
| 543 | Ape | Arg | Asp | Dab | Leu | (N-Me) Ile | ε -Lys | single bond | $NH_2$ |
| 544 | Ape | Arg | Asp | Dab | Leu | (N-Me) Ile | ε - (d) - Lys | single bond | $NH_2$ |
| 545 | Ape | Arg | Asp | Dab | Leu | (N-Me) Val | ε -Lys | single bond | $NH_2$ |
| 546 | Ape | Arg | As p | Dab | Leu | (N-Me) Val | ε - (d) - Lys | single bond | $NH_2$ |
| 547 | Ape | (d) - Thr | Asp | Dab | Leu | (N-Me) Ile | ε -Lys | single bond | $NH_2$ |
| 548 | Ape | (d) - Thr | Asp | Dab | Leu | (N-Me) Ile | ε - (d) - Lys | single bond | $NH_2$ |
| 549 | Ape | (d) - Thr | Asp | Dab | Leu | (N-Me) Val | ε -Lys | single bond | $NH_2$ |
| 550 | Ape | (d) - Thr | Asp | Dab | Leu | (N-Me) Val | ε - (d) - Lys | single bond | $NH_2$ |
| 551 | δ -Orn | Arg | Asp | Dab | Leu | (N-Me) Ile | ε -Lys | single bond | $NH_2$ |
| 552 | δ -Orn | Arg | Asp | Dab | Leu | (N-Me) Val | ε -Lys | single bond | $NH_2$ |
| 553 | δ -Orn | Arg | Asp | Dab | Leu | (N-Me) Val | ε - (d) - Lys | single bond | $NH_2$ |
| 554 | δ -Orn | (d) - Thr | Asp | Dab | Leu | (N-Me) lie | ε -Lys | single bond | $NH_2$ |
| 555 | δ -Orn | (d) - Thr | Asp | Dab | Leu | (N-Me) lie | ε - (d) - Lys | single bond | $NH_2$ |
| 556 | δ -Orn | (d) - Thr | Asp | Dab | Leu | (N-Me) Val | ε -Lys | single bond | $NH_2$ |
| 557 | δ -Orn | (d) - Thr | Asp | Dab | Leu | (N-Me) Val | ε -(d)-Lys | single bond | $NH_2$ |
| 658 | Ape | Gln | Asp | Dab | Leu | (N-Me) Ile | β -Dap | β -Ala | $NH_2$ |
| 559 | δ -Orn | Gln | Asp | Dab | Leu | (N-Me) Ile | β -Dap | β -Ala | $NH_2$ |
| 560 | δ -Orn | Gln | Asp | Dab | Leu | (N-Me) Val | β -Dap | β -Ala | $NH_2$ |
| 561 | δ -Orn | Gln | Asp | Dab | Leu | (N-Me) Ile | β -Dap | single bond | $NH_2$ |
| 562 | δ -Orn | Gln | Asp | Dab | Leu | (N-Me) Ile | β - (d) - Dap | single bond | $NH_2$ |
| 563 | δ -Orn | Gln | Asp | Dab | Leu | (N-Me) Ile | γ -Dab | single bond | $NH_2$ |
| 564 | δ -Orn | Gln | Asp | Dab | Leu | (N-Me) Ile | γ - (d) - Dab | single bond | $NH_2$ |
| 565 | δ -Orn | Gln | Asp | Dab | Leu | (N-Me) Ile | δ -Orn | single bond | $NH_2$ |
| 566 | δ -Orn | Gln | Asp | Dab | Leu | (N-Me) lie | δ - (d) - Orn | single bond | $NH_2$ |
| 567 | Ape | Asn | Asp | Dab | Leu | (N-Me) Ile | ε -Lys | single bond | $NH_2$ |

(continued)

| Compound No. | $L^{1''}$ | $L^{1-}$ | $AA^1$ | $AA^2$ | $AA^3$ | $AA^4$ | $AA^5$ | $W^c$ | $R^c$ |
|---|---|---|---|---|---|---|---|---|---|
| 568 | Ape | Asn | Asp | Dab | Leu | (N-Me) Ile | ε - (d) - Lys | single bond | $NH_2$ |
| 569 | Ape | Gln | As p | Dap | Leu | (N-Me) Ile | ε -Lys | single bond | $NH_2$ |
| 570 | Ape | Gln | Asp | Dap | Leu | (N-Me) Ile | ε - (d) - Lys | single bond | $NH_2$ |
| 671 | Ape | Gln | Asp | Dab | Leu | (N-Me) Glu | ε -Lys | single bond | $NH_2$ |
| 672 | Ape | Gln | Asp | Dab | Leu | (N-Me) Glu | ε - (d) - Lys | single bond | $NH_2$ |
| 573 | Ape | Gln | Asp | Dab | Leu | (N-Me) Ala | ε -Lys | single bond | $NH_2$ |
| 574 | Ape | Gln | Asp | Dab | Leu | (N-Me) Ala | ε -(d)-Lys | single bond | $NH_2$ |
| 575 | Ape | Gln | Asp | Dab | Leu | (N-Me) Leu | ε -Lys | single bond | $NH_2$ |
| 576 | Ape | Gln | Asp | Dab | Leu | (N-Me) Leu | ε -(d)-Lys | single bond | $NH_2$ |
| 577 | Ape | Gln | Asp | Dab | Leu | (N-Me) Ile | ε - (d) - Lys | single bond | OH |
| 578 | Ape | Gln | Asp | Dab | Leu | (N-Me) Ile | Aze (2) | single bond | OH |
| 579 | Ape | Gln | Asp | Aze (2) | Leu | (N-Me) Ile | β -homoPro | single bond | $NH_2$ |
| 580 | Ape | Lys | Asp | Aze (2) | Leu | (N-Me) Ile | Ape | single bond | $NH_2$ |
| 581 | Ape | Lys | Asp | Aze (2) | Leu | (N-Me) Ile | β -homoPro | single bond | $NH_2$ |
| 582 | Ape | Arg | Asp | Aze (2) | Leu | (N-Me) Ile | Ape | single bond | $NH_2$ |
| 583 | Ape | Arg | Asp | Aze (2) | Leu | (N-Me) Ile | β -homoPro | single bond | $NH_2$ |
| 584 | Ape | (d) - Ser | Asp | Aze (2) | Leu | (N-Me) Ile | β -homoPro | single bond | $NH_2$ |
| 585 | Ape | (d) - Thr | Asp | Aze (2) | Leu | (N-Me) Ile | Ape | single bond | $NH_2$ |
| 586 | Ape | (d) - Thr | Asp | Aze (2) | Leu | (N-Me) Ile | β -homoPro | single bond | $NH_2$ |

The structures of compounds represented by formula [I'-16], which were synthesized in Example 4 or with the same method as in Example 4, are shown in the following table.

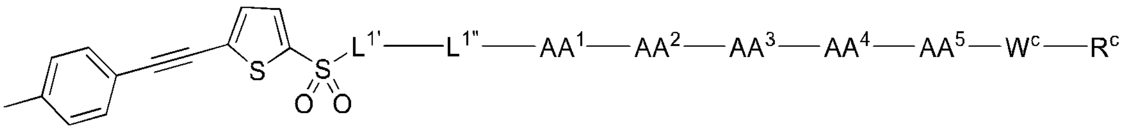

[I'-16]

[Table 23]

| Compound No. | $L^{1'}$ | $L^{1-}$ | $AA^1$ | $AA^2$ | $AA^3$ | $AA^4$ | $AA^5$ | $W^c$ | $R^c$ |
|---|---|---|---|---|---|---|---|---|---|
| 587 | β-Ala | Asn | Asp | Ala | Leu | (N-Me) Met | Pro | single bond | $NH_2$ |
| 588 | GABA | Asn | Asp | Ala | Leu | (N-Me) Met | Pro | single bond | $NH_2$ |
| 589 | β-Ala | Asn | Asp | Ala | Leu | (N-Me) Val | Pro | single bond | $NH_2$ |

The structure of a compound represented by formula [I'-17], which was synthesized with the same method as in Example 2, is shown in the following table.

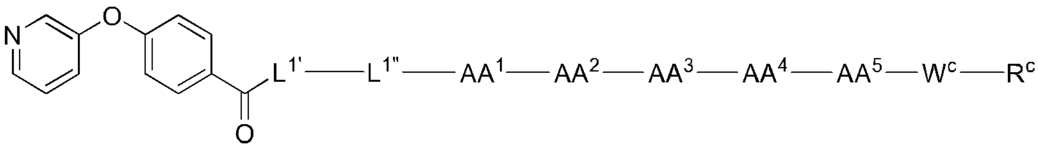

[I'-17]

[Table 24]

| Compound No. | $L^{1\cdot}$ | $L^{1-}$ | $AA^1$ | $AA^2$ | $AA^3$ | $AA^4$ | $AA^5$ | $W^c$ | $R^c$ |
|---|---|---|---|---|---|---|---|---|---|
| 590 | Ape | Asn | Asp | Ala | Leu | Met | Pro | single bond | $NH_2$ |

The structure of a compound represented by formula [I'-18], which was synthesized with the same method as in Example 2, is shown in the following table.

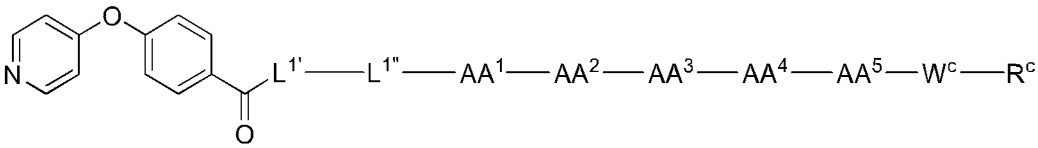

[I'-18]

[Table 25]

| Compound No. | $L^{1\cdot}$ | $L^{1-}$ | $AA^1$ | $AA^2$ | $AA^3$ | $AA^4$ | $AA^5$ | $W^C$ | $R^c$ |
|---|---|---|---|---|---|---|---|---|---|
| 591 | GABA | Asn | Asp | Ala | Leu | Met | Pro | single bond | $NH_2$ |

The structure of a compound represented by formula [I'-19], which was synthesized with the same method as in Example 2, is shown in the following table.

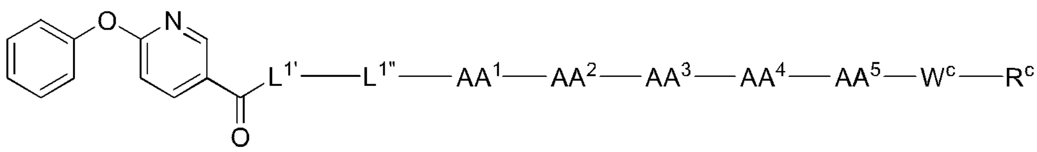

[I'-19]

[Table 26]

| Compound No. | $L^{1\cdot}$ | $L^{1-}$ | $AA^1$ | $AA^2$ | $AA^3$ | $AA^4$ | $AA^5$ | $W^c$ | $R^c$ |
|---|---|---|---|---|---|---|---|---|---|
| 592 | Ape | Asn | Asp | Ala | Leu | Met | Pro | single bond | $NH_2$ |

The structure of a compound represented by formula [I'-20], which was synthesized with the same method as in Example 1, is shown in the following table.

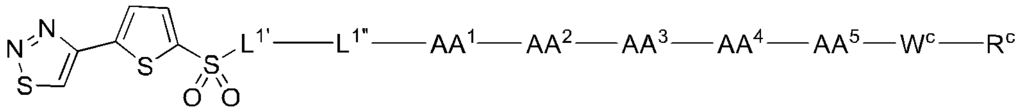

[I'-20]

[Table 27]

| Compound No. | $L^{1\cdot}$ | $L^{1-}$ | $AA^1$ | $AA^2$ | $AA^3$ | $AA^4$ | $AA^5$ | $W^c$ | $R^c$ |
|---|---|---|---|---|---|---|---|---|---|
| 593 | single bond | single bond | β-(d) -Asp | Ala | Leu | (N-Me) Glu | β-hom oPro | single bond | $NH_2$ |

The structures of compounds represented by formula [I'-21], which were synthesized with the same method as in Example 1, are shown in the following table.

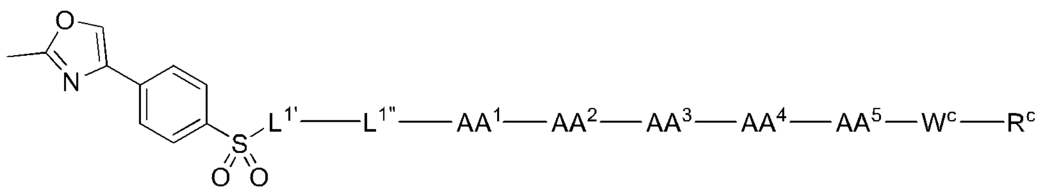

[I'-21]

[Table 28]

| Compound No. | $L^{1\cdot}$ | $L^{1-}$ | $AA^1$ | $AA^2$ | $AA^3$ | $AA^4$ | $AA^5$ | $W^c$ | $R^c$ |
|---|---|---|---|---|---|---|---|---|---|
| 594 | single bond | single bond | β-(d) -Asp | Ala | Leu | (N-Me) Ile | β-horn oPro | single bond | $NH_2$ |
| 595 | single bond | single bond | γ - (d) -Glu | (2S, 4S )-(4-a mino)P ro | Leu | (N-Me) Glu | Ape | single bond | $NH_2$ |

The structure of a compound represented by formula [I'-22], which was synthesized with the same method as in Example 2, is shown in the following table.

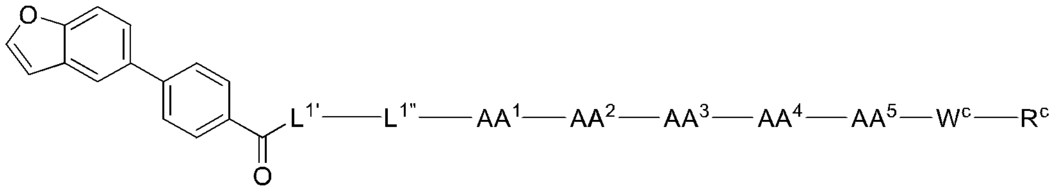

[I'-22]

[Table 29]

| Compound No. | L$^{1\cdot}$ | L$^{1-}$ | AA$^1$ | AA$^2$ | AA$^3$ | AA$^4$ | AA$^5$ | W$^c$ | R$^c$ |
|---|---|---|---|---|---|---|---|---|---|
| 596 | GABA | Asn | Asp | Dab | Leu | (N-Me) Ile | Pro | single bond | $NH_2$ |

The structure of a compound represented by formula [I'-23], was synthesized with the same method as in Example 2, is shown in the following table.

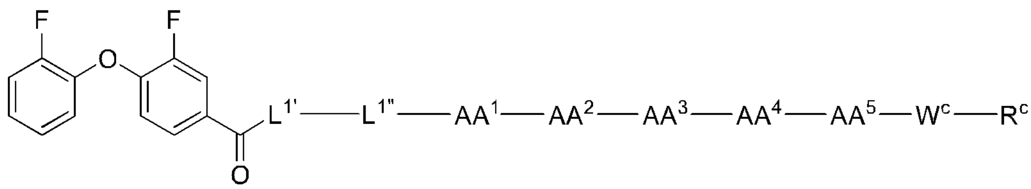

[I'-23]

[Table 30]

| Compound No. | L$^{1\cdot}$ | L$^{1-}$ | AA$^1$ | AA$^2$ | AA$^3$ | AA$^4$ | AA$^5$ | W$^c$ | R$^c$ |
|---|---|---|---|---|---|---|---|---|---|
| 597 | GABA | Asn | Asp | Ala | Leu | Met | Pro | single bond | $NH_2$ |

The structure of a compound represented by formula [I'-24], which was synthesized with the same method as in Example 2, is shown in the following table.

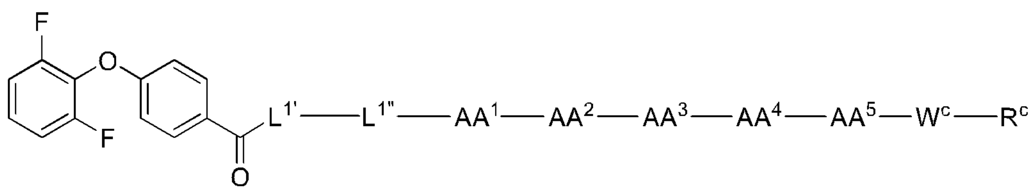

[I'-24]

[Table 31]

| Compound No. | L$^{1\cdot}$ | L$^{1-}$ | AA$^1$ | AA$^2$ | AA$^3$ | AA$^4$ | AA$^5$ | W$^c$ | R$^c$ |
|---|---|---|---|---|---|---|---|---|---|
| 598 | GABA | Asn | Asp | Ala | Leu | Met | Pro | single bond | $NH_2$ |

The structure of a compound represented by formula [I'-25], which was synthesized with the same method as in Example 2, is shown in the following table.

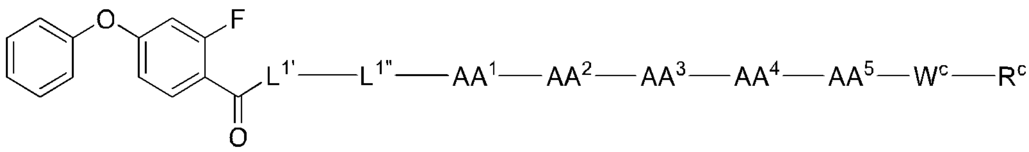

[I'-25]

[Table 32]

| Compound No. | $L^{1\cdot}$ | $L^{1-}$ | $AA^1$ | $AA^2$ | $AA^3$ | $AA^4$ | $AA^5$ | $W^c$ | $R^c$ |
|---|---|---|---|---|---|---|---|---|---|
| 599 | GABA | Asn | Asp | Ala | Leu | Met | Pro | single bond | $NH_2$ |

The structures of compounds represented by formula [I'-26], which were synthesized with the same method as in Example 2, are shown in the following table.

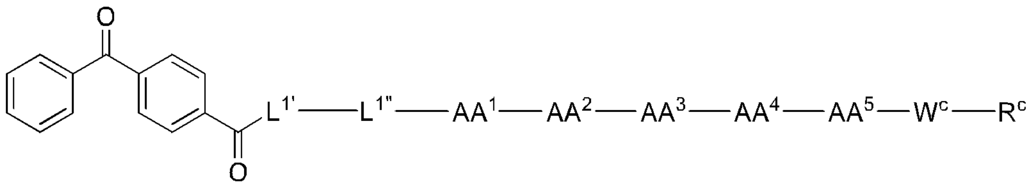

[I'-26]

[Table 33]

| Compound No. | $L^{1\cdot}$ | $L^{1-}$ | $AA^1$ | $AA^2$ | $AA^3$ | $AA^4$ | $AA^5$ | $W^c$ | $R^c$ |
|---|---|---|---|---|---|---|---|---|---|
| 600 | GABA | Asn | Asp | Dab | Leu | (N-Me) Ile | $\beta$-homo Pro | single bond | $NH_2$ |
| 601 | GABA | Asn | Asp | Dab | Leu | (N-Me) Glu | $\beta$-homo Pro | single bond | $NH_2$ |

The structures of compounds represented by formula [I'-27], which were synthesized with the same method as in Example 1, each are shown in the following table.

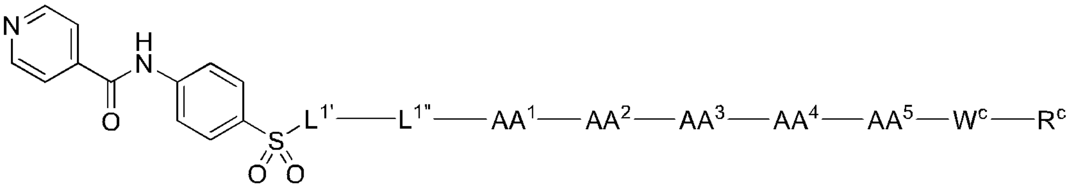

[I'-27]

[Table 34]

| Compound No. | $L^{1\cdot}$ | $L^{1-}$ | $AA^1$ | $AA^2$ | $AA^3$ | $AA^4$ | $AA^5$ | $W^c$ | $R^c$ |
|---|---|---|---|---|---|---|---|---|---|
| 602 | single bond | single bond | $\beta$-(d) -Asp | Dap | Leu | (N-Me) Glu | $\beta$-homo Pro | single bond | $NH_2$ |
| 603 | single bond | single bond | $\beta$-(d) -Asp | Dab | Leu | (N-Me) Glu | $\beta$-homo Pro | single bond | $NH_2$ |
| 604 | single bond | single bond | $\beta$-(d) -Asp | Dap | Leu | (N-Me) Ile | $\beta$-homo Pro | single bond | $NH_2$ |
| 605 | single bond | single bond | $\beta$-(d) -Asp | Dab | Leu | (N-Me) Ile | $\beta$-homo Pro | single bond | $NH_2$ |

The structure of a compound represented by formula [I'-28], which was synthesized with the same method as

in Example 1 is shown in the following table.

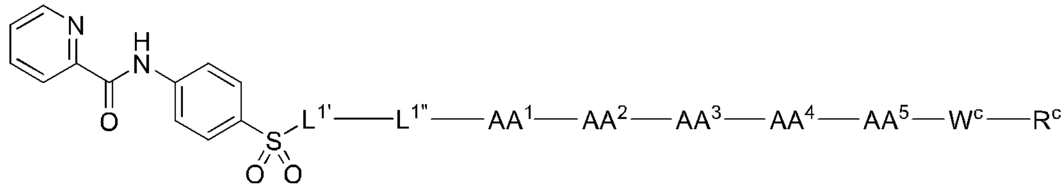

[I'-28]

[Table 35]

| Compound No. | $L^{1\cdot}$ | $L^{1-}$ | $AA^1$ | $AA^2$ | $AA^3$ | $AA^4$ | $AA^5$ | $W^c$ | $R^c$ |
|---|---|---|---|---|---|---|---|---|---|
| 606 | single bond | single bond | γ -(d) -Glu | (2S, 4S )-(4-a mino)P ro | Leu | (N-Me) Glu | Ape | single bond | 0H |

The structure of a compound represented by formula [I'-29], which was synthesized with the same method as in Example 1, is shown in the following table.

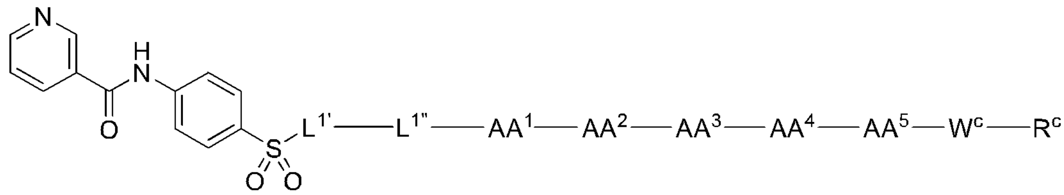

[I'-29]

[Table 36]

| Compound No. | $L^{1\cdot}$ | $L^{1-}$ | $AA^1$ | $AA^2$ | $AA^3$ | $AA^4$ | $AA^5$ | $W^c$ | $R^c$ |
|---|---|---|---|---|---|---|---|---|---|
| 607 | single bond | single bond | γ -(d) -Glu | (2S, 4S )- (4-a mino)P ro | Leu | (N-Me) Glu | Ape | single bond | 0H |

The structure of a compound represented by formula [I'-30], which was synthesized with the same method as in Example 5, is shown in the following table.

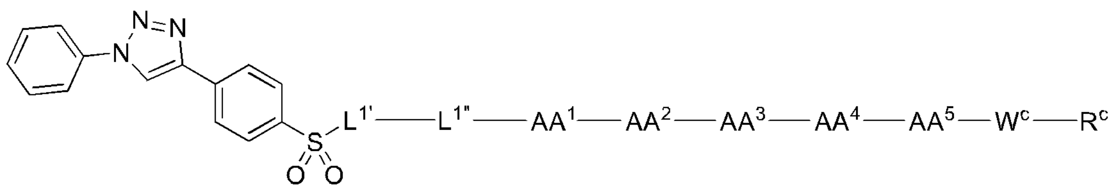

[I'-30]

[Table 37]

| Compound No. | $L^1$ | $L^2$ | $AA^1$ | $AA^2$ | $AA^3$ | $AA^4$ | $AA^5$ | $W^c$ | $R^c$ |
|---|---|---|---|---|---|---|---|---|---|
| 608 | single bond | single bond | γ - (d) -Glu | Dab | Leu | (N-Me) Ile | β -homo Pro | single bond | $NH_2$ |

The structure of a compound represented by formula [I'-31], which was synthesized with the same method as in Example 5, is shown in the following table.

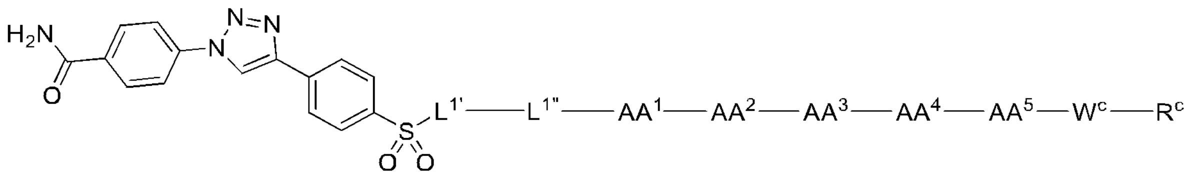

[I'-31]

[Table 38]

| Compound No. | $L^{1\cdot}$ | $L^{1-}$ | $AA^1$ | $AA^2$ | $AA^3$ | $AA^4$ | $AA^5$ | $W^c$ | $R^c$ |
|---|---|---|---|---|---|---|---|---|---|
| 609 | single bond | single bond | γ - (d) -Glu | Dab | Leu | (N-Me) Glu | Ape | single bond | $NH_2$ |

The structures of compounds represented by formula [I'-32], which were synthesized with the same method as in Example 1, are shown in the following table.

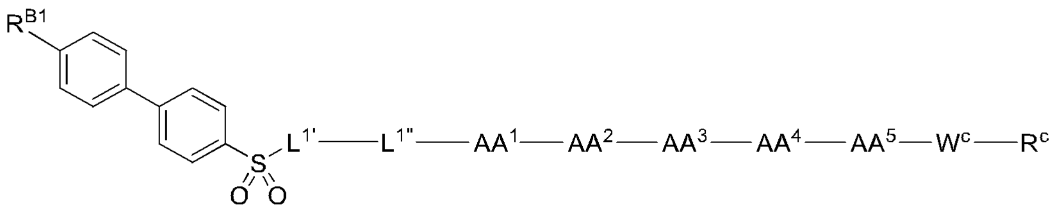

[I'-32]

[Table 39]

| Compound No. | $R^{B1}$ | $L^{1\cdot}$ | $L^{1-}$ | $AA^1$ | $AA^2$ | $AA^3$ | $AA^4$ | $AA^5$ | $W^c$ | $R^c$ |
|---|---|---|---|---|---|---|---|---|---|---|
| 610 | Me0 | single bond | single bond | β-(d) -Asp | Ala | Ala(2 -Pyr) | Met | Pro | single bond | $NH_2$ |
| 611 | Me0 | single bond | single bond | β-(d) -Asp | Dap | Leu | (N-Me) Glu | β-homo Pro | single bond | $NH_2$ |
| 612 | Me0 | single bond | single bond | β-(d)-Asp | Dab | Leu | (N-Me) Glu | β-homo Pro | single bond | $NH_2$ |
| 613 | Me0 | single bond | single bond | β-(d) -Asp | Dap | Leu | (N-Me) Ile | β-homo Pro | single bond | $NH_2$ |
| 614 | Me0 | single bond | single bond | β-(d) -Asp | Dab | Leu | (N-Me) Ile | β-homo Pro | single bond | $NH_2$ |
| 615 | $F_3C0$ | single bond | single bond | β-(d) -Asp | Dap | Leu | (N-Me) Ile | β-homo Pro | single bond | $NH_2$ |
| 616 | $F_3C0$ | single bond | single bond | β-(d) -Asp | Dab | Leu | (N-Me) Ile | β-homo Pro | single bond | $NH_2$ |

(continued)

| Compound No. | $R^{B1}$ | $L^{1-}$ | $L^{1-}$ | $AA^1$ | $AA^2$ | $AA^3$ | $AA^4$ | $AA^5$ | $W^c$ | $R^c$ |
|---|---|---|---|---|---|---|---|---|---|---|
| 617 | Et0 | single bond | single bond | β-(d)-Asp | Dap | Leu | (N-Me) Glu | β-homo Pro | single bond | $NH_2$ |
| 618 | Et0 | single bond | single bond | β-(d)-Asp | Dab | Leu | (N-Me) Glu | β-homo Pro | single bond | $NH_2$ |
| 619 | Et0 | single bond | single bond | β-(d)-Asp | Orn | Leu | (N-Me) Ile | β-homo Pro | single bond | $NH_2$ |
| 620 | H | single bond | single bond | β-(d)-Asp | (2S,4 S)-(4-amino )Pro | Leu | (N-Me) Glu | β-homo Pro | single bond | $NH_2$ |
| 621 | H | single bond | single bond | β-(d)-Asp | (2S,4 S)-(4-amino )Pro | Leu | (N-Me) Ile | β-homo Pro | single bond | $NH_2$ |
| 622 | MeO | single bond | single bond | β-(d)-Asp | (2S, 4 S)-(4-amino )Pro | Leu | (N-Me) Glu | β-homo Pro | single bond | $NH_2$ |
| 623 | MoO | single bond | single bond | β-(d)-Asp | (2S, 4 S)-(4-amino )Pro | Leu | (N-Me) Ile | β-homo Pro | single bond | $NH_2$ |
| 624 | F | single bond | single bond | β-(d)-Asp | (2S,4 S)-(4-amino )Pro | Leu | (N-Me) Glu | β-homo Pro | single bond | $NH_2$ |
| 625 | F | single bond | single bond | β-(d)-Asp | (2S,4 S)-(4-amino )Pro | Leu | (N-Me) Ile | β-homo Pro | single bond | $NH_2$ |
| 626 | C1 | single bond | single bond | β-(d)-Asp | (2S, 4 S)-(4-amino )Pro | Leu | (N-Me) Glu | β-homo Pro | single bond | $NH_2$ |
| 627 | C1 | single bond | single bond | β-(d)-Asp | (2S, 4 S)-(4-amino )Pro | Leu | (N-Me) Ile | β-homo Pro | single bond | $NH_2$ |
| 628 | Ac | single bond | single bond | β-(d)-Asp | (2S.4 S)-(4-amino )Pro | Leu | (N-Me) Glu | β-homo Pro | single bond | $NH_2$ |
| 629 | Ac | single bond | single bond | β-(d)--Asp | (2S, 4 S)-(4-amino )Pro | Leu | (N-Me) Ile | β-homo Pro | single bond | $NH_2$ |
| 630 | Me0 | single bond | single bond | γ-(d) -Glu | (2S, 4 S)-(4-amino )Pro | Leu | (N-Me) Glu | β-homo Pro | single bond | $NH_2$ |
| 631 | Me0 | single bond | single bond | γ-(d)-Glu | (2S, 4 S)-(4-amino )Pro | Leu | (N-Me) Ile | β-homo Pro | single bond | $NH_2$ |

(continued)

| Compound No. | $R^{B1}$ | $L^{1\cdot}$ | $L^{1-}$ | $AA^1$ | $AA^2$ | $AA^3$ | $AA^4$ | $AA^5$ | $W^c$ | $R^c$ |
|---|---|---|---|---|---|---|---|---|---|---|
| 632 | Me0 | single bond | single bond | γ-(d)-Glu | (2S, 4 S)-(4-amino ) Pro | Leu | (N-Me) Glu | Ape | single bond | $NH_2$ |
| 633 | MeO | single bond | single bond | γ-(d)-Glu | (2S,4 S)-(4-amino ) Pro | Leu | (N-Me) Glu | single bond | single bond | $NH_2$ |

The structures of compounds represented by formula [I'-32], which were synthesized in Example 6 or with the same method as in Example 6, are shown in the following table.

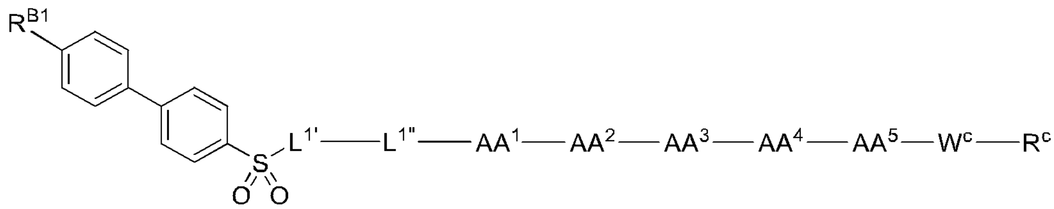

[I'-32]

[Table 40]

| Compound No. | $R^{B1}$ | $L^{1\cdot}$ | $L^{1-}$ | $AA^1$ | $AA^2$ | $AA^3$ | $AA^4$ | $AA^5$ | $W^c$ | $R^c$ |
|---|---|---|---|---|---|---|---|---|---|---|
| 634 | Ac | single bond | single bond | β-(d)-Asp | Ala | Leu | (N-Me) Ile | β-homo Pro | single bond | $NH_2$ |
| 635 | $H_2NCO$ | single bond | single bond | β-(d)-Asp | Ala | Leu | (N-Me) Ile | β-homo Pro | single bond | $NH_2$ |
| 636 | MeCONH | single bond | single bond | β-(d)-Asp | Ala | Leu | (N-Me) Ile | β-homo Pro | single bond | $NH_2$ |
| 637 | HO-$CH_2$ | single bond | single bond | β-(d)-Asp | Dap | Leu | (N-Me) Glu | β-homo Pro | single bond | $NH_2$ |
| 638 | HO-(C $H_2)_2$-O | single bond | single bond | β-(d)-Asp | Dap | Leu | (N-Me) Glu | β-homo Pro | single bond | $NH_2$ |
| 639 | HO-$CH_2$ | single bond | single bond | β-(d)-Asp | Dab | Leu | (N-Me) Glu | β-homo Pro | single bond | $NH_2$ |
| 640 | HO-$(CH_2)_2$-O | single bond | single bond | β-(d)-Asp | Dab | Leu | (N-Me) Glu | β-homo Pro | single bond | $NN_2$ |
| 641 | HO-$CH_2$ | single bond | single bond | β-(d)-Asp | Dap | Leu | (N-Me) Ile | β-homo Pro | single bond | $NH_2$ |

(continued)

| Compound No. | $R^{B1}$ | $L^{1\cdot}$ | $L^{1-}$ | $AA^1$ | $AA^2$ | $AA^3$ | $AA^4$ | $AA^5$ | $W^c$ | $R^c$ |
|---|---|---|---|---|---|---|---|---|---|---|
| 642 | HO-$CH_2$ | single bond | single bond | β- (d) -Asp | Dab | Leu | (N-Me) Ile | β -homo Pro | single bond | $NH_2$ |

The structure of a compound represented by formula [I'-33], which was synthesized with the same method as in Example 1, is shown in the following table.

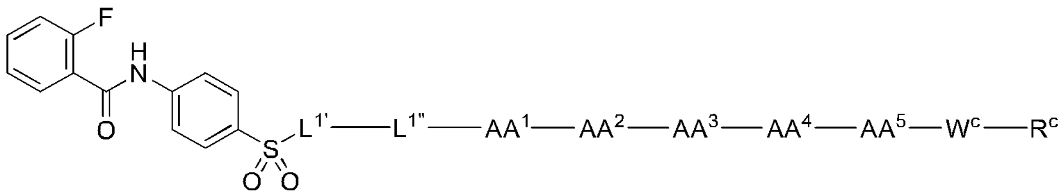

[I'-33]

[Table 41]

| Compound No. | $L^{1\cdot}$ | $L^{1-}$ | $AA^1$ | $AA^2$ | $AA^3$ | $AA^3$ | $AA^5$ | $W^c$ | $R^c$ |
|---|---|---|---|---|---|---|---|---|---|
| 643 | single bond | single bond | γ - (d) -Glu | (2S, 4S )-(4-a mino) Pro | Leu | (N-Me) Glu | Ape | single bond | OH |

The structures of compounds represented by formula [I'-34], which were synthesized with the same method as in Example 1, are shown in the following table.

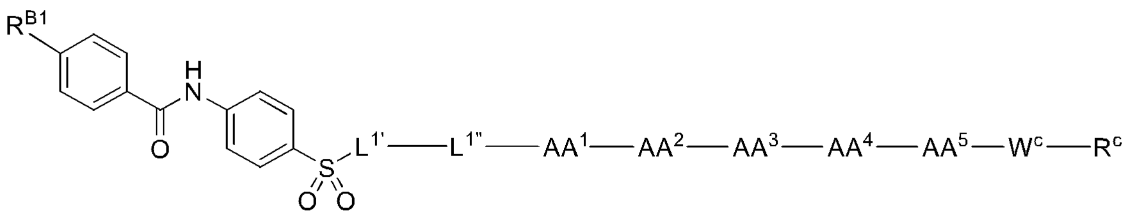

[I'-34]

[Table 42]

| Compound No. | $R^{B1}$ | $L^{1\cdot}$ | $L^{1-}$ | $AA^1$ | $AA^2$ | $AA^3$ | $AA^4$ | $AA^5$ | $W^c$ | $R^c$ |
|---|---|---|---|---|---|---|---|---|---|---|
| 644 | H | single bond | single bond | β-(d)-Asp | Dap | Leu | (N-Me) Gl u | β-homoP ro | single bond | $NH_2$ |
| 646 | H | single bond | single bond | β-(d)-Asp | Dap | Leu | (N-Me) Il e | β-homoP ro | single bond | $NH_2$ |
| 646 | H | single bond | single bond | β-(d)-Asp | Dab | Leu | (N-Me) Gl u | β-homoP ro | single bond | $NH_2$ |

(continued)

| Compound No. | $R^{B1}$ | $L^{1\cdot}$ | $L^{1-}$ | $AA^1$ | $AA^2$ | $AA^3$ | $AA^4$ | $AA^5$ | $W^c$ | $R^c$ |
|---|---|---|---|---|---|---|---|---|---|---|
| 647 | H | single bond | single bond | β-(d)-Asp | Da b | Leu | (N-Me) Il e | β-homoP ro | single bond | $NH_2$ |
| 648 | H | single bond | single bond | β-Asp | Dap | Leu | (N-Me) Gl u | β-homoP ro | single bond | $NH_2$ |
| 649 | H | single bond | single bond | γ-Glu | Dap | Leu | (N-Me) Il e | β-homoP ro | single bond | $NH_2$ |
| 650 | H | single bond | single bond | γ- (d) - Glu | Dap | Leu | (N-Me) Gl u | β-homoP ro | single bond | $NH_2$ |
| 651 | H | single bond | single bond | γ-(d) - Glu | Dap | Leu | (N-Me) Il β-( | β-homoP ro | single bond | $NH_2$ |
| 652 | H | single bond | single bond | γ-(d)-Glu | Dab | Leu | (N-Me) Gl u | β-homoP ro | single bond | $NH_2$ |
| 653 | H | single bond | single bond | γ-(d) - Glu | Dab | Leu | (N-Me) Il e | β-homoP ro | single bond | $NH_2$ |
| 654 | F | single bond | single bond | β-(d) - Asp | Dap | Leu | (N-Me) Gl u | β-homoP ro | single bond | $NH_2$ |
| 655 | F | single bond | single bond | β-(d) - Asp | Dap | Leu | (N-Me) Il e | β-homoP ro | single bond | $NH_2$ |
| 656 | F | single bond | single bond | β-(d) - Asp | Dab | Leu | (N-Me) Gl u | β-homoP ro | single bond | $NH_2$ |
| 657 | F | single bond | single bond | β-(d) -Asp | Dab | Leu | (N-Me) Il e | β-homoP ro | single bond | $NH_2$ |
| 658 | Me | single bond | single bond | β-(d) - Asp | Dap | Leu | (N-Me) Gl u | β-homoP ro | single bond | $NH_2$ |
| 659 | Me | single bond | single bond | β-(d)-Asp | Dap | Leu | (N-Me) Il e | β-homoP ro | single bond | $NH_2$ |
| 660 | Me | single bond | single bond | β-(d)-Asp | Dab | Leu | (N-Me) Gl u | β-homoP ro | single bond | $NH_2$ |
| 661 | Me | single bond | single bond | β-(d) - Asp | Dab | Leu | (N-Me) Il e | β-homoP ro | single bond | $NH_2$ |

(continued)

| Compound No. | $R^{B1}$ | $L^{1-}$ | $L^{1-}$ | $AA^1$ | $AA^2$ | $AA^3$ | $AA^4$ | $AA^5$ | $W^c$ | $R^c$ |
|---|---|---|---|---|---|---|---|---|---|---|
| 662 | $CP_3$ | single bond | single bond | β-(d)-Asp | Dap | Leu | (N-Me) Gl u | β-homoP ro | single bond | $NH_2$ |
| 663 | $CF_3$ | single bond | single bond | β-(d)-Asp | Dap | Leu | (N-Me) Il e | β-homoP ro | single bond | $NH_2$ |
| 664 | CFa | single bond | single bond | β-(d)-Asp | Dab | Leu | (N-Me) Gl u | β-homoP ro | single bond | $NH_2$ |
| 665 | CFa | single bond | single bond | β-(d)-Asp | Dab | Leu | (N-Me) Il e | β-homoP ro | single bond | $NH_2$ |
| 666 | Br | single bond | single bond | β-(d) - Asp | Dap | Leu | (N-Me) Gl u | β-homoP ro | single bond | $NH_2$ |
| 66T | Br | single bond | single bond | β-(d)-Asp | Dab | Leu | (N-Me) Gl u | β-homoP ro | single bond | $NH_2$ |
| 668 | CN | single bond | single bond | β-(d)-Asp | Dap | Le u | (N-Me) Gl u | β-homoP ro | single bond | $NH_2$ |
| 669 | CN | single bond | single bond | β-(d)-Asp | Dap | Leu | (N-Me) Gl u | β-homoP ro | single bond | $NH_2$ |
| 670 | H | Ape | Glu | Asp | Dab | Leu | (N-Me) Il e | β-homoP ro | single bond | $NH_2$ |
| 671 | Br | single bond | single bond | β - (d) - Asp | Dap | Leu | (N-Me) Il e | β-homoP ro | single bond | $NH_2$ |
| 672 | CN | single bond | single bond | β - (d) - Asp | Dap | Le u | (N-Me) Il e | β-homoP ro | single bond | $NH_2$ |
| 673 | Br | single bond | single bond | β-(d) - Asp | Dab | Leu | (N-Me) Il e | β -homoP ro | single bond | $NH_2$ |
| 674 | MeNHC 0 | single bond | single bond | β-(d)-Asp | Dap | Leu | (N-Me) Il e | β-homoP ro | single bond | $NH_2$ |
| **675** | **$Me_2NC$ 0** | single bond | single bond | β-(d)-Asp | **Dap** | Leu | (N-**Me) Ile** | β-**homoP** ro | single bond | $NH_2$ |
| **676** | **NeNHC 0** | single bond | single bond | β - (d) - Asp | Dab | Leu | (N-Me) 11 **e** | β-**homoP** ro | single bond | $NH_2$ |

(continued)

| Compound No. | $R^{B1}$ | $L^{1\cdot}$ | $L^{1-}$ | $AA^1$ | $AA^2$ | $AA^3$ | $AA^4$ | $AA^5$ | $W^c$ | $R^c$ |
|---|---|---|---|---|---|---|---|---|---|---|
| **677** | **$Me_2NC$ 0** | single bond | single bond | **$\beta$-(d)-Asp** | **Dab** | **Leu** | **(N-Me) Ile** | **$\beta$-homoPro** | single bond | **$NH_2$** |
| **678** | **MeNHC 0** | single bond | single bond | **$\beta$-(d)-Asp** | **Dap** | **Leu** | **(N-Me) Glu** | **$\beta$-homoPro** | single bond | **$NH_2$** |
| **679** | **$Me_2NC$ 0** | single bond | single bond | **$\beta$-(d)-Asp** | **Dap** | **Leu** | **(N-Me) Gl u** | **$\beta$-homoPro** | single bond | **$NH_2$** |
| **680** | **MeNHC 0** | single bond | single bond | **$\beta$-(d)-Asp** | **Dab** | **Leu** | **(N-Me) Gl u** | **$\beta$-homoPro** | single bond | **$NH_2$** |
| **681** | **$Me_2NC$ 0** | single bond | single bond | **$\beta$-(d)-Asp** | **Dab** | **Leu** | **(N-Me) Gl u** | **$\beta$-homoPro** | single bond | **$NH_2$** |
| **682** | **F** | single bond | single bond | **$\gamma$-Glu** | **(2S,4S)-(4-amino)Pro** | **Leu** | **(N-Me) Il e** | **Ape** | single bond | **$NH_2$** |
| **683** | **F** | single bond | single bond | **$\gamma$-Glu** | **(2S, 4 S)-(4-amino) Pro** | **Leu** | **(N-Me) Va 1** | **Ape** | single bond | **$NH_2$** |
| **684** | H | single bond | single bond | $\gamma$-(d)-Glu | **(2S,4S)**-(4-**amino**) Pro | Leu | (N-**Me) Gl** u | Ape | single bond | OH |
| **685** | **F** | single bond | single bond | **$\gamma$-(d)-Glu** | **(2S,4S)-(4-amino)Pro** | **Leu** | **(N-Me) Gl u** | **Ape** | single bond | **OH** |
| **686** | **Me** | single bond | single bond | **$\gamma$-(d)-Glu** | **(2S,4S)-(4-amino)Pro** | **Leu** | **(N-Me) Gl u** | **Ape** | single bond | **OH** |
| **687** | **$P_2CO$** | single bond | single bond | **$\gamma$-(d)-Glu** | **(2S,4S)-(4-amino)Pro** | **Leu** | **(N-Me) Gl** u | **Ape** | single bond | **OH** |
| **688** | **$F_2C$** | single bond | single bond | **$\gamma$-(d)-Glu** | **(2S,4S)-(4-amino)Pro** | **Leu** | **(N-Me) Gl u** | **Ape** | single bond | **OH** |
| **689** | **$F_2HC-CF_2$** | single bond | single bond | **$\gamma$-(d)-Glu** | **(2S,4S)-(4-amino)Pro** | **Leu** | **(N-Me) Gl u** | **Ape** | single bond | **OH** |
| **690** | **CN** | single bond | single bond | **$\gamma$-(d)-Glu** | **(2S,4S)-(4-amino)Pro** | **Leu** | **(N-Me) Gl u** | **Ape** | single bond | **OH** |

The structures of compounds represented by formula [I'-34], which were synthesized in Example 7 or with the same method as in Example 7, are shown in the following table.

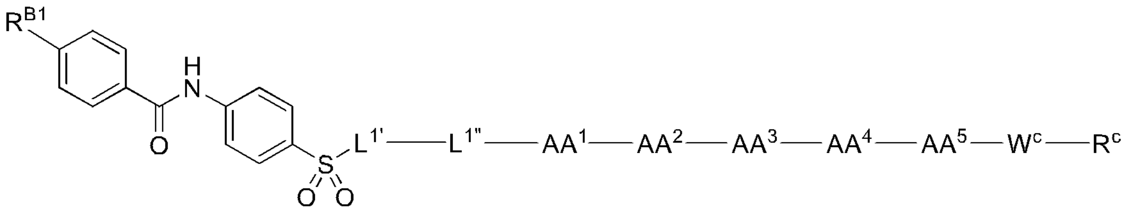

[I'-34]

[Table 43]

| Compound No. | $R^{B1}$ | $L^{1'}$ | $L^{1'}$ | $AA^1$ | $AA^2$ | $AA^3$ | $AA^4$ | $AA^5$ | $W^c$ | $R^c$ |
|---|---|---|---|---|---|---|---|---|---|---|
| **691** | **MeO** | single bond | single bond | **β - (d) -Asp** | **Dap** | **Leu** | **(N-Me) Glu** | **β-homo Pro** | single bond | **$NH_2$** |
| **692** | **MeO** | single bond | single bond | **β - (d) -Asp** | **Dap** | **Leu** | **(N-Me) Ile** | **β-homo Pro** | single bond | **$NH_2$** |
| **693** | **MeO** | single bond | single bond | **β - (d) -Asp** | **Dab** | **Leu** | **(N-Me) Glu** | **β-homo Pro** | single bond | **$NH_2$** |
| **694** | **MeO** | single bond | single bond | **β - (d) -Asp** | **Dab** | **Leu** | **(N-Me) Ile** | **β-homo Pro** | single bond | **$NH_2$** |
| **695** | **MeO** | single bond | single bond | **γ - (d) -Glu** | **Dab** | **Leu** | **(N-Me) Ile** | **β-homo Pro** | single bond | **$NH_2$** |
| **696** | **n-PrO** | single bond | single bond | **γ - (d) -Glu** | **Dab** | **Leu** | **(N-Me) Ile** | **β-homo Pro** | single bond | **$NH_2$** |
| **697** | **MeO** | single bond | single bond | **γ - (d) -Glu** | **Dab** | **Leu** | **(N-Me) Glu** | **β-homo Pro** | single bond | **$NH_2$** |
| **698** | **EtO** | single bond | single bond | **γ - (d) -Glu** | **Dab** | **Leu** | **(N-Me) Ile** | **β-homo Pro** | single bond | **$NH_2$** |
| **699** | **i-PrO** | single bond | single bond | **γ - (d) -Glu** | **Dab** | **Leu** | **(N-Me) Ile** | **β-homo Pro** | single bond | **$NH_2$** |
| **700** | **c-PrO** | single bond | single bond | **γ - (d) -Glu** | **Dab** | **Leu** | **(N-Me) Ile** | **β-homo Pro** | single bond | **$NH_2$** |
| **701** | **$F_3CO$** | single bond | single bond | **γ - (d) -Glu** | **Dab** | **Leu** | **(N-Me) Ile** | **β-homo Pro** | single bond | **$NH_2$** |
| **702** | **EtO** | single bond | single bond | **γ - (d) -Glu** | **Dab** | **Leu** | **(N-Me) Glu** | **β-homo Pro** | single bond | **$NH_2$** |
| **703** | **c-PrO** | single bond | single bond | **γ - (d) -Glu** | **Dab** | **Leu** | **(N-Me) Glu** | **β-homo Pro** | single bond | **$NH_2$** |
| **704** | **$F_3CO$** | single bond | single bond | **γ - (d) -Glu** | **Dab** | **Leu** | **(N-Me) Glu** | **β-homo Pro** | single bond | **$NH_2$** |

The structures of compounds represented by formula [I'-35], which were synthesized with the same method as in Example 1, are shown in the following table.

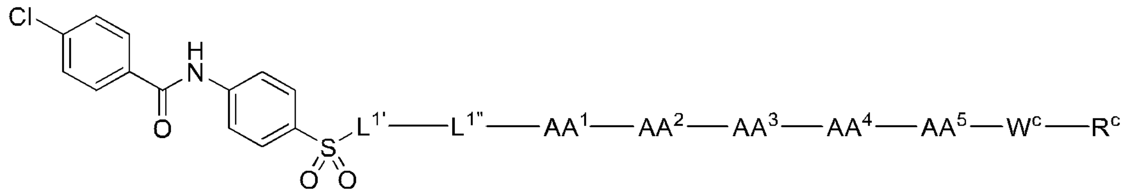

[I'-35]

[Table 44]

| Compound No. | $L^1$ | $L^1$ | $AA^1$ | $AA^2$ | $AA^3$ | $AA^4$ | $AA^5$ | $W^c$ | $R^c$ |
|---|---|---|---|---|---|---|---|---|---|
| **705** | single bond | single bond | **β - (d) - Asp** | **Dap** | **Leu** | **(N-Me)Glu** | **β - homoPr o** | single bond | **$NH_2$** |
| **706** | single bond | single bond | **β - (d) - Asp** | **Dap** | **Leu** | **(N-Me)Ile** | **β - homoPr o** | single bond | **$NH_2$** |
| **707** | single bond | single bond | **β - (d) - Asp** | **Dab** | **Leu** | **(N-Me)Glu** | **β - homoPr o** | single bond | **$NH_2$** |
| **708** | single bond | single bond | **β - (d) - Asp** | **Dab** | **Leu** | **(N-Me)Ile** | **β - homoPr o** | single bond | **$NH_2$** |
| **709** | single bond | single bond | **β - (d) - Asp** | **Dap** | **Leu** | **(N-Me)Glu** | single bond | single bond | **$NH_2$** |
| **710** | single bond | single bond | **β - (d) - Asp** | **Dap** | **Leu** | **(N-Me)Ile** | single bond | single bond | **$NH_2$** |
| **711** | single bond | single bond | **β - (d) - Asp** | **Dab** | **Leu** | **(N-Me)Glu** | single bond | single bond | **$NH_2$** |
| **712** | single bond | single bond | **β - (d) - Asp** | **Dab** | **Leu** | **(N-Me) Ile** | single bond | single bond | **$NH_2$** |
| **713** | single bond | single bond | **β - (d) - Asp** | **Ala** | **Leu** | **(N-Me)Glu** | single bond | single bond | **$NH_2$** |
| **714** | single bond | single bond | **β - (d) - Asp** | **Ala** | **Leu** | **(N-Me)Glu** | **β - homoPr o** | single bond | **$NH_2$** |
| **715** | single bond | single bond | **β - (d) - Asp** | **Ala** | **Leu** | **(N-Me) Ile** | **β - homoPro** | single bond | **$NH_2$** |
| **716** | single bond | single bond | **γ - (d) - Glu** | **Dap** | **Leu** | **(N-Me)Glu** | **β - homoPr o** | single bond | **$NH_2$** |
| **717** | single bond | single bond | **γ - (d) - Glu** | **Dap** | **Leu** | **(N-Me)Ile** | **β - homoPr o** | single bond | **$NH_2$** |
| **718** | single bond | single bond | **γ - (d) - Glu** | **Dab** | **Leu** | **(N-Me)Ile** | **β - homoPr o** | single bond | **$NH_2$** |

(continued)

| Compound No. | $L^1$ | $L^1$ | $AA^1$ | $AA^2$ | $AA^3$ | $AA^4$ | $AA^5$ | $W^c$ | $R^c$ |
|---|---|---|---|---|---|---|---|---|---|
| **719** | single bond | single bond | **γ - (d) - Glu** | **Dab** | **Leu** | **(N-Me)Glu** | **β - homoPr o** | single bond | **$NH_2$** |
| **720** | single bond | single bond | **γ - (d)-Glu** | **Dap** | **Leu** | **(N-Me) Ile** | **β - homoPr o** | **(d) - Lys** | **$NH_2$** |
| **721** | single bond | single bond | **γ - (d) - Glu** | **Dap** | **Leu** | **(N-Me)Ile** | **β - homoPr o** | **(d) - Arg** | **$NH_2$** |
| **722** | single bond | single bond | **γ - (d) - Glu** | **Dap** | **Leu** | **(N-Me)Ile** | **β - homoPr o** | **single bond** | **$NH-(CH_2)_2-NH_2$** |
| **723** | single bond | single bond | **γ - (d) - Glu** | **Dab** | **Leu** | **(N-Me) Ile** | **β - homoPr o** | **(d)-Lys** | **$NH_2$** |
| **724** | single bond | single bond | **γ - (d)-Glu** | **Dab** | **Leu** | **(N-Me)Ile** | **β - homoPr o** | **(d) - Arg** | **$NH_2$** |
| **725** | single bond | single bond | **γ - (d) - Glu** | **Dab** | **Leu** | **(N-Me)Ile** | **β - homoPr o** | **single bond** | **$NH-(CH_2)_2-NH_2$** |
| **726** | single bond | single bond | **γ - (d) - Glu** | **Dab** | **Nle** | **(N-Me)Ile** | **β - homoPr o** | single bond | **$NH_2$** |
| **727** | single bond | single bond | **γ - (d)-Glu** | **Dab** | **Leu** | **(N-Me)Ile** | **Ala** | single bond | **$NH_2$** |
| **728** | single bond | single bond | **γ - (d)-Glu** | **Dab** | **Leu** | **(N-Me) Ile** | **Thr** | single bond | **$NH_2$** |
| **729** | single bond | single bond | **γ - (d)-Glu** | **Dab** | **Leu** | **(N-Me) Ile** | **Phe** | single bond | **$NH_2$** |
| **730** | single bond | single bond | **γ - (d)-Glu** | **Dab** | **Leu** | **(N-Me) Ile** | **Lys** | single bond | **$NH_2$** |
| **731** | single bond | single bond | **γ - (d)-Glu** | **Dab** | **Leu** | **(N-Me)Ile** | **β -Ala** | single bond | **$NH_2$** |
| **732** | single bond | single bond | **γ - (d) - Glu** | **Dab** | **Leu** | **(N-Me) Ile** | **GABA** | single bond | **$NH_2$** |

(continued)

| Compound No. | $L^1$ | $L^1$ | $AA^1$ | $AA^2$ | $AA^3$ | $AA^4$ | $AA^5$ | $W^c$ | $R^c$ |
|---|---|---|---|---|---|---|---|---|---|
| **733** | single bond | single bond | γ - **(d) - Glu** | **Dab** | **Leu** | **(N-Me)Ile** | **Ape** | single bond | $NH_2$ |
| **734** | single bond | single bond | γ - **(d) - Glu** | **Dab** | **Leu** | **(N-Me)Ile** | **Acp** | single bond | $NH_2$ |
| **735** | single bond | single bond | γ - **(d) - Glu** | **Dab** | **Leu** | Lys (CO - $(CH_2)_{10}$ -$CO_2H$) | β - **homoPr o** | single bond | $NH_2$ |
| **736** | single bond | single bond | γ - **(d) - Glu** | **Dab** | **Leu** | Lys (CO - $(CH_2)_{12}$ -$CO_2H$) | β - **homoPro** | single bond | $NH_2$ |
| **737** | single bond | single bond | γ - **(d) - Glu** | **Dab** | **Leu** | Lys (CO - $(CH_2)_{14}$ -$CO_2H$) | β - **homoPr o** | single bond | $NH_2$ |
| **738** | single bond | single bond | γ - **(d) - Glu** | **Dab** | **Leu** | **(N-Me) Ile** | β **-Dap** | single bond | $NH_2$ |
| **739** | single bond | single bond | γ - **(d) - Glu** | **Dab** | **Leu** | **(N-Me)Ile** | β **- (d)-Dap** | single bond | $NH_2$ |
| **740** | single bond | single bond | γ - **(d) - Glu** | **Dab** | **Leu** | **(N-Me)Ile** | γ **-Dab** | single bond | $NH_2$ |
| **741** | single bond | single bond | γ - **(d) - Glu** | **Dab** | **Leu** | **(N-Me)Ile** | γ **- (d) - Dab** | single bond | $NH_2$ |
| **742** | single bond | single bond | γ - **(d) - Glu** | **Dab** | **Leu** | **(N-Me)Ile** | δ **-Orn** | single bond | $NH_2$ |
| **743** | single bond | single bond | γ - **(d) - Glu** | **Dab** | **Leu** | **(N-Me)Ile** | ε **-Lys** | single bond | $NH_2$ |
| **744** | single bond | single bond | γ - **(d) - Glu** | **Dab** | **Leu** | **(N-Me)Ile** | ε **- (d) - Lys** | single bond | $NH_2$ |
| **745** | single bond | single bond | γ - **(d) - Glu** | **Dab** | **Leu** | **(N-Me)Glu** | ε **-Lys** | single bond | $NH_2$ |
| **746** | single bond | single bond | γ - **(d) - Glu** | **Dab** | **Leu** | **(N-Me)Ile** | single bond | single bond | NH-$(CH_2)_4$ -$NH_2$ |

(continued)

| Compound No. | $L^1$ | $L^1$ | $AA^1$ | $AA^2$ | $AA^3$ | $AA^4$ | $AA^5$ | $W^c$ | $R^c$ |
|---|---|---|---|---|---|---|---|---|---|
| **747** | single bond | single bond | **γ - (d) - Glu** | **Dab** | **Leu** | **(N-Me)Ile** | **β - Ala** | single bond | **NH-$(CH_2)_2$ -$NH_2$** |
| **748** | single bond | single bond | **γ - (d) - Glu** | **Dab** | **Leu** | **(N-Me)Ile** | **β -Ala** | single bond | **NH-$(CH_2)_3$ -$NH_2$** |
| **749** | single bond | single bond | **γ - (d) - Glu** | **Dab** | **Leu** | **(N)-Me) Ile** | **β -Ala** | single bond | **NH-$(CH_2)_4$ -$NH_2$** |
| **750** | single bond | single bond | **γ - (d) - Glu** | **Dab** | **Leu** | **(N-Me) Ile** | **β -Ala** | single bond | **NH-$(CH_2)_5$ -$NH_2$** |
| **751** | single bond | single bond | **γ - (d)-Glu** | **Dab** | **Leu** | **(N-Me)Ile** | **GABA** | single bond | **NH-$(CH_2)_2$ -$NH_2$** |
| **752** | single bond | single bond | **γ - (d) - Glu** | **Dab** | **Leu** | **(N-Me)Ile** | **GABA** | single bond | **NH-$(CH_2)_3$ -$NH_2$** |
| **753** | single bond | single bond | **γ - (d)-Glu** | **Dab** | **Leu** | **(N-Me)Ile** | **GABA** | single bond | **NH-$(CH_2)_4$ -$NH_2$** |
| **754** | single bond | single bond | **γ - (d) - Glu** | **Dab** | **Leu** | **(N-Me) Ile** | **GABA** | single bond | **NH-$(CH_2)_5$ -$NH_2$** |
| **756** | single bond | single bond | **γ - (d) - Glu** | **Dab** | **Leu** | **(N-Me)Ile** | **Ape** | single bond | **NH-$(CH_2)_2$ -$NH_2$** |
| **756** | single bond | single bond | **γ - (d) - Glu** | **Dab** | **Leu** | **(N-Me)Ile** | **Ape** | single bond | **NH-$(CH_2)_3$ -$NH_2$** |
| **757** | single bond | single bond | **γ - (d) - Glu** | **Dab** | **Leu** | **(N-Me)Ile** | **Ape** | single bond | **NH-$(CH_2)_4$ -$NH_2$** |
| **758** | single bond | single bond | **γ - (d) - Glu** | **Dab** | **Leu** | **(N-Me) Ile** | **Ape** | single bond | **NH-$(CH_2)_5$ -$NH_2$** |
| **759** | single bond | single bond | **γ - (d) - Glu** | **Dab** | **Leu** | **(N-Me)Ile** | **(4)Abz** | single bond | **$NH_2$** |
| **760** | single bond | single bond | **γ - (d) - Glu** | **Dab** | **Leu** | **(N-Me)Ile** | **Ape** | single bond | **(4-Me)pip erazin -1-yl** |

(continued)

| Compound No. | $L^1$ | $L^1$ | $AA^1$ | $AA^2$ | $AA^3$ | $AA^4$ | $AA^5$ | $W^c$ | $R^c$ |
|---|---|---|---|---|---|---|---|---|---|
| **761** | single bond | single bond | **$\gamma$ - (d) - Glu** | **Dab** | **Leu** | **(N-Me)Ile** | **Ape** | single bond | **morpho lin-4-yl** |
| **762** | single bond | single bond | **$\gamma$ - (d) - Glu** | **Dab** | **Leu** | **(N-Me)Ile** | **Ape** | single bond | **[VI-8]** |
| **763** | single bond | single bond | **$\gamma$ - (d) - Glu** | **Dab** | **Leu** | **(N-Me)Ile** | **Ape** | single bond | **[VI-16]** |
| **764** | single bond | single bond | **$\gamma$ - (d) - Glu** | **Dab** | **Leu** | **(N-Me) Ile** | **Ape** | single bond | **[VI-18]** |
| **765** | single bond | single bond | **$\gamma$ - (d) - Glu** | **Dab** | **Leu** | **homoSe r** | **Ape** | single bond | **$NH_2$** |
| **766** | single bond | single bond | **$\beta$ - (d) - Asp** | **Dab** | **Leu** | **(N-Me)Ile** | **Ape** | **(d) - Lys** | **$NH_2$** |
| **767** | single bond | single bond | **$\beta$-- (d) - Asp** | **Dab** | **Leu** | **(N-Me)Ile** | **Ape** | **(d) - Arg** | **$NH_2$** |
| **768** | single bond | single bond | **$\beta$ - (d) - Asp** | **Dab** | **Leu** | **(N-Me)Ile** | **Ape** | **(d) - Arg (d) -Lys** | **$NH_2$** |
| **769** | single bond | single bond | **$\beta$ - (d) - Asp** | **Dab** | **Leu** | **(N-Me)Ile** | **Ape** | **(d) - Arg(d) -Arg** | **$NH_2$** |
| **770** | single bond | single bond | **$\beta$ - (d) - Asp** | **Dab** | **Leu** | **(N-Me) Ile** | **Ape** | **(d)-Lys (d) -Arg** | **$NH_2$** |
| **771** | single bond | single bond | **$\beta$ - (d) - Asp** | **Dab** | **Leu** | **(N-Me) Ile** | **Ape** | **(d) - Lys(d) -Lys** | **$NH_2$** |
| **772** | single bond | single bond | **$\beta$-(d) - Asp** | **(S)-pipera zine** | **Leu** | **(N-Me) Ile** | **Ape** | **(d) - Lys** | **$NH_2$** |
| **773** | single bond | single bond | **$\beta$ - (d) - Asp** | **Dab** | **Leu** | **(N-Me)Glu** | **Ape** | **(d) - Arg(d) -Lys** | **$NH_2$** |
| **774** | single bond | single bond | **$\beta$ - (d) - Asp** | **Dab** | **Leu** | **(N-Me)Glu** | **Ape** | **(d)-Arg(d) -Arg** | **$NH_2$** |

(continued)

| Compound No. | $L^1$ | $L^1$ | $AA^1$ | $AA^2$ | $AA^3$ | $AA^4$ | $AA^5$ | $W^c$ | $R^c$ |
|---|---|---|---|---|---|---|---|---|---|
| **775** | single bond | single bond | **β - (d) - Asp** | **Dab** | **Leu** | **(N-Me)Glu** | **Ape** | **(d)-Lys(d) -Arg** | **$NH_2$** |
| **776** | single bond | single bond | **β - (d) - Asp** | **Dab** | **Leu** | **(N-Me)Glu** | **Ape** | **(d) - Lys(d) -Lys** | **$NH_2$** |
| **777** | single bond | single bond | **β - (d) - Asp** | **Dab** | **Leu** | **(N-Me)Ile** | **γ - (d) - Dab** | single bond | **$NH_2$** |
| **778** | single bond | single bond | **β - (d) - Asp** | **Dab** | **Leu** | **(N-Me)Ile** | **δ -Orn** | single bond | **$NH_2$** |
| **779** | single bond | single bond | **β - (d) - Asp** | **Dab** | **Leu** | **(N-Me)Ile** | **δ - (d) - Orn** | single bond | **$NH_2$** |
| **780** | single bond | single bond | **β - (d) - Asp** | **Dab** | **Leu** | **(N-Me)Ile** | **ε -Lys** | single bond | **$NH_2$** |
| **781** | single bond | single bond | **β - (d) - Asp** | **Dab** | **Leu** | **(N-Me)Ile** | **ε - (d) - Lys** | single bond | **$NH_2$** |
| 782 | single bond | single bond | β-(d) -Asp | Dab | Leu | (N-Me)Val | Ape | (d)-Lys | $NH_2$ |
| 783 | single bond | single bond | β - (d)-Asp | Dab | Leu | (N-Me)Leu | Ape | (d)-Lys | $NH_2$ |
| 784 | single bond | single bond | β - (d)-Asp | Dab | Leu | (N-Me)Glu | Ape | (d) - Lys | $NH_2$ |
| 786 | single bond | single bond | β - (d)-Asp | Dab | Leu | (N-Me) Ile | Ape | (d)-Lys | OH |
| 786 | single bond | single bond | γ - (d)-Glu | (2S, 4S )-(4-amino) Pro | Leu | (N-Me)Glu | Pro | single bond | $NH_2$ |
| 787 | single bond | single bond | γ - (d) - Glu | (2S, 4S )-(4-amino) Pro | Leu | (N-Ne)Glu | GABA | single bond | $NH_2$ |
| 788 | single bond | single bond | γ - (d)-Glu | (2S, 4S )-(4-amino) Pro | Leu | (N-Me)Glu | Acp | single bond | $NH_2$ |

(continued)

| Compound No. | **$L^1$** | **$L^1$** | **$AA^1$** | **$AA^2$** | **$AA^3$** | **$AA^4$** | **$AA^5$** | **$W^c$** | **$R^c$** |
|---|---|---|---|---|---|---|---|---|---|
| 789 | single bond | single bond | $\gamma$ - (d) - Glu | (2S, 4S )-(4-amino) Pro | Leu | (N-Me)Asp | $\beta$-homoPr o | single bond | $NH_2$ |
| 790 | single bond | single bond | $\gamma$-(d) -Glu | (2S, 4S )-(4-amino) Pro | Leu | (N-Me) Asp | Pro | single bond | $NH_2$ |
| 791 | single bond | single bond | $\gamma$ -Glu | (2S, 4S ) - (4-amino) Pro | Leu | (N-Me)Ile | Ape | single bond | $NH_2$ |

The structures of compounds represented by formula [I'-36], which were synthesized with the same method as in Example 1, are shown in the following table.

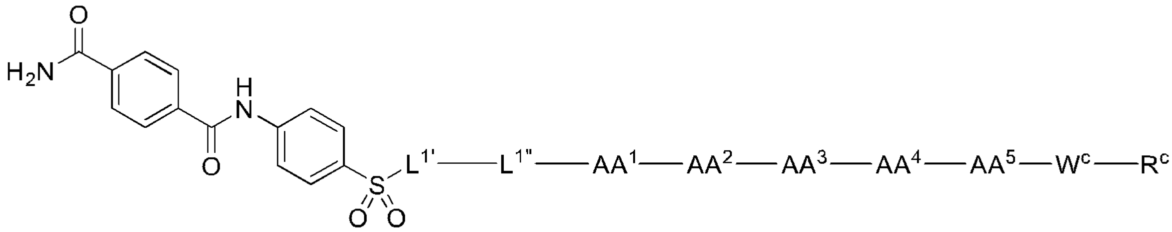

[I'-36]

[Table 45]

| Compound No. | $L^{1\cdot}$ | $L^{1-}$ | $AA^1$ | $AA^2$ | $AA^3$ | $AA^4$ | $AA^5$ | $W^c$ | $R^c$ |
|---|---|---|---|---|---|---|---|---|---|
| 792 | single bond | single bond | $\beta$ - (d)-Asp | Dap | Leu | (N-Me)Glu | $\beta$ - homoPr o | single bond | $NH_2$ |
| 793 | single bond | single bond | $\beta$ - (d)-Asp | Dab | Leu | (N-Me)Glu | $\beta$ - homoPr o | single bond | $NH_2$ |
| 794 | single bond | single bond | $\beta$ - (d)-Asp | Dap | Leu | (N-Me)Ile | $\beta$ - homoPr o | single bond | $NH_2$ |
| 795 | single bond | single bond | $\beta$ - (d)-Asp | Dab | Leu | (N-Me)Ile | $\beta$ - homoPr o | single bond | $NH_2$ |
| 796 | single bond | single bond | $\gamma$ - (d)-Glu | Dab | Leu | (N-Me)Ile | Ape | single bond | $NH_2$ |
| 797 | single bond | single bond | $\gamma$ - (d)-Glu | Dab | Leu | (N-Me) Ile | Acp | single bond | $NH_2$ |
| 798 | single bond | single bond | $\gamma$ - (d)-Glu | Dab | Leu | (N-Me)Glu | Ape | single bond | $NH_2$ |
| 799 | single bond | single bond | $\gamma$ - (d)-Glu | Dab | Leu | (N-Me)Glu | Acp | single bond | $NH_2$ |
| 800 | single bond | single bond | $\gamma$ - (d)-Glu | Dab | Leu | (N-Me)Glu | $\gamma$ -Dab | single bond | $NH_2$ |
| 801 | single bond | single bond | $\gamma$ - (d)-Glu | Dab | Ala(cP ropyl) | (N-Me)Ile | Ape | single bond | $NH_2$ |
| 802 | single bond | single bond | $\gamma$ - (d)-Glu | Dab | Ala(4-Thz) | (N-Me)Ile | Ape | single bond | $NH_2$ |
| 803 | single bond | single bond | $\beta$ - (d)-Asp | Dab | Leu | (N-Me)Ile | Ape | (d) - Lys | $NH_2$ |
| 804 | single bond | single bond | $\beta$ - (d)-Asp | Dab | Leu | (N-Me)Ile | Ape | (d)-Arg | $NH_2$ |
| 805 | single bond | single bond | $\beta$ - (d)-Asp | Dab | Leu | (N-Me)Ile | Ape | (d)-Arg-(d)-Lys | $NH_2$ |
| 806 | single bond | single bond | $\beta$ - (d)-Asp | Dab | Leu | (N-Me) Ile | Ape | (d)-Arg (d) -Arg | $NH_2$ |
| 807 | single bond | single bond | $\beta$ - (d)-Asp | Dab | Leu | (N-Me)Ile | Ape | (d)-Lys(d) -Lys | $NH_2$ |
| 808 | single bond | single bond | $\gamma$ - (d)-Glu | Dab | Leu | (N-Me)Ile | Ape | (d)-Arg | $NH_2$ |
| 809 | single bond | single bond | $\gamma$ - (d)-Glu | Dab | Leu | (N-Me)Ile | Ape | (d)-Arg(d) -Lys | $NH_2$ |
| 810 | single bond | single bond | $\gamma$ - (d)-Glu | Dab | Leu | (N-Me)Ile | Ape | (d)-Lys (d) -Arg | $NH_2$ |
| 811 | single bond | single bond | $\gamma$ - (d)-Glu | Dab | Leu | (N-Me)Ile | Ape | (d)-Lys (d) -Lys | $NH_2$ |
| 812 | single bond | single bond | $\gamma$ - (d)-Glu | Dab | Leu | (N-Me)Ile | Ape | (d)-Lys | $NH_2$ |
| 813 | single bond | single bond | $\gamma$ - (d)-Glu | Dab | Leu | (N-Me)Ile | Ape | (d)-Arg (d) -Arg | $NH_2$ |
| 814 | single bond | single bond | $\gamma$ - (d)-Glu | Dab | Leu | (N-Me)Ile | $\beta$ - (d)-Dap | single bond | $NH_2$ |
| 815 | single bond | single bond | $\gamma$ - (d)-Glu | Dab | Leu | (N-Me)Ile | $\delta$ -Orn | single bond | $NH_2$ |
| 816 | single bond | single bond | $\gamma$ - (d)-Glu | Dab | Leu | (N-Me)Ile | $\delta$-(d)-Orn | single bond | $NH_2$ |

(continued)

| Compound No. | $L^{1\cdot}$ | $L^{1-}$ | $AA^1$ | $AA^2$ | $AA^3$ | $AA^4$ | $AA^5$ | $W^c$ | $R^c$ |
|---|---|---|---|---|---|---|---|---|---|
| 817 | single bond | single bond | γ - (d)-Glu | Dab | Leu | (N-Me)Ile | ε -Lys | single bond | $NH_2$ |
| 818 | single bond | single bond | γ - (d)-Glu | Dab | Leu | (N-Me)Ile | ε-(d)-Lys | single bond | $NH_2$ |
| 819 | single bond | single bond | γ - (d)-Glu | Dab | Leu | (N-Me)Ile | β-homoPr o | single bond | $NH_2$ |
| 820 | single bond | single bond | γ - (d)-Glu | Dab | Leu | (N-Me)Ile | β - homoPr o | (d)-Lys | $NH_2$ |
| 821 | single bond | single bond | γ - (d)-Glu | Da b | Leu | (N-Me)Ile | β-homoPr o | (d)-Arg | $NH_2$ |
| 822 | single bond | single bond | γ-(d)-Glu | Dab | Leu | (N-Me)Ile | β - homoPr o | (d)-Arg(d) -Lys | $NH_2$ |
| 823 | single bond | single bond | γ - (d)-Glu | Dab | Leu | (N-Me)Ile | β - homoPr o | (d)-Arg(d) -Arg | $NH_2$ |
| 824 | single bond | single bond | γ - (d)-Glu | Dab | Leu | (N-Me)Ile | β - homoPr o | (d)-Lys(d) -Arg | $NH_2$ |
| 825 | single bond | single bond | γ - (d)-Glu | Dab | Leu | (N-Me)Ile | β - homoPr o | (d)-Lys(d) -Lys | $NH_2$ |
| 826 | single bond | single bond | γ - (d)-Glu | Dab | Leu | (N-Me)Val | Ape | single bond | $NH_2$ |
| 827 | single bond | single bond | γ - (d)-Glu | Dab | Leu | (N-Me)Val | β - homoPr o | single bond | $NH_2$ |
| 828 | single bond | single bond | γ-(d)-Glu | Dab | Leu | (N-Me)Ile | Ape | Gly-(d)-Lys | $NH_2$ |
| 829 | single bond | single bond | γ - (d)-Glu | Dab | Leu | (N-Me)Ile- | Ape | Gly-(d)-Arg | $NH_2$ |
| 830 | single bond | single bond | γ - (d)-Glu | Dab | Leu | (N-Me)Ile | Ape | Gly-(d) - Arg-(d)-Arg | $NH_2$ |
| 831 | single bond | single bond | γ - (d)-Glu | Dab | Leu | (N-Me)Ile | Ape | Gly-(d)-Lys(d) -Arg | $NH_2$ |
| 832 | single bond | single bond | γ - (d)-Glu | Dab | Leu | (N-Me)Ile | Ape | β - Ala-(d)-Lys | $NH_2$ |
| 833 | single bond | single bond | γ - (d)-Glu | Dab | Leu | (N-Me)Ile | Ape | β - Ala-(d) -Arg-(d) -Lys | $NH_2$ |
| 834 | single bond | single bond | γ - (d)-Glu | Dab | Leu | (N-Me)Ile | Ape | β-Ala-(d)-Arg-(d)-Arg | $NH_2$ |
| 835 | single bond | single bond | γ - (d)-Glu | Dab | Leu | (N-Me)Ile | Ape | β - Ala-(d) -Lys-(d)-Arg | $NH_2$ |
| 836 | single bond | single bond | γ - (d)-Glu | Dab | Leu | (N-Me)Ile | Ape | Gly-(d) -Arg-(d)-Lys | $NH_2$ |
| 837 | single bond | single bond | γ - (d)-Glu | Dab | Leu | (N-Me)Ile | Ape | Gly-(d)-Lys-(d) -Lys | $MH_2$ |
| 838 | single bond | single bond | β - (d)-Asp | Dab | Leu | (N-Me)Ile | β - homoPr o | single bond | $NH_2$ |
| 839 | single bond | single bond | β - (d)-Asp | Dab | Leu | (N-Me)Ile | Pro | single bond | $NH_2$ |
| 840 | single bond | single bond | β - (d)-Asp | Dab | Leu | (N-Me)Ile | β -Ala | single bond | $NH_2$ |
| 841 | single bond | single bond | β - (d)-Asp | Dab | Leu | (N-Me)Ile | GABA | single bond | $NH_2$ |

(continued)

| Compound No. | $L^{1\cdot}$ | $L^{1-}$ | $AA^1$ | $AA^2$ | $AA^3$ | $AA^4$ | $AA^5$ | $W^c$ | $R^c$ |
|---|---|---|---|---|---|---|---|---|---|
| 842 | single bond | single bond | $\beta$ - (d)-Asp | Dab | Leu | (N-Me)Ile | Ape | single bond | $NH_2$ |
| 843 | single bond | single bond | $\beta$ - (d)-Asp | Dab | Leu | (N-Me)Ile | Acp | single bond | $NH_2$ |
| 844 | single bond | single bond | $\beta$ - (d)-Asp | Dab | Leu | (N-Me)Ile | $\beta$ -Dap | single bond | $NH_2$ |
| 845 | single bond | single bond | $\gamma$ - (d)-Glu | Dab | Leu | (N-Me)Ile | Aze (2) | single bond | $MH_2$ |
| 846 | single bond | single bond | $\gamma$ - (d)-Glu | Dab | Leu | (N-Me)Ile | (d)-Aze (2) | single bond | $NH_2$ |
| 847 | single bond | single bond | $\gamma$ - (d)-Glu | Dab | Leu | (N-Me)Ile | Aze (3) | single bond | $NH_2$ |
| 848 | single bond | single bond | $\gamma$ - (d)-Glu | Dab | Leu | (N-Me)Ile | cis-NH(3)c Pen | single bond | $NH_2$ |
| 849 | single bond | single bond | $\gamma$ - (d)-Glu | Dab | Leu | (N-Me)Ile | cis-NIH(3)c Pen | single bond | $NH_2$ |
| 850 | single bond | single bond | $\gamma$- (d) - Glu | Dab | Leu | (N-Me)Ile | single bond | single bond | [VI-14] |
| 851 | single bond | single bond | β - (d)-Asp | (S)-pipera zine | Leu | (N-Me)Ile | Ape | single bond | $NH_2$ |
| 852 | single bond | single bond | $\gamma$ -Glu | Dab | Leu | (N-Me)Ile | Ape | single bond | $NH_2$ |
| 853 | single bond | single bond | $\gamma$ - (d)-Glu | Dab | Leu | (N-Me)Leu | Ape | single bond | $NH_2$ |
| 854 | single bond | single bond | $\gamma$ - (d)-Glu | Dab | Leu | (N-Me)Ile | Ape | single bond | OH |
| 855 | single bond | single bond | $\gamma$ - (d)-Glu | (S)-pipera 2ine | Leu | (N-Me)Ile | Ape | single bond | $NH_2$ |
| 856 | single bond | single bond | $\beta$ - (d)-Asp | (S)-pipera zine | Leu | (N-Me)Ile | $\beta$-hompPr o | single bond | $NH_2$ |
| 857 | single bond | single bond | $\gamma$ - (d)-Glu | Pro | Leu | (N-Me)Glu | Ape | single bond | $NH_2$ |
| 858 | single bond | single bond | $\gamma$ - (d)-Glu | (2S, 4S )-(4-amino) Pro | Leu | (N-Me)Glu | Ape | single bond | OH |
| 859 | single bond | single bond | $\gamma$-(d)-Glu | (2S, 4S )-(4-amino) Pro | Leu | (N-Me)Glu | single bond | single bond | OH |
| 860 | single bond | single bond | $\gamma$ - (d)-Glu | (2S, 4S )-(4-amino) Pro | Leu | single bond | Ape | single bond | $NH_2$ |
| 861 | single bond | single bond | $\gamma$ - (d)-Glu | (2S, 4S )-(4-amino) Pro | Leu | single bond | $\beta$ -Ala | single bond | $NH_2$ |
| 862 | single bond | single bond | $\gamma$ - (d)-Glu | (2S, 4S )-(4-amino) Pro | Leu | single bond | GABA | single bond | $NH_2$ |
| 863 | single bond | single bond | $\gamma$ - (d)-Glu | (2S, 4S )-(4-amino) Pro | Leu | single bond | Acp | single bond | $NH_2$ |
| 864 | single bond | single bond | $\gamma$ - (d)-Glu | (2S, 4S )-(4-amino) Pro | Leu | single bond | (d)-Lys | single bond | $NH_2$ |
| 865 | single bond | single bond | $\beta$- (d) - Asp | (2S, 4S )-(4-amino) Pro | Leu | (N-Me)Glu | single bond | single bond | $NH_2$ |
| 866 | single bond | single bond | $\gamma$ -Glu | (2S, 4S )-(4-amino) Pro | Leu | (N-Me)Glu | single bond | single bond | $NH_2$ |

(continued)

| Compound No. | $L^{1\cdot}$ | $L^{1-}$ | $AA^1$ | $AA^2$ | $AA^3$ | $AA^4$ | $AA^5$ | $W^c$ | $R^c$ |
|---|---|---|---|---|---|---|---|---|---|
| 867 | single bond | single bond | $\gamma$ - (d)-Glu | (2S, 4S )-(4-amino) Pro | Ile | (N-Me)Glu | single bond | single bond | $NH_2$ |
| 868 | single bond | single bond | $\gamma$ - (d)-Glu | (2S, 4S )-(4-amino) Pro | Leu | (N-Me)Ser | single bond | single bond | $NH_2$ |
| 869 | single bond | single bond | $\gamma$ - (d)-Glu | (2S, 4S )-(4-amino) Pro | Leu | (N-Me)Tyr | single bond | single bond | $NH_2$ |
| 870 | single bond | single bond | $\gamma$ - (d)-Glu | (2S, 4S )-(4-amino) Pro | Leu | (N-Me)Phe | single bond | single bond | $NH_2$ |
| 871 | single bond | single bond | $\gamma$ - (d)-Glu | (2S, 4S )-(4-amino) Pro | Leu | (N-Me)Ala | single bond | single bond | $NH_2$ |
| 872 | single bond | single bond | $\gamma$ - (d)-Glu | (2S, 4S )-(4-amino) Pro | Leu | (N-Me)Ile | single bond | single bond | $NM_2$ |
| 873 | single bond | single bond | $\gamma$ - (d)-Glu | (2S, 4S )-(4-amino) Pro | Leu | (N-Me)Val | single bond | single bond | $NH_2$ |
| 874 | single bond | single bond | $\gamma$ - (d)-Glu | (2S, 4S )-(4-amino) Pro | Leu | (N-Me)Leu | single bond | single bond | $NH_2$ |
| 875 | single bond | single bond | $\gamma$ - (d)-Glu | (2S, 4S )-(4-amino) Pro | Leu | (N-Me) Asp | single bond | single bond | $NH_2$ |
| 876 | single bond | single bond | $\gamma$ - (d)-Glu | (2S, 4S )-(4-amino) Pro | Leu | (N-Me)Lys | single bond | single bond | $MH_2$ |
| 877 | single bond | single bond | $\gamma$ - (d)-Glu | (2S, 4S )-(4-amino) Pro | Leu | Gly | single bond | single bond | $NH_2$ |
| 878 | single bond | single bond | $\gamma$ - (d)-Glu | (2S, 4S )-(4-amino) Pro | Leu | (d)-Arg | single bond | single bond | $NH_2$ |
| 879 | single bond | single bond | $\gamma$ - (d)-Glu | (2S, 4S )-(4-amino) Pro | Leu | (d)-Pro | single bond | single bond | $NH_2$ |
| 880 | single bond | single bond | $\gamma$ - (d)-Glu | (2S, 4S )-(4-amino) Pro | Leu | (d)-Ser | single bond | single bond | $NH_2$ |
| 881 | single bond | single bond | $\gamma$ - (d)-Glu | (2S, 4S )-(4-amino) Pro | Leu | (d)-Tyr | single bond | single bond | $NH_2$ |
| 882 | single bond | single bond | $\gamma$ - (d)-Glu | (2S, 4S )-(4-amino) Pro | Leu | (d)-Phe | single bond | single bond | $NH_2$ |
| 883 | single bond | single bond | $\gamma$ - (d)-Glu | (2S, 4S )-(4-amino) Pro | Leu | (d)-Ala | single bond | single bond | $NH_2$ |
| 884 | single bond | single bond | $\gamma$ - (d)-Glu | (2S, 4S )-(4-amino) Pro | Leu | (d)-Thr | single bond | single bond | $NH_2$ |
| 885 | single bond | single bond | $\gamma$ - (d)-Glu | (2S, 4S )-(4-amino) Pro | Leu | (N-Me)Arg | single bond | single bond | $NH_2$ |
| 886 | single bond | single bond | $\gamma$ - (d)-Glu | (2S, 4S )-(4-amino) Pro | Val | (N-Me)Glu | single bond | single bond | $NH_2$ |
| 887 | single bond | single bond | $\gamma$ - (d)-Glu | (2S, 4S )-(4-amino) Pro | Tyr | (N-Me)Glu | single bond | single bond | $NH_2$ |
| 888 | single bond | single bond | $\gamma$ - (d)-Glu | (2S, 4S )-(4-amino) Pro | Leu, | (d)-(N-Me)Glu | Ape | single bond | $NH_2$ |
| 889 | single bond | single bond | $\gamma$ - (d)-Glu | (2S, 4S )-(4-amino) Pro | Leu | Glu | Ape | single bond | $NH_2$ |
| 890 | single bond | single bond | $\gamma$ - (d)-Glu | (2S, 4S )-(4-amino) Pro | Leu | (N-Me)Glu | Ape | Ape | $NH_2$ |

The structures of compounds represented by formula [I'-37], which were synthesized in Example 8 or with the same method as in Example 8, are shown in the following table.

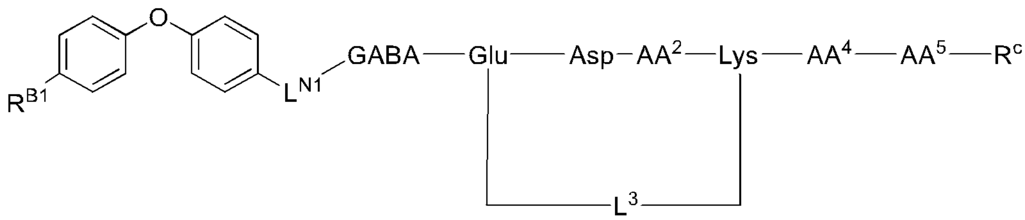

[I'-37]

[Table 46]

| Compound No. | $R^{BI}$ | $L^{NI}$ | $AA^2$ | $L^3$ | $AA^4$ | $AA^5$ | $R^c$ |
|---|---|---|---|---|---|---|---|
| 891 | H | S0$_2$ | Ala | Gly | (N-Me) Ile | β - homoPro | OH |
| 892 | H | S0$_2$ | Ala | Gly | (N-Me) Ile | β - homoPro | $NH_2$ |
| 893 | H | S0$_2$ | Ala | GABA | (N-Me) lie | β-homoPro | NII$_2$ |
| 894 | H | S0$_2$ | Dab | β-Ala | (N-Me) Ile | β - homoPro | $NH_2$ |
| 895 | H | S0$_2$ | Dab | β -Ala | (N-Me) Asp | β - homoPro | $NH_2$ |
| 896 | H | S0$_2$ | Dab | β -Ala | (N-Me) Ile | single bond | $NH_2$ |
| 897 | F | C0 | Ala | β -Ala | (N-Me) Ile | β - homoPro | $NH_2$ |
| 898 | F | C0 | Dab | β -Ala | (N-Me) Ile | β - homoPro | $NH_2$ |
| 899 | F | C0 | Dab | β -Ala | (N-Me) Asp | β - homoPro | $NH_2$ |

The structures of compounds represented by formula [I'-38], which were synthesized in Example 9 or with the same method as in Example 9, are shown in the following table.

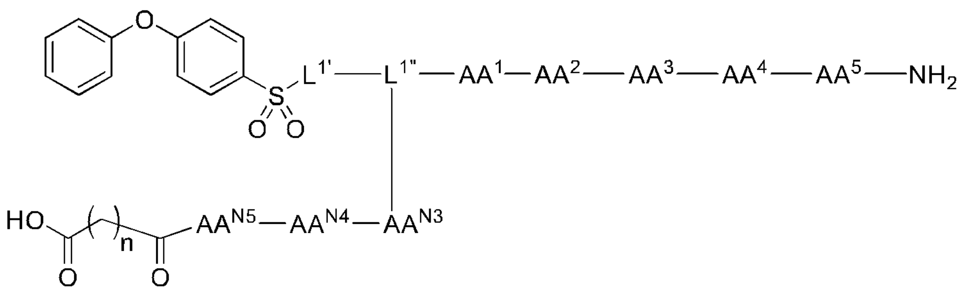

[I'-38]

[Table 47]

| Compound No. | L' | $L^{1-}$ | $AA^{N5}$ | $AA^{N4}$ | $AA^{N3}$ | n | $AA^1$ | $AA^2$ | $AA^3$ | $AA^2$ | $AA^5$ |
|---|---|---|---|---|---|---|---|---|---|---|---|
| 900 | GABA | Lys | γ-Glu | Adox | Adox | 4 | Asp | Ala | Leu | (N-Me) Ile | β - homoPro |
| 901 | GABA | Lys | γ - Glu | Adox | Adox | 6 | Asp | Ala | Leu | (N-Me) Ile | β-homoPro |
| 902 | GABA | Lys | Lys | Lys | Lys | 8 | Asp | Ala | Leu | (N-Me) Ile | β-homoPro |
| 903 | GABA | Lys | γ-Glu | Adox | Adox | 14 | Asp | Ala | Leu | (N-Me) Ile | β - homoPro |
| 904 | GABA | Lys | γ – Glu | Adox | Adox | 16 | Asp | Ala | Leu | (N-Me) Ile | β-homoPro |
| 905 | GABA | Lys | Lys | Lys | Lys | 14 | Asp | Ala | Leu | (N-Me) Ile | β-homoPro |

(continued)

| Compound No. | L' | L1- | $AA^{N5}$ | $AA^{N4}$ | $AA^{N3}$ | n | $AA^1$ | $AA^2$ | $AA^3$ | $AA^2$ | $AA^5$ |
|---|---|---|---|---|---|---|---|---|---|---|---|
| 906 | GABA | Lys | γ-Glu | Adox | Adox | 14 | Asp | Dab | Leu | (N-Me) Glu | β-homoPro |
| 907 | GABA | Lys | Lys | Lys | Lys | 14 | Asp | Dab | Leu | (N-Me) Glu | β - homoPro |
| 908 | GABA | Lys | (d)-Lys | (d) - Lys | (d)-Lys | 14 | Asp | Dab | Leu | (N-Me) Ile | β-homoPro |
| 909 | GABA | Lys | (d) - Lys | (d)-Lys | (d)-Lys | 14 | Asp | Dab | Leu | (N-Me) Glu | β-homoPro |
| 910 | GABA | (d)-Lys | γ - Glu | Adox | Adox | 14 | Asp | Dab | Leu | (N-Me) Glu | β-homoPro |
| 911 | GABA | (d)-Lys | (d) - Lys | (d) - Lys | (d) - Lys | 14 | Asp | Dab | Leu | (N-Me) Glu | β - homoPro |

The structures of compounds represented by formula [I'-39], which were synthesized with the same method as in Example 9, are shown in the following table.

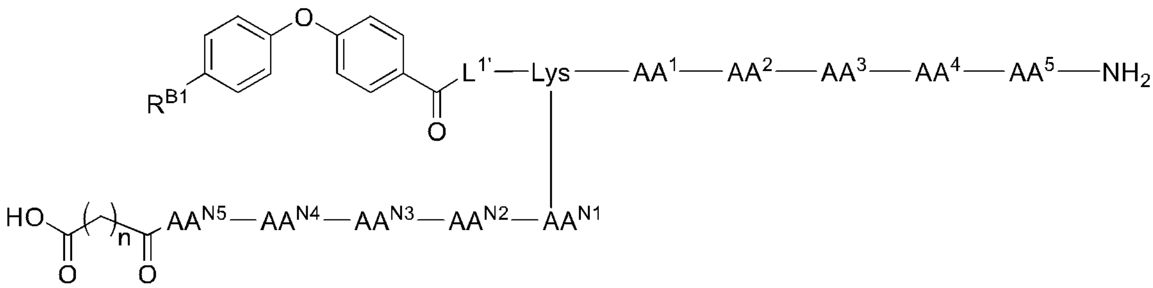

[I'-39]

[Table 48]

| Compound No. | $R^{B1}$ | L1' | $AA^{N5}$ | $AA^{N4}$ | $AA^{N3}$ | $AA^{N2}$ | $AA^{N1}$ | n | $AA^1$ | $AA^2$ | $AA^3$ | $AA^4$ | $AA^5$ |
|---|---|---|---|---|---|---|---|---|---|---|---|---|---|
| 912 | F | GAB A | (d) Lys | (d) - Lys | (d) - Lys | single bond | single bond | 14 | Asp | Dab | Leu | (N-Me) I le | β- hom oPr o |
| 913 | F | GAB A | (d) - Lys | (d) - Lys | (d) - Lys | single bond | single bond | 14 | Asp | Dab | Leu | (N-Me) A sp | β- hom oPr o |
| 914 | $H_2NC$ 0 | GAB A | γ-Glu | Ado x | Ado x | single bond | single bond | 14 | Asp | Dab | Leu | (N-Me) I le | β- hom oPr o |
| 915 | $H_2NC$ 0 | GAB A | γ-Glu | Ado x | Ado x | single bond | single bond | 14 | Asp | Dab | Leu | (N-Me) A sp | β" hom oPr o |
| 916 | $H_2NC$ 0 | GAB A | (d) - Lys | (d) - Lys | (d) - Lys | single bond | single bond | 14 | Asp | Dab | Leu | (N-Me) I le | β-hom oPr o |
| 917 | $H_2NC$ 0 | GAB A | (d) - Lys | (d) - Lys | (d) - Lys | single bond | single bond | 14 | Asp | Dab | Leu | (N-Me) A sp | β-hom oPr o |
| 918 | $H_2NC$ 0 | Ape | γ - Glu | Ado x | Ado x | single bond | single bond | 14 | Asp | Dab | Leu | (N-Me)G lu | β-hom oPr o |
| 919 | $H_2NC$· 0 | Ape | γ-Glu | Ado x | Ado x | single bond | single bond | 14 | Asp | Dab | Leu | (N-Me) I le | β-hom oPr o |
| 920 | $H_2NC$ 0 | Ape | (d) - Lys | (d) - Lys | (d) Lys | single bond | single bond | 14 | Asp | Dab | Leu | (N-Me)G lu | β- hom oPr o |
| 921 | $H_2NC$ 0 | Ape | (d) - Lys | (d) - Lys | (d) - Lys | single bond | single bond | 14 | Asp | Dab | Leu | (N-Me) I le | β-hom oPr o |
| 922 | $H_2NC$ 0 | Ape | γ - Glu | Ado x | Ado x | single bond | single bond | 12 | Asp | Dab | Leu | (N-Me)G lu | β- hom oPr o |
| 923 | $H_2NC$ 0 | Ape | γ - Glu | Ado x | Ado x | single bond | single bond | 12 | Asp | Dab | Leu | (N-Me) I le | β- hom oPr o |
| 924 | $H_2NC$ 0 | Ape | (d) - Lys | (d) - Lys | (d) - Lys | single bond | single bond | 12 | Asp | Dab | Leu | (N-Me) G lu | β- hom oPr o |
| 925 | $H_2NC$ 0 | Ape | (d) - Lys | (d) - Lys | (d) Lys | single bond | single bond | 12 | Asp | Dab | Leu | (N-Me) I le | β- hom oPr o |
| 926 | $H_2NC$ 0 | Ape | (d) - Lys | single bond | single bond | single bond | single bond | 14 | Asp | Dab | Leu | (N-Me) I le | β- hom oPr o |
| 927 | $H_2NC$ 0 | Ape | (d) - Lys | (d) - Lys | single bond | single bond | single bond | 14 | Asp | Dab | Leu | (N-Me)I le | β- hom oPr o |
| 928 | $H_2NC$ 0 | Ape | (d) - Lys | (d) - Lys | (d) - Lys | (d) - Lys | single bond | 14 | Asp | Dab | Leu | (N-Me) I le | β- hom oPr o |
| 929 | $H_2NC$ 0 | Ape | (d) Lys | (d) Lys | (d) Lys | (d) Lys | (d) Lys | 14 | Asp | Dab | Leu | (N-Me) I le | β-hom oPr o |
| 930 | $H_2NC$ 0 | Ape | (d) - Lys | single bond | single bond | single bond | single bond | 12 | Asp | Dab | Leu | (N-Me)G lu | β- hom oPr o |
| 931 | $H_2NC$ 0 | Ape | (d) - Lys | (d) - Lys | single bond | single bond | single bond | 12 | Asp | Dab | Leu | (N-Me) G lu | β- hom oPr o |
| 932 | $H_2NC$ 0 | · Ape | (d) - Lys | (d) - Lys | (d) - Lys | (d) Lys | single bond | 12 | Asp | Dab | Leu | (N-Me)G lu | β- hom oPr o |
| 933 | $H_2NC$ 0 | Ape | (d) - Lys | (d) Lys | (d) Lys | (d) Lys | (d) Lys | 12 | Asp | Dab | Leu | (N-Me)G lu | β- hom oPr o |
| 934 | $H_2NC$ 0 | Ape | Lys | Lys | Lys | single bond | single bond | 12 | Asp | Dab | Leu | (N-Me)G lu | β- hom oPr o |
| 935 | $H_2NC$ 0 | Ape | Arg | Arg | Arg | single bond | single bond | 12 | Asp | Dab | Leu | (N-Me)G lu | β- hom oPr o |
| 936 | $H_sNC$. 0 | Ape | (d) - Arg | (d) - Arg | (d) Arg | single bond | single bond | 12 | Asp | Dab | Leu | (N-Me)G lu | β- hom oPr o |

(continued)

| Compound No. | $R^{B1}$ | L1' | $AA^{N5}$ | $AA^{N4}$ | $AA^{N3}$ | $AA^{N2}$ | $AA^{N1}$ | n | $AA^1$ | $AA^2$ | $AA^3$ | $AA^4$ | $AA^5$ |
|---|---|---|---|---|---|---|---|---|---|---|---|---|---|
| 937 | $H_2$NC 0 | Ape | Ado x | Ado x | single bond | single bond | single bond | 12 | Asp | Dab | Leu | (N-Me)G lu | $\beta$- hom oPr o |
| 938 | $H_2$NG 0 | Ape | Ado x | single bond | single bond | single bond | single bond | 12 | Asp | Dab | Leu | (N-Me) G lu | $\beta$- hom oPr o |
| 939 | $H_2$NC 0 | Ape | $\gamma$ - Glu | single bond | single bond | single bond | single bond | 12 | Asp | Dab | Leu | (N-Me)G lu | $\beta$- hom oPr o |
| 940 | $H_2$NC 0 | Ape | Ado x | Ado x | $\gamma$-Glu | single bond | single bond | 12 | Asp | Dab | Leu | (N-Me) G lu | $\beta$- hom oPr o |
| 941 | $H_2$NC 0 | Ape | single bond | single bond | single bond | single bond | single bond | 12 | Asp | Dab | Leu | (N-Me)G lu | $\beta$- hom oPr o |
| 942 | $H_2$NC 0 | Ape | (d) - Lys | single bond | single bond | single bond | single bond | 12 | Asp | Dab | Leu | (N-Me) I le | $\beta$- hom oPr o |
| 943 | $H_2$NC 0 | Ape | (d) Lys | (d) - Lys | single bond | single bond | single bond | 12 | Asp | Dab | Leu | (N-Me) I le | $\beta$- hom oPr o |
| 944 | $H_2$NC 0 | Ape | (d) - Lys | single bond | single bond | single bond | single bond | 14 | Asp | Dab | Leu | (N-Me) G lu | $\beta$- hom oPr o |
| 946 | $H_2$NC 0 | Ape | (d) - Lys | (d) - Lys | single bond | single bond | single bond | 14 | Asp | Dab | Leu | (N-Me) G lu | $\beta$- hom oPr o |
| 946 | $H_2$NC 0 | Ape | (d) - Lys | (d) - Lys | (d) Lys | single bond | single bond | 10 | Asp | Dab | Leu | (N-Me)G lu | $\beta$- hom oPr o |
| 947 | $H_2$NC 0 | Ape | (d) - Lys | (d) - Lys | (d) - Lys | single bond | single bond | 10 | Asp | Dab | Leu | (N-Me) I le | $\beta$- hom oPr o |
| 948 | $H_2$NC 0 | Ape | (d) Arg | single bond | single bond | single bond | single bond | 12 | Asp | Dab | Leu | (N-Me) G lu | $\beta$- hom oPr o |
| 949 | $H_2$NG 0 | Ape | (d) - Arg | (d) - Arg | single bond | single bond | single bond | 12 | Asp | Dab | Leu | (N-Me) G lu | $\beta$- horn oPr o |
| 950 | $H_2$NC 0 | Ape | (d) - Lys | (d) - Lys | (d) - Lys | single bond | single bond | 12 | Asp | Dab | Leu | (N-Me)G lu | $\beta$- hom oPr o |
| 951 | $H_2$NC 0 | Ape | single bond | single bond | single bond | single bond | single bond | 12 | Asp | Dab | Leu | (N-Me) I le | $\beta$- hom oPr o |
| 952 | $H_2$NC 0 | Ape | single bond | single bond | single bond | single bond | single bond | 14 | Asp | Dab | Leu | (N-Me) G lu | $\beta$- hom oPr o |
| 953 | $H_2$NC 0 | Ape | single bond | single bond | single bond | single bond | single bond | 14 | Asp | Dab | Leu | (N-Me) I le | $\beta$- hom oPr o |
| 954 | $H_2$NC 0 | Ape | (d) - Lys | (d) Lys | (d) - Lys | single bond | single bond | 16 | Asp | Dab | Leu | (N-Me) G lu | $\beta$- hom oPr o |
| 956 | $H_2$NC 0 | Ape | (d) Lys | (d) - Lys | (d) - Lys | single bond | single bond | 16 | Asp | Dab | Leu | (N-Me)I le | $\beta$- hom oPr o |

The structures of compounds represented by formula [I'-40], which were synthesized with the same method as in Example 9, are shown in the following table:

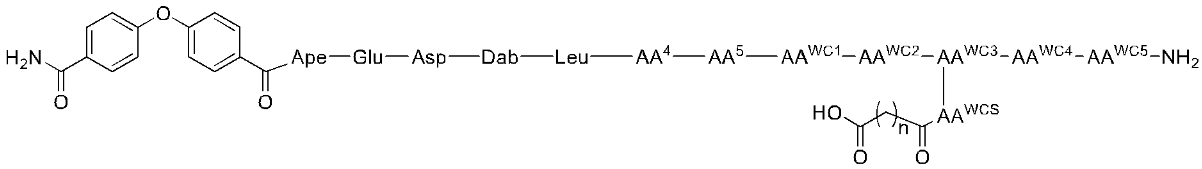

[I'-40]

wherein

the structure represented by formula [VII-2]:

$-AA^{WC1}-AA^{WC2}-AA^{WC3}-AA^{WC4}-AA^{WC5}-$ [VII-2]

corresponds to $W^C$, which is "linker consisting of one to three amino acids" in the compound represented by formula [I'], wherein

$AA^{WC1}$, $AA^{WC2}$, $AA^{WC4}$, and $AA^{WC5}$ are same or different and each selected from the group consisting of a single bond and (d)-Lys,
$AA^{WC3}$ is Lys or (d)-Lys, and
$AA^{WCS}$ is a single bond or (d)-Lys.

[Table 49]

| Compound No. | $AA^4$ | $AA^5$ | $AA^{WC1}$ | $AA^{WC2}$ | $AA^{WC3}$ | $AA^{WC4}$ | $AA^{WC5}$ | $AA^{WCS}$ | n |
|---|---|---|---|---|---|---|---|---|---|
| 956 | (N-Me) Ile | β - homoPro | (d) - Lys | (d)-Lys | Lys | single bond | single bond | single bond | 12 |
| 957 | (N-Me) Ile | β - homoPro | (d)-Lys | (d)-Lys | Lys | single bond | single bond | single bond | 14 |
| 958 | (N-Me) Glu | β - homoPro | (d) - Lys- | (d) -Lys | Lys | single bond | single bond | single bond | 14 |
| 959 | (N-Me) Ile | β - homoPro | (d) - Lys | single bond | Lys | (d)-Lys | single bond | single bond | 12 |
| 960 | (N-Me) Ile | β - homoPro | (d) - Lys | single bond | Lys | (d) - Lys | single bond | single bond | 14 |
| 961 | (N-Me) Glu | β - homoPro | (d)-Lys | single bond | Lys | (d)-Lys | single bond | single bond | 12 |
| 962 | (N-Me) Glu | β - homoPro | (d)-Lys | single bond | Lys | (d)-Lys | single bond | single bond | 14 |

(continued)

| Compound No. | AA4 | AA5 | AA$^{WC1}$ | AA$^{WC2}$ | AA$^{WC3}$ | AA$^{WC4}$ | AA$^{WC5}$ | AA$^{WCS}$ | n |
|---|---|---|---|---|---|---|---|---|---|
| 963 | (N-Me) Ile | $\beta$ - homoPro | single bond | single bond | Lys | (d)-Lys | (d)-Lys | single bond | 12 |
| 964 | (N-Me) Ile | $\beta$-homoPro | single bond | single bond | Lys | (d)-Lys | (d)-Lys | single bond | 14 |
| 965 | (N-Me) Glu | $\beta$ - homoPro | single bond | single bond | Lys | (d) - Lys | (d)-Lys | single bond | 12 |
| 966 | (N-Me) Glu | $\beta$ - homoPro | single bond | single bond | Lys | (d) - Lys | (d)-Lys | single bond | 14 |
| 967 | (N-Me) Ile | $\beta$ - homoPro | single bond | single bond | Lys | single bond | single bond | single bond | 12 |
| 968 | (N-Me) Ile | $\beta$ - homoPro | single bond | single bond | Lys | single bond | single bond | single bond | 14 |
| 969 | (N-Me) Ile | $\beta$ - homoPro | single bond | single bond | (d)-Lys | single bond | single bond | single bond | 12 |
| 970 | (N-Me) Ile | $\beta$ - homoPro | single bond | single bond | (d) - Lys | single bond | single bond | single bond | 14 |
| 971 | (N-Me) Ile | $\beta$ - homoPro | single bond | single bond | Lys | single bond | single bond | (d)-Lys | 12 |
| 972 | (N-Me) Ile | $\beta$ - homoPro | single bond | single bond | Lys | single bond | single bond | (d) - Lys | 14 |

Mass spectra of high-performance liquid chromatography (LCMS), retention time (RT), and analysis conditions for the present inventive compounds are shown in the following table.

[Table 50]

| Compound No. | LCMS analysis | | | |
|---|---|---|---|---|
| | MS (found) | MS (calc.) | Retention time RT (min) | Analysis condition |
| 1 | 916.7 $[M+H]^+$ | 916.4 $[M+H]^+$ | 0.97 | A |
| 2 | 902.5 $[M+H]^+$ | 902.4 $[M+H]^+$ | 0.98 | A |
| 3 | 902.5 $[M+H]^+$ | 902.4 $[M+H]^+$ | 0.99 | A |
| 4 | 916.4 $[M+H]^+$ | 916.4 $[M+H]^+$ | 0.94 | A |
| 5 | 902.4 $[M+H]^+$ | 902.4 $[M+H]^+$ | 0.81 | A |
| 6 | 916.4 $[M+H]^+$ | 916.4 $[M+H]^+$ | 0.90 | A |
| 7 | 950.4 $[M+H]^+$ | 950.4 $[M+H]^+$ | 0.99 | A |

(continued)

| Compound No. | LCMS analysis | | | |
|---|---|---|---|---|
| | MS (found) | MS (calc.) | Retention time RT (min) | Analysis condition |
| 8 | 989.4 $[M+H]^+$ | 989.4 $[M+H]^+$ | 1.01 | A |
| 9 | 900.6 $[M+H]^+$ | 900.4 $[M+H]^+$ | 1.08 | A |
| 10 | 886.4 $[M+H]^+$ | 886.4 $[M+H]^+$ | 1.02 | A |
| 11 | 900.5 $[M+H]^+$ | 900.4 $[M+H]^+$ | 1.10 | A |
| 12 | 858.4 $[M+H]^+$ | 858.4 $[M+H]^+$ | 0.98 | A |
| 13 | 934.4 $[M+H]^+$ | 934.4 $[M+H]^+$ | 1.20 | A |
| 14 | 950.5 $[M+H]^+$ | 950.4 $[M+H]^+$ | 1.02 | A |
| 15 | 874.4 $[M+H]^+$ | 874.4 $[M+H]^+$ | 0.88 | A |
| 16 | 870.4 $[M+H]^+$ | 870.4 $[M+H]^+$ | 0.96 | A |
| 17 | 914.5 $[M+H]^+$ | 914.4 $[M+H]^+$ | 0.99 | A |
| 18 | 900.4 $[M+H]^+$ | 900.3 $[M+H]^+$ | 0.95 | A |
| 19 | 900.3 $[M+H]^+$ | 900.3 $[M+H]^+$ | 0.92 | A |
| 20 | 928.6 $[M+H]^+$ | 928.4 $[M+H]^+$ | 1.01 | A |
| 21 | 914.4 $[M+H]^+$ | 914.4 $[M+H]^+$ | 0.94 | A |
| 22 | 888.3 $[M+H]^+$ | 888.3 $[M+H]^+$ | 0.87 | A |
| 23 | 902.4 $[M+H]^+$ | 902.4 $[M+H]^+$ | 0.89 | A |
| 24 | 930.4 $[M+H]^+$ | 930.4 $[M+H]^+$ | 0.99 | A |
| 25 | 914.4 $[M+H]^+$ | 914.4 $[M+H]^+$ | 0.91 | A |
| 26 | 945.5 $[M+H]^+$ | 945.4 $[M+H]^+$ | 0.69 | A |
| 27 | 945.4 $[M+H]^+$ | 945.4 $[M+H]^+$ | 0.69 | A |
| 28 | 973.4 $[M+H]^+$ | 973.4 $[M+H]^+$ | 0.70 | A |
| 29 | 973.4 $[M+H]^+$ | 973.4 $[M+H]^+$ | 0.71 | A |
| 30 | 817.3 $[M+H]^+$ | 817.3 $[M+H]^+$ | 0.89 | A |
| 31 | 674.1 $[M+H]^+$ | 674.3 $[M+H]^+$ | 0.85 | A |
| 32 | 1013.5 $[M+H]^+$ | 1013.4 $[M+H]^+$ | 1.00 | A |
| 33 | 1042.7 $[M-H]^-$ | 1042.5 $[M-H]^-$ | 0.74 | A |
| 34 | 1042.6 $[M-H]^-$ | 1042.5 $[M-H]^-$ | 0.74 | A |
| 35 | 1070.7 $[M-H]^-$ | 1070.5 $[M-H]^-$ | 0.75 | A |
| 36 | 1070.5 $[M-H]^-$ | 1070.5 $[M-H]^-$ | 0.76 | A |
| 37 | 587.0 $[M+2H]^{2+}$ | 586.8 $[M+2H]^{2+}$ | 0.64 | A |
| 38 | 898.7 $[M+H]^+$ | 898.4 $[M+H]^+$ | 1.07 | A |
| 39 | 912.6 $[M+H]^+$ | 912.4 $[M+H]^+$ | 1.06 | A |
| 40 | 830.3 $[M+H]^+$ | 830.4 $[M+H]^+$ | 1.28 | A |
| 41 | 860.4 $[M+H]^+$ | 860.4 $[M+H]^+$ | 1.27 | A |
| 42 | 864.4 $[M+H]^+$ | 864.3 $[M+H]^+$ | 1.38 | A |
| 43 | 848.4 $[M+H]^+$ | 848.4 $[M+H]^+$ | 1.30 | A |
| 44 | 846.4 $[M+H]^+$ | 846.4 $[M+H]^+$ | 1.28 | A |

(continued)

| Compound No. | LCMS analysis | | | |
|---|---|---|---|---|
| | MS (found) | MS (calc.) | Retention time RT (min) | Analysis condition |
| 45 | 877.5 $[M+H]^+$ | 877.4 $[M+H]^+$ | 1.46 | A |
| 46 | 877.5 $[M+H]^+$ | 877.4 $[M+H]^+$ | 1.47 | A |
| 47 | 875.5 $[M-H]^-$ | 875.4 $[M-H]^-$ | 1.48 | A |
| 48 | 888.5 $[M-H]^-$ | 888.3 $[M-H]^-$ | 1.74 | A |
| 49 | 876.5 $[M-H]^-$ | 876.3 $[M-H]^-$ | 1.71 | A |
| 50 | 905.6 $[M+H]^+$ | 905.3 $[M+H]^+$ | 1.36 | A |
| 51 | 889.5 $[M+H]^+$ | 889.4 $[M+H]^+$ | 1.44 | A |
| 52 | 893.5 $[M+H]^+$ | 893.3 $[M+H]^+$ | 1.32 | A |
| 53 | 907.5 $[M+H]^+$ | 907.3 $[M+H]^+$ | 1.29 | A |
| 54 | 903.6 $[M+H]^+$ | 903.4 $[M+H]^+$ | 1.40 | A |
| 55 | 903.6 $[M+H]^+$ | 903.4 $[M+H]^+$ | 1.40 | A |
| 56 | 976.4 $[M+H]^+$ | 976.4 $[M+H]^+$ | 1.66 | A |
| 57 | 956.6 $[M-H]^-$ | 956.4 $[M-H]^-$ | 1.63 | A |
| 58 | 970.7 $[M-H]^-$ | 970.4 $[M-H]^-$ | 1.65 | A |
| 59 | 970.6 $[M-H]^-$ | 970.4 $[M-H]^-$ | 1.64 | A |
| 60 | 985.0 $[M-H]^-$ | 984.5 $[M-H]^-$ | 1.72 | A |
| 61 | 1001.6 $[M+H]^+$ | 1001.5 $[M+H]^+$ | 1.37 | A |
| 62 | 1015.6 $[M+H]^+$ | 1015.5 $[M+H]^+$ | 1.35 | A |
| 63 | 1029.6 $[M+H]^+$ | 1029.5 $[M+H]^+$ | 1.35 | A |
| 64 | 988.6 $[M+H]^+$ | 988.4 $[M+H]^+$ | 1.59 | A |
| 65 | 984.6 $[M-H]^-$ | 984.5 $[M-H]^-$ | 1.66 | A |
| 66 | 1000.6 $[M-H]^-$ | 1000.4 $[M-H]^-$ | 1.58 | A |
| 67 | 1001.6 $[M+H]^+$ | 1001.5 $[M+H]^+$ | 1.33 | A |
| 68 | 1031.6 $[M+H]^+$ | 1031.4 $[M+H]^+$ | 1.29 | A |
| 69 | 1017.5 $[M+H]^+$ | 1017.4 $[M+H]^+$ | 1.29 | A |
| 70 | 1027.7 $[M-H]^-$ | 1027.5 $[M-H]^-$ | 1.40 | A |
| 71 | 1015.6 $[M+H]^+$ | 1015.5 $[M+H]^+$ | 1.37 | A |
| 72 | 1045.6 $[M+H]^+$ | 1045.5 $[M+H]^+$ | 1.31 | A |
| 73 | 1031.5 $[M+H]^+$ | 1031.4 $[M+H]^+$ | 1.30 | A |
| 74 | 973.6 $[M+H]^+$ | 973.4 $[M+H]^+$ | 1.31 | A |
| 75 | 1002.6 $[M+H]^+$ | 1002.4 $[M+H]^+$ | 1.60 | A |
| 76 | 1032.7 $[M+H]^+$ | 1032.4 $[M+H]^+$ | 1.35 | A |
| 77 | 1078.7 $[M+H]^+$ | 1078.4 $[M+H]^+$ | 1.39 | A |
| 78 | 1047.7 $[M+H]^+$ | 1047.5 $[M+H]^+$ | 1.44 | A |
| 79 | 1063.6 $[M+H]^+$ | 1063.4 $[M+H]^+$ | 1.37 | A |
| 80 | 1088.8 $[M+H]^+$ | 1088.5 $[M+H]^+$ | 1.06 | A |
| 81 | 1102.7 $[M+H]^+$ | 1102.5 $[M+H]^+$ | 1.06 | A |

(continued)

| Compound No. | LCMS analysis | | | |
|---|---|---|---|---|
| | MS (found) | MS (calc.) | Retention time RT (min) | Analysis condition |
| 82 | 1025.6 $[M+H]^+$ | 1025.5 $[M+H]^+$ | 1.03 | A |
| 83 | 1037.7 $[M-H]^-$ | 1037.5 $[M-H]^-$ | 1.02 | A |
| 84 | 1032.8 $[M+H]^+$ | 1032.5 $[M+H]^+$ | 1.37 | A |
| 85 | 1016.7 $[M+H]^+$ | 1016.5 $[M+H]^+$ | 1.45 | A |
| 86 | 1050.7 $[M+H]^+$ | 1050.5 $[M+H]^+$ | 1.42 | A |
| 87 | 1034.7 $[M+H]^+$ | 1034.5 $[M+H]^+$ | 1.48 | A |
| 88 | 1103.8 $[M-H]^-$ | 1103.5 $[M-H]^-$ | 1.20 | A |
| 89 | 1089.8 $[M+H]^+$ | 1089.5 $[M+H]^+$ | 1.25 | A |
| 90 | 1188.1 $[M-H]^-$ | 1187.6 $[M-H]^-$ | 1.16 | A |
| 91 | 1216.0 $[M-H]^-$ | 1215.6 $[M-H]^-$ | 1.19 | A |
| 92 | 905.7 $[M-H]^-$ | 905.5 $[M-H]^-$ | 1.72 | A |
| 93 | 923.6 $[M-H]^-$ | 923.5 $[M-H]^-$ | 1.73 | A |
| 94 | 948.7 $[M-H]^-$ | 948.5 $[M-H]^-$ | 1.62 | A |
| 95 | 966.6 $[M-H]^-$ | 966.5 $[M-H]^-$ | 1.65 | A |
| 96 | 893.7 $[M-H]^-$ | 893.5 $[M-H]^-$ | 1.80 | A |
| 97 | 911.7 $[M-H]^-$ | 911.5 $[M-H]^-$ | 1.82 | A |
| 98 | 938.7 $[M+H]^+$ | 938.5 $[M+H]^+$ | 1.71 | A |
| 99 | 956.7 $[M+H]^+$ | 956.5 $[M+H]^+$ | 1.74 | A |
| 100 | 919.7 $[M-H]^-$ | 919.5 $[M-H]^-$ | 1.73 | A |
| 101 | 937.7 $[M-H]^-$ | 937.5 $[M-H]^-$ | 1.75 | A |
| 102 | 933.7 $[M-H]^-$ | 933.5 $[M-H]^-$ | 1.81 | A |
| 103 | 962.6 $[M-H]^-$ | 962.5 $[M-H]^-$ | 1.63 | A |
| 104 | 980.7 $[M-H]^-$ | 980.5 $[M-H]^-$ | 1.66 | A |
| 105 | 976.7 $[M-H]^-$ | 976.5 $[M-H]^-$ | 1.71 | A |
| 106 | 979.7 $[M+H]^+$ | 979.5 $[M+H]^+$ | 1.33 | A |
| 107 | 965.7 $[M+H]^+$ | 965.5 $[M+H]^+$ | 1.28 | A |
| 108 | 995.6 $[M+H]^+$ | 995.5 $[M+H]^+$ | 1.25 | A |
| 109 | 981.6 $[M+H]^+$ | 981.5 $[M+H]^+$ | 1.23 | A |
| 110 | 993.7 $[M+H]^+$ | 993.5 $[M+H]^+$ | 1.34 | A |
| 111 | 979.6 $[M+H]^+$ | 979.5 $[M+H]^+$ | 1.31 | A |
| 112 | 1009.6 $[M+H]^+$ | 1009.5 $[M+H]^+$ | 1.26 | A |
| 113 | 995.6 $[M+H]^+$ | 995.5 $[M+H]^+$ | 1.25 | A |
| 114 | 934.7 $[M-H]^-$ | 934.5 $[M-H]^-$ | 1.60 | A |
| 115 | 891.6 $[M-H]^-$ | 891.5 $[M-H]^-$ | 1.66 | A |
| 116 | 903.6 $[M-H]^-$ | 903.5 $[M-H]^-$ | 1.68 | A |
| 117 | 917.6 $[M-H]^-$ | 917.5 $[M-H]^-$ | 1.74 | A |
| 118 | 931.7 $[M-H]^-$ | 931.5 $[M-H]^-$ | 1.76 | A |

(continued)

| Compound No. | LCMS analysis | | | |
|---|---|---|---|---|
| | MS (found) | MS (calc.) | Retention time RT (min) | Analysis condition |
| 119 | 933.7 $[M-H]^-$ | 933.5 $[M-H]^-$ | 1.80 | A |
| 120 | 947.7 $[M-H]^-$ | 947.5 $[M-H]^-$ | 1.85 | A |
| 121 | 947.7 $[M-H]^-$ | 947.5 $[M-H]^-$ | 1.85 | A |
| 122 | 965.6 $[M-H]^-$ | 965.5 $[M-H]^-$ | 1.79 | A |
| 123 | 948.7 $[M-H]^-$ | 948.5 $[M-H]^-$ | 1.33 | A |
| 124 | 981.7 $[M-H]^-$ | 981.5 $[M-H]^-$ | 1.87 | A |
| 125 | 949.6 $[M-H]^-$ | 949.5 $[M-H]^-$ | 1.65 | A |
| 126 | 963.7 $[M-H]^-$ | 963.5 $[M-H]^-$ | 1.67 | A |
| 127 | 962.6 $[M-H]^-$ | 962.5 $[M-H]^-$ | 1.35 | A |
| 128 | 948.7 $[M-H]^-$ | 948.5 $[M-H]^-$ | 1.60 | A |
| 129 | 997.7 $[M-H]^-$ | 997.5 $[M-H]^-$ | 1.74 | A |
| 130 | 1020.6 $[M-H]^-$ | 1020.5 $[M-H]^-$ | 1.85 | A |
| 131 | 935.7 $[M-H]^-$ | 935.5 $[M-H]^-$ | 1.41 | A |
| 132 | 932.8 $[M-H]^-$ | 932.5 $[M-H]^-$ | 1.40 | A |
| 133 | 946.7 $[M-H]^-$ | 946.5 $[M-H]^-$ | 1.39 | A |
| 134 | 996.6 $[M+H]^+$ | 996.5 $[M+H]^+$ | 1.50 | A |
| 135 | 962.7 $[M-H]^-$ | 962.5 $[M-H]^-$ | 1.49 | A |
| 136 | 978.7 $[M+H]^+$ | 978.6 $[M+H]^+$ | 1.54 | A |
| 137 | 997.7 $[M+H]^+$ | 997.5 $[M+H]^+$ | 1.32 | A |
| 138 | 1011.8 $[M+H]^+$ | 1011.5 $[M+H]^+$ | 1.35 | A |
| 139 | 1013.6 $[M+H]^+$ | 1013.5 $[M+H]^+$ | 1.29 | A |
| 140 | 1027.7 $[M+H]^+$ | 1027.5 $[M+H]^+$ | 1.28 | A |
| 141 | 999.6 $[M+H]^+$ | 999.5 $[M+H]^+$ | 1.28 | A |
| 142 | 1013.6 $[M+H]^+$ | 1013.5 $[M+H]^+$ | 1.28 | A |
| 143 | 980.8 $[M+H]^+$ | 980.5 $[M+H]^+$ | 1.38 | A |
| 144 | 1010.7 $[M+H]^+$ | 1010.5 $[M+H]^+$ | 1.27 | A |
| 145 | 995.7 $[M+H]^+$ | 995.5 $[M+H]^+$ | 1.07 | A |
| 146 | 964.7 $[M+H]^+$ | 964.5 $[M+H]^+$ | 1.46 | A |
| 147 | 994.7 $[M+H]^+$ | 994.5 $[M+H]^+$ | 1.34 | A |
| 148 | 977.9 $[M-H]^-$ | 977.6 $[M-H]^-$ | 1.10 | A |
| 149 | 1013.7 $[M+H]^+$ | 1013.5 $[M+H]^+$ | 1.26 | A |
| 150 | 998.7 $[M+H]^+$ | 998.5 $[M+H]^+$ | 1.42 | A |
| 151 | 1028.6 $[M+H]^+$ | 1028.5 $[M+H]^+$ | 1.30 | A |
| 152 | 1013.6 $[M+H]^+$ | 1013.5 $[M+H]^+$ | 1.09 | A |
| 153 | 997.7 $[M+H]^+$ | 997.5 $[M+H]^+$ | 1.35 | A |
| 154 | 993.7 $[M+H]^+$ | 993.5 $[M+H]^+$ | 1.37 | A |
| 155 | 996.7 $[M+H]^+$ | 996.5 $[M+H]^+$ | 1.30 | A |

(continued)

| Compound No. | LCMS analysis | | | |
|---|---|---|---|---|
| | MS (found) | MS (calc.) | Retention time RT (min) | Analysis condition |
| 156 | 980.7 $[M+H]^+$ | 980.5 $[M+H]^+$ | 1.39 | A |
| 157 | 854.4 $[M-2H]^{2-}$ | 854.5 $[M-2H]^{2-}$ | 1.44 | A |
| 158 | 870.7 $[M+2H]^{2+}$ | 870.5 $[M+2H]^{2+}$ | 1.56 | A |
| 159 | 1675.3 $[M-H]^-$ | 1675.0 $[M-H]^-$ | 1.01 | A |
| 160 | 853.4 $[M+2H]^{2+}$ | 853.0 $[M+2H]^{2+}$ | 1.10 | A |
| 161 | 1012.6 $[M-H]^-$ | 1012.5 $[M-H]^-$ | 1.36 | A |
| 162 | 1025.7 $[M-H]^-$ | 1025.5 $[M-H]^-$ | 1.28 | A |
| 163 | 1026.0 $[M-H]^-$ | 1025.5 $[M-H]^-$ | 1.31 | A |
| 164 | 1041.8 $[M+H]^+$ | 1041.5 $[M+H]^+$ | 1.28 | A |
| 165 | 1025.8 $[M+H]^+$ | 1025.5 $[M+H]^+$ | 1.35 | A |
| 166 | 1039.7 $[M+H]^+$ | 1039.5 $[M+H]^+$ | 1.33 | A |
| 167 | 1055.7 $[M+H]^+$ | 1055.5 $[M+H]^+$ | 1.25 | A |
| 168 | 1087.7 $[M+H]^+$ | 1087.5 $[M+H]^+$ | 1.10 | A |
| 169 | 1060.6 $[M+H]^+$ | 1060.5 $[M+H]^+$ | 1.10 | A |
| 170 | 1059.6 $[M-H]^-$ | 1059.5 $[M-H]^-$ | 1.94 | B |
| 171 | 1062.6 $[M+H]^+$ | 1062.5 $[M+H]^+$ | 1.98 | B |
| 172 | 1020.6 $[M+H]^+$ | 1020.5 $[M+H]^+$ | 1.97 | B |
| 173 | 996.8 $[M+H]^+$ | 996.5 $[M+H]^+$ | 1.30 | A |
| 174 | 980.8 $[M+H]^+$ | 980.5 $[M+H]^+$ | 1.38 | A |
| 175 | 1152.0 $[M-H]^-$ | 1151.6 $[M-H]^-$ | 1.11 | A |
| 176 | 1180.0 $[M-H]^-$ | 1179.6 $[M-H]^-$ | 1.14 | A |
| 177 | 995.7 $[M-H]^-$ | 995.4 $[M-H]^-$ | 1.30 | A |
| 178 | 1055.4 $[M+H]^+$ | 1055.4 $[M+H]^+$ | 1.29 | A |
| 179 | 548.7 $[M+2H]^{2+}$ | 548.3 $[M+2H]^{2+}$ | 0.82 | A |
| 180 | 555.7 $[M+2H]^{2+}$ | 555.3 $[M+2H]^{2+}$ | 0.84 | A |
| 181 | 1121.8 $[M-H]^-$ | 1121.6 $[M-H]^-$ | 0.86 | A |
| 182 | 1110.7 $[M+H]^+$ | 1110.5 $[M+H]^+$ | 1.03 | A |
| 183 | 1124.7 $[M+H]^+$ | 1124.5 $[M+H]^+$ | 1.04 | A |
| 184 | 1079.0 $[M-H]^-$ | 1078.6 $[M-H]^-$ | 0.91 | A |
| 185 | 1063.4 $[M-H]^-$ | 1062.6 $[M-H]^-$ | 1.00 | A |
| 186 | 1093.0 $[M-H]^-$ | 1092.6 $[M-H]^-$ | 0.97 | A |
| 187 | 1077.0 $[M-H]^-$ | 1076.6 $[M-H]^-$ | 1.01 | A |
| 188 | 1106.9 $[M-H]^-$ | 1106.6 $[M-H]^-$ | 0.94 | A |
| 189 | 1090.9 $[M-H]^-$ | 1090.6 $[M-H]^-$ | 1.00 | A |
| 190 | 1121.1 $[M-H]^-$ | 1120.6 $[M-H]^-$ | 0.97 | A |
| 191 | 1105.0 $[M-H]^-$ | 1104.6 $[M-H]^-$ | 1.02 | A |
| 192 | 1093.1 $[M-H]^-$ | 1092.6 $[M-H]^-$ | 0.99 | A |

(continued)

| Compound No. | LCMS analysis | | | |
|---|---|---|---|---|
| | MS (found) | MS (calc.) | Retention time RT (min) | Analysis condition |
| 193 | 1077.0 $[M-H]^-$ | 1076.6 $[M-H]^-$ | 1.03 | A |
| 194 | 1106.9 $[M-H]^-$ | 1106.6 $[M-H]^-$ | 1.01 | A |
| 195 | 1091.0 $[M-H]^-$ | 1090.6 $[M-H]^-$ | 1.05 | A |
| 196 | 1121.0 $[M-H]^-$ | 1120.6 $[M-H]^-$ | 1.00 | A |
| 197 | 1105.0 $[M-H]^-$ | 1104.6 $[M-H]^-$ | 1.04 | A |
| 198 | 1118.9 $[M-H]^-$ | 1118.6 $[M-H]^-$ | 1.06 | A |
| 199 | 1164.8 $[M-H]^-$ | 1164.6 $[M-H]^-$ | 1.05 | A |
| 200 | 1151.7 $[M+H]^+$ | 1151.6 $[M+H]^+$ | 1.17 | A |
| 201 | 1150.7 $[M+H]^+$ | 1150.6 $[M+H]^+$ | 1.12 | A |
| 202 | 958.5 $[M+H]^+$ | 958.4 $[M+H]^+$ | 1.62 | A |
| 203 | 1059.7 $[M+H]^+$ | 1059.5 $[M+H]^+$ | 1.36 | A |
| 204 | 1073.7 $[M+H]^+$ | 1073.5 $[M+H]^+$ | 1.34 | A |
| 205 | 1016.7 $[M+H]^+$ | 1016.5 $[M+H]^+$ | 1.40 | A |
| 206 | 1084.7 $[M+H]^+$ | 1084.5 $[M+H]^+$ | 1.07 | A |
| 207 | 1098.7 $[M+H]^+$ | 1098.5 $[M+H]^+$ | 1.07 | A |
| 208 | 1041.7 $[M+H]^+$ | 1041.5 $[M+H]^+$ | 1.12 | A |
| 209 | 1070.6 $[M+H]^+$ | 1070.5 $[M+H]^+$ | 1.04 | A |
| 210 | 1038.7 $[M+H]^+$ | 1038.5 $[M+H]^+$ | 1.49 | B |
| 211 | 1023.7 $[M+H]^+$ | 1023.5 $[M+H]^+$ | 1.62 | B |
| 212 | 1053.7 $[M+H]^+$ | 1053.5 $[M+H]^+$ | 1.52 | B |
| 213 | 1038.7 $[M+H]^+$ | 1038.5 $[M+H]^+$ | 1.33 | B |
| 214 | 1022.7 $[M+H]^+$ | 1022.5 $[M+H]^+$ | 1.57 | B |
| 215 | 1007.7 $[M+H]^+$ | 1007.6 $[M+H]^+$ | 1.70 | B |
| 216 | 1037.6 $[M+H]^+$ | 1037.5 $[M+H]^+$ | 1.59 | B |
| 217 | 1020.6 $[M-H]^-$ | 1020.6 $[M-H]^-$ | 1.44 | B |
| 218 | 1036.7 $[M+H]^+$ | 1036.5 $[M+H]^+$ | 1.08 | A |
| 219 | 1052.7 $[M+H]^+$ | 1052.5 $[M+H]^+$ | 1.03 | A |
| 220 | 1066.6 $[M+H]^+$ | 1066.5 $[M+H]^+$ | 1.03 | A |
| 221 | 1024.7 $[M+H]^+$ | 1024.5 $[M+H]^+$ | 0.98 | A |
| 222 | 1052.7 $[M+H]^+$ | 1052.5 $[M+H]^+$ | 1.02 | A |
| 223 | 1067.7 $[M+H]^+$ | 1067.5 $[M+H]^+$ | 1.04 | A |
| 224 | 1010.6 $[M+H]^+$ | 1010.5 $[M+H]^+$ | 1.00 | A |
| 225 | 1024.5 $[M+H]^+$ | 1024.5 $[M+H]^+$ | 1.01 | A |
| 226 | 1038.6 $[M+H]^+$ | 1038.5 $[M+H]^+$ | 1.03 | A |
| 227 | 1039.6 $[M+H]^+$ | 1039.5 $[M+H]^+$ | 1.03 | A |
| 228 | 1053.6 $[M+H]^+$ | 1053.5 $[M+H]^+$ | 1.04 | A |
| 229 | 1008.7 $[M+H]^+$ | 1008.5 $[M+H]^+$ | 1.05 | A |

(continued)

| Compound No. | LCMS analysis | | | |
|---|---|---|---|---|
| | MS (found) | MS (calc.) | Retention time RT (min) | Analysis condition |
| 230 | 1036.7 $[M+H]^+$ | 1036.5 $[M+H]^+$ | 1.09 | A |
| 231 | 1023.7 $[M+H]^+$ | 1023.5 $[M+H]^+$ | 1.07 | A |
| 232 | 1051.7 $[M+H]^+$ | 1051.5 $[M+H]^+$ | 1.10 | A |
| 233 | 1066.6 $[M+H]^+$ | 1066.5 $[M+H]^+$ | 1.02 | A |
| 234 | 1050.6 $[M+H]^+$ | 1050.6 $[M+H]^+$ | 1.07 | A |
| 235 | 1039.7 $[M+H]^+$ | 1039.5 $[M+H]^+$ | 1.04 | A |
| 236 | 1023.7 $[M+H]^+$ | 1023.5 $[M+H]^+$ | 1.12 | A |
| 237 | 1053.7 $[M+H]^+$ | 1053.5 $[M+H]^+$ | 1.04 | A |
| 238 | 1039.7 $[M+H]^+$ | 1039.5 $[M+H]^+$ | 1.04 | A |
| 239 | 1023.7 $[M+H]^+$ | 1023.5 $[M+H]^+$ | 1.11 | A |
| 240 | 1025.6 $[M+H]^+$ | 1025.5 $[M+H]^+$ | 1.04 | A |
| 241 | 1024.6 $[M+H]^+$ | 1024.5 $[M+H]^+$ | 1.02 | A |
| 242 | 1053.6 $[M+H]^+$ | 1053.5 $[M+H]^+$ | 1.04 | A |
| 243 | 1052.6 $[M+H]^+$ | 1052.5 $[M+H]^+$ | 1.00 | A |
| 244 | 1036.7 $[M-H]^+$ | 1036.5 $[M-H]^+$ | 1.05 | A |
| 245 | 1065.8 $[M+H]^+$ | 1065.6 $[M+H]^+$ | 0.95 | A |
| 246 | 1079.8 $[M+H]^+$ | 1079.6 $[M+H]^+$ | 0.95 | A |
| 247 | 533.6 $[M+2H]^{2+}$ | 533.3 $[M+2H]^{2+}$ | 0.90 | A |
| 248 | 1050.7 $[M+H]^+$ | 1050.6 $[M+H]^+$ | 1.06 | A |
| 249 | 1064.7 $[M+H]^+$ | 1064.6 $[M+H]^+$ | 1.07 | A |
| 250 | 1038.7 $[M+H]^+$ | 1038.5 $[M+H]^+$ | 0.99 | A |
| 251 | 1022.7 $[M+H]^+$ | 1022.5 $[M+H]^+$ | 1.05 | A |
| 252 | 1024.7 $[M+H]^+$ | 1024.5 $[M+H]^+$ | 1.00 | A |
| 253 | 1052.6 $[M+H]^+$ | 1052.5 $[M+H]^+$ | 1.00 | A |
| 254 | 1064.8 $[M+H]^+$ | 1064.6 $[M+H]^+$ | 1.16 | A |
| 255 | 1104.8 $[M+H]^+$ | 1104.6 $[M+H]^+$ | 1.31 | A |
| 256 | 1066.7 $[M+H]^+$ | 1066.5 $[M+H]^+$ | 1.04 | A |
| 257 | 1080.7 $[M+H]^+$ | 1080.5 $[M+H]^+$ | 1.04 | A |
| 258 | 1081.7 $[M+H]^+$ | 1081.5 $[M+H]^+$ | 1.07 | A |
| 259 | 1053.6 $[M+H]^+$ | 1053.5 $[M+H]^+$ | 1.07 | A |
| 260 | 1067.7 $[M+H]^+$ | 1067.5 $[M+H]^+$ | 1.06 | A |
| 261 | 1051.6 $[M+H]^+$ | 1051.5 $[M+H]^+$ | 1.13 | A |
| 262 | 1082.7 $[M+H]^+$ | 1082.5 $[M+H]^+$ | 1.09 | A |
| 263 | 1098.6 $[M+H]^+$ | 1098.5 $[M+H]^+$ | 1.03 | A |
| 264 | 1097.7 $[M+H]^+$ | 1097.5 $[M+H]^+$ | 1.11 | A |
| 265 | 1113.6 $[M+H]^+$ | 1113.5 $[M+H]^+$ | 1.05 | A |
| 266 | 1096.6 $[M+H]^+$ | 1096.6 $[M+H]^+$ | 1.07 | A |

(continued)

| Compound No. | LCMS analysis | | | |
|---|---|---|---|---|
| | MS (found) | MS (calc.) | Retention time RT (min) | Analysis condition |
| 267 | 1112.7 $[M+H]^+$ | 1112.5 $[M+H]^+$ | 1.03 | A |
| 268 | 1066.7 $[M+H]^+$ | 1066.5 $[M+H]^+$ | 1.03 | A |
| 269 | 1092.7 $[M+H]^+$ | 1092.5 $[M+H]^+$ | 1.06 | A |
| 270 | 1106.7 $[M+H]^+$ | 1106.6 $[M+H]^+$ | 1.11 | A |
| 271 | 1136.7 $[M+H]^+$ | 1136.6 $[M+H]^+$ | 1.03 | A |
| 272 | 1096.6 $[M+H]^+$ | 1096.5 $[M+H]^+$ | 1.01 | A |
| 273 | 1076.7 $[M+H]^+$ | 1076.6 $[M+H]^+$ | 1.16 | A |
| 274 | 1092.6 $[M+H]^+$ | 1092.6 $[M+H]^+$ | 1.07 | A |
| 275 | 1090.7 $[M+H]^+$ | 1090.6 $[M+H]^+$ | 1.22 | A |
| 276 | 1120.7 $[M+H]^+$ | 1120.6 $[M+H]^+$ | 1.10 | A |
| 277 | 1080.6 $[M+H]^+$ | 1080.6 $[M+H]^+$ | 1.07 | A |
| 278 | 1103.7 $[M+H]^+$ | 1103.5 $[M+H]^+$ | 1.09 | A |
| 279 | 1036.8 $[M+H]^+$ | 1036.5 $[M+H]^+$ | 1.06 | A |
| 280 | 1052.7 $[M+H]^+$ | 1052.5 $[M+H]^+$ | 1.02 | A |
| 281 | 1050.7 $[M+H]^+$ | 1050.6 $[M+H]^+$ | 1.08 | A |
| 282 | 1066.7 $[M+H]^+$ | 1066.5 $[M+H]^+$ | 1.03 | A |
| 283 | 1036.8 $[M+H]^+$ | 1036.5 $[M+H]^+$ | 1.06 | A |
| 284 | 1052.6 $[M+H]^+$ | 1052.5 $[M+H]^+$ | 1.00 | A |
| 285 | 1025.7 $[M+H]^+$ | 1025.5 $[M+H]^+$ | 1.02 | A |
| 286 | 1007.7 $[M-H]^-$ | 1007.5 $[M-H]^-$ | 1.04 | A |
| 287 | 1085.7 $[M+H]^+$ | 1085.5 $[M+H]^+$ | 1.22 | A |
| 288 | 1085.7 $[M+H]^+$ | 1085.5 $[M+H]^+$ | 1.21 | A |
| 289 | 1101.7 $[M+H]^+$ | 1101.5 $[M+H]^+$ | 1.11 | A |
| 290 | 1101.5 $[M+H]^+$ | 1101.5 $[M+H]^+$ | 1.11 | A |
| 291 | 527.6 $[M+2H]^{2+}$ | 527.3 $[M+2H]^{2+}$ | 0.83 | A |
| 292 | 534.5 $[M+2H]^{2+}$ | 534.3 $[M+2H]^{2+}$ | 0.80 | A |
| 293 | 505.9 $[M+2H]^{2+}$ | 505.8 $[M+2H]^{2+}$ | 0.86 | A |
| 294 | 506.0 $[M+2H]^{2+}$ | 505.8 $[M+2H]^{2+}$ | 0.86 | A |
| 295 | 993.5 $[M-H]^-$ | 993.5 $[M-H]^-$ | 1.03 | A |
| 296 | 520.0 $[M+2H]^{2+}$ | 519.8 $[M+2H]^{2+}$ | 0.85 | A |
| 297 | 513.1 $[M+2H]^{2+}$ | 512.8 $[M+2H]^{2+}$ | 0.87 | A |
| 298 | 1036.8 $[M-H]^-$ | 1036.5 $[M-H]^-$ | 0.86 | A |
| 299 | 1051.8 $[M-H]^-$ | 1051.5 $[M-H]^-$ | 0.76 | A |
| 300 | 991.7 $[M+H]^+$ | 991.5 $[M+H]^+$ | 1.11 | A |
| 301 | 1007.6 $[M+H]^+$ | 1007.5 $[M+H]^+$ | 1.04 | A |
| 302 | 1005.6 $[M+H]^+$ | 1005.5 $[M+H]^+$ | 1.12 | A |
| 303 | 1021.6 $[M+H]^+$ | 1021.5 $[M+H]^+$ | 1.06 | A |

(continued)

| Compound No. | LCMS analysis | | | |
|---|---|---|---|---|
| | MS (found) | MS (calc.) | Retention time RT (min) | Analysis condition |
| 304 | 1051.7 $[M+H]^+$ | 1051.5 $[M+H]^+$ | 1.11 | A |
| 305 | 534.5 $[M+2H]^{2+}$ | 534.2 $[M+2H]^{2+}$ | 1.05 | A |
| 306 | 1037.7 $[M-H]^-$ | 1037.5 $[M-H]^-$ | 0.78 | A |
| 307 | 1037.7 $[M-H]^-$ | 1037.5 $[M-H]^-$ | 0.75 | A |
| 308 | 1081.8 $[M+H]^+$ | 1081.5 $[M+H]^+$ | 1.07 | A |
| 309 | 1035.8 $[M+H]^+$ | 1035.6 $[M+H]^+$ | 1.31 | A |
| 310 | 1048.9 $[M-H]^-$ | 1048.6 $[M-H]^-$ | 0.98 | A |
| 311 | 1066.8 $[M+H]^+$ | 1066.6 $[M+H]^+$ | 0.88 | A |
| 312 | 1062.8 $[M-H]^-$ | 1062.6 $[M-H]^-$ | 1.00 | A |
| 313 | 1078.9 $[M-H]^-$ | 1078.6 $[M-H]^-$ | 0.91 | A |
| 314 | 1038.7 $[M+H]^+$ | 1038.5 $[M+H]^+$ | 1.06 | A |
| 315 | 1011.7 $[M+H]^+$ | 1011.5 $[M+H]^+$ | 1.07 | A |
| 316 | 1071.7 $[M+H]^+$ | 1071.5 $[M+H]^+$ | 1.18 | A |
| 317 | 1044.7 $[M+H]^+$ | 1044.5 $[M+H]^+$ | 1.18 | A |
| 318 | 1065.7 $[M+H]^+$ | 1065.6 $[M+H]^+$ | 1.11 | A |
| 319 | 1039.8 $[M+H]^+$ | 1039.5 $[M+H]^+$ | 1.04 | A |
| 320 | 1039.7 $[M+H]^+$ | 1039.5 $[M+H]^+$ | 1.03 | A |
| 321 | 1035.7 $[M+H]^+$ | 1035.5 $[M+H]^+$ | 1.09 | A |
| 322 | 1124.7 $[M+H]^+$ | 1124.5 $[M+H]^+$ | 1.22 | A |
| 323 | 1049.8 $[M+H]^+$ | 1049.6 $[M+H]^+$ | 1.32 | A |
| 324 | 1064.8 $[M+H]^+$ | 1065.6 $[M+H]^+$ | 1.24 | A |
| 325 | 1067.8 $[M+H]^+$ | 1067.6 $[M+H]^+$ | 1.26 | A |
| 326 | 1082.7 $[M+H]^+$ | 1083.5 $[M+H]^+$ | 1.17 | A |
| 327 | 1124.7 $[M+H]^+$ | 1124.5 $[M+H]^+$ | 1.21 | A |
| 328 | 538.6 $[M+2H]^{2+}$ | 538.3 $[M+2H]^{2+}$ | 0.85 | A |
| 329 | 1073.8 $[M-H]^-$ | 1073.5 $[M-H]^-$ | 0.85 | A |
| 330 | 1041.7 $[M+H]^+$ | 1041.5 $[M+H]^+$ | 1.08 | A |
| 331 | 1041.7 $[M+H]^+$ | 1041.5 $[M+H]^+$ | 1.07 | A |
| 332 | 1092.7 $[M-H]^-$ | 1092.6 $[M-H]^-$ | 0.86 | A |
| 333 | 1092.9 $[M-H]^-$ | 1092.6 $[M-H]^-$ | 0.86 | A |
| 334 | 534.6 $[M+2H]^{2+}$ | 534.3 $[M+2H]^{2+}$ | 1.03 | A |
| 335 | 1027.7 $[M+H]^+$ | 1027.5 $[M+H]^+$ | 1.00 | A |
| 336 | 1092.8 $[M+H]^+$ | 1092.6 $[M+H]^+$ | 1.09 | A |
| 337 | 1108.7 $[M+H]^+$ | 1108.6 $[M+H]^+$ | 1.05 | A |
| 338 | 1079.8 $[M+H]^+$ | 1079.6 $[M+H]^+$ | 1.06 | A |
| 339 | 1079.8 $[M+H]^+$ | 1079.6 $[M+H]^+$ | 1.05 | A |
| 340 | 1095.7 $[M+H]^+$ | 1095.5 $[M+H]^+$ | 1.01 | A |

(continued)

| Compound No. | LCMS analysis | | | |
|---|---|---|---|---|
| | MS (found) | MS (calc.) | Retention time RT (min) | Analysis condition |
| 341 | 1095.7 $[M+H]^+$ | 1095.5 $[M+H]^+$ | 1.01 | A |
| 342 | 1053.7 $[M+H]^+$ | 1053.5 $[M+H]^+$ | 1.04 | A |
| 343 | 1053.6 $[M+H]^+$ | 1053.5 $[M+H]^+$ | 1.04 | A |
| 344 | 1048.9 $[M-H]^-$ | 1048.6 $[M-H]^-$ | 0.92 | A |
| 345 | 1053.7 $[M+H]^+$ | 1053.5 $[M+H]^+$ | 1.20 | A |
| 346 | 1069.7 $[M+H]^+$ | 1069.5 $[M+H]^+$ | 1.14 | A |
| 347 | 1009.8 $[M+H]^+$ | 1009.5 $[M+H]^+$ | 1.06 | A |
| 348 | 1023.8 $[M+H]^+$ | 1023.5 $[M+H]^+$ | 1.08 | A |
| 349 | 984.6 $[M+H]^+$ | 984.5 $[M+H]^+$ | 1.04 | A |
| 350 | 1422.4 $[M-H]^-$ | 1421.8 $[M-H]^-$ | 0.60 | A |
| 351 | 470.1 $[M+3M]^{3+}$ | 469.9 $[M+3M]^{3+}$ | 0.67 | A |
| 352 | 1025.7 $[M+H]^+$ | 1025.5 $[M+H]^+$ | 1.02 | A |
| 353 | 1009.7 $[M+M]^+$ | 1009.5 $[M+M]^+$ | 1.07 | A |
| 354 | 1053.7 $[M+M]^+$ | 1053.5 $[M+M]^+$ | 1.06 | A |
| 355 | 1037.7 $[M+M]^+$ | 1037.6 $[M+M]^+$ | 1.13 | A |
| 356 | 1076.8 $[M-H]^-$ | 1076.6 $[M-H]^-$ | 0.99 | A |
| 357 | 1064.7 $[M+H]^+$ | 1064.6 $[M+M]^+$ | 1.09 | A |
| 358 | 1080.7 $[M+H]^+$ | 1080.5 $[M+M]^+$ | 1.04 | A |
| 359 | 1070.6 $[M+H]^+$ | 1070.5 $[M+M]^+$ | 1.07 | A |
| 360 | 1086.6 $[M+H]^+$ | 1086.5 $[M+M]^+$ | 1.04 | A |
| 361 | 1038.9 $[M-H]^-$ | 1038.5 $[M-H]^-$ | 0.80 | A |
| 362 | 1052.7 $[M-H]^-$ | 1052.5 $[M-H]^-$ | 0.83 | A |
| 363 | 1079.8 $[M-H]^-$ | 1079.6 $[M-H]^-$ | 0.71 | A |
| 364 | 513.1 $[M+2H]^{2+}$ | 512.8 $[M+2H]^{2+}$ | 0.91 | A |
| 365 | 520.1 $[M+2H]^{2+}$ | 519.8 $[M+2H]^{2+}$ | 0.98 | A |
| 366 | 1064.0 $[M-H]^-$ | 1063.6 $[M-H]^-$ | 0.78 | A |
| 367 | 1107.0 $[M-H]^-$ | 1106.6 $[M-H]^-$ | 0.94 | A |
| 368 | 1091.1 $[M-H]^-$ | 1090.6 $[M-H]^-$ | 1.00 | A |
| 369 | 1064.9 $[M-H]^-$ | 1064.5 $[M-H]^-$ | 0.86 | A |
| 370 | 1049.1 $[M-H]^-$ | 1048.6 $[M-H]^-$ | 0.99 | A |
| 371 | 1009.9 $[M+H]^+$ | 1009.5 $[M+H]^+$ | 0.66 | A |
| 372 | 1021.8 $[M-H]^-$ | 1021.5 $[M-H]^-$ | 0.66 | A |
| 373 | 1035.9 $[M-H]^-$ | 1035.6 $[M-H]^-$ | 0.62 | A |
| 374 | 1092.0 $[M-H]^-$ | 1091.6 $[M-H]^-$ | 0.73 | A |
| 375 | 1035.9 $[M-H]^-$ | 1035.5 $[M-H]^-$ | 0.68 | A |
| 376 | 1049.9 $[M-H]^-$ | 1049.6 $[M-H]^-$ | 0.68 | A |
| 377 | 1065.8 $[M+H]^+$ | 1065.6 $[M+M]^+$ | 0.67 | A |

(continued)

| Compound No. | LCMS analysis | | | |
|---|---|---|---|---|
| | MS (found) | MS (calc.) | Retention time RT (min) | Analysis condition |
| 378 | 1093.8 [M+H]+ | 1093.6 [M+M]+ | 0.73 | A |
| 379 | 925.5 [M+H]+ | 925.5 [M+H]+ | 1.13 | A |
| 380 | 955.5 [M+H]+ | 955.5 [M+H]+ | 1.00 | A |
| 381 | 939.6 [M+H]+ | 939.5 [M+H]+ | 1.13 | A |
| 382 | 812.5 [M+H]+ | 812.4 [M+H]+ | 0.97 | A |
| 383 | 1032.7 [M+H]+ | 1032.6 [M+H]+ | 1.09 | A |
| 384 | 1192.9 [M-H]- | 1192.6 [M-H]- | 0.86 | A |
| 385 | 1176.9 [M-H]- | 1176.6 [M-H]- | 0.89 | A |
| 386 | 1221.0 [M-H]- | 1220.6 [M-H]- | 0.85 | A |
| 387 | 1204.9 [M-H]- | 1204.6 [M-H]- | 0.93 | A |
| 388 | 1220.9 [M-H]- | 1220.6 [M-H]- | 0.72 | A |
| 389 | 1205.0 [M-H]- | 1204.7 [M-H]- | 0.80 | A |
| 390 | 1249.0 [M-H]- | 1248.6 [M-H]- | 0.74 | A |
| 391 | 1232.9 [M-H]- | 1232.7 [M-H]- | 0.81 | A |
| 392 | 1036.7 [M+H]+ | 1036.5 [M+H]+ | 1.05 | A |
| 393 | 1052.7 [M+H]+ | 1052.5 [M+H]+ | 1.01 | A |
| 394 | 1037.7 [M+H]+ | 1037.5 [M+H]+ | 1.07 | A |
| 395 | 1053.6 [M+H]+ | 1053.5 [M+H]+ | 1.04 | A |
| 396 | 1062.9 [M-H]- | 1062.6 [M-H]- | 0.93 | A |
| 397 | 1078.9 [M-H]- | 1078.5 [M-H]- | 0.85 | A |
| 398 | 1009.7 [M+H]+ | 1009.5 [M+H]+ | 1.06 | A |
| 399 | 1025.7 [M+H]+ | 1025.5 [M+H]+ | 1.02 | A |
| 400 | 1206.8 [M-H]- | 1206.6 [M-H]- | 0.83 | A |
| 401 | 1191.0 [M-H]- | 1190.6 [M-H]- | 0.90 | A |
| 402 | 1178.9 [M-H]- | 1178.6 [M-H]- | 0.82 | A |
| 403 | 1163.0 [M-H]- | 1162.6 [M-H]- | 0.86 | A |
| 404 | 1179.0 [M-H]- | 1178.6 [M-H]- | 0.83 | A |
| 405 | 1163.1 [M-H]- | 1162.6 [M-H]- | 0.86 | A |
| 406 | 1206.9 [M-H]- | 1206.6 [M-H]- | 0.84 | A |
| 407 | 1191.0 [M-H]- | 1190.6 [M-H]- | 0.88 | A |
| 408 | 1110.0 [M-H]- | 1109.5 [M-H]- | 0.79 | A |
| 409 | 1093.9 [M-H]- | 1093.6 [M-H]- | 0.99 | A |
| 410 | 1081.9 [M-H]- | 1081.5 [M-H]- | 0.78 | A |
| 411 | 1065.7 [M-H]- | 1065.6 [M-H]- | 0.98 | A |
| 412 | 1026.7 [M+H]+ | 1026.5 [M+H]+ | 0.97 | A |
| 413 | 1010.7 [M+H]+ | 1010.5 [M+H]+ | 1.09 | A |
| 414 | 1040.7 [M+H]+ | 1040.5 [M+H]+ | 0.99 | A |

(continued)

| Compound No. | LCMS analysis | | | |
|---|---|---|---|---|
| | MS (found) | MS (calc.) | Retention time RT (min) | Analysis condition |
| 415 | 1024.8 $[M+H]^+$ | 1024.5 $[M+H]^+$ | 1.10 | A |
| 416 | 1054.7 $[M+H]^+$ | 1054.5 $[M+H]^+$ | 1.01 | A |
| 417 | 1092.7 $[M+H]^+$ | 1092.5 $[M+H]^+$ | 0.81 | A |
| 418 | 1093.9 $[M-H]^-$ | 1093.6 $[M-H]^-$ | 0.97 | A |
| 419 | 1109.9 $[M-H]^-$ | 1109.5 $[M-H]^-$ | 0.81 | A |
| 420 | 1065.8 $[M-H]^-$ | 1065.6 $[M-H]^-$ | 0.95 | A |
| 421 | 1081.8 $[M-H]^-$ | 1081.5 $[M-H]^-$ | 0.78 | A |
| 422 | 1039.8 $[M-H]^-$ | 1039.5 $[M-H]^-$ | 0.79 | A |
| 423 | 1053.7 $[M-H]^-$ | 1053.5 $[M-H]^-$ | 0.78 | A |
| 424 | 1051.9 $[M-H]^-$ | 1051.6 $[M-H]^-$ | 0.97 | A |
| 425 | 1067.8 $[M-H]^-$ | 1067.5 $[M-H]^-$ | 0.77 | A |
| 426 | 1066.7 $[M+H]^+$ | 1066.5 $[M+H]^+$ | 1.07 | A |
| 427 | 1038.8 $[M+H]^+$ | 1038.6 $[M+H]^+$ | 1.11 | A |
| 428 | 1049.8 $[M-H]^-$ | 1049.5 $[M-H]^-$ | 0.85 | A |
| 429 | 1377.2 $[M-H]^-$ | 1376.7 $[M-H]^-$ | 0.75 | A |
| 430 | 1361.0 $[M-H]^-$ | 1360.7 $[M-H]^-$ | 0.80 | A |
| 431 | 680.9 $[M-2H]^{2-}$ | 680.8 $[M-2H]^{2-}$ | 0.72 | A |
| 432 | 1347.1 $[M-H]^-$ | 1346.7 $[M-H]^-$ | 0.75 | A |
| 433 | 1376.8 $[M-H]^-$ | 1376.7 $[M-H]^-$ | 0.74 | A |
| 434 | 1361.2 $[M-H]^-$ | 1360.7 $[M-H]^-$ | 0.80 | A |
| 435 | 1363.0 $[M-H]^-$ | 1362.7 $[M-H]^-$ | 0.73 | A |
| 436 | 1347.1 $[M-H]^-$ | 1346.7 $[M-H]^-$ | 0.77 | A |
| 437 | 1025.8 $[M+H]^+$ | 1025.5 $[M+H]^+$ | 0.96 | A |
| 438 | 1023.8 $[M-H]^-$ | 1023.5 $[M-H]^-$ | 0.96 | A |
| 439 | 1038.1 $[M-H]^-$ | 1037.5 $[M-H]^-$ | 0.95 | A |
| 440 | 1321.1 $[M-H]^-$ | 1320.7 $[M-H]^-$ | 0.72 | A |
| 441 | 1305.1 $[M-H]^-$ | 1304.7 $[M-H]^-$ | 0.76 | A |
| 442 | 1307.1 $[M-H]^-$ | 1306.7 $[M-H]^-$ | 0.71 | A |
| 443 | 1291.1 $[M-H]^-$ | 1290.7 $[M-H]^-$ | 0.73 | A |
| 444 | 1321.1 $[M-H]^-$ | 1320.7 $[M-H]^-$ | 0.70 | A |
| 445 | 1305.1 $[M-H]^-$ | 1304.7 $[M-H]^-$ | 0.75 | A |
| 446 | 1307.0 $[M-H]^-$ | 1306.7 $[M-H]^-$ | 0.70 | A |
| 447 | 1291.2 $[M-H]^-$ | 1290.7 $[M-H]^-$ | 0.73 | A |
| 448 | 1037.9 $[M-H]^-$ | 1037.5 $[M-H]^-$ | 0.97 | A |
| 449 | 1053.8 $[M+H]^+$ | 1053.6 $[M+H]^+$ | 0.97 | A |
| 450 | 1051.9 $[M-H]^-$ | 1051.6 $[M-H]^-$ | 0.98 | A |
| 451 | 1065.8 $[M-H]^-$ | 1065.6 $[M-H]^-$ | 0.99 | A |

(continued)

| Compound No. | LCMS analysis | | | |
|---|---|---|---|---|
| | MS (found) | MS (calc.) | Retention time RT (min) | Analysis condition |
| 452 | 1065.9 [M-H]- | 1065.6 [M-H]- | 0.97 | A |
| 453 | 1050.8 [M+H]+ | 1050.6 [M+H]+ | 1.08 | A |
| 454 | 1020.8 [M-H]- | 1020.5 [M-H]- | 1.04 | A |
| 455 | 1020.9 [M-H]- | 1020.5 [M-H]- | 1.08 | A |
| 456 | 1022.9 [M-H]- | 1022.5 [M-H]- | 1.05 | A |
| 457 | 1179.0 [M-H]- | 1178.6 [M-H]- | 1.00 | A |
| 458 | 1179.0 [M-H]- | 1178.6 [M-H]- | 0.98 | A |
| 459 | 1150.9 [M-H]- | 1150.6 [M-H]- | 0.98 | A |
| 460 | 1151.0 [M-H]- | 1150.6 [M-H]- | 0.98 | A |
| 461 | 1335.1 [M-H]- | 1334.7 [M-H]- | 0.85 | A |
| 462 | 669.1 [M+2H]2+ | 668.9 [M+2H]2+ | 0.86 | A |
| 463 | 1279.3 [M-H]- | 1278.7 [M-H]- | 0.82 | A |
| 464 | 1194.9 [M-H]- | 1194.6 [M-H]- | 0.82 | A |
| 465 | 1195.0 [M-H]- | 1194.6 [M-H]- | 0.83 | A |
| 466 | 1167.1 [M-H]- | 1166.6 [M-H]- | 0.80 | A |
| 467 | 1166.9 [M-H]- | 1166.6 [M-H]- | 0.79 | A |
| 468 | 1351.2 [M-H]- | 1350.7 [M-H]- | 0.71 | A |
| 469 | 1351.1 [M-H]- | 1350.7 [M-H]- | 0.70 | A |
| 470 | 1295.1 [M-H]- | 1294.7 [M-H]- | 0.69 | A |
| 471 | 1295.0 [M-H]- | 1294.7 [M-H]- | 0.68 | A |
| 472 | 1193.0 [M-H]- | 1192.7 [M-H]- | 0.99 | A |
| 473 | 1193.0 [M-H]- | 1192.7 [M-H]- | 0.99 | A |
| 474 | 1165.1 [M-H]- | 1164.6 [M-H]- | 0.99 | A |
| 475 | 1165.0 [M-H]- | 1164.6 [M-H]- | 0.99 | A |
| 476 | 1349.2 [M-H]- | 1348.8 [M-H]- | 0.87 | A |
| 477 | 1349.5 [M-H]- | 1348.8 [M-H]- | 0.86 | A |
| 478 | 1293.1 [M-H]- | 1292.7 [M-H]- | 0.84 | A |
| 479 | 648.0 [M+2H]2+ | 647.9 [M+2H]2+ | 0.83 | A |
| 480 | 1052.9 [M+H]+ | 1052.6 [M+M]+ | 1.13 | A |
| 481 | 1052.9 [M-H]- | 1052.5 [M-H]- | 1.01 | A |
| 482 | 1037.0 [M-H]- | 1036.6 [M-H]- | 1.07 | A |
| 483 | 1205.2 [M-H]- | 1204.7 [M-H]- | 0.94 | A |
| 484 | 1177.2 [M-H]- | 1176.6 [M-H]- | 0.91 | A |
| 485 | 1022.9 [M-H]- | 1022.5 [M-H]- | 1.08 | A |
| 486 | 1037.0 [M-H]- | 1036.6 [M-H]- | 1.15 | A |
| 487 | 1164.9 [M-H]- | 1164.7 [M-H]- | 0.91 | A |
| 488 | 1193.0 [M-H]- | 1192.7 [M-H]- | 0.96 | A |

(continued)

| Compound No. | LCMS analysis | | | |
|---|---|---|---|---|
| | MS (found) | MS (calc.) | Retention time RT (min) | Analysis condition |
| 489 | 1178.9 [M-H]$^-$ | 1178.6 [M-H]$^-$ | 0.97 | A |
| 490 | 1165.1 [M-H]$^-$ | 1164.6 [M-H]$^-$ | 1.01 | A |
| 491 | 1193.1 [M-H]$^-$ | 1192.7 [M-H]$^-$ | 1.01 | A |
| 492 | 980.9 [M-H]$^-$ | 980.5 [M-H]$^-$ | 1.00 | A |
| 493 | 1080.1 [M-H]$^-$ | 1079.6 [M-H]$^-$ | 0.99 | A |
| 494 | 1008.9 [M-H]$^-$ | 1008.6 [M-H]$^-$ | 0.98 | A |
| 495 | 1023.0 [M-H]$^-$ | 1022.6 [M-H]$^-$ | 0.99 | A |
| 496 | 1063.9 [M-H]$^-$ | 1063.6 [M-H]$^-$ | 0.92 | A |
| 497 | 1051.9 [M-H]$^-$ | 1051.6 [M-H]$^-$ | 0.98 | A |
| 498 | 1006.9 [M-H]$^-$ | 1006.5 [M-H]$^-$ | 0.94 | A |
| 499 | 994.8 [M-H]$^-$ | 994.5 [M-H]$^-$ | 0.99 | A |
| 500 | 1105.7 [M-H]$^-$ | 1105.6 [M-H]$^-$ | 0.96 | A |
| 501 | 1093.8 [M-H]$^-$ | 1093.6 [M-H]$^-$ | 1.00 | A |
| 502 | 1021.9 [M-H]$^-$ | 1021.6 [M-H]$^-$ | 0.77 | A |
| 503 | 1009.9 [M-H]$^-$ | 1009.6 [M-H]$^-$ | 0.81 | A |
| 504 | 992.9 [M-H]$^-$ | 992.5 [M-H]$^-$ | 0.91 | A |
| 505 | 980.8 [M-H]$^-$ | 980.5 [M-H]$^-$ | 0.98 | A |
| 506 | 1050.0 [M-H]$^-$ | 1049.5 [M-H]$^-$ | 0.86 | A |
| 507 | 1037.9 [M-H]$^-$ | 1037.5 [M-H]$^-$ | 0.87 | A |
| 508 | 993.9 [M-H]$^-$ | 994.5 [M-H]$^-$ | 1.04 | A |
| 509 | 1191.0 [M-H]$^-$ | 1190.6 [M-H]$^-$ | 0.88 | A |
| 510 | 1163.0 [M-H]$^-$ | 1162.6 [M-H]$^-$ | 0.87 | A |
| 511 | 994.8 [M-H]$^-$ | 994.5 [M-H]$^-$ | 1.04 | A |
| 512 | 1008.8 [M-H]$^-$ | 1008.5 [M-H]$^-$ | 1.05 | A |
| 513 | 1052.0 [M-H]$^-$ | 1051.6 [M-H]$^-$ | 0.92 | A |
| 514 | 1179.0 [M-H]$^-$ | 1178.6 [M-H]$^-$ | 0.94 | A |
| 515 | 1150.9 [M-H]$^-$ | 1150.6 [M-H]$^-$ | 0.92 | A |
| 516 | 1007.7 [M-H]$^-$ | 1007.5 [M-H]$^-$ | 0.76 | A |
| 517 | 995.7 [M-H]$^-$ | 995.5 [M-H]$^-$ | 0.77 | A |
| 518 | 1221.9 [M-H]$^-$ | 1221.7 [M-H]$^-$ | 0.84 | A |
| 519 | 1193.9 [M-H]$^-$ | 1193.7 [M-H]$^-$ | 0.83 | A |
| 520 | 1109.0 [M-H]$^-$ | 1108.6 [M-H]$^-$ | 0.93 | A |
| 521 | 1123.0 [M-H]$^-$ | 1122.6 [M-H]$^-$ | 0.93 | A |
| 522 | 937.8 [M-H]$^-$ | 937.5 [M-H]$^-$ | 1.00 | A |
| 523 | 937.8 [M-H]$^-$ | 937.5 [M-H]$^-$ | 1.01 | A |
| 524 | 1067.1 [M-H]$^-$ | 1066.6 [M-H]$^-$ | 0.72 | A |
| 525 | 1067.0 [M-H]$^-$ | 1066.6 [M-H]$^-$ | 0.72 | A |

(continued)

| Compound No. | LCMS analysis | | | |
|---|---|---|---|---|
| | MS (found) | MS (calc.) | Retention time RT (min) | Analysis condition |
| 526 | 1080.8 $[M-H]^-$ | 1080.6 $[M-H]^-$ | 0.77 | A |
| 527 | 1080.5 $[M-H]^-$ | 1080.6 $[M-H]^-$ | 0.77 | A |
| 528 | 1108.9 $[M-H]^-$ | 1108.6 $[M-H]^-$ | 0.68 | A |
| 529 | 1009.9 $[M-H]^-$ | 1009.5 $[M-H]^-$ | 0.88 | A |
| 530 | 1009.9 $[M-H]^-$ | 1009.5 $[M-H]^-$ | 0.89 | A |
| 531 | 1023.8 $[M-H]^-$ | 1023.5 $[M-H]^-$ | 0.88 | A |
| 532 | 1023.9 $[M-H]^-$ | 1023.5 $[M-H]^-$ | 0.88 | A |
| 533 | 1037.9 $[M-H]^-$ | 1037.5 $[M-H]^-$ | 0.89 | A |
| 534 | 1051.9 $[M-H]^-$ | 1051.6 $[M-H]^-$ | 0.89 | A |
| 535 | 514.3 $[M+2H]^{2+}$ | 513.8 $[M+2H]^{2+}$ | 0.73 | A |
| 536 | 514.2 $[M+2H]^{2+}$ | 513.8 $[M+2H]^{2+}$ | 0.69 | A |
| 537 | 521.2 $[M+2H]^{2+}$ | 520.8 $[M+2H]^{2+}$ | 0.69 | A |
| 538 | 1053.0 $[M-H]^-$ | 1052.6 $[M-H]^-$ | 0.72 | A |
| 539 | 1052.9 $[M-H]^-$ | 1052.6 $[M-H]^-$ | 0.70 | A |
| 540 | 1095.0 $[M-H]^-$ | 1094.6 $[M-H]^-$ | 0.88 | A |
| 541 | 1108.9 $[M-H]^-$ | 1108.6 $[M-H]^-$ | 0.88 | A |
| 542 | 1038.0 $[M-H]^-$ | 1037.5 $[M-H]^-$ | 0.87 | A |
| 543 | 1094.0 $[M-H]^-$ | 1093.6 $[M-H]^-$ | 0.82 | A |
| 544 | 1094.0 $[M-H]^-$ | 1093.6 $[M-H]^-$ | 0.82 | A |
| 545 | 1080.0 $[M-H]^-$ | 1079.6 $[M-H]^-$ | 0.76 | A |
| 546 | 1079.8 $[M-H]^-$ | 1079.6 $[M-H]^-$ | 0.78 | A |
| 547 | 1038.8 $[M-H]^-$ | 1038.6 $[M-H]^-$ | 0.99 | A |
| 548 | 1038.9 $[M-H]^-$ | 1038.6 $[M-H]^-$ | 1.00 | A |
| 549 | 1024.9 $[M-H]^-$ | 1024.6 $[M-H]^-$ | 0.93 | A |
| 550 | 1024.9 $[M-H]^-$ | 1024.6 $[M-H]^-$ | 0.98 | A |
| 551 | 1109.0 $[M-H]^-$ | 1108.6 $[M-H]^-$ | 0.71 | A |
| 552 | 1095.0 $[M-H]^-$ | 1094.6 $[M-H]^-$ | 0.64 | A |
| 553 | 1095.0 $[M-H]^-$ | 1094.6 $[M-H]^-$ | 0.66 | A |
| 554 | 1053.7 $[M-H]^-$ | 1053.6 $[M-H]^-$ | 0.80 | A |
| 555 | 1053.8 $[M-H]^-$ | 1053.6 $[M-H]^-$ | 0.80 | A |
| 556 | 1039.8 $[M-H]^-$ | 1039.6 $[M-H]^-$ | 0.74 | A |
| 557 | 1039.8 $[M-H]^-$ | 1039.6 $[M-H]^-$ | 0.74 | A |
| 558 | 1094.9 $[M-H]^-$ | 1094.6 $[M-H]^-$ | 0.99 | A |
| 559 | 1109.9 $[M-H]^-$ | 1109.6 $[M-H]^-$ | 0.78 | A |
| 560 | 1095.8 $[M-H]^-$ | 1095.6 $[M-H]^-$ | 0.72 | A |
| 561 | 1039.0 $[M-H]^-$ | 1038.5 $[M-H]^-$ | 0.77 | A |
| 562 | 1038.8 $[M-H]^-$ | 1038.5 $[M-H]^-$ | 0.76 | A |

(continued)

| Compound No. | LCMS analysis | | | |
|---|---|---|---|---|
| | MS (found) | MS (calc.) | Retention time RT (min) | Analysis condition |
| 563 | 1053.0 $[M-H]^-$ | 1052.6 $[M-H]^-$ | 0.76 | A |
| 564 | 1052.9 $[M-H]^-$ | 1052.6 $[M-H]^-$ | 0.75 | A |
| 565 | 1066.9 $[M-H]^-$ | 1066.6 $[M-H]^-$ | 0.75 | A |
| 566 | 1067.0 $[M-H]^-$ | 1066.6 $[M-H]^-$ | 0.75 | A |
| 567 | 1051.9 $[M-H]^-$ | 1051.6 $[M-H]^-$ | 0.99 | A |
| 568 | 1051.9 $[M-H]^-$ | 1051.6 $[M-H]^-$ | 0.99 | A |
| 569 | 1051.9 $[M-H]^-$ | 1051.6 $[M-H]^-$ | 0.99 | A |
| 570 | 1051.9 $[M-H]^-$ | 1051.6 $[M-H]^-$ | 0.99 | A |
| 571 | 1081.9 $[M-H]^-$ | 1081.5 $[M-H]^-$ | 0.82 | A |
| 572 | 1081.9 $[M-H]^-$ | 1081.5 $[M-H]^-$ | 0.80 | A |
| 573 | 1023.9 $[M-H]^-$ | 1023.5 $[M-H]^-$ | 0.82 | A |
| 574 | 1023.9 $[M-H]^-$ | 1023.5 $[M-H]^-$ | 0.82 | A |
| 575 | 1065.9 $[M-H]^-$ | 1065.6 $[M-H]^-$ | 1.02 | A |
| 576 | 1065.8 $[M-H]^-$ | 1065.6 $[M-H]^-$ | 0.99 | A |
| 577 | 1066.9 $[M-H]^-$ | 1066.6 $[M-H]^-$ | 1.05 | A |
| 578 | 1021.8 $[M-H]^-$ | 1021.5 $[M-H]^-$ | 1.10 | A |
| 579 | 1031.9 $[M-H]^-$ | 1031.5 $[M-H]^-$ | 1.30 | A |
| 580 | 1019.8 $[M-H]^-$ | 1019.6 $[M-H]^-$ | 1.14 | A |
| 581 | 1031.8 $[M-H]^-$ | 1031.6 $[M-H]^-$ | 1.12 | A |
| 582 | 1047.8 $[M-H]^-$ | 1047.6 $[M-H]^-$ | 1.17 | A |
| 583 | 1059.8 $[M-H]^-$ | 1059.6 $[M-H]^-$ | 1.14 | A |
| 584 | 990.8 $[M-H]^-$ | 990.5 $[M-H]^-$ | 1.32 | A |
| 585 | 992.7 $[M-H]^-$ | 992.5 $[M-H]^-$ | 1.38 | A |
| 586 | 1004.7 $[M-H]^-$ | 1004.5 $[M-H]^-$ | 1.35 | A |
| 587 | 1002.7 $[M-H]^-$ | 1002.4 $[M-H]^-$ | 1.81 | A |
| 588 | 1016.7 $[M-H]^-$ | 1016.4 $[M-H]^-$ | 1.83 | A |
| 589 | 970.7 $[M-H]^-$ | 970.4 $[M-H]^-$ | 1.79 | A |
| 590 | 939.5 $[M-H]^-$ | 939.4 $[M-H]^-$ | 1.08 | A |
| 591 | 941.6 $[M+H]^+$ | 941.4 $[M+H]^+$ | 0.89 | A |
| 592 | 939.4 $[M-H]^-$ | 939.4 $[M-H]^-$ | 1.49 | A |
| 593 | 801.3 $[M+H]^+$ | 801.2 $[M+H]^+$ | 1.89 | B |
| 594 | 774.5 $[M-H]^-$ | 774.4 $[M-H]^-$ | 1.53 | A |
| 595 | 835.3 $[M+H]^+$ | 835.4 $[M+H]^+$ | 1.04 | A |
| 596 | 989.6 $[M+H]^+$ | 989.5 $[M+H]^+$ | 1.34 | A |
| 597 | 974.5 $[M-H]^-$ | 974.4 $[M-H]^-$ | 1.65 | A |
| 598 | 974.5 $[M-H]^-$ | 974.4 $[M-H]^-$ | 1.63 | A |
| 599 | 958.6 $[M+H]^+$ | 958.4 $[M+H]^+$ | 1.64 | A |

(continued)

| Compound No. | LCMS analysis | | | |
|---|---|---|---|---|
| | MS (found) | MS (calc.) | Retention time RT (min) | Analysis condition |
| 600 | 991.7 [M+H]+ | 991.5 [M+H]+ | 1.22 | A |
| 601 | 1007.7 [M+M]+ | 1007.5 [M+H]+ | 1.15 | A |
| 602 | 844.6 [M-H]- | 844.3 [M-H]- | 0.98 | A |
| 603 | 858.5 [M-H]- | 858.4 [M-H]- | 0.97 | A |
| 604 | 828.4 [M-H]- | 828.4 [M-H]- | 1.06 | A |
| 605 | 842.5 [M-H]- | 842.5 [M-H]- | 1.04 | A |
| 606 | 872.7 [M-H]- | 872.4 [M-H]- | 1.07 | A |
| 607 | 872.5 [M-H]- | 872.4 [M-H]- | 0.87 | A |
| 608 | 881.6 [M+H]+ | 881.4 [M+H]+ | 1.38 | A |
| 609 | 928.6 [M+H]+ | 928.4 [M+H]+ | 1.07 | A |
| 610 | 826.4 [M+H]+ | 826.3 [M+H]+ | 1.39 | A |
| 611 | 830.8 [M-H]- | 830.3 [M-H]- | 1.37 | A |
| 612 | 846.5 [M+H]+ | 846.4 [M+H]+ | 1.34 | A |
| 613 | 816.5 [M+H]+ | 816.4 [M+H]+ | 1.45 | A |
| 614 | 830.5 [M+H]+ | 830.4 [M+H]+ | 1.42 | A |
| 615 | 870.4 [M+H]+ | 870.4 [M+H]+ | 1.63 | A |
| 616 | 884.4 [M+H]+ | 884.4 [M+H]+ | 1.61 | A |
| 617 | 846.4 [M+H]+ | 846.4 [M+H]+ | 1.48 | A |
| 618 | 860.4 [M+H]+ | 860.4 [M+H]+ | 1.45 | A |
| 619 | 858.5 [M+H]+ | 858.4 [M+H]+ | 1.51 | A |
| 620 | 826.5 [M-H]- | 826.4 [M-H]- | 1.35 | A |
| 621 | 810.5 [M-H]- | 810.4 [M-H]- | 1.44 | A |
| 622 | 856.5 [M-H]- | 856.4 [M-H]- | 1.36 | A |
| 623 | 840.6 [M-H]- | 840.4 [M-H]- | 1.43 | A |
| 624 | 844.5 [M-H]- | 844.3 [M-H]- | 1.40 | A |
| 625 | 828.5 [M-H]- | 828.4 [M-H]- | 1.47 | A |
| 626 | 860.5 [M-H]- | 860.3 [M-H]- | 1.49 | A |
| 627 | 844.6 [M-H]- | 844.3 [M-H]- | 1.55 | A |
| 628 | 868.6 [M-H]- | 868.4 [M-H]- | 1.28 | A |
| 629 | 852.6 [M-H]- | 852.4 [M-H]- | 1.36 | A |
| 630 | 870.5 [M-H]- | 870.4 [M-H]- | 1.33 | A |
| 631 | 854.6 [M-H]- | 854.4 [M-H]- | 1.40 | A |
| 632 | 858.5 [M-H]- | 858.4 [M-H]- | 1.29 | A |
| 633 | 759.3 [M-H]- | 759.3 [M-H]- | 1.28 | A |
| 634 | 811.6 [M-H]- | 811.4 [M-H]- | 1.67 | A |
| 635 | 812.6 [M-H]- | 812.4 [M-H]- | 1.38 | A |
| 636 | 826.5 [M-H]- | 826.4 [M-H]- | 1.50 | A |

(continued)

| Compound No. | LCMS analysis | | | |
|---|---|---|---|---|
| | MS (found) | MS (calc.) | Retention time RT (min) | Analysis condition |
| 637 | 832.6 [M+H]+ | 832.3 [M+H]+ | 1.14 | A |
| 638 | 862.5 [M+H]+ | 862.4 [M+H]+ | 1.16 | A |
| 639 | 846.6 [M+H]+ | 846.4 [M+H]+ | 1.12 | A |
| 640 | 876.6 [M+H]+ | 876.4 [M+H]+ | 1.15 | A |
| 641 | 816.5 [M+H]+ | 816.4 [M+H]+ | 1.22 | A |
| 642 | 830.5 [M+H]+ | 830.4 [M+H]+ | 1.20 | A |
| 643 | 889.6 [M-H]- | 889.4 [M-H]- | 1.10 | A |
| 644 | 845.5 [M+H]+ | 845.3 [M+H]+ | 1.22 | A |
| 645 | 827.6 [M-H]- | 827.4 [M-H]- | 1.32 | A |
| 646 | 859.5 [M+H]+ | 859.4 [M+H]+ | 1.21 | A |
| 647 | 843.5 [M+H]+ | 843.4 [M+H]+ | 1.29 | A |
| 648 | 845.5 [M+H]+ | 845.3 [M+H]+ | 1.71 | B |
| 649 | 843.5 [M+H]+ | 843.4 [M+H]+ | 1.79 | B |
| 650 | 859.5 [M+H]+ | 859.4 [M+H]+ | 1.70 | B |
| 651 | 843.5 [M+H]+ | 843.4 [M+H]+ | 1.80 | B |
| 652 | 873.6 [M+H]+ | 873.4 [M+H]+ | 1.71 | B |
| 653 | 857.6 [M+H]+ | 857.4 [M+H]+ | 1.79 | B |
| 654 | 863.5 [M+H]+ | 863.3 [M+H]+ | 1.27 | A |
| 655 | 847.6 [M+H]+ | 847.4 [M+H]+ | 1.35 | A |
| 656 | 877.6 [M+H]+ | 877.3 [M+H]+ | 1.25 | A |
| 657 | 861.6 [M+H]+ | 861.4 [M+H]+ | 1.33 | A |
| 658 | 859.5 [M+H]+ | 859.4 [M+H]+ | 1.32 | A |
| 659 | 843.6 [M+H]+ | 843.4 [M+H]+ | 1.44 | A |
| 660 | 873.6 [M+H]+ | 873.4 [M+H]+ | 1.30 | A |
| 661 | 857.6 [M+H]+ | 857.4 [M+H]+ | 1.39 | A |
| 662 | 913.5 [M+H]+ | 913.3 [M+H]+ | 1.48 | A |
| 663 | 897.5 [M+H]+ | 897.4 [M+H]+ | 1.54 | A |
| 664 | 927.6 [M+H]+ | 927.3 [M+H]+ | 1.45 | A |
| 665 | 911.6 [M+H]+ | 911.4 [M+H]+ | 1.51 | A |
| 666 | 921.5 [M-H]- | 921.4 [M-H]- | 1.42 | A |
| 667 | 937.3 [M+H]+ | 937.3 [M+H]+ | 1.36 | A |
| 668 | 870.5 [M+H]+ | 870.3 [M+H]+ | 1.23 | A |
| 669 | 884.6 [M+H]+ | 884.4 [M+H]+ | 1.21 | A |
| 670 | 1071.7 [M+H]+ | 1071.5 [M+M]+ | 1.28 | A |
| 671 | 907.3 [M+H]+ | 907.3 [M+H]+ | 1.48 | A |
| 672 | 852.5 [M-H]- | 852.4 [M-H]- | 1.32 | A |
| 673 | 921.3 [M+H]+ | 921.3 [M+H]+ | 1.44 | A |

(continued)

| Compound No. | LCMS analysis | | | |
|---|---|---|---|---|
| | MS (found) | MS (calc.) | Retention time RT (min) | Analysis condition |
| 674 | 886.5 [M+H]+ | 886.4 [M+H]+ | 1.13 | A |
| 675 | 900.6 [M+H]+ | 900.4 [M+H]+ | 1.18 | A |
| 676 | 900.6 [M+H]+ | 900.4 [M+H]+ | 1.12 | A |
| 677 | 912.6 [M-H]- | 912.4 [M-H]- | 1.17 | A |
| 678 | 902.5 [M+H]+ | 902.4 [M+H]+ | 1.06 | A |
| 679 | 916.5 [M+H]+ | 916.4 [M+H]+ | 1.11 | A |
| 680 | 914.6 [M-H]- | 914.4 [M-H]- | 1.05 | A |
| 681 | 930.5 [M+H]+ | 930.4 [M+H]+ | 1.10 | A |
| 682 | 848.4 [M+H]+ | 848.4 [M+H]+ | 1.44 | A |
| 683 | 834.4 [M+H]+ | 834.4 [M+H]+ | 1.39 | A |
| 684 | 871.6 [M-H]- | 871.4 [M-H]- | 1.10 | A |
| 685 | 889.6 [M-H]- | 889.4 [M-H]- | 1.15 | A |
| 686 | 885.6 [M-H]- | 885.4 [M-H]- | 1.19 | A |
| 687 | 955.6 [M-H]- | 955.4 [M-H]- | 1.36 | A |
| 688 | 939.7 [M-H]- | 939.4 [M-H]- | 1.34 | A |
| 689 | 987.6 [M-H]- | 987.4 [M-H]- | 1.38 | A |
| 690 | 898.4 [M+H]+ | 898.4 [M+H]+ | 1.10 | A |
| 691 | 875.4 [M+H]+ | 875.4 [M+H]+ | 1.26 | A |
| 692 | 859.5 [M+H]+ | 859.4 [M+H]+ | 1.35 | A |
| 693 | 889.4 [M+H]+ | 889.4 [M+H]+ | 1.24 | A |
| 694 | 873.4 [M+H]+ | 873.4 [M+H]+ | 1.33 | A |
| 695 | 887.6 [M+H]+ | 887.4 [M+H]+ | 1.31 | A |
| 696 | 915.6 [M+H]+ | 915.5 [M+H]+ | 1.51 | A |
| 697 | 903.5 [M+H]+ | 903.4 [M+H]+ | 1.23 | A |
| 698 | 901.6 [M+H]+ | 901.4 [M+H]+ | 1.37 | A |
| 699 | 915.6 [M+H]+ | 915.5 [M+H]+ | 1.46 | A |
| 700 | 913.6 [M+H]+ | 913.4 [M+H]+ | 1.43 | A |
| 701 | 941.5 [M+H]+ | 941.4 [M+H]+ | 1.51 | A |
| 702 | 917.6 [M+H]+ | 917.4 [M+H]+ | 1.31 | A |
| 703 | 929.6 [M+H]+ | 929.4 [M+H]+ | 1.36 | A |
| 704 | 957.5 [M+H]+ | 957.4 [M+H]+ | 1.45 | A |
| 705 | 879.5 [M+H]+ | 879.3 [M+H]+ | 1.39 | A |
| 706 | 863.5 [M+H]+ | 863.3 [M+H]+ | 1.47 | A |
| 707 | 893.5 [M+H]+ | 893.3 [M+H]+ | 1.37 | A |
| 708 | 877.5 [M+H]+ | 877.4 [M+H]+ | 1.45 | A |
| 709 | 768.4 [M+H]+ | 768.2 [M+H]+ | 1.37 | A |
| 710 | 752.4 [M+H]+ | 752.3 [M+H]+ | 1.53 | A |

(continued)

| Compound No. | LCMS analysis | | | |
|---|---|---|---|---|
| | MS (found) | MS (calc.) | Retention time RT (min) | Analysis condition |
| 711 | 782.4 $[M+H]^+$ | 782.3 $[M+H]^+$ | 1.35 | A |
| 712 | 766.4 $[M+H]^+$ | 766.3 $[M+H]^+$ | 1.52 | A |
| 713 | 751.2 $[M-H]^-$ | 751.2 $[M-H]^-$ | 1.64 | A |
| 714 | 864.5 $[M+H]^+$ | 864.3 $[M+H]^+$ | 1.65 | A |
| 715 | 846.3 $[M-H]^-$ | 846.3 $[M-H]^-$ | 1.75 | A |
| 716 | 893.5 $[M+H]^+$ | 893.3 $[M+H]^+$ | 1.34 | A |
| 717 | 877.5 $[M+H]^+$ | 877.4 $[M+H]^+$ | 1.43 | A |
| 718 | 891.5 $[M+H]^+$ | 891.4 $[M+H]^+$ | 1.43 | A |
| 719 | 907.5 $[M+H]^+$ | 907.3 $[M+H]^+$ | 1.34 | A |
| 720 | 1003.8 $[M-H]^-$ | 1003.5 $[M-H]^-$ | 1.15 | A |
| 721 | 1031.7 $[M-H]^-$ | 1031.5 $[M-H]^-$ | 1.17 | A |
| 722 | 918.7 $[M-H]^-$ | 918.4 $[M-H]^-$ | 1.16 | A |
| 723 | 1017.8 $[M-H]^-$ | 1017.5 $[M-H]^-$ | 1.15 | A |
| 724 | 1045.8 $[M-H]^-$ | 1045.5 $[M-H]^-$ | 1.17 | A |
| 725 | 932.7 $[M-H]^-$ | 932.4 $[M-H]^-$ | 1.15 | A |
| 726 | 891.6 $[M+H]^+$ | 891.4 $[M+H]^+$ | 1.40 | A |
| 727 | 851.5 $[M+H]^+$ | 851.3 $[M+H]^+$ | 1.46 | A |
| 728 | 881.5 $[M+H]^+$ | 881.4 $[M+H]^+$ | 1.47 | A |
| 729 | 927.5 $[M+H]^+$ | 927.4 $[M+H]^+$ | 1.58 | A |
| 730 | 906.6 $[M-H]^-$ | 906.4 $[M-H]^-$ | 1.21 | A |
| 731 | 851.5 $[M+H]^+$ | 851.3 $[M+H]^+$ | 1.45 | A |
| 732 | 865.5 $[M+H]^+$ | 865.4 $[M+H]^+$ | 1.47 | A |
| 733 | 879.5 $[M+H]^+$ | 879.4 $[M+H]^+$ | 1.47 | A |
| 734 | 893.6 $[M+H]^+$ | 893.4 $[M+H]^+$ | 1.48 | A |
| 735 | 1104.7 $[M+H]^+$ | 1104.5 $[M+H]^+$ | 1.61 | A |
| 736 | 1132.8 $[M+H]^+$ | 1132.5 $[M+H]^+$ | 1.68 | A |
| 737 | 1159.0 $[M-H]^-$ | 1158.6 $[M-H]^-$ | 1.77 | A |
| 738 | 864.5 $[M-H]^-$ | 864.4 $[M-H]^-$ | 1.27 | A |
| 739 | 864.5 $[M-H]^-$ | 864.4 $[M-H]^-$ | 1.23 | A |
| 740 | 878.7 $[M-H]^-$ | 878.4 $[M-H]^-$ | 1.22 | A |
| 741 | 878.6 $[M-H]^-$ | 878.4 $[M-H]^-$ | 1.21 | A |
| 742 | 892.6 $[M-H]^-$ | 892.4 $[M-H]^-$ | 1.20 | A |
| 743 | 906.6 $[M-H]^-$ | 906.4 $[M-H]^-$ | 1.20 | A |
| 744 | 906.6 $[M-H]^-$ | 906.4 $[M-H]^-$ | 1.18 | A |
| 745 | 922.6 $[M-H]^-$ | 922.4 $[M-H]^-$ | 1.10 | A |
| 746 | 849.6 $[M-H]^-$ | 849.4 $[M-H]^-$ | 1.23 | A |
| 747 | 892.6 $[M-H]^-$ | 892.4 $[M-H]^-$ | 1.20 | A |

(continued)

| Compound No. | LCMS analysis | | | |
|---|---|---|---|---|
| | MS (found) | MS (calc.) | Retention time RT (min) | Analysis condition |
| 748 | 906.6 [M-H]- | 906.4 [M-H]- | 1.22 | A |
| 749 | 920.7 [M-H]- | 920.4 [M-H]- | 1.20 | A |
| 750 | 934.9 [M-H]- | 934.4 [M-H]- | 1.23 | A |
| 751 | 906.6 [M-H]- | 906.4 [M-H]- | 1.22 | A |
| 752 | 920.6 [M-H]- | 920.4 [M-H]- | 1.22 | A |
| 753 | 934.7 [M-H]- | 934.4 [M-H]- | 1.22 | A |
| 754 | 948.6 [M-H]- | 948.4 [M-H]- | 1.24 | A |
| 755 | 920.6 [M-H]- | 920.4 [M-H]- | 1.21 | A |
| 756 | 934.7 [M-H]- | 934.4 [M-H]- | 1.23 | A |
| 757 | 948.7 [M-H]- | 948.6 [M-H]- | 1.23 | A |
| 758 | 962.7 [M-H]- | 962.7 [M-H]- | 1.24 | A |
| 759 | 899.6 [M+H]+ | 899.4 [M+H]+ | 1.55 | A |
| 760 | 960.7 [M-H]- | 960.6 [M-H]- | 1.22 | A |
| 761 | 949.5 [M+H]+ | 949.4 [M+H]+ | 1.53 | A |
| 762 | 990.8 [M-H]- | 990.5 [M-H]- | 1.23 | A |
| 763 | 975.6 [M+H]+ | 975.4 [M+H]+ | 1.54 | A |
| 764 | 972.7 [M-H]- | 972.6 [M-H]- | 1.23 | A |
| 765 | 853.5 [M+H]+ | 853.3 [M+H]+ | 1.26 | A |
| 766 | 991.7 [M-H]- | 991.8 [M-H]- | 1.22 | A |
| 767 | 1019.8 [M-H]- | 1019.6 [M-H]- | 1.23 | A |
| 768 | 1147.9 [M-H]- | 1147.9 [M-H]- | 1.09 | A |
| 769 | 1175.9 [M-H]- | 1175.7 [M-H]- | 1.11 | A |
| 770 | 1147.9 [M-H]- | 1147.9 [M-H]- | 1.08 | A |
| 771 | 1119.7 [M-H]- | 1119.7 [M-H]- | 1.09 | A |
| 772 | 1003.7 [M-H]- | 1003.5 [M-H]- | 1.22 | A |
| 773 | 1164.0 [M-H]- | 1163.7 [M-H]- | 1.00 | A |
| 774 | 595.5 [M-2H]2- | 595.3 [M-2H]2- | 1.01 | A |
| 775 | 1163.9 [M-H]- | 1163.7 [M-H]- | 1.00 | A |
| 776 | 1136.0 [M-H]- | 1135.6 [M-H]- | 0.98 | A |
| 777 | 864.5 [M-H]- | 864.6 [M-H]- | 1.25 | A |
| 778 | 878.5 [M-H]- | 878.6 [M-H]- | 1.23 | A |
| 779 | 878.5 [M-H]- | 878.6 [M-H]- | 1.23 | A |
| 780 | 892.7 [M-H]- | 892.5 [M-H]- | 1.23 | A |
| 781 | 892.5 [M-H]- | 892.5 [M-H]- | 1.23 | A |
| 782 | 977.8 [M-H]- | 977.4 [M-H]- | 1.19 | A |
| 783 | 991.8 [M-H]- | 991.4 [M-H]- | 1.27 | A |
| 784 | 1007.8 [M-H]- | 1007.4 [M-H]- | 1.11 | A |

(continued)

| Compound No. | LCMS analysis | | | |
|---|---|---|---|---|
| | MS (found) | MS (calc.) | Retention time RT (min) | Analysis condition |
| 785 | 992.8 [M-H]- | 992.4 [M-H]- | 1.28 | A |
| 786 | 905.5 [M+H]+ | 905.3 [M+H]+ | 1.30 | A |
| 787 | 893.5 [M+H]+ | 893.3 [M+H]+ | 1.26 | A |
| 788 | 921.5 [M+H]+ | 921.4 [M+H]+ | 1.32 | A |
| 789 | 905.5 [M+H]+ | 905.3 [M+H]+ | 1.31 | A |
| 790 | 891.4 [M+H]+ | 891.3 [M+H]+ | 1.31 | A |
| 791 | 864.4 [M+H]+ | 864.4 [M+H]+ | 1.51 | A |
| 792 | 888.6 [M+H]+ | 888.5 [M+H]+ | 1.03 | A |
| 793 | 902.6 [M+H]+ | 902.4 [M+H]+ | 1.02 | A |
| 794 | 870.5 [M-H]- | 870.6 [M-H]- | 1.10 | A |
| 795 | 886.5 [M+H]+ | 886.6 [M+H]+ | 1.08 | A |
| 796 | 888.6 [M+H]+ | 888.4 [M+H]+ | 1.13 | A |
| 797 | 902.7 [M+H]+ | 902.7 [M+H]+ | 1.15 | A |
| 798 | 904.6 [M+H]+ | 904.7 [M+H]+ | 1.00 | A |
| 799 | 918.6 [M+H]+ | 918.5 [M+H]+ | 1.02 | A |
| 800 | 903.7 [M-H]- | 903.5 [M-H]- | 0.77 | A |
| 801 | 884.6 [M-H]- | 884.6 [M-H]- | 1.05 | A |
| 802 | 927.5 [M-H]- | 927.5 [M-H]- | 1.02 | A |
| 803 | 1000.8 [M-H]- | 1000.5 [M-H]- | 1.00 | A |
| 804 | 1028.7 [M-H]- | 1028.8 [M-H]- | 1.00 | A |
| 805 | 1156.9 [M-H]- | 1156.8 [M-H]- | 0.89 | A |
| 806 | 1184.9 [M-H]- | 1184.6 [M-H]- | 0.91 | A |
| 807 | 1129.0 [M-H]- | 1128.7 [M-H]- | 0.87 | A |
| 808 | 1043.0 [M-H]- | 1042.5 [M-H]- | 1.00 | A |
| 809 | 1171.0 [M-H]- | 1170.7 [M-H]- | 0.85 | A |
| 810 | 1171.0 [M-H]- | 1170.7 [M-H]- | 0.84 | A |
| 811 | 1142.8 [M-H]- | 1142.6 [M-H]- | 0.83 | A |
| 812 | 1014.8 [M-H]- | 1014.7 [M-H]- | 0.99 | A |
| 813 | 1199.0 [M-H]- | 1198.6 [M-H]- | 0.86 | A |
| 814 | 873.5 [M-H]- | 873.4 [M-H]- | 1.01 | A |
| 815 | 901.6 [M-H]- | 901.4 [M-H]- | 1.00 | A |
| 816 | 901.7 [M-H]- | 901.4 [M-H]- | 0.99 | A |
| 817 | 915.8 [M-H]- | 915.7 [M-H]- | 0.99 | A |
| 818 | 915.6 [M-H]- | 915.7 [M-H]- | 0.99 | A |
| 819 | 900.6 [M+H]+ | 900.4 [M+H]+ | 1.08 | A |
| 820 | 1026.8 [M-H]- | 1026.7 [M-H]- | 0.90 | A |
| 821 | 1054.7 [M-H]- | 1054.5 [M-H]- | 0.92 | A |

(continued)

| Compound No. | LCMS analysis | | | |
|---|---|---|---|---|
| | MS (found) | MS (calc.) | Retention time RT (min) | Analysis condition |
| 822 | 1183.0 [M-H]- | 1182.7 [M-H]- | 0.75 | A |
| 823 | 1211.0 [M-H]- | 1210.6 [M-H]- | 0.76 | A |
| 824 | 1183.1 [M-H]- | 1182.7 [M-H]- | 0.75 | A |
| 825 | 1155.0 [M-H]- | 1154.6 [M-H]- | 0.73 | A |
| 826 | 874.6 [M+H]+ | 874.6 [M+H]+ | 1.07 | A |
| 827 | 886.6 [M+H]+ | 886.6 [M+H]+ | 1.04 | A |
| 828 | 1072.0 [M-H]- | 1071.8 [M-H]- | 0.99 | A |
| 829 | 1099.9 [M-H]- | 1099.5 [M-H]- | 1.00 | A |
| 830 | 1256.0 [M-H]- | 1255.6 [M-H]- | 0.86 | A |
| 831 | 1228.1 [M-H]- | 1227.6 [M-H]- | 0.82 | A |
| 832 | 1085.8 [M-H]- | 1085.6 [M-H]- | 0.99 | A |
| 833 | 1242.1 [M-H]- | 1241.7 [M-H]- | 0.83 | A |
| 834 | 1270.1 [M-H]- | 1269.8 [M-H]- | 0.85 | A |
| 835 | 1242.1 [M-H]- | 1241.7 [M-H]- | 0.83 | A |
| 836 | 1228.0 [M-H]- | 1227.9 [M-H]- | 0.86 | A |
| 837 | 1199.8 [M-H]- | 1199.6 [M-H]- | 0.85 | A |
| 838 | 884.5 [M-H]- | 884.6 [M-H]- | 1.09 | A |
| 839 | 872.5 [M+H]+ | 872.6 [M+H]+ | 1.06 | A |
| 840 | 844.5 [M-H]- | 844.5 [M-H]- | 1.13 | A |
| 841 | 858.5 [M-H]- | 858.6 [M-H]- | 1.13 | A |
| 842 | 872.8 [M-H]- | 872.6 [M-H]- | 1.14 | A |
| 843 | 886.7 [M-H]- | 886.4 [M-H]- | 1.16 | A |
| 844 | 859.6 [M-H]- | 859.6 [M-H]- | 1.00 | A |
| 845 | 870.5 [M-H]- | 870.6 [M-H]- | 1.06 | A |
| 846 | 872.5 [M+H]+ | 872.6 [M+H]+ | 1.09 | A |
| 847 | 872.5 [M+H]+ | 872.6 [M+H]+ | 1.06 | A |
| 848 | 900.5 [M+H]+ | 900.4 [M+H]+ | 1.17 | A |
| 849 | 900.6 [M+H]+ | 900.4 [M+H]+ | 1.20 | A |
| 850 | 914.6 [M+H]+ | 914.7 [M+H]+ | 1.10 | A |
| 851 | 886.5 [M+H]+ | 886.4 [M+H]+ | 1.12 | A |
| 852 | 886.7 [M-H]- | 886.4 [M-H]- | 1.11 | A |
| 853 | 886.7 [M-H]- | 886.4 [M-H]- | 1.13 | A |
| 854 | 887.7 [M-H]- | 887.4 [M-H]- | 1.18 | A |
| 855 | 891.6 [M+H]+ | 891.4 [M+H]+ | 1.45 | A |
| 856 | 887.6 [M-H]- | 887.4 [M-H]- | 1.44 | A |
| 857 | 901.4 [M+H]+ | 901.4 [M+H]+ | 1.24 | A |
| 858 | 915.5 [M-H]- | 915.4 [M-H]- | 1.02 | A |

(continued)

| Compound No. | LCMS analysis | | | |
|---|---|---|---|---|
| | MS (found) | MS (calc.) | Retention time RT (min) | Analysis condition |
| 859 | 816.3 [M-H]- | 816.3 [M-H]- | 0.96 | A |
| 860 | 771.3 [M-H]- | 771.3 [M-H]- | 0.90 | A |
| 861 | 743.2 [M-H]- | 743.3 [M-H]- | 0.82 | A |
| 862 | 757.2 [M-H]- | 757.3 [M-H]- | 0.86 | A |
| 863 | 785.4 [M-H]- | 785.3 [M-H]- | 0.94 | A |
| 864 | 800.4 [M-H]- | 800.3 [M-H]- | 0.64 | A |
| 865 | 803.3 [M+H]+ | 803.3 [M+H]+ | 0.93 | A |
| 866 | 817.4 [M+H]+ | 817.3 [M+H]+ | 0.91 | A |
| 867 | 817.4 [M+H]+ | 817.3 [M+H]+ | 0.86 | A |
| 868 | 775.3 [M+H]+ | 775.3 [M+H]+ | 0.84 | A |
| 869 | 851.4 [M+H]+ | 851.3 [M+H]+ | 1.01 | A |
| 870 | 835.3 [M+H]+ | 835.3 [M+H]+ | 1.22 | A |
| 871 | 759.3 [M+H]+ | 759.3 [M+H]+ | 0.96 | A |
| 872 | 799.4 [M-H]- | 799.4 [M-H]- | 1.08 | A |
| 873 | 787.4 [M+H]+ | 787.3 [M+H]+ | 1.03 | A |
| 874 | 801.4 [M+H]+ | 801.4 [M+H]+ | 1.12 | A |
| 875 | 803.3 [M+H]+ | 803.3 [M+H]+ | 0.89 | A |
| 876 | 816.4 [M+H]+ | 816.4 [M+H]+ | 0.68 | A |
| 877 | 729.3 [M-H]- | 729.3 [M-H]- | 0.85 | A |
| 878 | 828.3 [M-H]- | 828.3 [M-H]- | 0.64 | A |
| 879 | 769.5 [M-H]- | 769.3 [M-H]- | 1.01 | A |
| 880 | 759.5 [M-H]- | 759.3 [M-H]- | 0.81 | A |
| 881 | 835.3 [M-H]- | 835.3 [M-H]- | 1.00 | A |
| 882 | 819.3 [M-H]- | 819.3 [M-H]- | 1.20 | A |
| 883 | 743.3 [M-H]- | 743.3 [M-H]- | 0.94 | A |
| 884 | 773.3 [M-H]- | 773.3 [M-H]- | 0.89 | A |
| 885 | 844.4 [M+H]+ | 844.4 [M+H]+ | 0.72 | A |
| 886 | 803.3 [M+H]+ | 803.3 [M+H]+ | 0.78 | A |
| 887 | 867.4 [M+H]+ | 867.3 [M+H]+ | 0.77 | A |
| 888 | 914.7 [M-H]- | 914.4 [M-H]- | 0.92 | A |
| 889 | 900.6 [M-H]- | 900.4 [M-H]- | 0.83 | A |
| 890 | 1015.5 [M+H]+ | 1015.4 [M+H]+ | 0.96 | A |
| 891 | 1054.7 [M-H]- | 1054.5 [M-H]- | 1.67 | A |
| 892 | 1053.7 [M-H]- | 1053.5 [M-H]- | 1.62 | A |
| 893 | 1105.8 [M+Na]+ | 1105.5 [M+Na]+ | 1.60 | A |
| 894 | 1098.6 [M+H]+ | 1098.5 [M+H]+ | 1.26 | A |
| 895 | 1100.6 [M+H]+ | 1100.5 [M+H]+ | 1.19 | A |

(continued)

| Compound No. | LCMS analysis | | | |
|---|---|---|---|---|
| | MS (found) | MS (calc.) | Retention time RT (min) | Analysis condition |
| 896 | 987.6 $[M+H]^+$ | 987.5 $[M+H]^+$ | 1.29 | A |
| 897 | 1073.6 $[M+Na]^+$ | 1073.5 $[M+Na]^+$ | 1.59 | A |
| 898 | 1080.8 $[M+H]^+$ | 1080.5 $[M+H]^+$ | 1.23 | A |
| 899 | 1082.7 $[M+H]^+$ | 1082.5 $[M+H]^+$ | 1.17 | A |
| 900 | 1546.2 $[M-H]^-$ | 1545.8 $[M-H]^-$ | 1.64 | A |
| 901 | 1598.2 $[M+Na]^+$ | 1597.8 $[M+Na]^+$ | 1.67 | A |
| 902 | 785.1 $[M+2H]^{2+}$ | 785.0 $[M+2H]^{2+}$ | 1.17 | A |
| 903 | 1686.5 $[M-H]^-$ | 1685.9 $[M-H]^-$ | 1.95 | A |
| 904 | 1714.3 $[M-H]^-$ | 1713.9 $[M-H]^-$ | 2.06 | A |
| 905 | 1651.7 $[M-H]^-$ | 1651.0 $[M-H]^-$ | 1.40 | A |
| 906 | 1733.4 $[M+H]^+$ | 1732.9 $[M+H]^+$ | 1.67 | A |
| 907 | 1696.5 $[M-H]^-$ | 1696.0 $[M-H]^-$ | 1.21 | A |
| 908 | 841.6 $[M+2H]^{2+}$ | 841.5 $[M+2H]^{2+}$ | 1.25 | A |
| 909 | 1696.4 $[M-H]^-$ | 1696.0 $[M-H]^-$ | 1.22 | A |
| 910 | 1733.2 $[M+H]^+$ | 1732.9 $[M+H]^+$ | 1.67 | A |
| 911 | 1695.9 $[M-H]^-$ | 1696.0 $[M-H]^-$ | 1.23 | A |
| 912 | 1662.8 $[M-H]^-$ | 1662.1 $[M-H]^-$ | 1.23 | A |
| 913 | 1664.4 $[M-H]^-$ | 1664.0 $[M-H]^-$ | 1.20 | A |
| 914 | 1724.6 $[M+H]^+$ | 1724.0 $[M+H]^+$ | 2.05 | B |
| 915 | 1726.5 $[M+H]^+$ | 1725.9 $[M+H]^+$ | 1.54 | A |
| 916 | 1687.6 $[M-H]^-$ | 1687.1 $[M-H]^-$ | 1.10 | A |
| 917 | 1689.6 $[M-H]^-$ | 1689.0 $[M-H]^-$ | 1.06 | A |
| 918 | 1754.5 $[M+H]^+$ | 1753.9 $[M+H]^+$ | 2.05 | B |
| 919 | 1736.6 $[M-H]^-$ | 1736.0 $[M-H]^-$ | 2.07 | B |
| 920 | 1717.6 $[M-H]^-$ | 1717.0 $[M-H]^-$ | 1.60 | B |
| 921 | 1701.2 $[M-H]^-$ | 1701.1 $[M-H]^-$ | 1.63 | B |
| 922 | 1723.9 $[M-H]^-$ | 1723.9 $[M-H]^-$ | 1.95 | B |
| 923 | 1710.6 $[M+H]^+$ | 1710.0 $[M+H]^+$ | 1.97 | B |
| 924 | 1689.5 $[M-H]^-$ | 1689.0 $[M-H]^-$ | 1.53 | B |
| 925 | 1673.0 $[M-H]^-$ | 1673.0 $[M-H]^-$ | 1.57 | B |
| 926 | 1445.0 $[M-H]^-$ | 1444.9 $[M-H]^-$ | 1.94 | B |
| 927 | 1573.7 $[M-H]^-$ | 1573.0 $[M-H]^-$ | 1.77 | B |
| 928 | 1829.7 $[M-H]^-$ | 1829.2 $[M-H]^-$ | 1.56 | B |
| 929 | 1957.9 $[M-H]^-$ | 1957.3 $[M-H]^-$ | 1.52 | B |
| 930 | 1433.4 $[M-H]^-$ | 1432.8 $[M-H]^-$ | 1.77 | B |
| 931 | 1561.4 $[M-H]^-$ | 1560.9 $[M-H]^-$ | 1.63 | B |
| 932 | 1817.3 $[M-H]^-$ | 1817.1 $[M-H]^-$ | 1.49 | B |

(continued)

| Compound No. | LCMS analysis | | | |
|---|---|---|---|---|
| | MS (found) | MS (calc.) | Retention time RT (min) | Analysis condition |
| 933 | 1945.8 $[M-H]^-$ | 1945.2 $[M-H]^-$ | 1.39 | B |
| 934 | 1689.7 $[M-H]^-$ | 1689.0 $[M-H]^-$ | 1.52 | B |
| 935 | 1773.7 $[M-H]^-$ | 1773.0 $[M-H]^-$ | 1.55 | B |
| 936 | 886.3 $[M-2H]^{2-}$ | 886.0 $[M-2H]^{2-}$ | 1.60 | B |
| 937 | 1597.3 $[M+H]^+$ | 1596.9 $[M+H]^+$ | 1.97 | B |
| 938 | 1452.3 $[M+H]^+$ | 1451.8 $[M+H]^+$ | 1.98 | B |
| 939 | 1434.3 $[M-H]^-$ | 1433.8 $[M-H]^-$ | 1.97 | B |
| 940 | 1726.5 $[M+H]^+$ | 1725.9 $[M+H]^+$ | 1.98 | B |
| 941 | 1307.0 $[M+H]^+$ | 1306.7 $[M+H]^+$ | 2.01 | B |
| 942 | 1417.5 $[M-H]^-$ | 1416.9 $[M-H]^-$ | 1.32 | A |
| 943 | 1545.6 $[M-H]^-$ | 1545.0 $[M-H]^-$ | 1.16 | A |
| 944 | 1461.0 $[M-H]^-$ | 1460.9 $[M-H]^-$ | 1.39 | A |
| 945 | 1589.7 $[M-H]^-$ | 1588.9 $[M-H]^-$ | 1.21 | A |
| 946 | 1661.6 $[M-H]^-$ | 1661.0 $[M-H]^-$ | 0.97 | A |
| 947 | 1645.8 $[M-H]^-$ | 1645.0 $[M-H]^-$ | 1.00 | A |
| 948 | 1461.3 $[M-H]^-$ | 1460.8 $[M-H]^-$ | 1.29 | A |
| 949 | 1617.2 $[M-H]^-$ | 1616.9 $[M-H]^-$ | 1.14 | A |
| 950 | 1675.4 $[M-H]^-$ | 1675.0 $[M-H]^-$ | 1.03 | A |
| 951 | 1291.0 $[M+H]^+$ | 1290.8 $[M+H]^+$ | 1.55 | A |
| 952 | 1335.1 $[M+H]^+$ | 1334.8 $[M+H]^+$ | 1.61 | A |
| 953 | 1319.1 $[M+H]^+$ | 1318.8 $[M+H]^+$ | 1.64 | A |
| 954 | 1745.5 $[M-H]^-$ | 1745.1 $[M-H]^-$ | 1.20 | A |
| 955 | 1729.8 $[M-H]^-$ | 1729.1 $[M-H]^-$ | 1.23 | A |
| 956 | 839.2 $[M+2H]^{2+}$ | 838.5 $[M+2H]^{2+}$ | 1.18 | A |
| 957 | 1702.8 $[M-H]^-$ | 1702.0 $[M-H]^-$ | 1.30 | A |
| 958 | 1718.3 $[M-H]^-$ | 1718.0 $[M-H]^-$ | 1.26 | A |
| 959 | 1674.3 $[M-H]^-$ | 1674.0 $[M-H]^-$ | 1.16 | A |
| 960 | 1702.4 $[M-H]^-$ | 1702.0 $[M-H]^-$ | 1.25 | A |
| 961 | 1690.2 $[M-H]^-$ | 1690.0 $[M-H]^-$ | 1.15 | A |
| 962 | 1719.3 $[M-H]^-$ | 1719.0 $[M-H]^-$ | 1.28 | A |
| 963 | 1675.2 $[M-H]^-$ | 1675.0 $[M-H]^-$ | 1.21 | A |
| 964 | 1703.4 $[M-H]^-$ | 1703.0 $[M-H]^-$ | 1.34 | A |
| 965 | 1691.1 $[M-H]^-$ | 1691.0 $[M-H]^-$ | 1.20 | A |
| 966 | 1719.4 $[M-H]^-$ | 1719.0 $[M-H]^-$ | 1.32 | A |
| 967 | 1418.0 $[M-H]^-$ | 1417.8 $[M-H]^-$ | 1.54 | A |
| 968 | 1448.0 $[M+H]^+$ | 1447.8 $[M+H]^+$ | 1.63 | A |
| 969 | 1420.0 $[M+H]^+$ | 1419.8 $[M+H]^+$ | 1.53 | A |

(continued)

| Compound No. | LCMS analysis | | | |
|---|---|---|---|---|
| | MS (found) | MS (calc.) | Retention time RT (min) | Analysis condition |
| 970 | 1446.1 [M-H]$^-$ | 1445.8 [M-H]$^-$ | 1.63 | A |
| 971 | 1546.3 [M-H]$^-$ | 1545.9 [M-H]$^-$ | 1.30 | A |
| 972 | 1573.9 [M-H]$^-$ | 1573.9 [M-H]$^-$ | 1.41 | A |

The inhibitory effects of the present inventive compounds on MMP2 were determined with the method described in Test Examples 1-1 and 1-2 shown below.

Test Example 1-1: Evaluation Test for Inhibitory Effects of Compounds of Present Invention on Human MMP2 (Method 1)

The human-MMP2-inhibitory effects of the compounds were determined through enzyme assay using MOCAc-Pro-Leu-Gly-Leu-A2pr(Dnp)-Ala-Arg-$NH_2$ as a substrate. In a reaction solution [50 mmol/L Tris-HCl (pH 7.5), 150 mmol/L NaCl, 10 mmol/L $CaCl_2$, 0.05% Brij L23], 100 μg/mL human recombinant MMP2 enzyme and 1 mmol/L 4-Aminophenylmercuric acetate were mixed together, and reacted at 37°C for 60 minutes. The human MMP2 activated through the reaction was poured into a 96-well microplate to reach a final concentration of 0.7 or 7 ng/mL. Further, the compounds of the present invention, which had been diluted to different concentrations, were added, and left to stand at room temperature for 15 minutes. Then, MOCAc-Pro-Leu-Gly-Leu-A2pr(Dnp)-Ala-Arg-$NH_2$ was added to reach a final concentration of 5 or 16 μmol/L, and enzymatic reaction was initiated. After reacting at room temperature for 2 hours, fluorescence intensity (Ex 320 nm/Em 400 nm) was measured by using a microplate reader. Enzyme inhibition rates (%) were calculated with the measured fluorescence values in accordance with the following formula, and the 50% inhibitory concentrations ($IC_{50}$ values) of the inventive compounds were calculated.

$$\text{Enzyme inhibition rate (\%)} = [1-(A-B)/(C-B)] * 100$$

A: Fluorescence value with addition of compound
B: Fluorescence value without addition of compound and enzyme
C: Fluorescence value without addition of compound

Test Example 1-2: Evaluation Test for Inhibitory Effects of Compounds of Present Invention on Human MMP2 (Method 2)

The human-MMP2-inhibitory effects of the compounds were determined through enzyme assay using MOCAc-Pro-Leu-Gly-Leu-A2pr(Dnp)-Ala-Arg-$NH_2$ as a substrate. In a reaction solution [50 mmol/L Tris-HCl (pH 7.5), 150 mmol/L NaCl, 10 mmol/L $CaCl_2$, 0.05% Brij L23], 12.5 μg/mL human recombinant MMP2 enzyme and 1 mmol/L 4-Aminophenylmercuric acetate were mixed together, and reacted at 37°C for 60 minutes. The human MMP2 activated through the reaction was poured into a 384-well microplate to reach a final concentration of 7 or 2.8 ng/mL. Further, the compounds of the present invention, which had been diluted to different concentrations, were added, and left to stand at room temperature for 10 minutes. Then, MOCAc-Pro-Leu-Gly-Leu-A2pr(Dnp)-Ala-Arg-$NH_2$ was added to reach a final concentration of 16 or 5 μmol/L, and enzymatic reaction was initiated. After reacting at room temperature for 1 to 2 hours, fluorescence intensity (Ex 320 nm/Em 405 nm) was measured by using a microplate reader. Enzyme inhibition rates (%) were calculated with the measured fluorescence values in accordance with the following formula, and the 50% inhibitory concentrations ($IC_{50}$ values) of the inventive compounds were calculated.

$$\text{Enzyme inhibition rate (\%)} = [1-(A-B)/(C-B)] * 100$$

A: Fluorescence value with addition of compound
B: Fluorescence value without addition of compound and enzyme
C: Fluorescence value without addition of compound

The results on the inhibitory effects of the present inventive compounds on human MMP2 enzyme activity are shown in the following tables.

[Table 51-1]

| Compound No. | Human MMP2, $IC_{50}$ (nmol/L) | Test Example No. | Compound No. | Human MMP2, $IC_{50}$ (nmol/L) | Test Example No. |
|---|---|---|---|---|---|
| 1 | 0.22 | 1-1 | 29 | 0.140 | 1-2 |
| 2 | 2.04 | 1-2 | 30 | 0.107 | 1-2 |
| 3 | 0.25 | 1-1 | 31 | 0.199 | 1-2 |
| 4 | 0.128 | 1-2 | 32 | 0.0996 | 1-2 |
| 5 | 0.608 | 1-2 | 33 | 0.144 | 1-2 |
| 6 | 0.198 | 1-2 | 34 | 0.130 | 1-2 |
| 7 | 0.898 | 1-2 | 35 | 0.147 | 1-2 |
| 8 | 3.15 | 1-2 | 36 | 0.152 | 1-2 |
| 9 | 0.29 | 1-1 | 37 | 0.178 | 1-2 |
| 10 | 0.107 | 1-2 | 38 | 0.27 | 1-1 |
| 11 | 0.109 | 1-2 | 39 | 0.27 | 1-1 |
| 12 | 0.109 | 1-2 | 40 | 0.451 | 1-2 |
| 13 | 0.0784 | 1-2 | 41 | 0.0719 | 1-2 |
| 14 | 0.0897 | 1-2 | 42 | 0.113 | 1-2 |
| 15 | 0.108 | 1-2 | 43 | 0.594 | 1-2 |
| 16 | 0.119 | 1-2 | 44 | 4.75 | 1-2 |
| 17 | 0.199 | 1-2 | 45 | 0.32 | 1-1 |
| 18 | 0.135 | 1-2 | 46 | 0.31 | 1-1 |
| 19 | 0.148 | 1-2 | 47 | 4.1 | 1-1 |
| 20 | 0.20 | 1-1 | 48 | 0.0740 | 1-2 |
| 21 | 0.101 | 1-2 | 49 | 0.113 | 1-2 |
| 22 | 0.109 | 1-2 | 50 | 0.089 | 1-1 |
| 23 | 0.0923 | 1-2 | 51 | 0.21 | 1-1 |
| 24 | 0.105 | 1-2 | 52 | 0.12 | 1-1 |
| 25 | 0.178 | 1-2 | 53 | 0.0702 | 1-2 |
| 26 | 0.151 | 1-2 | 54 | 0.095 | 1-1 |
| 27 | 0.152 | 1-2 | 55 | 0.13 | 1-1 |
| 28 | 0.136 | 1-2 | | | |

[Table 51-2]

| Compound No. | Human MMP2, $IC_{50}$ (nmol/L) | Test Example No. | Compound No. | Human MMP2, $IC_{50}$ (nmol/L) | Test Example No. |
|---|---|---|---|---|---|
| 56 | 0.083 | 1-1 | 86 | 0.40 | 1-1 |
| 57 | 0.078 | 1-1 | 87 | 0.84 | 1-1 |
| 58 | 0.097 | 1-1 | 88 | 0.97 | 1-1 |
| 59 | 0.046 | 1-1 | 89 | 0.58 | 1-1 |

(continued)

| Compound No. | Human MMP2, $IC_{50}$ (nmol/L) | Test Example No. | Compound No. | Human MMP2, $IC_{50}$ (nmol/L) | Test Example No. |
|---|---|---|---|---|---|
| 60 | 0.97 | 1-1 | 90 | 1.4 | 1-1 |
| 61 | 0.13 | 1-1 | 91 | 1.5 | 1-1 |
| 62 | 0.090 | 1-1 | 92 | 0.31 | 1-1 |
| 63 | 0.61 | 1-1 | 93 | 0.34 | 1-1 |
| 64 | 0.060 | 1-1 | 94 | 0.088 | 1-1 |
| 65 | 0.083 | 1-1 | 95 | 0.13 | 1-1 |
| 66 | 0.047 | 1-1 | 96 | 0.74 | 1-1 |
| 67 | 0.11 | 1-1 | 97 | 0.84 | 1-1 |
| 68 | 0.068 | 1-1 | 98 | 0.27 | 1-1 |
| 69 | 0.19 | 1-1 | 99 | 0.33 | 1-1 |
| 70 | 0.34 | 1-1 | 100 | 1.2 | 1-1 |
| 71 | 0.89 | 1-1 | 101 | 1.2 | 1-1 |
| 72 | 0.52 | 1-1 | 102 | 0.55 | 1-1 |
| 73 | 1.9 | 1-1 | 103 | 0.67 | 1-1 |
| 74 | 8.1 | 1-1 | 104 | 0.51 | 1-1 |
| 75 | 0.097 | 1-1 | 105 | 0.30 | 1-1 |
| 76 | 0.055 | 1-1 | 106 | 0.16 | 1-1 |
| 77 | 0.25 | 1-1 | 107 | 0.32 | 1-1 |
| 78 | 0.037 | 1-1 | 108 | 0.19 | 1-1 |
| 79 | 0.091 | 1-1 | 109 | 0.70 | 1-1 |
| 80 | 0.22 | 1-1 | 110 | 0.37 | 1-1 |
| 81 | 0.48 | 1-1 | 111 | 0.96 | 1-1 |
| 82 | 0.83 | 1-1 | 112 | 0.55 | 1-1 |
| 83 | 0.37 | 1-1 | 113 | 2.2 | 1-1 |
| 84 | 0.17 | 1-1 | 114 | 0.072 | 1-1 |
| 85 | 0.49 | 1-1 | 115 | 0.42 | 1-1 |

[Table 51-3]

| Compound No. | Human MMP2, $IC_{50}$ (nmol/L) | Test Example No. | Compound No. | Human MMP2, $IC_{50}$ (nmol/L) | Test Example No. |
|---|---|---|---|---|---|
| 116 | 0.055 | 1-1 | 146 | 1.7 | 1-1 |
| 117 | 0.55 | 1-1 | 147 | 0.14 | 1-1 |
| 118 | 0.40 | 1-1 | 148 | 0.48 | 1-1 |
| 119 | 0.17 | 1-1 | 149 | 0.11 | 1-1 |
| 120 | 0.21 | 1-1 | 150 | 3.3 | 1-1 |
| 121 | 0.19 | 1-1 | 151 | 0.33 | 1-1 |
| 122 | 0.16 | 1-1 | 152 | 0.85 | 1-1 |

(continued)

| Compound No. | Human MMP2, $IC_{50}$ (nmol/L) | Test Example No. | Compound No. | Human MMP2, $IC_{50}$ (nmol/L) | Test Example No. |
|---|---|---|---|---|---|
| 123 | 1.11 | 1-1 | 153 | 0.066 | 1-1 |
| 124 | 0.31 | 1-1 | 154 | 0.44 | 1-1 |
| 125 | 0.47 | 1-1 | 155 | 0.19 | 1-1 |
| 126 | 0.29 | 1-1 | 156 | 0.088 | 1-1 |
| 127 | 0.24 | 1-1 | 157 | 1.24 | 1-1 |
| 128 | 0.15 | 1-1 | 158 | 1.38 | 1-1 |
| 129 | 0.46 | 1-1 | 159 | 0.37 | 1-1 |
| 130 | 0.36 | 1-1 | 160 | 0.68 | 1-1 |
| 131 | 0.36 | 1-1 | 161 | 0.33 | 1-1 |
| 132 | 0.43 | 1-1 | 162 | 0.60 | 1-1 |
| 133 | 0.88 | 1-1 | 163 | 0.12 | 1-1 |
| 134 | 2.0 | 1-1 | 164 | 0.37 | 1-1 |
| 135 | 9.3 | 1-1 | 165 | 0.23 | 1-1 |
| 136 | 4.7 | 1-1 | 166 | 0.39 | 1-1 |
| 137 | 0.60 | 1-1 | 167 | 0.58 | 1-1 |
| 138 | 0.26 | 1-1 | 168 | 1.2 | 1-1 |
| 139 | 1.1 | 1-1 | 169 | 2.5 | 1-1 |
| 140 | 0.43 | 1-1 | 170 | 0.072 | 1-1 |
| 141 | 1.0 | 1-1 | 171 | 0.068 | 1-1 |
| 142 | 2.1 | 1-1 | 172 | 0.13 | 1-1 |
| 143 | 2.6 | 1-1 | 173 | 0.19 | 1-1 |
| 144 | 0.26 | 1-1 | 174 | 0.55 | 1-1 |
| 145 | 0.72 | 1-1 | 175 | 2.0 | 1-1 |

[Table 51-4]

| Compound No. | Human MMP2, $IC_{50}$ (nmol/L) | Test Example No. | Compound No. | Human MMP2, $IC_{50}$ (nmol/L) | Test Example No. |
|---|---|---|---|---|---|
| 176 | 1.9 | 1-1 | 206 | 0.101 | 1-1 |
| 177 | 0.21 | 1-1 | 207 | 0.49 | 1-1 |
| 178 | 3.6 | 1-1 | 208 | 0.61 | 1-1 |
| 179 | 3.5 | 1-1 | 209 | 0.094 | 1-1 |
| 180 | 2.6 | 1-1 | 210 | 0.17 | 1-1 |
| 181 | 3.1 | 1-1 | 211 | 2.9 | 1-1 |
| 182 | 0.49 | 1-1 | 212 | 0.62 | 1-1 |
| 183 | 7.0 | 1-1 | 213 | 1.9 | 1-1 |
| 184 | 0.61 | 1-1 | 214 | 0.096 | 1-1 |
| 185 | 1.0 | 1-1 | 215 | 2.6 | 1-1 |

(continued)

| Compound No. | Human MMP2, $IC_{50}$ (nmol/L) | Test Example No. | Compound No. | Human MMP2, $IC_{50}$ (nmol/L) | Test Example No. |
|---|---|---|---|---|---|
| 186 | 2.0 | 1-1 | 216 | 0.21 | 1-1 |
| 187 | 2.8 | 1-1 | 217 | 0.72 | 1-1 |
| 188 | 0.81 | 1-1 | 218 | 0.32 | 1-1 |
| 189 | 0.86 | 1-1 | 219 | 0.47 | 1-1 |
| 190 | 1.8 | 1-1 | 220 | 0.12 | 1-1 |
| 191 | 2.7 | 1-1 | 221 | 0.53 | 1-1 |
| 192 | 1.9 | 1-1 | 222 | 0.051 | 1-1 |
| 193 | 1.7 | 1-1 | 223 | 0.20 | 1-1 |
| 194 | 3.7 | 1-1 | 224 | 1.1 | 1-1 |
| 195 | 2.7 | 1-1 | 225 | 0.33 | 1-1 |
| 196 | 1.7 | 1-1 | 226 | 0.12 | 1-1 |
| 197 | 2.6 | 1-1 | 227 | 0.49 | 1-1 |
| 198 | 5.1 | 1-1 | 228 | 0.19 | 1-1 |
| 199 | 1.4 | 1-1 | 229 | 0.39 | 1-1 |
| 200 | 7.4 | 1-1 | 230 | 0.103 | 1-1 |
| 201 | 1.4 | 1-1 | 231 | 1.1 | 1-1 |
| 202 | 0.097 | 1-1 | 232 | 0.18 | 1-1 |
| 203 | 0.13 | 1-1 | 233 | 0.17 | 1-1 |
| 204 | 0.42 | 1-1 | 234 | 0.14 | 1-1 |
| 205 | 0.80 | 1-1 | 235 | 0.22 | 1-1 |

[Table 51-5]

| Compound No. | Human MMP2, $IC_{50}$ (nmol/L) | Test Example No. | Compound No. | Human MMP2, $IC_{50}$ (nmol/L) | Test Example No. |
|---|---|---|---|---|---|
| 236 | 0.10 | 1-1 | 266 | 1.5 | 1-1 |
| 237 | 0.53 | 1-1 | 267 | 1.6 | 1-1 |
| 238 | 0.11 | 1-1 | 268 | 0.16 | 1-1 |
| 239 | 0.108 | 1-1 | 269 | 0.15 | 1-1 |
| 240 | 0.44 | 1-1 | 270 | 0.12 | 1-1 |
| 241 | 0.32 | 1-1 | 271 | 0.084 | 1-1 |
| 242 | 6.3 | 1-1 | 272 | 0.13 | 1-1 |
| 243 | 0.33 | 1-1 | 273 | 0.071 | 1-1 |
| 244 | 0.27 | 1-1 | 274 | 0.12 | 1-1 |
| 245 | 0.16 | 1-1 | 275 | 0.096 | 1-1 |
| 246 | 0.64 | 1-1 | 276 | 0.103 | 1-1 |
| 247 | 1.9 | 1-1 | 277 | 0.12 | 1-1 |
| 248 | 6.0 | 1-1 | 278 | 0.71 | 1-1 |

(continued)

| Compound No. | Human MMP2, $IC_{50}$ (nmol/L) | Test Example No. | Compound No. | Human MMP2, $IC_{50}$ (nmol/L) | Test Example No. |
|---|---|---|---|---|---|
| 249 | 8.9 | 1-1 | 279 | 0.084 | 1-1 |
| 250 | 0.093 | 1-1 | 280 | 0.15 | 1-1 |
| 251 | 0.18 | 1-1 | 281 | 0.047 | 1-1 |
| 252 | 0.31 | 1-1 | 282 | 0.059 | 1-1 |
| 253 | 3.5 | 1-1 | 283 | 0.046 | 1-1 |
| 254 | 0.18 | 1-1 | 284 | 0.064 | 1-1 |
| 255 | 0.072 | 1-1 | 285 | 0.091 | 1-1 |
| 256 | 0.13 | 1-1 | 286 | 1.15 | 1-1 |
| 257 | 0.68 | 1-1 | 287 | 0.35 | 1-1 |
| 258 | 0.94 | 1-1 | 288 | 1.9 | 1-1 |
| 259 | 0.083 | 1-1 | 289 | 0.29 | 1-1 |
| 260 | 0.15 | 1-1 | 290 | 1.3 | 1-1 |
| 261 | 0.13 | 1-1 | 291 | 0.11 | 1-1 |
| 262 | 0.14 | 1-1 | 292 | 0.14 | 1-1 |
| 263 | 0.17 | 1-1 | 293 | 2.8 | 1-1 |
| 264 | 0.97 | 1-1 | 294 | 2.1 | 1-1 |
| 265 | 2.0 | 1-1 | 295 | 2.8 | 1-1 |

[Table 51-6]

| Compound No. | Human MMP2, $IC_{50}$ (nmol/L) | Test Example No. | Compound No. | Human MMP2, $IC_{50}$ (nmol/L) | Test Example No. |
|---|---|---|---|---|---|
| 296 | 0.96 | 1-1 | 326 | 1.9 | 1-1 |
| 297 | 0.35 | 1-1 | 327 | 0.92 | 1-1 |
| 298 | 3.4 | 1-1 | 328 | 0.43 | 1-1 |
| 299 | 0.62 | 1-1 | 329 | 2.5 | 1-1 |
| 300 | 0.86 | 1-1 | 330 | 0.22 | 1-1 |
| 301 | 2.0 | 1-1 | 331 | 0.22 | 1-1 |
| 302 | 0.29 | 1-1 | 332 | 0.14 | 1-1 |
| 303 | 0.21 | 1-1 | 333 | 0.84 | 1-1 |
| 304 | 1.05 | 1-1 | 334 | 0.63 | 1-1 |
| 305 | 1.9 | 1-1 | 335 | 0.56 | 1-1 |
| 306 | 2.2 | 1-1 | 336 | 0.13 | 1-1 |
| 307 | 3.7 | 1-1 | 337 | 0.17 | 1-1 |
| 308 | 0.48 | 1-1 | 338 | 0.13 | 1-1 |
| 309 | 1.4 | 1-1 | 339 | 0.62 | 1-1 |
| 310 | 3.2 | 1-1 | 340 | 0.16 | 1-1 |
| 311 | 4.5 | 1-1 | 341 | 0.77 | 1-1 |

(continued)

| Compound No. | Human MMP2, $IC_{50}$ (nmol/L) | Test Example No. | Compound No. | Human MMP2, $IC_{50}$ (nmol/L) | Test Example No. |
|---|---|---|---|---|---|
| 312 | 0.29 | 1-1 | 342 | 3.9 | 1-1 |
| 313 | 0.57 | 1-1 | 343 | 7.0 | 1-1 |
| 314 | 0.11 | 1-1 | 344 | 0.13 | 1-1 |
| 315 | 0.54 | 1-1 | 345 | 0.10 | 1-1 |
| 316 | 1.06 | 1-1 | 346 | 0.11 | 1-1 |
| 317 | 2.3 | 1-1 | 347 | 0.059 | 1-1 |
| 318 | 0.15 | 1-1 | 348 | 0.083 | 1-1 |
| 319 | 0.75 | 1-1 | 349 | 1.4 | 1-1 |
| 320 | 0.061 | 1-1 | 350 | 0.21 | 1-1 |
| 321 | 5.8 | 1-1 | 351 | 0.34 | 1-1 |
| 322 | 0.27 | 1-1 | 352 | 0.26 | 1-1 |
| 323 | 0.17 | 1-1 | 353 | 0.28 | 1-1 |
| 324 | 7.8 | 1-1 | 354 | 0.076 | 1-1 |
| 325 | 0.17 | 1-1 | 355 | 0.12 | 1-1 |

[Table 51-7]

| Compound No. | Human MMP2, $IC_{50}$ (nmol/L) | Test Example No. | Compound No. | Human MMP2, $IC_{50}$ (nmol/L) | Test Example No. |
|---|---|---|---|---|---|
| 356 | 0.19 | 1-1 | 386 | 0.12 | 1-1 |
| 357 | 0.10 | 1-1 | 387 | 0.12 | 1-1 |
| 358 | 0.14 | 1-1 | 388 | 0.20 | 1-1 |
| 359 | 5.1 | 1-1 | 389 | 0.39 | 1-1 |
| 360 | 2.5 | 1-1 | 390 | 0.36 | 1-1 |
| 361 | 0.20 | 1-1 | 391 | 0.37 | 1-1 |
| 362 | 0.27 | 1-1 | 392 | 0.15 | 1-1 |
| 363 | 0.24 | 1-1 | 393 | 0.28 | 1-1 |
| 364 | 0.25 | 1-1 | 394 | 0.17 | 1-1 |
| 365 | 0.49 | 1-1 | 395 | 0.48 | 1-1 |
| 366 | 0.33 | 1-1 | 396 | 0.40 | 1-1 |
| 367 | 0.17 | 1-1 | 397 | 0.61 | 1-1 |
| 368 | 0.28 | 1-1 | 398 | 0.12 | 1-1 |
| 369 | 0.76 | 1-1 | 399 | 0.13 | 1-1 |
| 370 | 1.2 | 1-1 | 400 | 0.43 | 1-1 |
| 371 | 2.1 | 1-1 | 401 | 0.43 | 1-1 |
| 372 | 6.5 | 1-1 | 402 | 0.31 | 1-1 |
| 373 | 4.7 | 1-1 | 403 | 0.20 | 1-1 |
| 374 | 1.9 | 1-1 | 404 | 0.42 | 1-1 |

(continued)

| Compound No. | Human MMP2, $IC_{50}$ (nmol/L) | Test Example No. | Compound No. | Human MMP2, $IC_{50}$ (nmol/L) | Test Example No. |
|---|---|---|---|---|---|
| 375 | 2.4 | 1-1 | 405 | 0.15 | 1-1 |
| 376 | 3.4 | 1-1 | 406 | 0.45 | 1-1 |
| 377 | 2.8 | 1-1 | 407 | 0.15 | 1-1 |
| 378 | 2.6 | 1-1 | 408 | 2.0 | 1-1 |
| 379 | 0.20 | 1-1 | 409 | 1.8 | 1-1 |
| 380 | 0.75 | 1-1 | 410 | 1.1 | 1-1 |
| 381 | 0.23 | 1-1 | 411 | 0.59 | 1-1 |
| 382 | 5.3 | 1-1 | 412 | 0.30 | 1-1 |
| 383 | 6.3 | 1-1 | 413 | 0.13 | 1-1 |
| 384 | 0.13 | 1-1 | 414 | 0.27 | 1-1 |
| 385 | 0.17 | 1-1 | 415 | 0.082 | 1-1 |

[Table 51-8]

| Compound No. | Human MMP2, $IC_{50}$ (nmol/L) | Test Example No. | Compound No. | Human MMP2, $IC_{50}$ (nmol/L) | Test Example No. |
|---|---|---|---|---|---|
| 416 | 0.29 | 1-1 | 446 | 0.46 | 1-1 |
| 417 | 1.5 | 1-1 | 447 | 0.50 | 1-1 |
| 418 | 2.1 | 1-1 | 448 | 0.19 | 1-1 |
| 419 | 1.8 | 1-1 | 449 | 0.17 | 1-1 |
| 420 | 1.5 | 1-1 | 450 | 0.24 | 1-1 |
| 421 | 2.6 | 1-1 | 451 | 0.16 | 1-1 |
| 422 | 1.8 | 1-1 | 452 | 0.14 | 1-1 |
| 423 | 1.9 | 1-1 | 453 | 30 | 1-1 |
| 424 | 0.44 | 1-1 | 454 | 0.19 | 1-1 |
| 425 | 0.62 | 1-1 | 455 | 0.46 | 1-1 |
| 426 | 0.27 | 1-1 | 456 | 0.61 | 1-1 |
| 427 | 0.073 | 1-1 | 457 | 0.24 | 1-1 |
| 428 | 1.0 | 1-1 | 458 | 0.27 | 1-1 |
| 429 | 0.15 | 1-1 | 459 | 0.25 | 1-1 |
| 430 | 0.071 | 1-1 | 460 | 0.29 | 1-1 |
| 431 | 0.69 | 1-1 | 461 | 0.29 | 1-1 |
| 432 | 0.38 | 1-1 | 462 | 0.33 | 1-1 |
| 433 | 0.19 | 1-1 | 463 | 0.76 | 1-1 |
| 434 | 0.23 | 1-1 | 464 | 0.79 | 1-1 |
| 435 | 0.45 | 1-1 | 465 | 0.82 | 1-1 |
| 436 | 0.30 | 1-1 | 466 | 1.1 | 1-1 |
| 437 | 0.21 | 1-1 | 467 | 0.85 | 1-1 |

(continued)

| Compound No. | Human MMP2, $IC_{50}$ (nmol/L) | Test Example No. | Compound No. | Human MMP2, $IC_{50}$ (nmol/L) | Test Example No. |
|---|---|---|---|---|---|
| 438 | 0.26 | 1-1 | 468 | 0.52 | 1-1 |
| 439 | 0.16 | 1-1 | 469 | 0.85 | 1-1 |
| 440 | 0.28 | 1-1 | 470 | 0.81 | 1-1 |
| 441 | 0.29 | 1-1 | 471 | 1.6 | 1-1 |
| 442 | 0.67 | 1-1 | 472 | 0.21 | 1-1 |
| 443 | 0.32 | 1-1 | 473 | 0.18 | 1-1 |
| 444 | 0.17 | 1-1 | 474 | 0.30 | 1-1 |
| 445 | 0.24 | 1-1 | 475 | 0.19 | 1-1 |

[Table 51-9]

| Compound No. | Human MMP2, $IC_{50}$ (nmol/L) | Test Example No. | Compound No. | Human MMP2, $IC_{50}$ (nmol/L) | Test Example No. |
|---|---|---|---|---|---|
| 476 | 0.20 | 1-1 | 506 | 0.15 | 1-1 |
| 477 | 0.46 | 1-1 | 507 | 0.16 | 1-1 |
| 478 | 0.53 | 1-1 | 508 | 0.30 | 1-1 |
| 479 | 0.66 | 1-1 | 509 | 0.28 | 1-1 |
| 480 | 0.45 | 1-1 | 510 | 0.40 | 1-1 |
| 481 | 2.4 | 1-1 | 511 | 0.20 | 1-1 |
| 482 | 0.90 | 1-1 | 512 | 0.15 | 1-1 |
| 483 | 0.22 | 1-1 | 513 | 0.18 | 1-1 |
| 484 | 0.26 | 1-1 | 514 | 0.22 | 1-1 |
| 485 | 0.096 | 1-1 | 515 | 0.29 | 1-1 |
| 486 | 0.39 | 1-1 | 516 | 1.3 | 1-1 |
| 487 | 1.4 | 1-1 | 517 | 1.3 | 1-1 |
| 488 | 1.8 | 1-1 | 518 | 1.4 | 1-1 |
| 489 | 0.27 | 1-1 | 519 | 1.6 | 1-1 |
| 490 | 0.86 | 1-1 | 520 | 0.32 | 1-1 |
| 491 | 0.81 | 1-1 | 521 | 0.41 | 1-1 |
| 492 | 0.68 | 1-1 | 522 | 3.1 | 1-1 |
| 493 | 0.55 | 1-1 | 523 | 1.9 | 1-1 |
| 494 | 0.37 | 1-1 | 524 | 0.12 | 1-1 |
| 495 | 0.44 | 1-1 | 525 | 0.15 | 1-1 |
| 496 | 0.23 | 1-1 | 526 | 0.21 | 1-1 |
| 497 | 0.24 | 1-1 | 527 | 0.32 | 1-1 |
| 498 | 0.32 | 1-1 | 528 | 0.23 | 1-1 |
| 499 | 0.31 | 1-1 | 529 | 0.49 | 1-1 |
| 500 | 0.25 | 1-1 | 530 | 0.42 | 1-1 |

(continued)

| Compound No. | Human MMP2, $IC_{50}$ (nmol/L) | Test Example No. | Compound No. | Human MMP2, $IC_{50}$ (nmol/L) | Test Example No. |
|---|---|---|---|---|---|
| 501 | 0.32 | 1-1 | 531 | 0.35 | 1-1 |
| 502 | 0.30 | 1-1 | 532 | 0.31 | 1-1 |
| 503 | 0.45 | 1-1 | 533 | 0.34 | 1-1 |
| 504 | 0.34 | 1-1 | 534 | 0.29 | 1-1 |
| 505 | 0.38 | 1-1 | 535 | 0.31 | 1-1 |

[Table 51-10]

| Compound No. | Human MMP2, $IC_{50}$ (nmol/L) | Test Example No. | Compound No. | Human MMP2, $IC_{50}$ (nmol/L) | Test Example No. |
|---|---|---|---|---|---|
| 536 | 0.35 | 1-1 | 566 | 0.59 | 1-1 |
| 537 | 0.28 | 1-1 | 567 | 0.54 | 1-1 |
| 538 | 0.22 | 1-1 | 568 | 0.92 | 1-1 |
| 539 | 0.23 | 1-1 | 569 | 0.93 | 1-1 |
| 540 | 0.29 | 1-1 | 570 | 1.0 | 1-1 |
| 541 | 0.35 | 1-1 | 571 | 1.7 | 1-1 |
| 542 | 0.45 | 1-1 | 572 | 1.8 | 1-1 |
| 543 | 1.3 | 1-1 | 573 | 4.3 | 1-1 |
| 544 | 1.3 | 1-1 | 574 | 4.8 | 1-1 |
| 545 | 1.0 | 1-1 | 575 | 2.0 | 1-1 |
| 546 | 0.98 | 1-1 | 576 | 2.8 | 1-1 |
| 547 | 0.59 | 1-1 | 577 | 0.34 | 1-1 |
| 548 | 0.67 | 1-1 | 578 | 0.36 | 1-1 |
| 549 | 0.52 | 1-1 | 579 | 0.249 | 1-2 |
| 550 | 0.52 | 1-1 | 580 | 0.436 | 1-2 |
| 551 | 0.92 | 1-1 | 581 | 0.412 | 1-2 |
| 552 | 0.62 | 1-1 | 582 | 0.254 | 1-2 |
| 553 | 1.1 | 1-1 | 583 | 0.217 | 1-2 |
| 554 | 0.89 | 1-1 | 584 | 1.82 | 1-2 |
| 555 | 0.87 | 1-1 | 585 | 1.20 | 1-2 |
| 556 | 0.55 | 1-1 | 586 | 1.03 | 1-2 |
| 557 | 0.44 | 1-1 | 587 | 0.19 | 1-1 |
| 558 | 0.66 | 1-1 | 588 | 0.12 | 1-1 |
| 559 | 0.42 | 1-1 | 589 | 0.12 | 1-1 |
| 560 | 0.42 | 1-1 | 590 | 3.2 | 1-1 |
| 561 | 0.74 | 1-1 | 591 | 0.98 | 1-1 |
| 562 | 0.58 | 1-1 | 592 | 0.38 | 1-1 |
| 563 | 0.49 | 1-1 | 593 | 2.6 | 1-1 |

(continued)

| Compound No. | Human MMP2, $IC_{50}$ (nmol/L) | Test Example No. | Compound No. | Human MMP2, $IC_{50}$ (nmol/L) | Test Example No. |
|---|---|---|---|---|---|
| 564 | 0.44 | 1-1 | 594 | 1.8 | 1-1 |
| 565 | 0.42 | 1-1 | 595 | 3.83 | 1-2 |

[Table 51-11]

| Compound No. | Human MMP2, $IC_{50}$ (nmol/L) | Test Example No. | Compound No. | Human MMP2, $IC_{50}$ (nmol/L) | Test Example No. |
|---|---|---|---|---|---|
| 596 | 1.3 | 1-1 | 626 | 0.18 | 1-1 |
| 597 | 0.27 | 1-1 | 627 | <0.05 | 1-2 |
| 598 | 4.1 | 1-1 | 628 | 0.0914 | 1-2 |
| 599 | 0.39 | 1-1 | 629 | 0.0655 | 1-2 |
| 600 | 0.98 | 1-1 | 630 | 0.11 | 1-1 |
| 601 | 1.03 | 1-1 | 631 | <0.05 | 1-2 |
| 602 | 0.59 | 1-1 | 632 | 0.0682 | 1-2 |
| 603 | 1.6 | 1-1 | 633 | 0.135 | 1-2 |
| 604 | 0.94 | 1-1 | 634 | 0.13 | 1-1 |
| 605 | 0.80 | 1-1 | 635 | 0.83 | 1-1 |
| 606 | 0.355 | 1-2 | 636 | 0.94 | 1-1 |
| 607 | 2.68 | 1-2 | 637 | 0.48 | 1-1 |
| 608 | 0.34 | 1-1 | 638 | 0.14 | 1-1 |
| 609 | 0.24 | 1-1 | 639 | 1.0 | 1-1 |
| 610 | 0.70 | 1-1 | 640 | 0.38 | 1-1 |
| 611 | 0.15 | 1-1 | 641 | 0.57 | 1-1 |
| 612 | 0.31 | 1-1 | 642 | 1.6 | 1-1 |
| 613 | 0.12 | 1-1 | 643 | 0.320 | 1-2 |
| 614 | 0.35 | 1-1 | 644 | 0.26 | 1-1 |
| 615 | 0.18 | 1-1 | 645 | 0.41 | 1-1 |
| 616 | 0.36 | 1-1 | 646 | 0.29 | 1-1 |
| 617 | 0.087 | 1-1 | 647 | 0.35 | 1-1 |
| 618 | 0.17 | 1-1 | 648 | 5.9 | 1-1 |
| 619 | 0.30 | 1-1 | 649 | 0.70 | 1-1 |
| 620 | 0.132 | 1-2 | 650 | 1.6 | 1-1 |
| 621 | 0.0867 | 1-2 | 651 | 2.0 | 1-1 |
| 622 | <0.05 | 1-2 | 652 | 1.1 | 1-1 |
| 623 | <0.05 | 1-2 | 653 | 0.74 | 1-1 |
| 624 | 0.159 | 1-2 | 654 | 0.21 | 1-1 |
| 625 | 0.103 | 1-2 | 655 | 0.27 | 1-1 |

[Table 51-12]

| Compound No. | Human MMP2, $IC_{50}$ (nmol/L) | Test Example No. | Compound No. | Human MMP2, $IC_{50}$ (nmol/L) | Test Example No. |
|---|---|---|---|---|---|
| 656 | 0.72 | 1-1 | 686 | 0.0488 | 1-2 |
| 657 | 0.56 | 1-1 | 687 | 0.0577 | 1-2 |
| 658 | 0.055 | 1-1 | 688 | 0.0513 | 1-2 |
| 659 | 0.065 | 1-1 | 689 | 0.0319 | 1-2 |
| 660 | 0.12 | 1-1 | 690 | 0.0974 | 1-2 |
| 661 | 0.11 | 1-1 | 691 | 0.065 | 1-1 |
| 662 | 0.093 | 1-1 | 692 | 0.068 | 1-1 |
| 663 | 0.13 | 1-1 | 693 | 0.079 | 1-1 |
| 664 | 0.19 | 1-1 | 694 | 0.094 | 1-1 |
| 665 | 0.17 | 1-1 | 695 | 0.53 | 1-1 |
| 666 | 0.035 | 1-1 | 696 | 0.22 | 1-1 |
| 667 | 0.079 | 1-1 | 697 | 0.28 | 1-1 |
| 668 | 0.25 | 1-1 | 698 | 0.31 | 1-1 |
| 669 | 0.37 | 1-1 | 699 | 0.27 | 1-1 |
| 670 | 5.3 | 1-1 | 700 | 0.41 | 1-1 |
| 671 | 0.069 | 1-1 | 701 | 0.27 | 1-1 |
| 672 | 0.23 | 1-1 | 702 | 0.25 | 1-1 |
| 673 | 0.061 | 1-1 | 703 | 0.33 | 1-1 |
| 674 | 0.22 | 1-1 | 704 | 0.42 | 1-1 |
| 675 | 3.7 | 1-1 | 705 | 0.066 | 1-1 |
| 676 | 0.49 | 1-1 | 706 | 0.080 | 1-1 |
| 677 | 6.3 | 1-1 | 707 | 0.14 | 1-1 |
| 678 | 0.20 | 1-1 | 708 | 0.16 | 1-1 |
| 679 | 1.5 | 1-1 | 709 | 0.41 | 1-1 |
| 680 | 0.33 | 1-1 | 710 | 0.29 | 1-1 |
| 681 | 4.1 | 1-1 | 711 | 1.0 | 1-1 |
| 682 | 1.68 | 1-2 | 712 | 0.27 | 1-1 |
| 683 | 1.86 | 1-2 | 713 | 0.11 | 1-1 |
| 684 | 0.126 | 1-2 | 714 | 0.065 | 1-1 |
| 685 | 0.134 | 1-2 | 715 | 0.069 | 1-1 |

[Table 51-13]

| Compound No. | Human MMP2, $IC_{50}$ (nmol/L) | Test Example No. | Compound No. | Human MMP2, $IC_{50}$ (nmol/L) | Test Example No. |
|---|---|---|---|---|---|
| 716 | 0.48 | 1-1 | 746 | 1.0 | 1-1 |
| 717 | 0.80 | 1-1 | 747 | 0.88 | 1-1 |
| 718 | 0.43 | 1-1 | 748 | 0.96 | 1-1 |

(continued)

| Compound No. | Human MMP2, $IC_{50}$ (nmol/L) | Test Example No. | Compound No. | Human MMP2, $IC_{50}$ (nmol/L) | Test Example No. |
|---|---|---|---|---|---|
| 719 | 0.55 | 1-1 | 749 | 1.1 | 1-1 |
| 720 | 0.72 | 1-1 | 750 | 0.78 | 1-1 |
| 721 | 0.43 | 1-1 | 751 | 1.4 | 1-1 |
| 722 | 0.66 | 1-1 | 752 | 1.6 | 1-1 |
| 723 | 0.41 | 1-1 | 753 | 1.7 | 1-1 |
| 724 | 0.45 | 1-1 | 754 | 2.3 | 1-1 |
| 725 | 0.51 | 1-1 | 755 | 1.9 | 1-1 |
| 726 | 2.4 | 1-1 | 756 | 1.3 | 1-1 |
| 727 | 0.39 | 1-1 | 757 | 1.4 | 1-1 |
| 728 | 1.9 | 1-1 | 758 | 0.86 | 1-1 |
| 729 | 1.4 | 1-1 | 759 | 2.1 | 1-1 |
| 730 | 2.9 | 1-1 | 760 | 1.4 | 1-1 |
| 731 | 0.20 | 1-1 | 761 | 1.5 | 1-1 |
| 732 | 0.16 | 1-1 | 762 | 1.1 | 1-1 |
| 733 | 0.11 | 1-1 | 763 | 0.78 | 1-1 |
| 734 | 0.10 | 1-1 | 764 | 1.7 | 1-1 |
| 735 | 0.65 | 1-1 | 765 | 1.7 | 1-1 |
| 736 | 1.2 | 1-1 | 766 | 0.27 | 1-1 |
| 737 | 4.5 | 1-1 | 767 | 0.29 | 1-1 |
| 738 | 1.2 | 1-1 | 768 | 0.35 | 1-1 |
| 739 | 1.0 | 1-1 | 769 | 0.34 | 1-1 |
| 740 | 1.4 | 1-1 | 770 | 0.31 | 1-1 |
| 741 | 0.61 | 1-1 | 771 | 0.44 | 1-1 |
| 742 | 0.57 | 1-1 | 772 | 0.81 | 1-1 |
| 743 | 0.78 | 1-1 | 773 | 5.4 | 1-1 |
| 744 | 1.0 | 1-1 | 774 | 5.1 | 1-1 |
| 745 | 1.7 | 1-1 | 775 | 4.4 | 1-1 |

[Table 51-14]

| Compound No. | Human MMP2, $IC_{50}$ (nmol/L) | Test Example No. | Compound No. | Human MMP2, $IC_{50}$ (nmol/L) | Test Example No. |
|---|---|---|---|---|---|
| 776 | 5.2 | 1-1 | 806 | 0.28 | 1-1 |
| 777 | 1.0 | 1-1 | 807 | 1.0 | 1-1 |
| 778 | 1.1 | 1-1 | 808 | 0.55 | 1-1 |
| 779 | 1.3 | 1-1 | 809 | 1.9 | 1-1 |
| 780 | 0.97 | 1-1 | 810 | 0.99 | 1-1 |
| 781 | 1.8 | 1-1 | 811 | 0.82 | 1-1 |

(continued)

| Compound No. | Human MMP2, $IC_{50}$ (nmol/L) | Test Example No. | Compound No. | Human MMP2, $IC_{50}$ (nmol/L) | Test Example No. |
|---|---|---|---|---|---|
| 782 | 0.41 | 1-1 | 812 | 1.3 | 1-1 |
| 783 | 2.1 | 1-1 | 813 | 1.6 | 1-1 |
| 784 | 2.1 | 1-1 | 814 | 1.5 | 1-1 |
| 785 | 0.51 | 1-1 | 815 | 0.98 | 1-1 |
| 786 | 0.0830 | 1-2 | 816 | 1.3 | 1-1 |
| 787 | 0.0670 | 1-2 | 817 | 0.93 | 1-1 |
| 788 | 0.0645 | 1-2 | 818 | 1.1 | 1-1 |
| 789 | 0.0553 | 1-2 | 819 | 0.42 | 1-1 |
| 790 | 0.0605 | 1-2 | 820 | 0.70 | 1-1 |
| 791 | 0.584 | 1-2 | 821 | 1.2 | 1-1 |
| 792 | 0.11 | 1-1 | 822 | 1.1 | 1-1 |
| 793 | 0.23 | 1-1 | 823 | 0.95 | 1-1 |
| 794 | 0.15 | 1-1 | 824 | 1.0 | 1-1 |
| 795 | 0.19 | 1-1 | 825 | 1.1 | 1-1 |
| 796 | 0.22 | 1-1 | 826 | 0.32 | 1-1 |
| 797 | 0.30 | 1-1 | 827 | 0.58 | 1-1 |
| 798 | 0.59 | 1-1 | 828 | 1.4 | 1-1 |
| 799 | 0.72 | 1-1 | 829 | 1.5 | 1-1 |
| 800 | 1.6 | 1-1 | 830 | 1.7 | 1-1 |
| 801 | 1.3 | 1-1 | 831 | 1.1 | 1-1 |
| 802 | 1.4 | 1-1 | 832 | 1.1 | 1-1 |
| 803 | 0.46 | 1-1 | 833 | 1.4 | 1-1 |
| 804 | 0.32 | 1-1 | 834 | 1.2 | 1-1 |
| 805 | 0.46 | 1-1 | 835 | 1.2 | 1-1 |

[Table 51-15]

| Compound No. | Human MMP2, $IC_{50}$ (nmol/L) | Test Example No. | Compound No. | Human MMP2, $IC_{50}$ (nmol/L) | Test Example No. |
|---|---|---|---|---|---|
| 836 | 1.2 | 1-1 | 866 | 0.137 | 1-2 |
| 837 | 1.0 | 1-1 | 867 | 0.213 | 1-2 |
| 838 | 0.61 | 1-1 | 868 | 0.143 | 1-2 |
| 839 | 0.62 | 1-1 | 869 | 0.106 | 1-2 |
| 840 | 0.80 | 1-1 | 870 | 0.124 | 1-2 |
| 841 | 0.74 | 1-1 | 871 | 0.140 | 1-2 |
| 842 | 0.82 | 1-1 | 872 | 0.123 | 1-2 |
| 843 | 1.1 | 1-1 | 873 | 0.135 | 1-2 |
| 844 | 1.4 | 1-1 | 874 | 0.133 | 1-2 |

(continued)

| Compound No. | Human MMP2, $IC_{50}$ (nmol/L) | Test Example No. | Compound No. | Human MMP2, $IC_{50}$ (nmol/L) | Test Example No. |
|---|---|---|---|---|---|
| 845 | 1.3 | 1-1 | 875 | 0.191 | 1-2 |
| 846 | 2.3 | 1-1 | 876 | 0.158 | 1-2 |
| 847 | 2.1 | 1-1 | 877 | 0.136 | 1-2 |
| 848 | 1.1 | 1-1 | 878 | 0.124 | 1-2 |
| 849 | 1.3 | 1-1 | 879 | 0.133 | 1-2 |
| 850 | 0.92 | 1-1 | 880 | 0.146 | 1-2 |
| 851 | 0.66 | 1-1 | 881 | 0.106 | 1-2 |
| 852 | 0.64 | 1-1 | 882 | 0.0996 | 1-2 |
| 853 | 0.57 | 1-1 | 883 | 0.143 | 1-2 |
| 854 | 0.89 | 1-1 | 884 | 0.153 | 1-2 |
| 855 | 0.67 | 1-1 | 885 | 0.128 | 1-2 |
| 856 | 0.19 | 1-1 | 886 | 1.09 | 1-2 |
| 857 | 0.307 | 1-2 | 887 | 1.34 | 1-2 |
| 858 | 0.17 | 1-1 | 888 | 0.153 | 1-2 |
| 859 | 0.25 | 1-1 | 889 | 0.170 | 1-2 |
| 860 | 0.108 | 1-2 | 890 | 0.158 | 1-2 |
| 861 | 0.124 | 1-2 | 891 | 1.6 | 1-1 |
| 862 | 0.114 | 1-2 | 892 | 1.7 | 1-1 |
| 863 | 0.118 | 1-2 | 893 | 1.15 | 1-1 |
| 864 | 0.107 | 1-2 | 894 | 1.1 | 1-1 |
| 865 | 0.150 | 1-2 | 895 | 1.3 | 1-1 |

[Table 51-16]

| Compound No. | Human MMP2, $IC_{50}$ (nmol/L) | Test Example No. | Compound No. | Human MMP2, $IC_{50}$ (nmol/L) | Test Example No. |
|---|---|---|---|---|---|
| 896 | 1.2 | 1-1 | 926 | 0.090 | 1-1 |
| 897 | 0.87 | 1-1 | 927 | 0.090 | 1-1 |
| 898 | 6.4 | 1-1 | 928 | 0.081 | 1-1 |
| 899 | 4.2 | 1-1 | 929 | 0.12 | 1-1 |
| 900 | 0.055 | 1-1 | 930 | 0.074 | 1-1 |
| 901 | 0.054 | 1-1 | 931 | 0.081 | 1-1 |
| 902 | 0.049 | 1-1 | 932 | 0.077 | 1-1 |
| 903 | 0.127 | 1-1 | 933 | 0.084 | 1-1 |
| 904 | 0.20 | 1-1 | 934 | 0.086 | 1-1 |
| 905 | 0.092 | 1-1 | 935 | 0.079 | 1-1 |
| 906 | 0.19 | 1-1 | 936 | 0.11 | 1-1 |
| 907 | 0.080 | 1-1 | 937 | 0.13 | 1-1 |

(continued)

| Compound No. | Human MMP2, $IC_{50}$ (nmol/L) | Test Example No. | Compound No. | Human MMP2, $IC_{50}$ (nmol/L) | Test Example No. |
|---|---|---|---|---|---|
| 908 | 0.049 | 1-1 | 938 | 0.21 | 1-1 |
| 909 | 0.13 | 1-1 | 939 | 0.20 | 1-1 |
| 910 | 2.5 | 1-1 | 940 | 0.26 | 1-1 |
| 911 | 3.1 | 1-1 | 941 | 0.22 | 1-1 |
| 912 | 0.111 | 1-1 | 942 | 0.080 | 1-1 |
| 913 | 0.38 | 1-1 | 943 | 0.081 | 1-1 |
| 914 | 0.23 | 1-1 | 944 | 0.15 | 1-1 |
| 915 | 1.19 | 1-1 | 945 | 0.11 | 1-1 |
| 916 | 0.19 | 1-1 | 946 | 0.093 | 1-1 |
| 917 | 0.61 | 1-1 | 947 | 0.082 | 1-1 |
| 918 | 0.31 | 1-1 | 948 | 0.12 | 1-1 |
| 919 | 0.18 | 1-1 | 949 | 0.12 | 1-1 |
| 920 | 0.18 | 1-1 | 950 | 0.19 | 1-1 |
| 921 | 0.085 | 1-1 | 951 | 0.088 | 1-1 |
| 922 | 0.27 | 1-1 | 952 | 0.29 | 1-1 |
| 923 | 0.13 | 1-1 | 953 | 0.21 | 1-1 |
| 924 | 0.096 | 1-1 | 954 | 0.11 | 1-1 |
| 925 | 0.16 | 1-1 | 955 | 0.17 | 1-1 |

[Table 51-17]

| Compound No. | Human MMP2, $IC_{50}$ (nmol/L) | Test Example No. | Compound No. | Human MMP2, $IC_{50}$ (nmol/L) | Test Example No. |
|---|---|---|---|---|---|
| 956 | 0.18 | 1-1 | 965 | 0.50 | 1-1 |
| 957 | 0.12 | 1-1 | 966 | 0.37 | 1-1 |
| 958 | 0.35 | 1-1 | 967 | 0.18 | 1-1 |
| 959 | 0.23 | 1-1 | 968 | 0.23 | 1-1 |
| 960 | 0.28 | 1-1 | 969 | 0.14 | 1-1 |
| 961 | 0.28 | 1-1 | 970 | 0.25 | 1-1 |
| 962 | 0.38 | 1-1 | 971 | 0.16 | 1-1 |
| 963 | 0.26 | 1-1 | 972 | 0.36 | 1-1 |
| 964 | 0.27 | 1-1 | | | |

The inhibitory effects of the present inventive compounds on different types of MMP can be determined with the methods described in Test Examples 2 to 9.

Test Example 2: Evaluation Test for Inhibitory Effects of Compounds of Present Invention on Human MMP 1

The human-MMP1-inhibitory effects of the compounds are determined through enzyme assay using MOCAc-Lys-Pro-Leu-Gly-Leu-A2pr(Dnp)-Ala-Arg-$NH_2$ as a substrate. In a reaction solution [50 mmol/L Tris-HCl (pH 7.5), 150 mmol/L NaCl, 10 mmol/L $CaCl_2$, 0.05% Brij L23], 50 μg/mL human recombinant MMP1 enzyme and 1 mmol/L 4-Aminophenylmercuric acetate are mixed together, and reacted at 37°C for 120 minutes. The human MMP1 activated through the reaction is poured into a 96-well microplate to reach a final concentration of 8 ng/mL. Further, the compounds of the present invention, which have been diluted to different concentrations, are added, and left to stand at room temperature for 15 minutes. Then, MOCAc-Lys-Pro-Leu-Gly-Leu-A2pr(Dnp)-Ala-Arg-$NH_2$ is added to reach a final concentration of 11 μmol/L, and enzymatic reaction is initiated. After reacting at room temperature for 1 hour, fluorescence intensity (Ex 320 nm/Em 400 nm) is determined by using a microplate reader. Enzyme inhibition rates (%) are calculated with the measured fluorescence values in accordance with the following formulation, and the 50% inhibitory concentrations ($IC_{50}$ values) of the inventive compounds are calculated.

$$\text{Enzyme inhibition rate (\%)} = [1-(A-B)/(C-B)] * 100$$

A: Fluorescence value with addition of compound
B: Fluorescence value without addition of compound and enzyme
C: Fluorescence value without addition of compound

Test Example 3: Evaluation Test for Inhibitory Effects of Compounds of Present Invention on Human MMP3

The human-MMP3-inhibitory effects of the compounds are determined through enzyme assay using MOCAc-Arg-Pro-Lys-Pro-Val-Glu-Nva-Trp-Arg-Lys(Dnp)-$NH_2$ as a substrate. In a reaction solution [50 mmol/L Tris-HCl (pH 7.5), 150 mmol/L NaCl, 10 mmol/L $CaCl_2$, 0.05% Brij L23], 3 μg/mL human recombinant MMP3 enzyme and 5 μg/mL Chymotrypsin are mixed together. After reacting at 37°C for 30 minutes, PMSF is added to reach 2 mmol/L, thereby terminating the reaction. The human MMP3 activated through the reaction is poured into a 96-well microplate to reach a final concentration of 52 ng/mL. Further, the compounds of the present invention, which have been diluted to different concentrations, are added, and left to stand at room temperature for 15 minutes. Then, MOCAc-Arg-Pro-Lys-Pro-Val-Glu-Nva-Trp-Arg-Lys(Dnp)-$NH_2$ is added to reach a final concentration of 26 μmol/L, and enzymatic reaction is initiated. After reacting at room temperature for 1 hour, fluorescence intensity (Ex 320 nm/Em 400 nm) is determined by using a microplate reader. Enzyme inhibition rates (%) are calculated with the measured fluorescence values in accordance with the following formulation, and the 50% inhibitory concentrations ($IC_{50}$ values) of the inventive compounds are calculated.

$$\text{Enzyme inhibition rate (\%)} = [1-(A-B)/(C-B)] * 100$$

A: Fluorescence value with addition of compound
B: Fluorescence value without addition of compound and enzyme
C: Fluorescence value without addition of compound

Test Example 4: Evaluation Test for Inhibitory Effects of Compounds of Present Invention on Human MMP7

The human-MMP7-inhibitory effects of the compounds are determined through enzyme assay using MOCAc-Pro-Leu-Gly-Leu-A2pr(Dnp)-Ala-Arg-$NH_2$ as a substrate. In a reaction solution [50 mmol/L Tris-HCl (pH 7.5), 150 mmol/L NaCl, 10 mmol/L $CaCl_2$, 0.05% Brij L23], 50 μg/mL human recombinant MMP7 enzyme and 1 mmol/L 4-Aminophenylmercuric acetate are mixed together, and reacted at 37°C for 60 minutes. The human MMP7 activated through the reaction is poured into a 96-well microplate to reach a final concentration of 18 ng/mL. Further, the compounds of the present invention, which have been diluted to different concentrations, are added, and left to stand at room temperature for 15 minutes. Then, MOCAc-Pro-Leu-Gly-Leu-A2pr(Dnp)-Ala-Arg-$NH_2$ is added to reach a final concentration of 22 μmol/L, and enzymatic reaction is initiated. After reacting at room temperature for 1 hour, fluorescence intensity (Ex 320 nm/Em 400 nm) is determined by using a microplate reader. Enzyme inhibition rates (%) are calculated with the measured fluorescence values in accordance with the following formulation, and the 50% inhibitory concentrations ($IC_{50}$ values) of the inventive compounds are calculated.

$$\text{Enzyme inhibition rate (\%)} = [1-(A-B)/(C-B)] * 100$$

A: Fluorescence value with addition of compound
B: Fluorescence value without addition of compound and enzyme
C: Fluorescence value without addition of compound

Test Example 5: Evaluation Test for Inhibitory Effects of Compounds of Present Invention on Human MMP8

The human-MMP8-inhibitory effects of the compounds are determined through enzyme assay using MOCAc-Pro-Leu-Gly-Leu-A2pr(Dnp)-Ala-Arg-$NH_2$ as a substrate. In a reaction solution [50 mmol/L Tris-HCl (pH 7.5), 150 mmol/L NaCl, 10 mmol/L $CaCl_2$, 0.05% Brij L23], 100 μg/mL human recombinant MMP8 enzyme and 1 mmol/L 4-Aminophenylmercuric acetate are mixed together, and reacted at 37°C for 60 minutes. The human MMP8 activated through the reaction is poured into a 96-well microplate to reach a final concentration of 149 ng/mL. Further, the compounds of the present invention, which have been diluted to different concentrations, are added, and left to stand at room temperature for 15 minutes. Then, MOCAc-Pro-Leu-Gly-Leu-A2pr(Dnp)-Ala-Arg-$NH_2$ is added to reach a final concentration of 25 μmol/L, and enzymatic reaction is initiated. After reacting at room temperature for 1 hour, fluorescence intensity (Ex 320 nm/Em 400 nm) is determined by using a microplate reader. Enzyme inhibition rates (%) are calculated with the measured fluorescence values in accordance with the following formulation, and the 50% inhibitory concentrations ($IC_{50}$ values) of the inventive compounds are calculated.

$$\text{Enzyme inhibition rate (\%)} = [1-(A-B)/(C-B)] * 100$$

A: Fluorescence value with addition of compound
B: Fluorescence value without addition of compound and enzyme
C: Fluorescence value without addition of compound

Test Example 6: Evaluation Test for Inhibitory Effects of Compounds of Present Invention on Human MMP9

The human-MMP9-inhibitory effects of the compounds are determined through enzyme assay using MOCAc-Pro-Leu-Gly-Leu-A2pr(Dnp)-Ala-Arg-$NH_2$ as a substrate. In a reaction solution [50 mmol/L Tris-HCl (pH 7.5), 150 mmol/L NaCl, 10 mmol/L $CaCl_2$, 0.05% Brij L23], 50 μg/mL human recombinant MMP9 enzyme and 1 mmol/L 4-Aminophenylmercuric acetate are mixed together, and reacted at 37°C for 24 hours. The human MMP9 activated through the reaction is poured into a 96-well microplate to reach a final concentration of 58 ng/mL. Further, the compounds of the present invention, which have been diluted to different concentrations, are added, and left to stand at room temperature for 15 minutes. Then, MOCAc-Pro-Leu-Gly-Leu-A2pr(Dnp)-Ala-Arg-$NH_2$ is added to reach a final concentration of 3 μmol/L, and enzymatic reaction is initiated. After reacting at room temperature for 1 hour, fluorescence intensity (Ex 320 nm/Em 400 nm) is determined by using a microplate reader. Enzyme inhibition rates (%) are calculated with the measured fluorescence values in accordance with the following formulation, and the 50% inhibitory concentrations ($IC_{50}$ values) of the inventive compounds are calculated.

$$\text{Enzyme inhibition rate (\%)} = [1-(A-B)/(C-B)] * 100$$

A: Fluorescence value with addition of compound
B: Fluorescence value without addition of compound and enzyme
C: Fluorescence value without addition of compound

Test Example 7: Evaluation Test for Inhibitory Effects of Compounds of Present Invention on Human MMP12

The human-MMP12-inhibitory effects of the compounds were determined through enzyme assay using MOCAc-Pro-Leu-Gly-Leu-A2pr(Dnp)-Ala-Arg-$NH_2$ as a substrate. In a reaction solution [50 mmol/L Tris-HCl (pH 7.5), 150 mmol/L NaCl, 10 mmol/L $CaCl_2$, 0.05% Brij L23], 50 μg/mL human recombinant MMP12 enzyme and 1 mmol/L 4-Aminophenylmercuric acetate were mixed together, and reacted at 37°C for 4 or 24 hours. The human MMP12 activated through the reaction was poured into a 96-well microplate to reach a final concentration of 17 or 5.2 ng/mL. Further, the compounds of the present invention, which had been diluted to different concentrations, were added, and left to stand at room temperature for 15 minutes. Then, MOCAc-Pro-Leu-Gly-Leu-A2pr(Dnp)-Ala-Arg-$NH_2$ was added to reach a final concentration of 5 or 14 μmol/L, and enzymatic reaction was initiated. After reacting at room temperature for 1 hour, fluorescence intensity (Ex 320 nm/Em 400 nm) was determined by using a microplate reader. Enzyme inhibition rates (%) were calculated with the measured fluorescence values in accordance with the following formulation, and the 50% inhibitory concentrations ($IC_{50}$ values) of the inventive compounds were calculated.

$$\text{Enzyme inhibition rate (\%)} = [1-(A-B)/(C-B)] * 100$$

A: Fluorescence value with addition of compound
B: Fluorescence value without addition of compound and enzyme
C: Fluorescence value without addition of compound

The results on the inhibitory effects of the present inventive compounds on human MMP12 enzyme activity are shown in the following table.

[Table 52]

| Compound No. | Human MMP 12, $IC_{50}$ (nmol/L) |
|---|---|
| 1 | 253 |
| 3 | 502 |
| 9 | 160 |
| 20 | 254 |
| 52 | 190 |
| 149 | 4478 |
| 223 | 1367 |
| 230 | 1270 |
| 320 | 1897 |
| 347 | 2769 |
| 666 | 80 |
| 691 | 101 |
| 701 | 169 |
| 766 | 756 |
| 796 | 411 |

Test Example 8: Evaluation Test for Inhibitory Effects of Compounds of Present Invention on Human MMP13

The human-MMP 13-inhibitory effects of the compounds were determined through enzyme assay using MOCAc-Pro-Leu-Gly-Leu-A2pr(Dnp)-Ala-Arg-$NH_2$ as a substrate. In a reaction solution [50 mmol/L Tris-HCl (pH 7.5), 150 mmol/L NaCl, 10 mmol/L $CaCl_2$, 0.05% Brij L23], 50 $\mu$g/mL human recombinant MMP13 enzyme and 1 mmol/L 4-Aminophenylmercuric acetate were mixed together, and reacted at 37°C for 2 hours. The human MMP13 activated through the reaction was poured into a 96-well microplate to reach a final concentration of 5 ng/mL. Further, the compounds of the present invention, which had been diluted to different concentrations, were added, and left to stand at room temperature for 15 minutes. Then,

MOCAc-Pro-Leu-Gly-Leu-A2pr(Dnp)-Ala-Arg-$NH_2$ was added to reach a final concentration of 6 $\mu$mol/L, and enzymatic reaction was initiated. After reacting at room temperature for 1 hour, fluorescence intensity (Ex 320 nm/Em 400 nm) was determined by using a microplate reader. Enzyme inhibition rates (%) were calculated with the measured fluorescence values in accordance with the following formulation, and the 50% inhibitory concentrations ($IC_{50}$ values) of the inventive compounds were calculated.

$$\text{Enzyme inhibition rate (\%)} = [1-(A-B)/(C-B)] * 100$$

A: Fluorescence value with addition of compound
B: Fluorescence value without addition of compound and enzyme
C: Fluorescence value without addition of compound

The results on the inhibitory effects of the present inventive compounds on human MMP13 enzyme activity are shown in the following table.

[Table 53]

| Compound No. | Human MMP 13, $IC_{50}$ (nmol/L) |
|---|---|
| 1 | 240 |
| 3 | 732 |
| 9 | 250 |
| 20 | 224 |
| 52 | 679 |
| 149 | 1293 |
| 223 | 174 |
| 230 | 114 |
| 320 | 94 |
| 347 | 162 |
| 666 | 81 |
| 691 | 142 |
| 701 | 116 |
| 766 | 699 |
| 796 | 685 |

Test Example 9: Evaluation Test for Inhibitory Effects of Compounds of Present Invention on Human MMP14

The human-MMP14-inhibitory effects of the compounds are determined through enzyme assay using MOCAc-Lys-Pro-Leu-Gly-Leu-A2pr(Dnp)-Ala-Arg-$NH_2$ as a substrate. In a reaction solution [50 mmol/L Tris-HCl (pH 7.5), 200 mmol/L NaCl, 5 mmol/L $CaCl_2$, 20 μmol/L $ZnSO_4$, 0.05% Brij L23], 100 μg/mL human recombinant MMP14 enzyme and 5 μg/mL trypsin are mixed together. After reacting at room temperature for 25 minutes, a trypsin inhibitor is added to reach 25 μg/mL, thereby terminating the reaction. The human MMP14 activated through the reaction is poured into a 96-well microplate to reach a final concentration of 6 ng/mL. Further, the compounds of the present invention, which have been diluted to different concentrations, are added, and left to stand at room temperature for 15 minutes. Then, MOCAc-Lys-Pro-Leu-Gly-Leu-A2pr(Dnp)-Ala-Arg-$NH_2$ is added to reach a final concentration of 5 μmol/L, and enzymatic reaction is initiated. After reacting at room temperature for 1 hour, fluorescence intensity (Ex 320 nm/Em 400 nm) is determined by using a microplate reader. Enzyme inhibition rates (%) are calculated with the measured fluorescence values in accordance with the following formulation, and the 50% inhibitory concentrations ($IC_{50}$ values) of the inventive compounds are calculated.

$$\text{Enzyme inhibition rate (\%)} = [1-(A-B)/(C-B)] * 100$$

A: Fluorescence value with addition of compound
B: Fluorescence value without addition of compound and enzyme
C: Fluorescence value without addition of compound

INDUSTRIAL APPLICABILITY

The compounds of the present invention have a superior effect to inhibit MMP2, and the present invention can provide a pharmaceutical product effective for preventing or treating cancerous disease and organ fibrosis, and symptoms relating to cancerous disease and organ fibrosis, and is expected to reduce burdens on patients, thereby contributing to the development of the pharmaceutical industry.

## Claims

1. A substituted polypeptide represented by formula [I']:

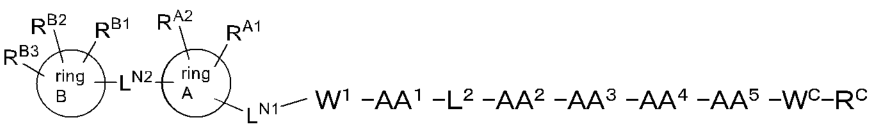

[I'],

or a pharmaceutically acceptable salt thereof,

wherein
$AA^1$ represents:

Asp,
β-Asp, β-(d)-Asp, γ-Glu, or γ-(d)-Glu;

$AA^2$ represents one group selected from the group consisting of:

Ala,
a group represented by any of formulas [IV-7], [IV-8], [IV-9], [IV-11], [IV-12], and [IV-13]:

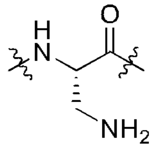 [IV-7], 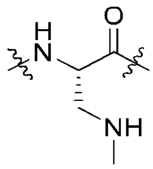 [IV-8],

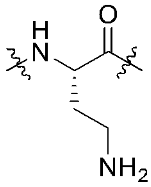 [IV-9], 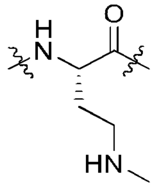 [IV-11], 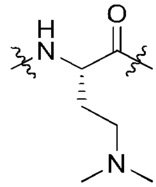 [IV-12],

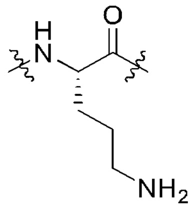 [IV-13]

a group represented by formula [IV-27]:

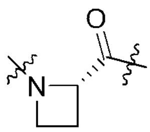 [IV-27]

Pro, and a group represented by any of formulas [II-1] and [II-2]:

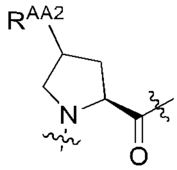 [II-1], 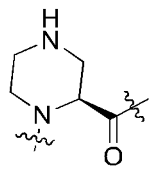 [II-2]

wherein $R^{AA2}$ represents hydroxy or amino;

$AA^1$ and $AA^2$ may be taken together to form a structure represented by formula [IV-32]:

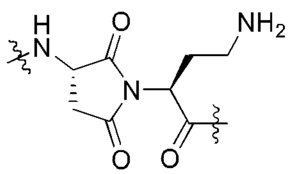 [IV-32];

$AA^3$ represents one group selected from the group consisting of:

Val, Leu, Ile, a group represented by formula [IV-2]:

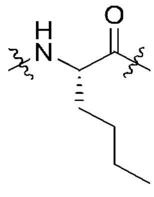 [IV-2]

Phe, Trp,
Tyr, Lys, a group represented by any of formulas [IV-3], [IV-4], and [IV-5]:

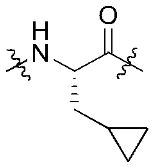 [IV-3], 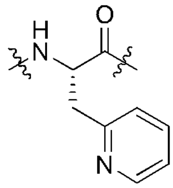 [IV-4], 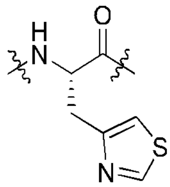 [IV-5]

and
a group represented by formula [IV-9]:

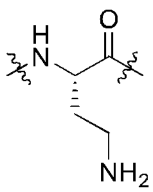 [IV-9];

AA4 represents one group selected from the group consisting of:

a single bond,
Gly, (d)-Ala, (N-Me)Ala, (N-Me)Val, (N-Me)Leu, (N-Me)Ile, Pro, (d)-Pro,
(N-Me)Phe, (d)-Phe,
(N-Me)Tyr, (d)-Tyr,
(N-Me)Ser, (d)-Ser, homoSer, (d)-Thr,
Met, (N-Me)Met,
(N-Me)Asp, Glu, (N-Me)Glu, (d)-(N-Me)Glu, homoGlu, (N-Me)Asn,
(N-Me)Arg, (d)-Arg,
a group represented by any of formulas [IV-7], [IV-9], and [IV-13]:

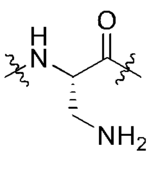 [IV-7], 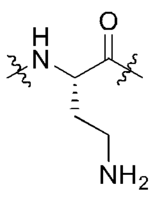 [IV-9], 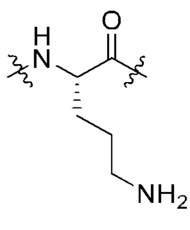 [IV-13]

Lys, and (N-Me)Lys,

wherein if AA4 represents Lys, then
the amino in the side chain of the Lys is optionally substituted with $C_{2-16}$ alkylcarbonyl terminally-substituted with carboxy;

AA5 represents one group selected from the group consisting of:

a single bond,
Ala, a group represented by formula [IV-1]:

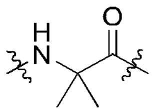

[IV-1]

a group represented by any of formulas [IV-27], [IV-28], and [IV-29]:

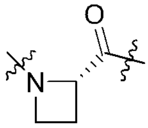

[IV-27],

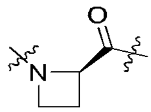

[IV-28],

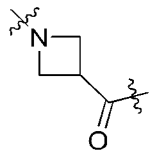

[IV-29]

Pro, (d)-Pro, β-homoPro, homoPro, a group represented by formula [II-1']:

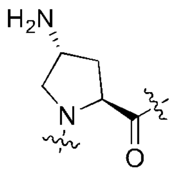

[II-1']

Phe, His,

Thr,
Arg, (d)-Arg,
a group represented by any of formulas [IV-7], [IV-9], and [IV-13]:

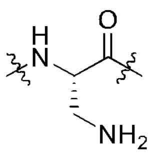

[IV-7],

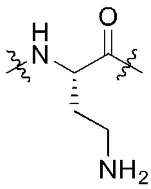

[IV-9],

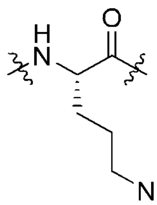

[IV-13]

Lys, (d)-Lys,
β-Ala, (N-Me)-β-Ala, GABA, Ape, Acp,
a group represented by any of formulas [III-6] to [III-13]:

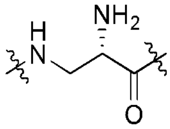

[III-6],

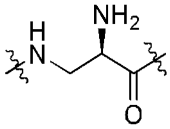

[III-7]

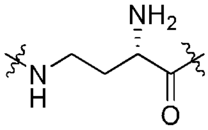

[III-8],

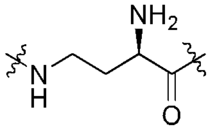

[III-9],

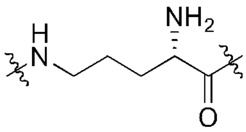 [III-10], 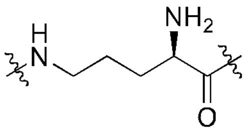 [III-11],

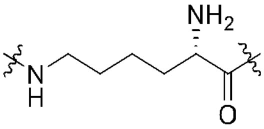 [III-12], 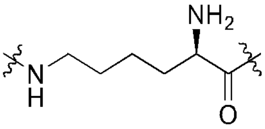 [III-13]

and

a group represented by any of formula [IV-25] and [IV-26]:

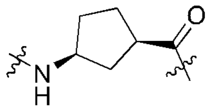 [IV-25], 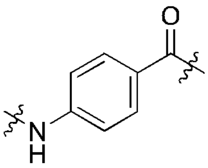 [IV-26];

$W^1$ represents $-L^1-$ or $-L^{1'}-L^{1''}-$; wherein

$L^1$ represents a single bond; and

$L^{1'}$ represents one group selected from the group consisting of:

a single bond,

β-Ala, GABA, (N-Me)GABA, Ape, Acp,

a group represented by any of formulas [III-6] to [III-13]

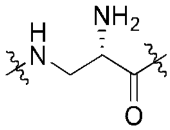 [III-6], 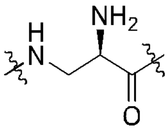 [III-7],

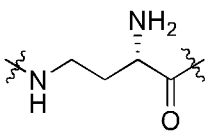 [III-8], 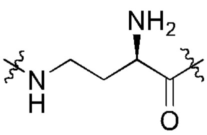 [III-9],

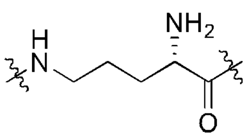 [III-10], 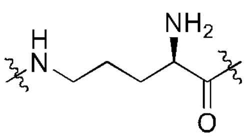 [III-11],

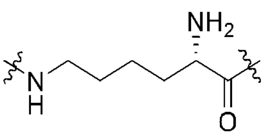 [III-12], 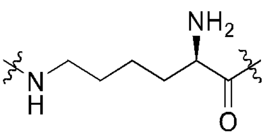 [III-13]

and

a group represented by any of formulas [IV-23] and [IV-24]:

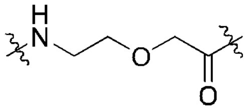 [IV-23], 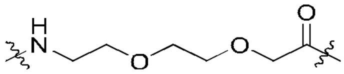 [IV-24];

and

L[1]" represents one group selected from the group consisting of:

a single bond,
Gly, (N-Me)Gly,
Ala, (N-Me)Ala, (d)-Ala, Val, (N-Me)Val, (N-Me)Leu, (N-Me)Ile,
a group represented by formula [IV-27]:

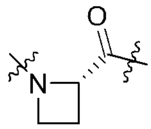 [IV-27]

Pro, (d)-Pro, homoPro, Phe, (N-Me)Phe, (d)-Phe,

His, (d)-His, Trp, (N-Me)Trp, (d)-Trp,
Tyr, (N-Me)Tyr, (d)-Tyr,
(d)-Ser, homoSer, Thr, (N-Me)Thr, (d)-Thr,
Cys, (d)-Cys, Met, (N-Me)Met,
(N-Me)Asp, Glu, (N-Me)Glu, (d)-Glu,
Asn, (N-Me)Asn, (d)-Asn, Gln, (N-Me)Gln, (d)-Gln,
Arg, (N-Me)Arg, (d)-Arg, Cit, (d)-Cit,

a group represented by any of formulas [IV-7], [IV-9], [IV-10], and [IV-13]:

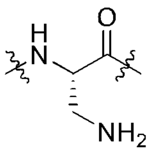 [IV-7], 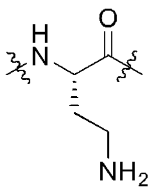 [IV-9], 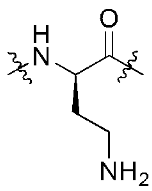 [IV-10],

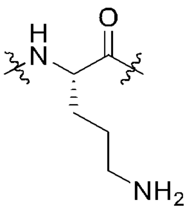 [IV-13]

Lys, (N-Me)Lys, (d)-Lys, a group represented by formula [IV-14]:

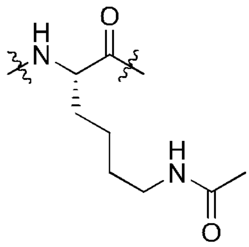

[IV-14]

β-Ala,

β-Asp, β-(d)-Asp, and

a group represented by any of formulas [III-6] and [III-7]:

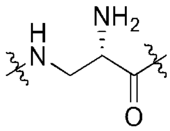

[III-6],

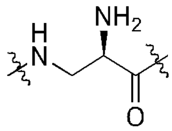

[III-7];

wherein if $L^{1"}$ represents Lys or (d)-Lys, then

the amino in the side chain of the Lys or (d)-Lys is optionally substituted with a group represented by formula [VII-1]:

$$FA^{N}\text{-}AA^{N5}\text{-}AA^{N4}\text{-}AA^{N3}\text{-}AA^{N2}\text{-}AA^{N1}\text{-} \quad [VII-1]$$

wherein

$FA^{N}$ represents $C_{2-16}$ alkylcarbonyl terminally-substituted with carboxy;

$AA^{N5}$ represents:

a single bond,
Arg, (d)-Arg,
Lys, (d)-Lys,
γ-Glu, or
a group represented by formula [IV-24]:

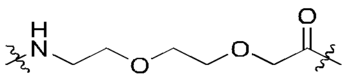

[IV-24];

$AA^{N4}$ represents:

a single bond,
Arg, (d)-Arg,
Lys, (d)-Lys, or
a group represented by formula [IV-24]:

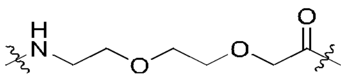

[IV-24];

$AA^{N3}$ represents:

a single bond,
Arg, (d)-Arg,
Lys, (d)-Lys,
γ-Glu, or
a group represented by formula [IV-24]:

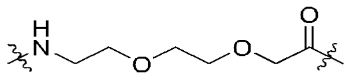

[IV-24];

$AA^{N2}$ represents:

a single bond, or
(d)-Lys; and

$AA^{N1}$ represents:

a single bond, or
(d)-Lys;

wherein if $L^{1"}$ represents by Glu and $AA^3$ represents Lys, then

the compound represented by formula [I'] may be taken together with $L^3$ attached to each of functional groups in the side chains of the two amino acids to form a cyclic structure, as represented by formula [I'-α]:

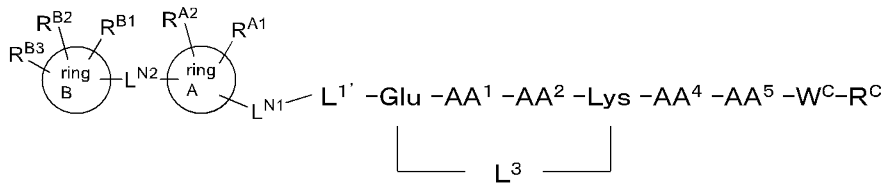

[I'-α]

wherein the $L^3$ represents Gly, β-Ala, or GABA;

$L^{N1}$ represents the formula -C(=O)- or the formula $-S(=O)_2-$;
$L^{N2}$ represents:

a single bond,
$C_{1-3}$ alkanediyl,
$C_{2-3}$ alkenediyl,
ethynediyl,
the formula -O-,
the formula -C(=O)-, the formula -C(=O)-NH-, or
triazolediyl;

$L^2$ represents a single bond;
ring A represents an aromatic ring or a heteroaromatic ring;
$R^{A1}$ and $R^{A2}$ each independently represent:

a hydrogen atom,
a halogen atom,
$C_{1-6}$ alkyl, or
$C_{1-6}$ alkoxy;

ring B represents:
aryl or heteroaryl;
$R^{B1}$, $R^{B2}$, and $R^{B3}$ each independently represent:

a hydrogen atom,
carbamoyl,
cyano,
a halogen atom,

$C_{1-6}$ alkyl optionally substituted with one hydroxy, halo $C_{1-6}$ alkyl,
$C_{1-6}$ alkoxy optionally substituted with one hydroxy, halo $C_{1-6}$ alkoxy,
$C_{1-6}$ alkylcarbonyl,
$C_{1-6}$ alkylcarbonylamino,
mono $C_{1-6}$ alkylaminocarbonyl, di $C_{1-6}$ alkylaminocarbonyl, wherein the alkyl in each of the mono $C_{1-6}$ alkylaminocarbonyl and the di $C_{1-6}$ alkylaminocarbonyl is optionally substituted with one group selected from the group consisting of hydroxy, carboxy, carbamoyl, and amino,
$C_{1-6}$ alkylsulfonyl, or
aryl;

$W^C$ is a single bond or a linker consisting of one to three amino acids, wherein the one to three amino acids forming the linker are same or different and each selected from the group consisting of:

Gly,
Pro,
Arg, (d)-Arg,
Lys, (d)-Lys,
β-Ala, GABA, and Ape,
wherein if Lys or (d)-Lys is included in the group represented by $W^C$, then
the amino in the side chain of the Lys or (d)-Lys is optionally substituted with:

$C_{2-16}$ alkylcarbonyl terminally-substituted with carboxy,
Lys, wherein the amino in the side chain of the Lys is optionally substituted with $C_{2-16}$ alkylcarbonyl terminally-substituted with carboxy, or
(d)-Lys, wherein the amino in the side chain of the (d)-Lys is optionally substituted with $C_{2-16}$ alkylcarbonyl terminally-substituted with carboxy; and

$R^C$ is:

the formula -OH, the formula $-NH_2$,
$C_{1-6}$ alkylamino, wherein the $C_{1-6}$ alkyl of the $C_{1-6}$ alkylamino is optionally substituted with one group selected from the group consisting of hydroxy, amino, $C_{1-6}$ alkoxy, and four- to seven-membered saturated heterocyclyl containing one nitrogen atom and optionally further containing one heteroatom, or
four- to seven-membered saturated heterocyclyl containing one nitrogen atom and optionally further containing one heteroatom; wherein the four- to seven-membered saturated heterocyclyl containing one nitrogen atom and optionally further containing one heteroatom is optionally substituted with one group selected from the group consisting of hydroxy, amino, and $C_{1-6}$ alkyl, wherein the $C_{1-6}$ alkyl is optionally substituted with one carbamoyl; and two carbon atoms in the four- to seven-membered saturated heterocyclyl containing one nitrogen atom and optionally further containing one heteroatom are optionally crosslinked with $C_{1-4}$ alkanediyl.

**2.** The substituted polypeptide or pharmaceutically acceptable salt thereof according to claim 1, wherein $W^C$ is:

a single bond,
Pro,
Arg, (d)-Arg,
Lys, (d)-Lys,
β-Ala, GABA, Ape,
Gly-(d)-Lys, Gly-(d)-Lys-(d)-Lys, Gly-(d)-Lys-(d)-Arg, Gly-(d)-Arg-(d)-Lys, Lys-Lys, (d)-Lys-(d)-Lys, (d)-Lys-(d)-Lys-(d)-Lys,
Arg-Arg, (d)-Arg-(d)-Arg, (d)-Arg-(d)-Lys,
Lys-(d)-Lys-(d)-Lys, (d)-Lys-Lys-(d)-Lys, (d)-Lys-(d)-Lys-Lys, β-Ala-(d)-Lys, β-Ala-(d)-Lys-(d)-Arg, β-Ala-(d)-Arg-(d)-Lys, or β-Ala-(d)-Arg-(d)-Arg,
wherein if Lys is contained in the group represented by $W^C$, then
the amino in the side chain of the Lys is optionally substituted with:

$C_{2-16}$ alkylcarbonyl terminally-substituted with carboxy, or

(d)-Lys, wherein the amino in the side chain of the (d)-Lys is optionally substituted with $C_{2-16}$ alkylcarbonyl terminally-substituted with carboxy.

**3.** The substituted polypeptide or pharmaceutically acceptable salt thereof according to claim 1 or 2, wherein

ring A is a benzene ring, a thiophene ring, or a pyridine ring;
$R^{A1}$ and $R^{A2}$ are each independently:

a hydrogen atom, or
a halogen atom;

ring B is:
phenyl, oxazolyl, thiadiazolyl, pyridyl, or benzofuranyl;
$R^{B1}$, $R^{B2}$, and $R^{B3}$ are each independently:

a hydrogen atom,
carbamoyl,
cyano,
a halogen atom,
$C_{1-6}$ alkyl, wherein the $C_{1-6}$ alkyl is optionally substituted with one hydroxy, halo $C_{1-6}$ alkyl,
$C_{1-6}$ alkoxy, wherein the $C_{1-6}$ alkoxy is optionally substituted with one hydroxy,
halo $C_{1-6}$ alkoxy,
$C_{1-6}$ alkylcarbonyl,
mono $C_{1-6}$ alkylaminocarbonyl, di $C_{1-6}$ alkylaminocarbonyl, wherein the alkyl in each of the mono $C_{1-6}$ alkylaminocarbonyl and the di $C_{1-6}$ alkylaminocarbonyl is optionally substituted with one group selected from the group consisting of hydroxy, carboxy, carbamoyl, and amino, or
$C_{1-6}$ alkylsulfonyl; and

$R^C$ is:

the formula -OH, the formula $-NH_2$,
$C_{1-6}$ alkylamino, wherein the $C_{1-6}$ alkyl of the $C_{1-6}$ alkylamino is optionally substituted with one group selected from the group consisting of hydroxy, amino, $C_{1-6}$ alkoxy, and morpholinyl,
azetidinyl, pyrrolidinyl, piperidinyl, morpholinyl, or piperazinyl, wherein the azetidinyl, pyrrolidinyl, piperidinyl, morpholinyl, or piperazinyl is optionally substituted with one group selected from the group consisting of hydroxy, amino, and $C_{1-6}$ alkyl, wherein the $C_{1-6}$ alkyl is optionally substituted with one carbamoyl, wherein two carbon atoms in the azetidinyl, pyrrolidinyl, piperidinyl, morpholinyl, or piperazinyl are optionally crosslinked with $C_{1-4}$ alkanediyl.

**4.** The substituted polypeptide or pharmaceutically acceptable salt thereof according to any one of claims 1 to 3, wherein in the substituted polypeptide represented by formula [I'],

ring A is a benzene ring;
ring B is phenyl;
$L^{1''}$ is one group selected from the group consisting of:

a single bond,
Gly, (N-Me)Gly,
Ala, (N-Me)Ala, (d)-Ala, Val, (N-Me)Val, (N-Me)Leu, (N-Me)Ile,
a group represented by formula [IV-27]:

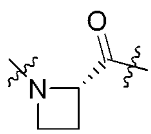
[IV-27]

Pro, (d)-Pro, homoPro, Phe, (N-Me)Phe, (d)-Phe,

His, (d)-His, Trp, (N-Me)Trp, (d)-Trp,
Tyr, (N-Me)Tyr, (d)-Tyr,
(d)-Ser, homoSer, Thr, (N-Me)Thr, (d)-Thr,
Cys, (d)-Cys, Met, (N-Me)Met,
(N-Me)Asp, Glu, (N-Me)Glu, (d)-Glu,
Asn, (N-Me)Asn, (d)-Asn, Gln, (N-Me)Gln, (d)-Gln,
Arg, (N-Me)Arg, (d)-Arg, Cit, (d)-Cit,
a group represented by any of formulas [IV-7], [IV-9], [IV-10], and [IV-13]:

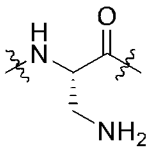 [IV-7], 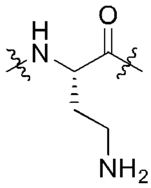 [IV-9], 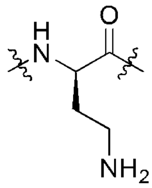 [IV-10],

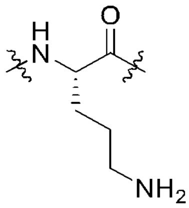 [IV-13]

Lys, (N-Me)Lys, (d)-Lys, a group represented by formula [IV-14]:

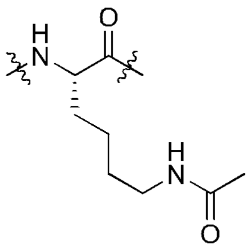 [IV-14]

β-Ala,
β-Asp, β-(d)-Asp, and
a group represented by any of formulas [III-6] and [III-7]:

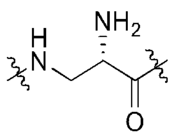 [III-6], 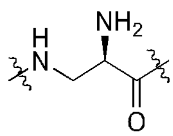 [III-7]; and

W$^C$ is:

a single bond,
Pro,
Arg, (d)-Arg,
Lys, (d)-Lys,
β-Ala, GABA, Ape,
Gly-(d)-Lys, Gly-(d)-Lys-(d)-Lys, Gly-(d)-Lys-(d)-Arg, Gly-(d)-Arg-(d)-Lys, Lys-Lys, (d)-Lys-(d)-Lys, (d)-Lys-(d)-Lys-(d)-Lys,
Arg-Arg, (d)-Arg-(d)-Arg, (d)-Arg-(d)-Lys,

Lys-(d)-Lys-(d)-Lys, (d)-Lys-Lys-(d)-Lys, (d)-Lys-(d)-Lys-Lys,
β-Ala-(d)-Lys, β-Ala-(d)-Lys-(d)-Arg,
β-Ala-(d)-Arg-(d)-Lys, or β-Ala-(d)-Arg-(d)-Arg.

**5.** The substituted polypeptide or pharmaceutically acceptable salt thereof according to any one of claims 1 to 4, wherein in the substituted polypeptide represented by formula [I'], $AA^2$ is one group selected from the group consisting of:

a group represented by formula [II-1]:

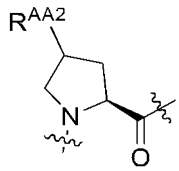 [II-1]

and
a group represented by any of formulas [IV-7], [IV-8], [IV-9], [IV-11], and [IV-12]:

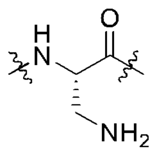 [IV-7], 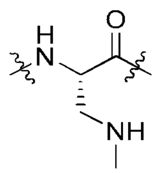 [IV-8],

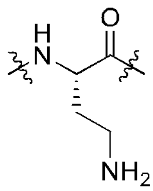 [IV-9], 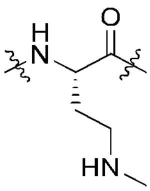 [IV-11], 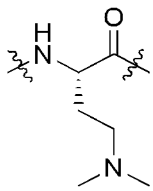 [IV-12];

wherein $R^{AA2}$ is amino;
$AA^3$ is Val, Leu, Ile, Phe, or Trp;
$AA^4$ is (N-Me)Val, (N-Me)Leu, (N-Me)Ile, (N-Me)Asp, or (N-Me)Glu;
$AA^5$ is β-Ala, GABA, Ape, Acp, Pro, (d)-Pro, or β-homoPro;
$W^C$ is a single bond, Arg, (d)-Arg, Lys, or (d)-Lys; and
$R^C$ is the formula -OH or the formula $-NH_2$.

**6.** The substituted polypeptide or pharmaceutically acceptable salt thereof according to any one of claims 1 to 5, wherein in the substituted polypeptide represented by formula [I'],

$W^1$ is $-L^{1'}-L^{1''}-$;
$L^2$ is a single bond;
$AA^1$ is Asp;

$L^{1'}$ is one group selected from the group consisting of β-Ala, GABA, Ape, Acp, and a group represented by any of formulas [IV-23] and [IV-24]:

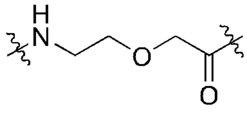 [IV-23], 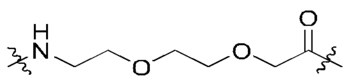 [IV-24];

$L^{1''}$ is a single bond, Asn, (d)-Ser, (d)-Thr, or Glu;

$L^{N1}$ is the formula -C(=O)- or the formula -S(=O)$_2$-;
$L^{N2}$ is the formula -O- or the formula -C(=O)-NH-;
$R^{A1}$ and $R^{A2}$ are each a hydrogen atom; and
$R^{B1}$, $R^{B2}$, and $R^{B3}$ are each independently a hydrogen atom, carbamoyl, a halogen atom, $C_{1-6}$ alkoxy, or halo $C_{1-6}$ alkoxy.

**7.** The substituted polypeptide or pharmaceutically acceptable salt thereof according to any one of claims 1 to 5, wherein in the substituted polypeptide represented by formula [I'],

$W^1$ is -$L^1$-, wherein $L^1$ is a single bond;
$L^2$ is a single bond;
$AA^1$ is β-Asp, β-(d)-Asp, γ-Glu, or γ-(d)-Glu;
$AA^2$ is one group selected from the group consisting of:

a group represented by formula [II-1]:

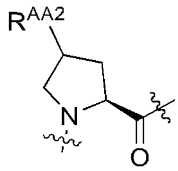
[II-1]

and
a group represented by any of formulas [IV-7], [IV-8], [IV-9], [IV-11], and [IV-12]:

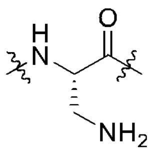
[IV-7],
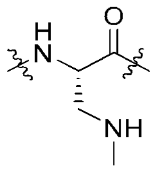
[IV-8],

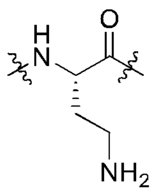
[IV-9],
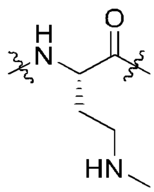
[IV-11],
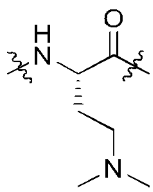
[IV-12];

wherein $R^{AA2}$ is amino;

$AA^3$ is Val, Leu, Ile, Phe, or Trp;
$AA^4$ is (N-Me)Val, (N-Me)Leu, (N-Me)Ile, (N-Me)Asp, or (N-Me)Glu;
$AA^5$ is β-Ala, GABA, Ape, Acp, or β-homoPro;
$L^{N1}$ is the formula -C(=O)- or the formula -S(=O)$_2$-;
$L^{N2}$ is a single bond, the formula -O-, or the formula -C(=O)-NH-;
$R^{A1}$ and $R^{A2}$ are each a hydrogen atom; and
$R^{B1}$, $R^{B2}$, and $R^{B3}$ are each independently a hydrogen atom, carbamoyl, a halogen atom, $C_{1-6}$ alkoxy, or halo $C_{1-6}$ alkoxy.

**8.** The substituted polypeptide or pharmaceutically acceptable salt thereof according to any one of claims 1 to 4, wherein the substituted polypeptide represented by formula [I'] is a substituted polypeptide represented by formula [I]:

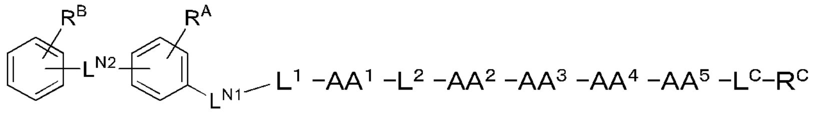

[I]

wherein

AA$^1$ is β-Asp, γ-Glu, or γ-(d)-Glu;
AA$^2$ is a group represented by formula [II-1] or formula [II-2]:

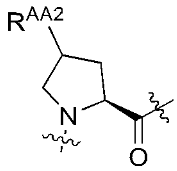
[II-1],
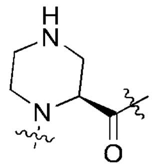
[II-2]

wherein $R^{AA2}$ is hydroxy or amino;
AA$^3$ is Val, Leu, Ile, Phe, or Trp;
AA$^4$ is a single bond, Pro, (N-Me)Ala, (N-Me)Val, (N-Me)Leu, (N-Me)Ile, (N-Me)Phe, (N-Me)Tyr, (N-Me)Ser, (N-Me)Asp, or (N-Me)Glu;
AA$^5$ is a single bond, Pro, (d)-Pro, β-homoPro, Arg, (d)-Arg, Lys, (d)-Lys, β-Ala, GABA, Ape, or Acp;
$L^1$ is a single bond;
$L^2$ is a single bond;
$L^{N1}$ is the formula -C(=O)- or the formula $-S(=O)_2-$;
$L^{N2}$ is a single bond, $C_{1-3}$ alkanediyl, the formula -O-, or the formula -C(=O)-NH-;
$R^A$ is a hydrogen atom, a halogen atom, $C_{1-6}$ alkyl, or $C_{1-6}$ alkoxy;
$R^B$ is a hydrogen atom, carbamoyl, a halogen atom, $C_{1-6}$ alkyl, or $C_{1-6}$ alkoxy;
$L^C$ is a single bond, Pro, Arg, (d)-Arg, Lys, (d)-Lys, or (d)-Lys-(d)-Lys; and
$R^C$ is the formula -OH or the formula $-NH_2$.

**9.** The substituted polypeptide or pharmaceutically acceptable salt thereof according to any one of claims 1 to 5, wherein in the substituted polypeptide represented by formula [I'],

AA$^1$ is Asp, β-(d)-Asp, or γ-(d)-Glu;
AA$^2$ is one group selected from the group consisting of:

a group represented by formula [II-1]:

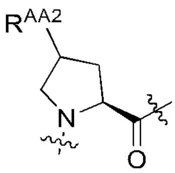
[II-1]

and
a group represented by any of formulas [IV-7] and [IV-9]:

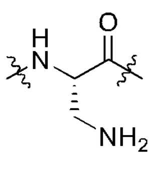
[IV-7],
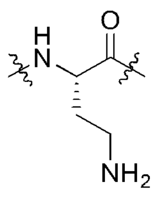
[IV-9]

wherein $R^{AA2}$ is amino;

$AA^3$ is Val, Leu, or Ile;
$AA^4$ is (N-Me)Ile or (N-Me)Glu;
$AA^5$ is Ape or β-homoPro;
$W^1$ is $-L^1-$ or $-L^{1'}-L^{1''}-$,
wherein

$L^1$ is a single bond,
$L^{1'}$ is GABA or Ape, and
$L^{1''}$ is Asn, (d)-Ser, (d)-Thr, or Glu;

$L^{N1}$ is the formula -C(=O)- or the formula $-S(=O)_2-$;
$L^{N2}$ is the formula -O- or the formula -C(=O)-NH-;
$R^{A1}$ and $R^{A2}$ are each a hydrogen atom;
$R^{B1}$, $R^{B2}$, and $R^{B3}$ are each independently a hydrogen atom, carbamoyl, a halogen atom, $C_{1-6}$ alkoxy, or halo $C_{1-6}$ alkoxy;
$W^C$ is a single bond or (d)-Lys; and
$R^C$ is the formula $-NH_2$.

**10.** The substituted polypeptide or pharmaceutically acceptable salt thereof according to any one of claims 1 to 5, wherein the substituted polypeptide is selected from compounds shown in the following:

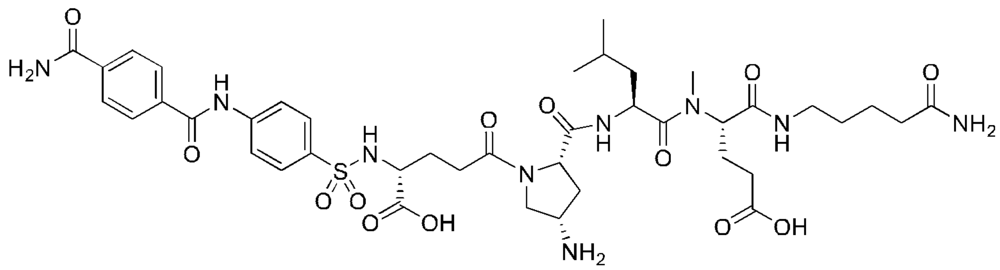

,

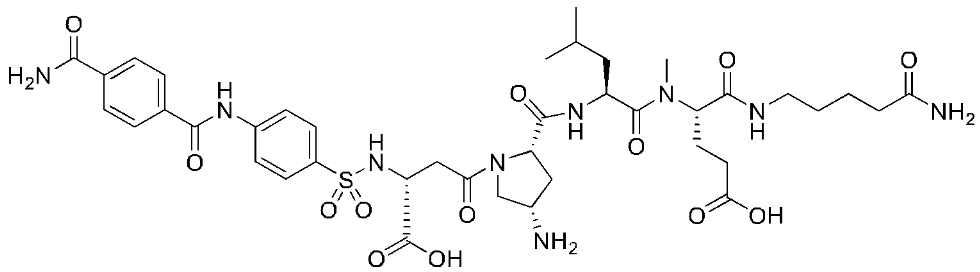

,

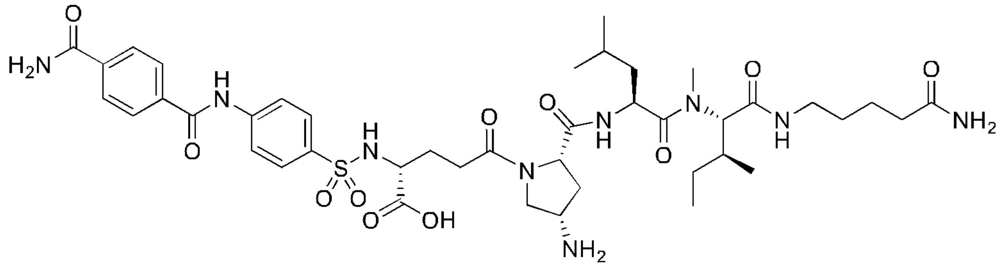

,

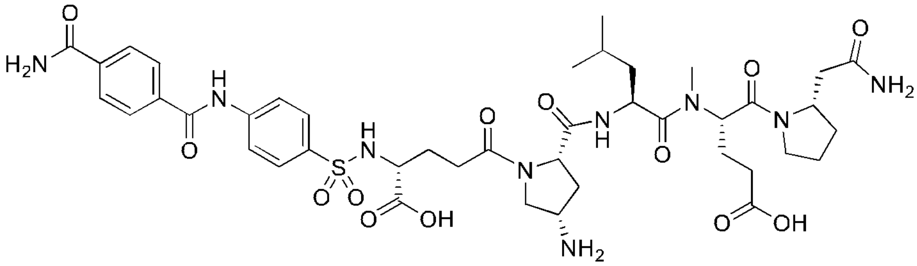

,

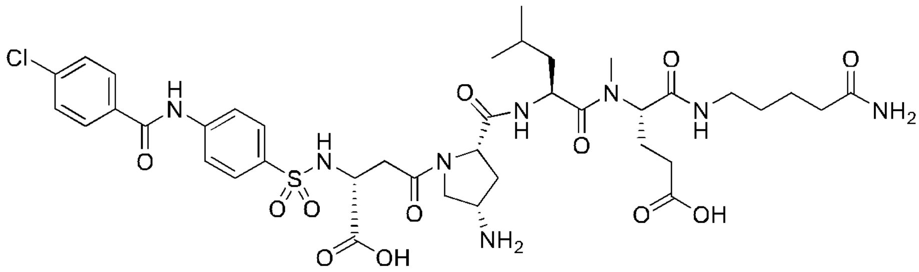

,

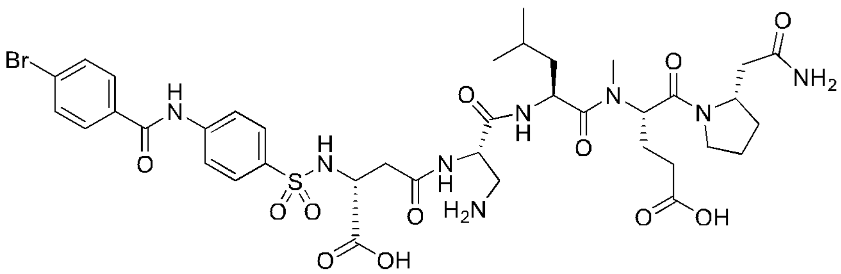

,

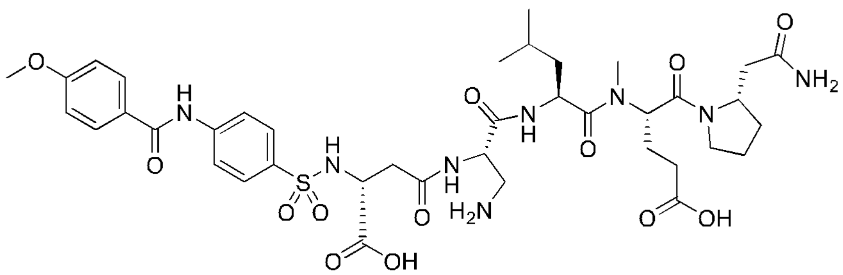

,

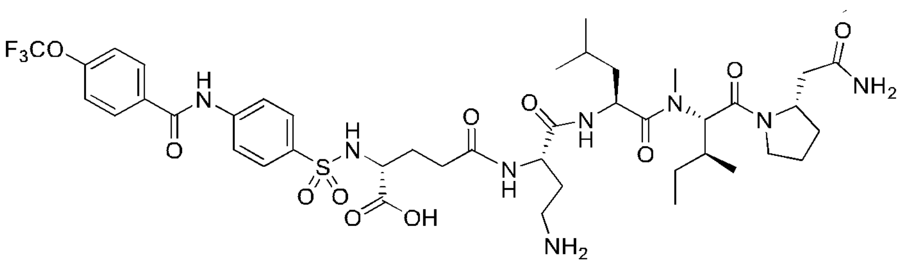

,

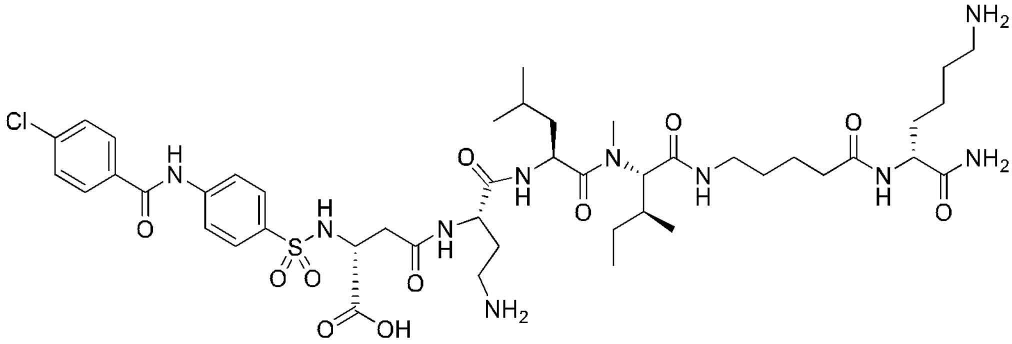

,

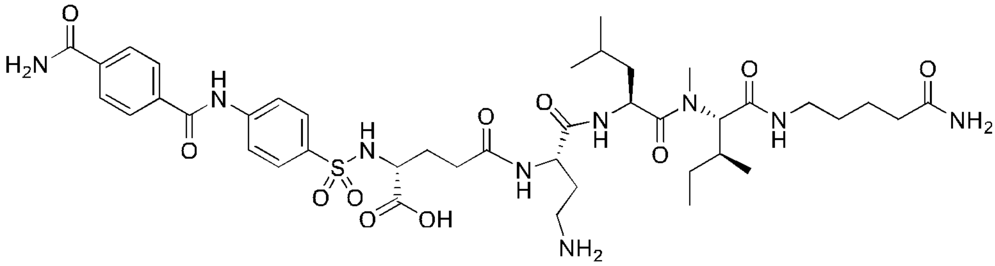

,

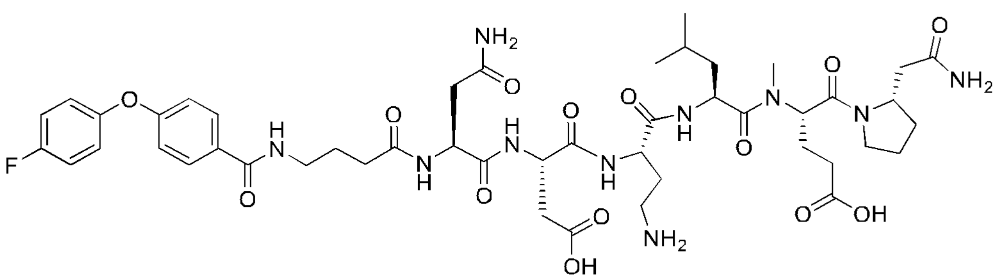

,

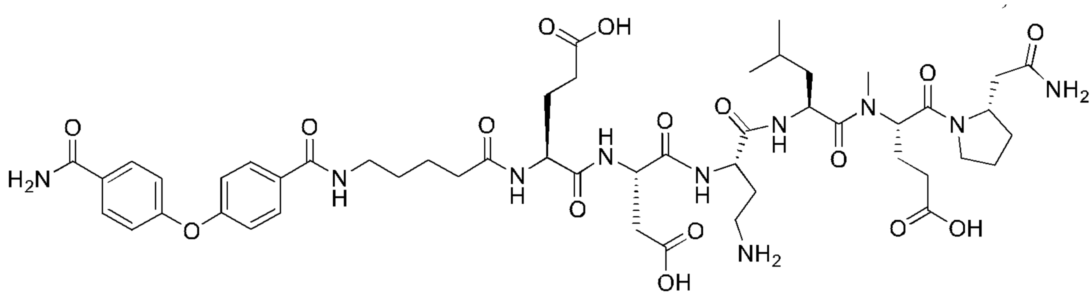

,

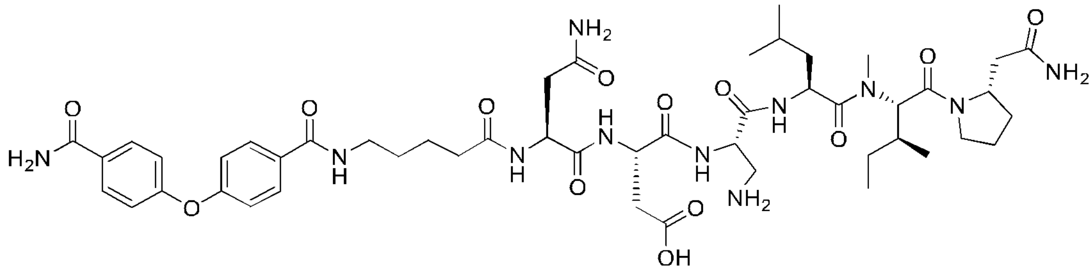

,

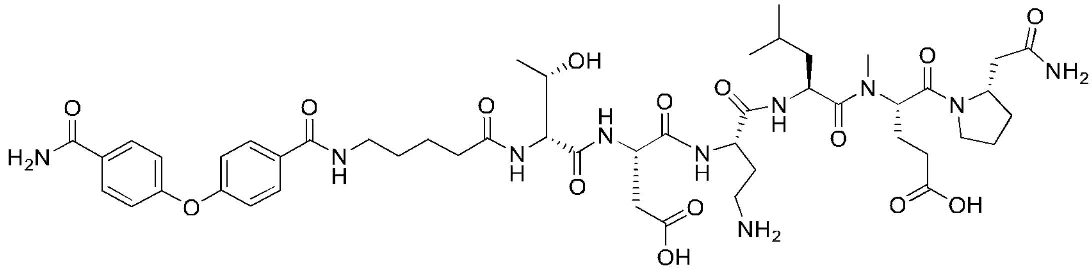

,

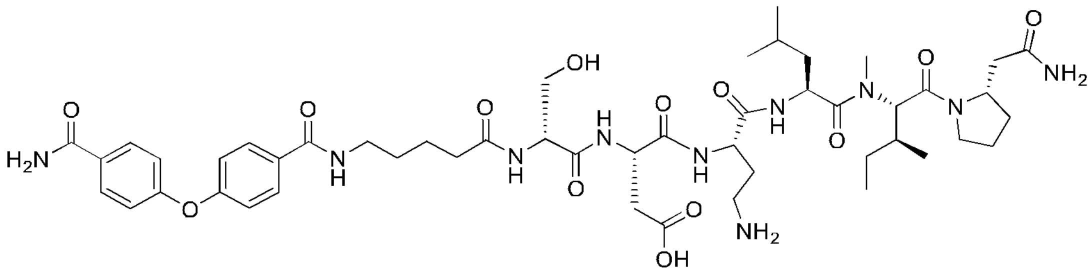

**11.** A pharmaceutical comprising the substituted polypeptide or pharmaceutically acceptable salt thereof according to any one of claims 1 to 10 as an active ingredient.

**12.** An MMP2 inhibitor comprising the substituted polypeptide or pharmaceutically acceptable salt thereof according to any one of claims 1 to 10 as an active ingredient.

**13.** A drug for preventing or treating cancerous disease or organ fibrosis, or a symptom associated with cancerous disease or organ fibrosis, comprising the substituted polypeptide or pharmaceutically acceptable salt thereof according to any one of claims 1 to 10 as an active ingredient.

## INTERNATIONAL SEARCH REPORT

International application No.
PCT/JP2020/042350

A. CLASSIFICATION OF SUBJECT MATTER
C07K 7/06(2006.01)i; A61K 38/07(2006.01)i; A61K 38/08(2019.01)i; A61P 35/00(2006.01)i; A61P 43/00(2006.01)i; C12N 9/99(2006.01)i
FI: C07K7/06 ZNA; C12N9/99; A61P35/00; A61P43/00 111; A61P43/00 105; A61K38/07; A61K38/08
According to International Patent Classification (IPC) or to both national classification and IPC

B. FIELDS SEARCHED
Minimum documentation searched (classification system followed by classification symbols)
A61P35/00; A61P43/00; C07K7/06; A61K38/07; A61K38/08; C12N9/99

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580 (JDreamIII);
CAplus/REGISTRY/MEDLINE/EMBASE/BIOSIS/WPIDS (STN)

C. DOCUMENTS CONSIDERED TO BE RELEVANT

| Category* | Citation of document, with indication, where appropriate, of the relevant passages | Relevant to claim No. |
|---|---|---|
| Y<br>A | US 2011/0171133 A1 (VAN ECK-SMIT Bertha Louise Frederike et al.) 14 July 2011 (2011-07-14) claims, examples, table 1 | 1-3, 11-13<br>4-10 |
| Y<br>A | SANTOS, M. Amelia et al., "Design, synthesis and molecular modeling study of iminodiacetyl monohydroxamic acid derivatives as MMP inhibitors", Bioorganic & Medicinal Chemistry, 2006, vol. 14, pp. 7539-7550, DOI: 10.1016/j.bmc.2006.07.011, abstract, chart 1, table 1, etc. | 1, 3, 11-13<br>2, 4-10 |
| Y<br>A | HIGASHI, Shouichi et al., "Identification of a Region of β-Amyloid Precursor Protein Essential for Its Gelatinase A Inhibitory Activity", The Journal of Biological Chemistry, 2003, vol. 278, no. 16, pp. 14020-14028, DOI:10.1074/jbc.M212264200, abstract, table I | 1-3, 11-13<br>4-10 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 07 January 2021 (07.01.2021) | 19 January 2021 (19.01.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2020/042350

C (Continuation). DOCUMENTS CONSIDERED TO BE RELEVANT

| Category* | Citation of document, with indication, where appropriate, of the relevant passages | Relevant to claim No. |
|---|---|---|
| Y<br>A | JP 2018-197251 A (CAREGEN CO., LTD.) 13 December 2018 (2018-12-13) claims, examples, table 1 | 1-3, 11-13<br>4-10 |
| A | JP 2014-526459 A (CAREGEN CO., LTD.) 06 October 2014 (2014-10-06) entire text | 1-13 |
| A | JP 2009-235071 A (NOVARTIS AG) 15 October 2009 (2009-10-15) entire text | 1-13 |

Form PCT/ISA/210 (continuation of second sheet) (January 2015)

**INTERNATIONAL SEARCH REPORT**
Information on patent family members

International application no.
PCT/JP2020/042350

| Patent Documents referred in the Report | Publication Date | Patent Family | Publication Date |
|---|---|---|---|
| US 2011/0171133 A1 | 14 Jul. 2011 | WO 2009/139634 A1<br>claims, examples, table 1<br>EP 2119690 A1 | |
| JP 2018-197251 A | 13 Dec. 2018 | US 2017/0267721 A1<br>claims, examples, table 1<br>US 2019/0040100 A1<br>WO 2016/088968 A1<br>EP 3228626 A1<br>KR 10-2016-0069054 A<br>CN 107001420 A<br>JP 2018-505850 A | |
| JP 2014-526459 A | 06 Oct. 2014 | US 2014/0328782 A1<br>whole document<br>WO 2013/035931 A1<br>EP 2754665 A1<br>KR 10-2013-0028403 A<br>CN 104024271 A | |
| JP 2009-235071 A | 15 Oct. 2009 | US 2008/0275127 A1<br>whole document<br>WO 2001/010827 A1<br>EP 2085379 A1<br>CA 2378310 A<br>CN 1368958 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **H. J. RA ; W. C. PARKS.** *Matrix Biol.,* 2007, vol. 26 (8), 587-596
- **A. H. DRUMMOND et al.** *Ann N Y Acad Sci.,* 1999, vol. 878, 228-235
- **A. D. BAXTER et al.** *Bioorg Med Chem Lett.,* 2001, vol. 11, 1465-1468
- **T. ITOH et al.** *Cancer Res.,* 1998, vol. 58, 1048-1051
- **R. ROY et al.** *J Clin Oncol.,* 2009, vol. 27, 5287-5297
- **T. TURPEENNIEMI-HUJANEN.** *Biochimie,* 2005, vol. 87, 287-297
- **S. CHENG et al.** *FASEB J.,* 2006, vol. 20, 1898-1900
- **K. PAWLAK et al.** *Clin Biochem.,* 2011, vol. 44, 838-843
- **H. R. CHANG et al.** *Clin Chim Acta.,* 2006, vol. 366, 243-248
- **X. DU et al.** *Lab Invest.,* 2012, vol. 92, 1149-1160
- **M. K. TVEITARAS et al.** *PLoS One.,* 2015, vol. 10, e0143390
- **M. SELMAN et al.** *Am J Physiol Lung Cell Mol Physiol.,* 2000, vol. 279, L562-L574
- **M. SUGA et al.** *Am J Respir Crit Care Med.,* 2000, vol. 162, 1949-1956
- **M. CORBEL et al.** *J Pathol.,* 2001, vol. 193, 538-545
- **J. MICHAEL et al.** *Am J Respir Cell Mol Biol.,* 2009, vol. 41, 731-741
- **A. TOKITO et al.** *Int J Mol Sci.,* 2016, vol. 17, E1178
- **D. E. LEVY et al.** *J Med Chem.,* 1998, vol. 41, 199-223
- **Y. TAMURA et al.** *J Med Chem.,* 1998, vol. 41, 640-649
- **S. HIGASHI et al.** *J Biol Chem.,* 2003, vol. 278, 14020-14028